# EUROPEAN PATENT SPECIFICATION

(11) **EP 1 282 718 B1**
(45) Date of publication and mention of the grant of the patent: **20.12.2006**
(21) Application number: 01931274.3
(22) Date of filing: 08.05.2001
(51) Int. Cl.: C12N 15/85, A61K 48/00, C07K 19/00, C07K 16/12, A61K 39/118, A61K 39/40

(54) **CHLAMYDIA ANTIGENS AND CORRESPONDING DNA FRAGMENTS AND USES THEREOF**
CHLAMYDIA ANTIGENE, ENTSPRECHENDE DNS FRAGMENTE UND IHRE VERWENDUNGEN
ANTIGENES DE CHLAMYDIA, FRAGMENTS D'ADN CORRESPONDANTS ET UTILISATIONS DE TELS ANTIGENES

(30) Priority: 08.05.2000 US 202672 P; 30.05.2000 US 207852 P; 16.06.2000 US 211801 P; 16.06.2000 US 212044 P; 16.06.2000 US 211797 P; 16.06.2000 US 211796 P; 16.06.2000 US 211798 P; 26.09.2000 US 235335 P; 26.09.2000 US 235361 P; 26.09.2000 US 235398 P
(43) Date of publication of application: 12.02.2003
(73) Proprietor: Sanofi Pasteur Limited, Toronto, Ontario M2R 3T4 (CA)
(72) Inventor: MURDIN, Andrew, D., Richmond Hill, Ontario L4B 3C2 (CA); OOMEN, Raymond, P., Aurora, Ontario L4G 2L6 (CA); WANG, Joe, Toronto, Ontario M2H 2E8 (CA); DUNN, Pamela, Woodbridge, Ontario L4L 3Z1 (CA)
(74) Representative: Desaix, Anne
(86) International application number: PCT/CA2001/000653
(87) International publication number: WO 2001/085972

(56) References cited:
- DATABASE GENESEQ [Online] 13 September 1999 (1999-09-13) GRIFFAIS R: "C. pneumoniae protein involved in metabolism of poylpeptides" retrieved from EBI Database accession no. AAY35040 XP002298776 & WO 99 27105 A2 (GENSET SA ; GRIFFAIS REMY (FR)) 03 June 1999
- DATABASE EMBL [Online] 15 March 1999 (1999-03-15) KALMAN S, ET AL.: "Chlamydia section 42 of 103 of the complete genome" retrieved from EBI Database accession no. AE001626 XP002298777 & KALMAN S ET AL: "COMPARATIVE GENOMES OF CHLAMYDIA PNEUMONIAE AND C. TRACHOMATIS" NATURE GENETICS, NEW YORK, NY, US, vol. 21, no. 4, April 1999 (1999-04), pages 385-389, XP000853883 ISSN: 1061-4036
- DATABASE EMBL [Online] 14 March 2000 (2000-03-14) READ ET AL.: "Chlamydophila pneumoniae AR39, section 27 of 94 of the complete genome" retrieved from EBI Database accession no. AE002194 XP002298778 & READ T D ET AL: "Genome sequences of Chlamydia trachomatis MoPn and Chlamydia pneumoniae AR39" NUCLEIC ACIDS RESEARCH, OXFORD UNIVERSITY PRESS, SURREY, GB, vol. 28, no. 6, 15 March 2000 (2000-03-15), pages 1397-1406, XP002164566 ISSN: 0305-1048
- DATABASE GENESEQ [Online] 7 October 1999 (1999-10-07) GRIFFAIS R: "Protein involved in intermediate metabolism of polypeptides" retrieved from EBI Database accession no. AAY36903 XP002298779 & WO 99 28475 A2 (GENSET SA ; GRIFFAIS REMY (FR)) 10 June 1999
- SHIRAI MUTSUNORI ET AL: "Comparison of whole genome sequences of Chlamydia pneumoniae J138 from Japan and CWL029 from USA" NUCLEIC ACIDS RESEARCH, OXFORD UNIVERSITY PRESS, SURREY, GB, vol. 28, no. 12, 15 June 2000 (2000-06-15), pages 2311-2314, XP002166059 ISSN: 0305-1048

## Description

### FIELD OF INVENTION

The present invention relates to a vaccine comprising a CLPc protease and its corresponding DNA molecule for the prevention and treatment of *Chlamydia* infection in mammals.

The application also discloses a number of *Chlamydia* antigens, including an ATP-binding cassette protein, a secretory locus ORF, an endopeptidase, a protease, a metalloprotease, CLP protease ATPase, a CLP protease subunit, a translycolase/transpeptidase and thioredoxin, and their corresponding DNA molecules, for the prevention and treatment of *Chlamydia* infection in mammals.

### BACKGROUND OF THE INVENTION

*Chlamydiae* are prokaryotes. They exhibit morphologic and structural similarities to gram-negative bacteria including a trilaminar outer membrane, which contains lipopolysaccharide and several membrane proteins that are structurally and functionally analogous to proteins found in *E coli.* They are obligate intracellular parasites with a unique biphasic life cycle consisting of a metabolically inactive but infectious extracellular stage and a replicating but non-infectious intracellular stage. The replicative stage of the life-cycle takes place within a membrane-bound inclusion which sequesters the bacteria away from the cytoplasm of the infected host cell.

*C. pneumoniae* is a common human pathogen, originally described as the TWAR strain of *Chlamydia psittaci* but subsequently recognised to be a new species. *C. pneumoniae* is antigenically, genetically and morphologically distinct from other *Chlamydia* species (*C. trachomatis, C. pecorum* and *C. psittaci*). It shows 10% or less DNA sequence homology with either of *C. trachomatis* or *C. psittaci.*

In general, all chlamydiae share a common developmental microbiology and appear to share a common immunobiology. Genome analysis shown that over 80% of *C. pneumoniae* and *C. trachomatis* protein-coding genes are orthologs that share a similar genome organization.

*C. pneumoniae* is the third most common cause of community acquired pneumonia, only less frequent than *Streptococcus pneumoniae* and *Mycoplasma pneumoniae* (Grayston *et al.* (1995) Journal of Infectious Diseases 168:1231; Campos *et al.* (1995) Investigation of Ophthalmology and Visual Science 36:1477). It can also cause upper respiratory tract symptoms and disease, including bronchitis and sinusitis (Grayston *et al.* (1995) Journal of Infectious Diseases 168:1231; Grayston *et al* (1990) Journal of Infectious Diseases 161:618-625; Marrie (1993) Clinical Infectious Diseases. 18:501-513; Wang *et al* (1986) Chlamydial infections Cambridge University Press, Cambridge. p. 329. The great majority of the adult population (over 60%) has antibodies to *C. pneumoniae* (Wang *et al* (1986) Chlamydial infections. Cambridge University Press, Cambridge. p. 329), indicating past infection which was unrecognized or asymptomatic.

*C. pneumoniae* infection usually presents as an acute respiratory disease (*i.e.,* cough, sore throat, hoarseness, and fever; abnormal chest sounds on auscultation). For most patients, the cough persists for 2 to 6 weeks, and recovery is slow. In approximately 10% of these cases, upper respiratory tract infection is followed by bronchitis or pneumonia. Furthermore, during a *C. pneumoniae* epidemic, subsequent co-infection with pneumococcus has been noted in about half of these pneumonia patients, particularly in the infirm and the elderly. As noted above, there is increasing evidence that *C. pneumoniae* infection is also linked to diseases other than respiratory infections.

The reservoir for the organism is presumably people. In contrast to *C. psittaci* infections, there is no known bird or animal reservoir. Transmission has not been clearly defined. It may result from direct contact with secretions, from fomites, or from airborne spread. There is a long incubation period, which may last for many months. Based on analysis of epidemics, *C. pneumoniae* appears to spread slowly through a population (case-to-case interval averaging 30 days) because infected persons are inefficient transmitters of the organism. Susceptibility to *C. pneumoniae* is universal. Reinfections occur during adulthood, following the primary infection as a child. *C. pneumoniae* appears to be an endemic disease throughout the world, noteworthy for superimposed intervals of increased incidence (epidemics) that persist for 2 to 3 years. *C. trachomatis* infection does not confer cross-immunity to *C. pneumoniae.* Infections are easily treated with oral antibiotics, tetracycline or erythromycin (2 g/d, for at least 10 to 14 d). A recently developed drug, azithromycin, is highly effective as a single-dose therapy against chlamydial infections.

In most instances, *C. pneumoniae* infection is often mild and without complications, and up to 90% of infections are subacute or unrecognized. Among children in industrialized countries, infections have been thought to be rare up to the age of 5 y, although a recent study (E Normann *et al, Chlamydia pneumoniae* in children with acute respiratory tract infections, Acta Paediatrica, 1998, Vol 87, Iss 1, pp 23-27) has reported that many children in this age group show PCR evidence of infection despite being seronegative, and estimates a prevalence of 17-19% in 2-4 y olds. In developing countries, the seroprevalence of *C. pneumoniae* antibodies among young children is elevated, and there are suspicions that *C. pneumoniae* may be an important cause of acute lower respiratory tract disease and mortality for infants and children in tropical regions of the world.

From seroprevalence studies and studies of local epidemics, the initial *C. pneumoniae* infection usually happens between the ages of 5 and 20 y. In the USA, for example, there are estimated to be 30,000 cases of childhood pneumonia each year caused by *C. pneumoniae.* Infections may cluster among groups of children or young adults (*e.g.*, school pupils or military conscripts).

*C. pneumoniae* causes 10 to 25% of community-acquired lower respiratory tract infections (as reported from Sweden, Italy, Finland, and the USA). During an epidemic, *C. pneumonia* infection may account for 50 to 60% of the cases of pneumonia. During these periods, also, more episodes of mixed infections with *S. pneumoniae* have been reported.

Reinfection during adulthood is common; the clinical presentation tends to be milder. Based on population seroprevalence studies, there tends to be increased exposure with age, which is particularly evident among men. Some investigators have speculated that a persistent, asymptomatic *C. pneumoniae* infection state is common.

In adults of middle age or older, *C. pneumoniae* infection may progress to chronic bronchitis and sinusitis. A study in the USA revealed that the incidence of pneumonia caused by *C. pneumoniae* in persons younger than 60 years is 1 case per 1,000 persons per year; but in the elderly, the disease incidence rose three-fold. *C. pneumoniae* infection rarely leads to hospitalization, except in patients with an underlying illness.

Of considerable importance is the association of atherosclerosis and *C. pneumoniae* infection. There are several epidemiological studies showing a correlation of previous infections with *C. pneumoniae* and heart attacks, coronary artery and carotid artery disease (Saikku *et al.* (1988) Lancet;ii:983-986; Thom *et al.* (1992) JAMA 268:68-72; Linnanmaki *et al.* (1993), Circulation 87:1030; Saikku *et al.* (1992)Annals Internal Medicine 116:273-287; Melnick *et al* (1993) American Journal of Medicine 95:499). Moreover, the organisms has been detected in atheromas and fatty streaks of the coronary, carotid, peripheral arteries and aorta (Shor *et al.* (1992) South African. Medical Journal 82:158-161; Kuo *et al.* (1993) Journal of Infectious Diseases 167:841-849; Kuo *et al.* (1993) Arteriosclerosis and Thrombosis 13:1501-1504; Campbell *et al* (1995) Journal of Infectious Diseases 172:585; Chiu *et al.* Circulation, 1997. Circulation. 96:2144-2148). Viable *C. pneumoniae* has been recovered from the coronary and carotid artery (Ramirez *et al* (1996) Annals of Internal Medicine 125:979-982; Jackson *et al.* 1997. J. Infect. Dis. 176:292-295). Furthermore, it has been shown that *C. pneumoniae* can induce changes of atherosclerosis in a rabbit model (Fong *et al.* 1997. Journal of Clinical Microbiolology 35:48 and Laitinen *et al.* 1997. Infect. Immun. 65:4832-4835). Taken together, these results indicate that it is highly probable that *C. pneumoniae* can cause atherosclerosis in humans, though the epidemiological importance of chlamydial atherosclerosis remains to be demonstrated.

A number of recent studies have also indicated an association between *C. pneumoniae* infection and asthma. Infection has been linked to wheezing, asthmatic bronchitis, adult-onset asthma and acute exacerbations of asthma in adults, and small-scale studies have shown that prolonged antibiotic treatment was effective at greatly reducing the severity of the disease in some individuals (Hahn DL, *et al.* Evidence for *Chlamydia pneumoniae* infection in steroid-dependent asthma.Ann Allergy Asthma Immunol. 1998 Jan; 80(1): 45-49.; Hahn DL, *et al.* Association of *Chlamydia pneumoniae* IgA antibodies with recently symptomatic asthma. Epidemiol Infect. 1996 Dec; 117(3): 513-517; Bjornsson E, *et al.* Serology of *chlamydia* in relation to asthma and bronchial hyperresponsiveness. Scand J Infect Dis. 1996; 28(1): 63-69.; Hahn DL. Treatment of *Chlamydia pneumoniae* infection in adult asthma: a before-after trial. J Fam Pract. 1995 Oct; 41(4): 345-351.; Allegra L, *et al.* Acute exacerbations of asthma in adults: role of *Chlamydia pneumoniae* infection. Eur Respir J. 1994 Dec; 7(12): 2165-2168.; Hahn DL, *et al.* Association of *Chlamydia pneumoniae* (strain TWAR) infection with wheezing, asthmatic bronchitis, and adult-onset asthma. JAMA. 1991 Jul 10; 266(2): 225-230).

In light of these results a protective vaccine against *C. pneumoniae* infection would be of considerable importance. There is not yet an effective vaccine for any human chlamydial infection. It is conceivable that an effective vaccine can be developed using physically or chemically inactivated Chlamydiae. However, such a vaccine does not have a high margin of safety. In general, safer vaccines are made by genetically manipulating the organism by attenuation or by recombinant means.

A disease associated with *C. trachomatis* infection is trachoma, a sequela of ocular infection. This disease continues to be a major cause of preventable blindness, with an estimated 500 million cases of active trachoma worldwide (seven million include blindness from conjunctival scarring and eyelid deformities). In the last two decades, genital chlamydial infection has been identified as a major public health problem because of the recognition that chlamydial infection is associated with disease syndromes such as non-gonococcal urethritis, mucopurulent cervicitis, pelvic inflammatory disease (PID), ectopic pregnancy, and tubal infertility. The World Health Organization estimated 89 million new cases of genital chlamydial infections worldwide in 1995. In the United States, each year an estimated four million new cases occur and 50,000 women become infertile as a result of infection.

Studies with *C. trachomatis* and *C. psittaci* indicate that safe and effective vaccine against *Chlamydia* is an attainable goal. For example, mice which have recovered from a lung infection with *C. trachomatis* are protected from infertility induced by a subsequent vaginal challenge (Pal *et al.* (1996) Infection and Immunity.64:5341). Similarly, sheep immunized with inactivated *C. psittaci* were protected from subsequent chlamydial-induced abortions and stillbirths (Jones *et al.* (1995) Vaccine 13:715). In a mouse model, protection from chlamydial infections has been associated with Th1 immune responses, particularly CD8+ CTL response (Rottenberg *et al.* 1999. J. Immunol. 162:2829-2836 and Penttila *et al.* 1999. Immunology. 97:490-496) and it is unlikely that similar responses will need to be induced in humans to confer protection. However, antigens able to elicit a protective immune response against *C. pneumoniae* are largely unknown. The presence of sufficiently high titres of neutralising antibody at mucosal surfaces can also exert a protective effect (Cotter *et al.* (1995) Infection and Immunity 63:4704).

Antigenic variation within the species *C. pneumoniae* is not well documented due to insufficient genetic information, though variation is expected to exist based on *C. trachomatis.* Serovars of *C. trachomatis* are defined on the basis of antigenic variation in the major outer membrane protein (MOMP), but published *C. pneumoniae* MOMP gene sequences show no variation between several diverse isolates of the organism (Campbell *et al.* Infection and Immunity (1990) 58:93; McCafferty *et al* Infection and Immunity (1995) 63:2387-9; Gaydos *et al.* Infection and Immunity. (1992) 60(12):5319-5323). The gene encoding a 76 kDa antigen has been cloned from a single strain of *C. pneumoniae* and the sequence published (Perez Melgosa *et al.* Infection and Immunity.(1994) 62:880). An operon encoding the 9 kDa and 60 kDa cyteine-rich outer membrane protein genes has been described (Watson *et al.,* Nucleic Acids Res (1990) 18:5299; Watson *et al.,* Microbiology (1995) 141:2489). Many antigens recognized by immune sera to *C. pneumoniae* are conserved across all *chlamydiae,* but 98 kDa, 76 kDa and several other proteins may be *C. pneumoniae*-specific (Knudsen *et al.* Infect. Immun. 1999. 67:375-383; Perez Melgosa *et al.* Infection and Immunity. 1994. 62:880; Melgosa *et al.,* FEMS Microbiol Lett 1993. 112 :199;, Campbell *et al.,* J. Clin. Microbiol. 1990. 28 :1261; Iijima *et al.,* J. Clin. Microbiol. 1994. 32:583). Antisera to 76kDa and 54kDa antigens have been reported to neutralize *C. pneumoniae in vitro* (Perez Melgosa *et al.* 1994. Infect. Immun. 62:880-886 and Wiedman-Al-Ahmad *et al.* 1997. Clin. Diagn. Lab. Immunol. 4:700-704). An assessment of the number and relative frequency of any *C. pneumoniae* serotypes, and the defining antigens, is not yet possible. The entire genome sequence of *C. pneumoniae* strain CWL-029 is now known (http://chlamydia-www.berkeley.edu:4231/) and as further sequences become available a better understanding of antigenic variation may be gained.

Many antigens recognised by immune sera to *C. pneumoniae* are conserved across all *chlamydiae,* but 98kDa, 76 kDa and 54 kDa proteins appear to be *C. pneumoniae*-specific (Campos *et al.* (1995) Investigation of Ophthalmology and Visual Science 36:1477; Marrie (1993) Clinical Infectious Diseases. 18:501-513; Wiedmann-Al-Ahmad M, *et al.* Reactions of polyclonal and neutralizing anti-p54 monoclonal antibodies with an isolated, species-specific 54-kilodalton protein of *Chlamydia pneumoniae.* Clin Diagn Lab Immunol. 1997 Nov; 4(6): 700-704).

Immunoblotting of isolates with sera from patients does show variation of blotting patterns between isolates, indicating that serotypes *C. pneumoniae* may exist (Grayston *et al.* (1995) Journal of Infectious Diseases 168:1231; Ramirez *et al* (1996) Annals of Internal Medicine 125:979-982). However, the results are potentially confounded by the infection status of the patients, since immunoblot profiles of a patient's sera change with time post-infection. An assessment of the number and relative frequency of any serotypes, and the defining antigens, is not yet possible.

The use of DNA immunization to elicit a protective immune response in Balb/c mice against pulmonary infection with the mouse pneumonitis (MoPn) strain of *Chlamydia trachomatis* has recently been described (Zhang *et al.* 1997. J. Infect. Dis. 76:1035-1040 and Zhang *et al.* 1999. Immunology. 96:314-321). Recently the genome sequence from *C. pneumoniae* strain CM1 (ATCC #1360-VR) has been disclosed by Griffais in WO99/27105 on June 3, 1999.

Accordingly, a need exists for identifying and isolating polynucleotide sequences of *C. pneumoniae* for use in preventing and treating *Chlamydia* infection.

### SUMMARY OF THE INVENTION

The application discloses purified and isolated polynucleotide molecules that encode a *Chlamydia* polypeptide selected from: an ATP-binding cassette protein, a secretory locus ORF, an endopeptidase, a protease, a metalloprotease, CLP protease ATPase, a CLP protease subunit, a translycolase / transpeptidase, a CLPc protease and thioredoxin. The polynucleotide molecules disclosed in the application can be used in methods to prevent, treat, and diagnose *Chlamydia* infection. For instance, the polynucleotide molecules disclosed in the application is DNA that encode a polypeptide of any one of SEQ ID Nos: 2, 4, 6, 8, 10, 12, 14, 16, 18 and 20.

Another aspect disclosed in the application is polypeptides corresponding to an isolated DNA molecule. Amino acid sequences of the corresponding encoded polypeptides are shown in one embodiment as SEQ ID Nos: 2, 4, 6, 8, 10, 12, 14, 16, 18 and 20.

Those skilled in the art will readily understand that the application, having disclosed the polynucleotide sequences encoding *Chlamydia* polypeptides, also discloses polynucleotides encoding fragments derived from such polypeptides. Moreover, the application further discloses mutants and derivatives of such polypeptides and fragments derived therefrom, which result from the addition, deletion, or substitution of non-essential amino acids as described herein. Those skilled in the art would also readily understand that the application having disclosed the polynucleotide sequences encoding *Chlamydia* polypeptides, further discloses monospecific antibodies that specifically bind to such polypeptides.

The present application discloses expression cassettes, vectors, and cells transformed or transfected with the polynucleotides described in the application. Accordingly, the present application further discloses (i) a method for producing a polypeptide described in the application in a recombinant host system and related expression cassettes, vectors, and transformed or transfected cells;(ii) a method for diagnosing the presence of *Chlamydia* in a biological sample, which can involve the use of a DNA or RNA molecule, a monospecific antibody, or a polypeptide described in the application; and (iii) a method for purifying a polypeptide described in the application by antibody-based affinity chromatography.

The present invention provides (i) a vaccine, or a live vaccine vector such as a pox virus, *Salmonella typhimurium,* or *Vibrio cholerae* vector, containing a polypeptide or a polynucleotide described in the application, such vaccines and vaccine vectors being useful for, e. g., preventing and treating *Chlamydia* infection, in combination with a diluent or carrier, and related pharmaceutical compositions and associated therapeutic and/or prophylactic methods; (ii) a therapeutic and/or prophylactic use of an RNA or DNA molecule described in the application, either in a naked form or formulated with a delivery vehicle, a polypeptide or combination of polypeptides, or a monospecific antibody disclosed in the application, and related pharmaceutical compositions;

One aspect of the invention provides a vaccine comprising a vaccine vector and at least one first nucleic acid selected from any one of:
(i) a nucleic acid sequence set forth in SEQ ID No:17;
(ii) a nucleic acid sequence which encodes a polypeptide encoded by SEQ ID No:17;
(iii) a nucleic acid sequence which encodes a polypeptide which is at least75% identical in amino acid sequence to the polypeptide encoded by SEQ ID No: 17; and
(iv) a nucleic acid sequence which encodes a polypeptide whose sequence is set forth in SEQ ID No: 18;
(v) a nucleic acid sequence as defined in (i), (ii) or (iv), which has been modified to encode a modified polypeptide, wherein the modified polypeptide retains immunogenicity and is at least 75% identical in amino acid sequence to the corresponding polypeptide encoded by the nucleic acid of (i), (ii) or (iv);
wherein each first nucleic acid is capable of being expressed.

Another aspect of the invention provides a vaccine comprising a vaccine vector and at least one first nucleic acid selected from any one of:
(i) a nucleic acid sequence comprising at least 36 consecutive nucleotides from SEQ ID No: 17;
(ii) a nucleic acid sequence which encodes an immunogenic fragment comprising at least 12 consecutive amino acids from SEQ ID No: 18;
(iii) a nucleic acid sequence as defined in (i) or (ii), which has been modified to encode a modified polypeptide,
wherein the modified polypeptide retains immunogenicity and is at least 75% identical in amino acid sequence to the corresponding fragment of (i) or (ii);
wherein each first nucleic acid is capable of being expressed.

### BRIEF DESCRIPTION OF THE DRAWINGS

The present invention will be further understood from the following description with reference to the drawings, in which:

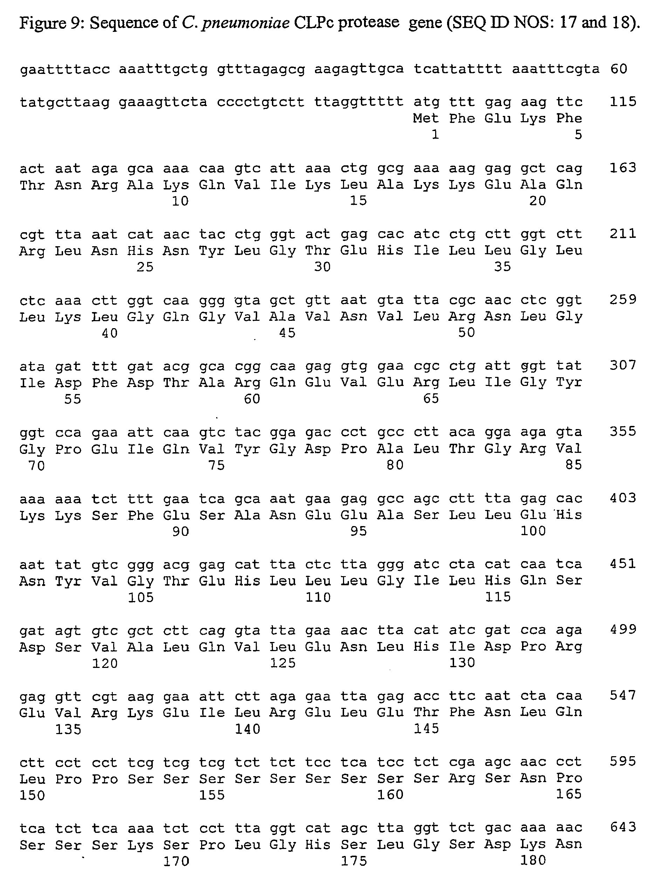

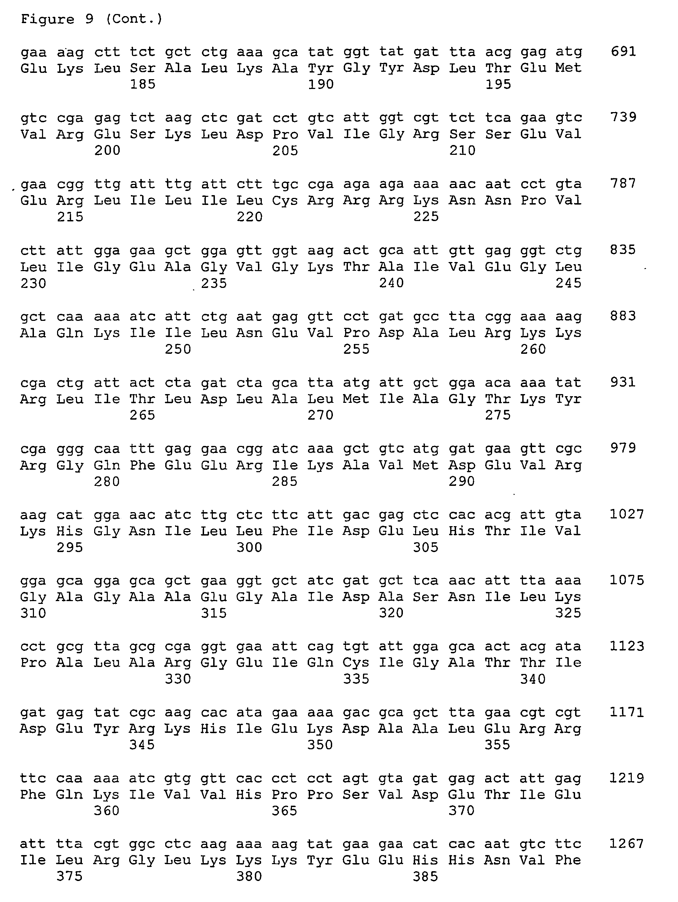

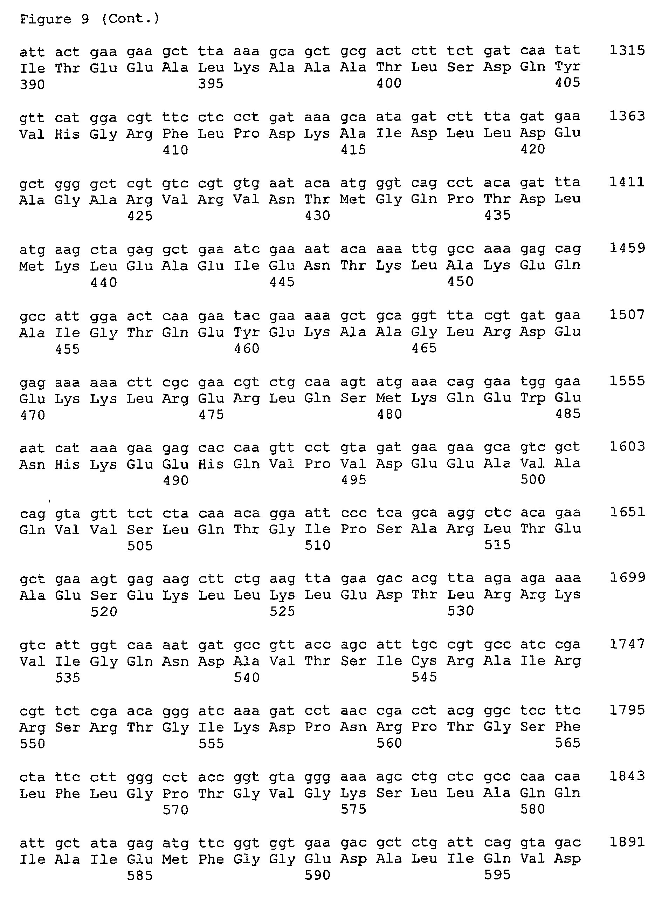

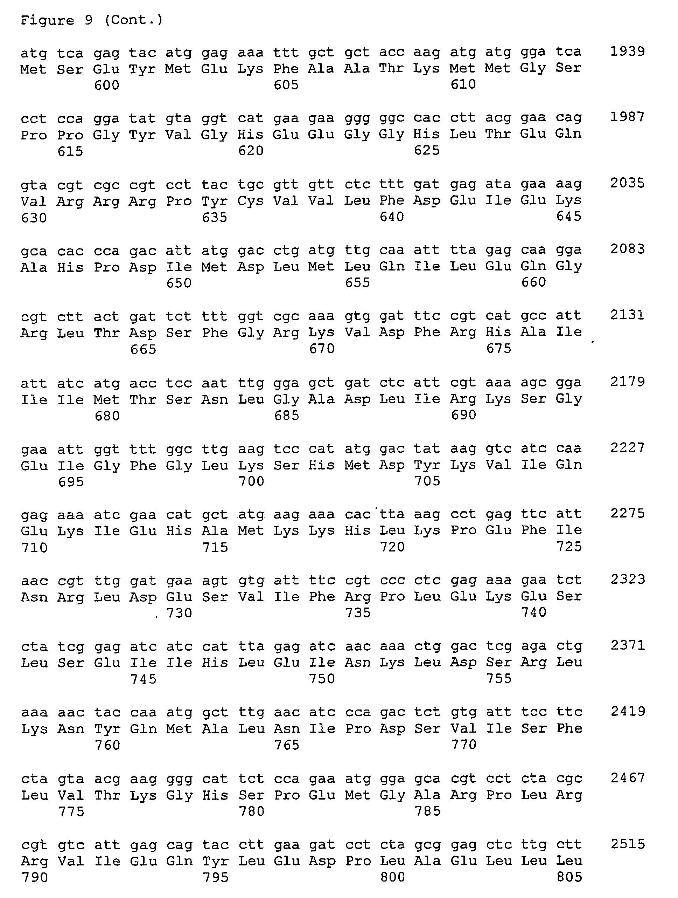

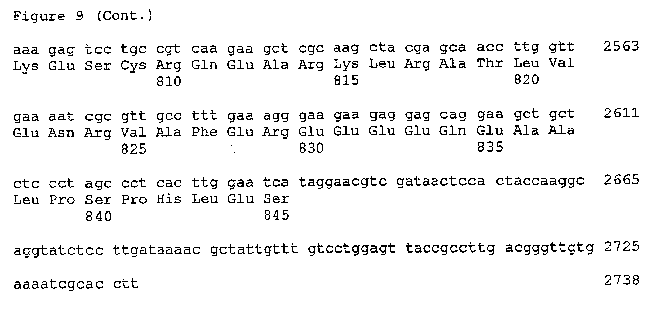

Figure 9 shows the nucleotide sequence of the gene encoding a CLPc protease (SEQ ID No: 17) and the deduced amino acid sequence of the CLPc protease from *Chlamydia pneumoniae* (SEQ ID No: 18).

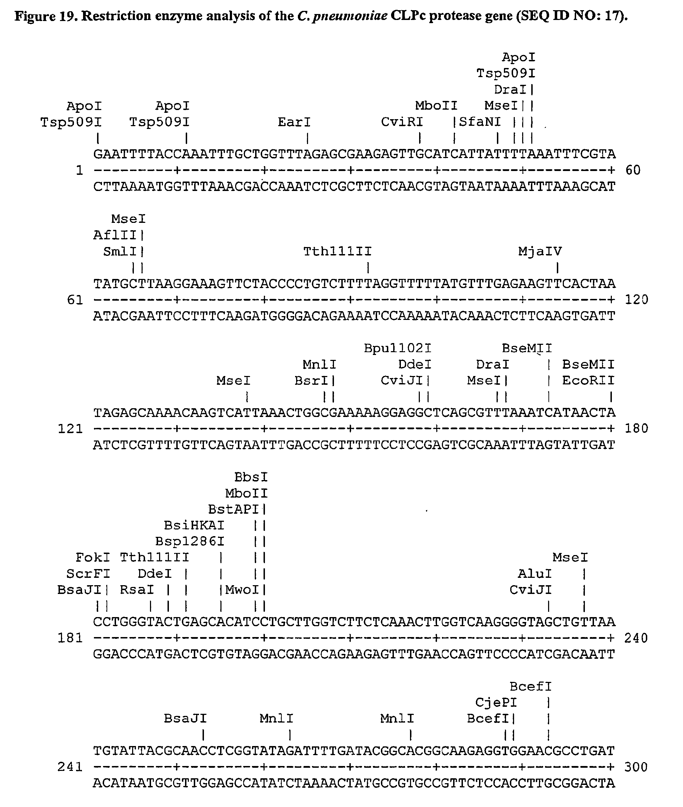

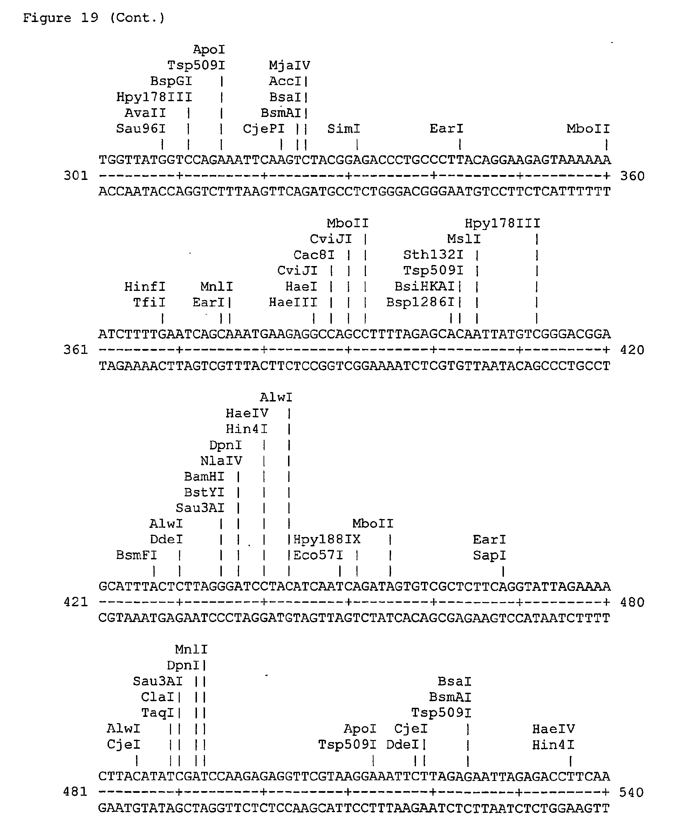

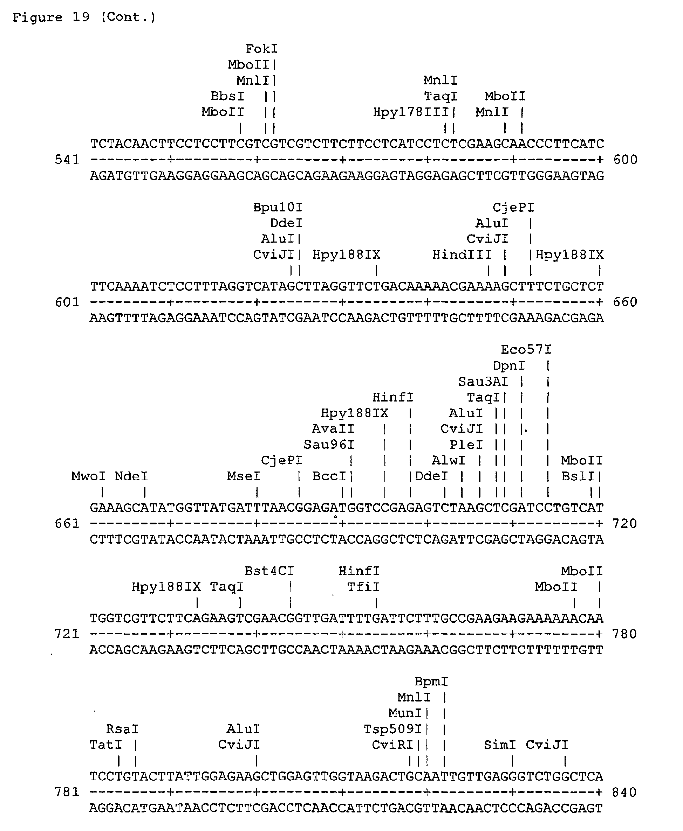

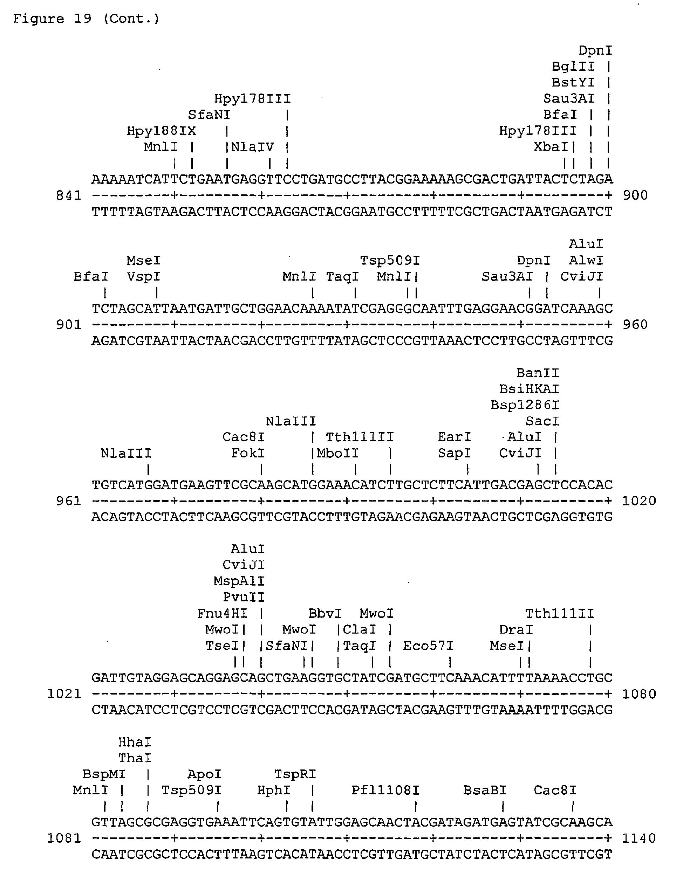

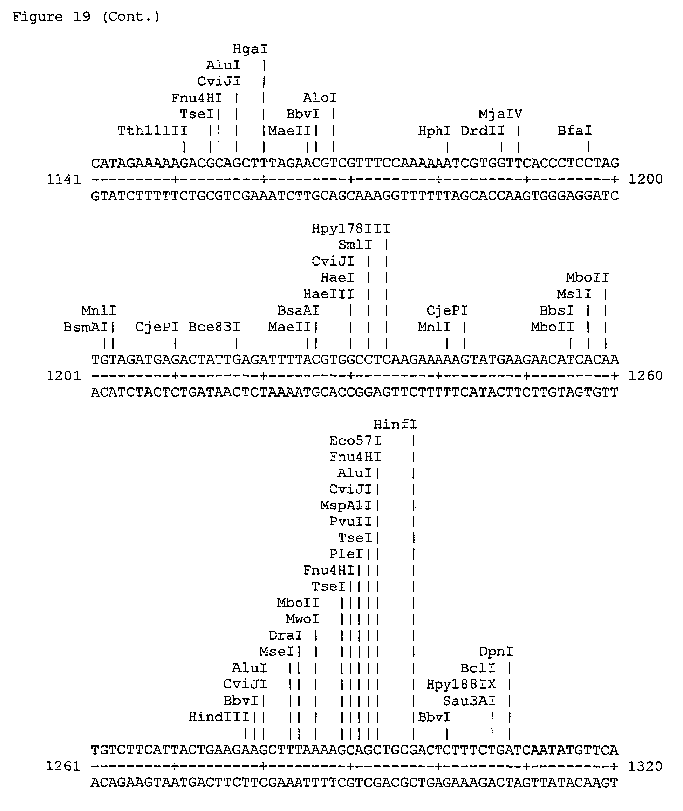

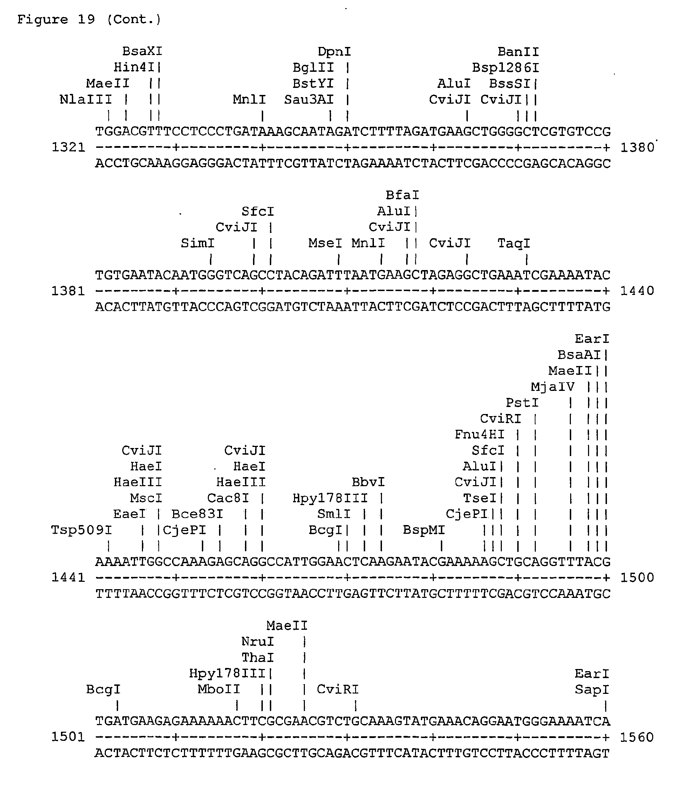

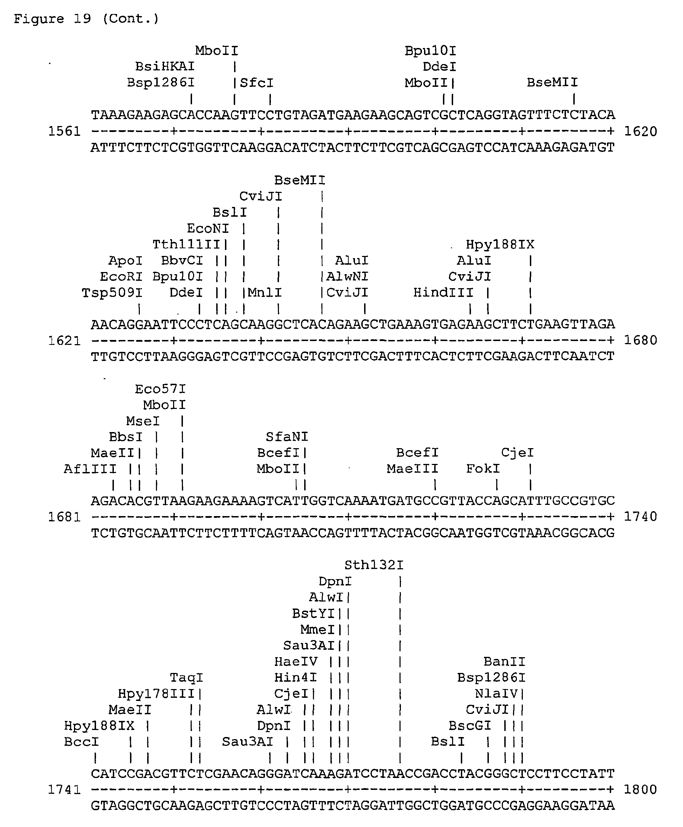

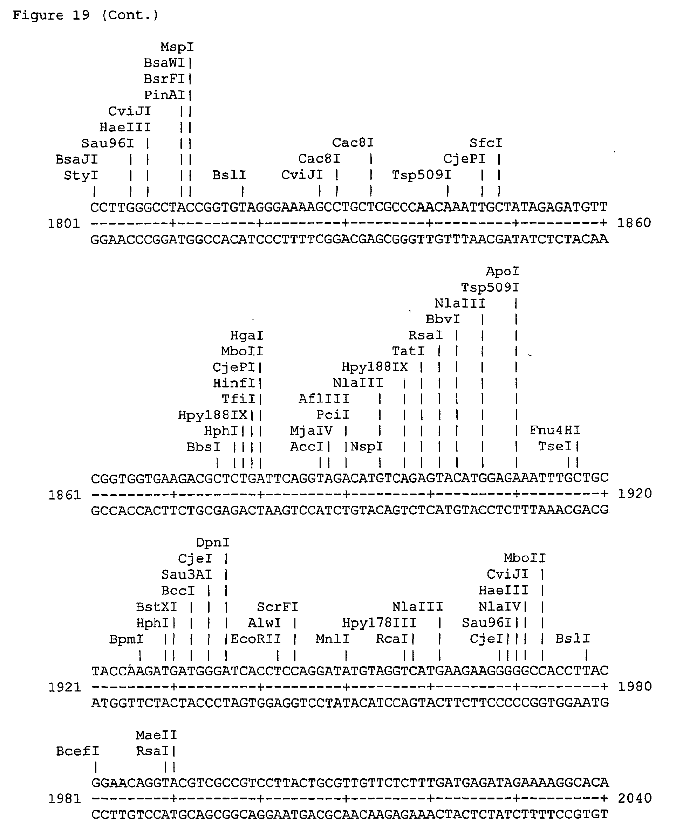

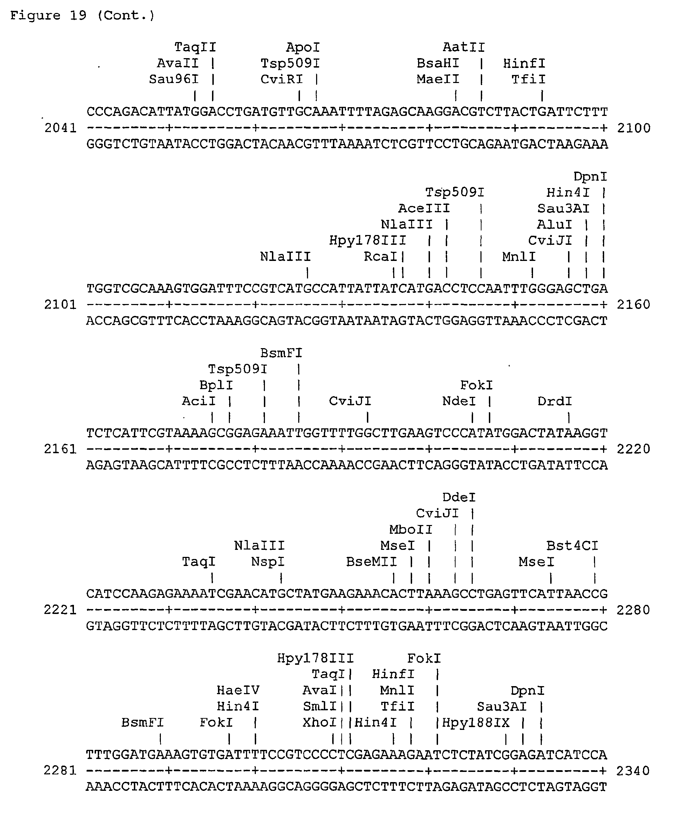

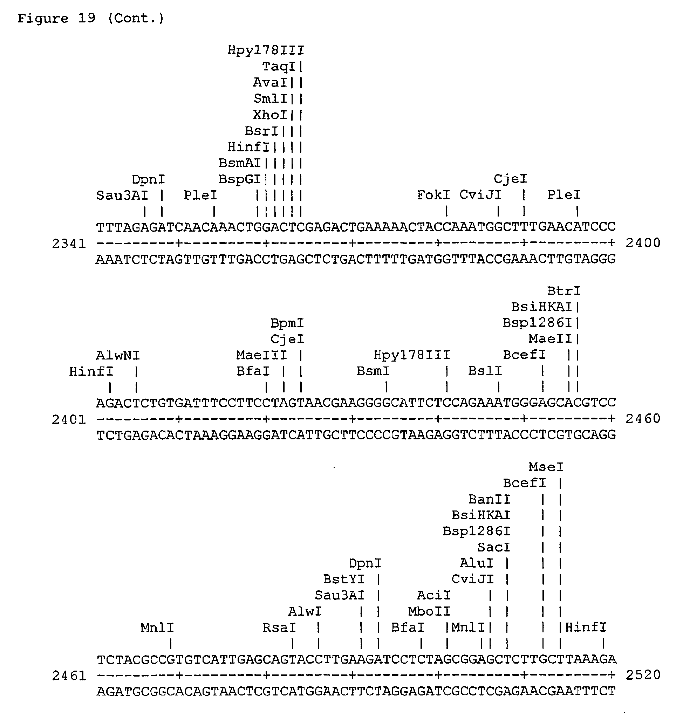

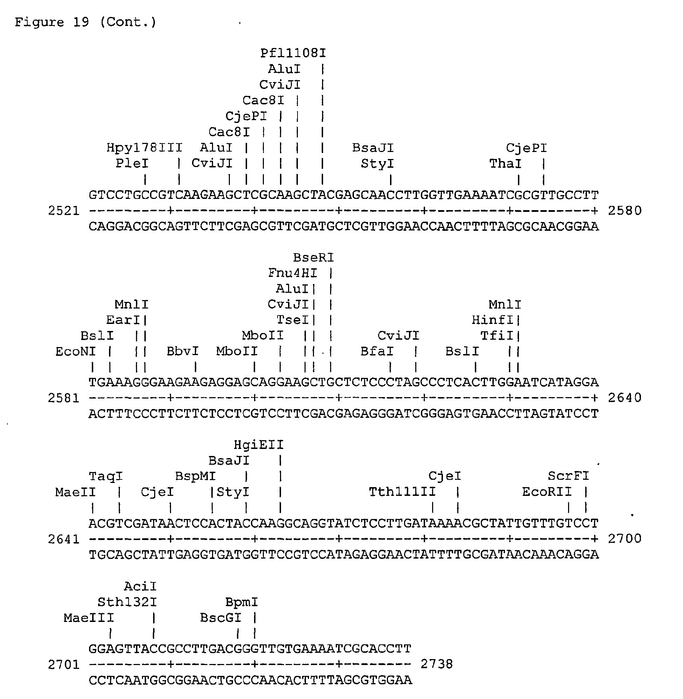

Figure 19 shows the restriction enzyme analysis of the *C. pneumoniae* gene encoding a CPLc protease.
Figure 29 shows the construction and elements of plasmid pCACPNM459.
Figure 39 illustrates protection against *C. pneumoniae* infection by pCACPNM459 following DNA immunization.

The application also discloses further drawings, in which:

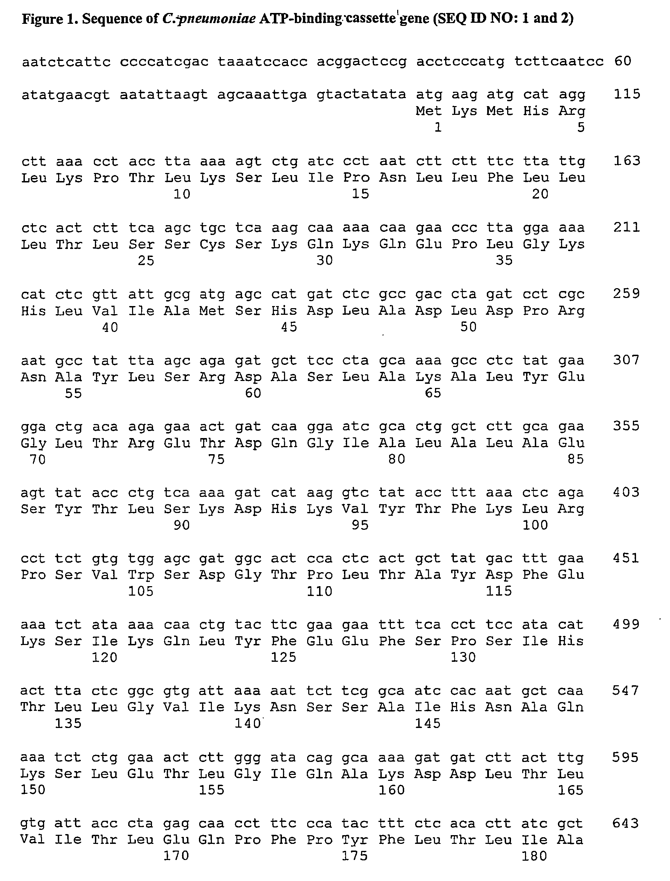

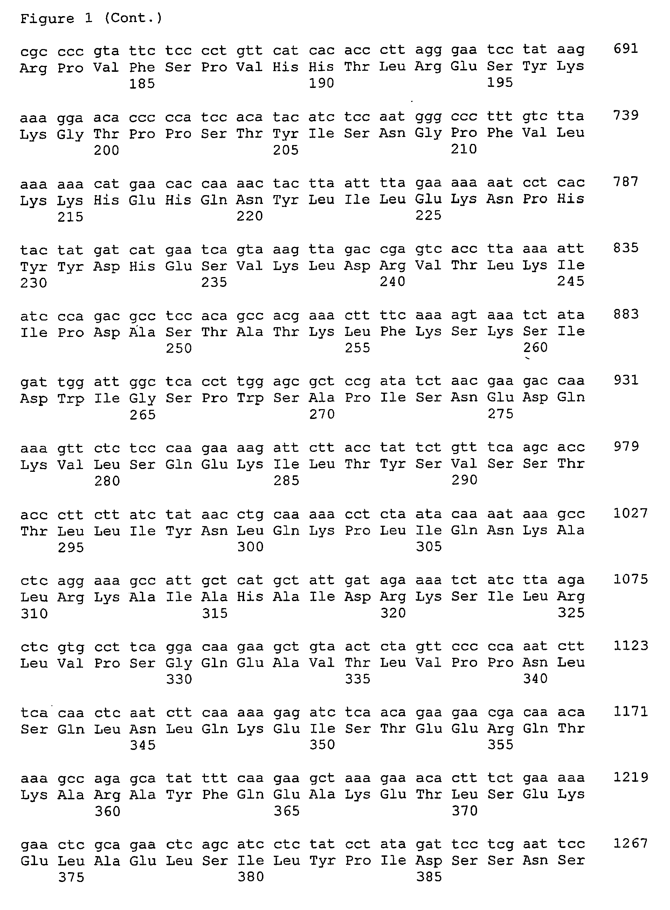

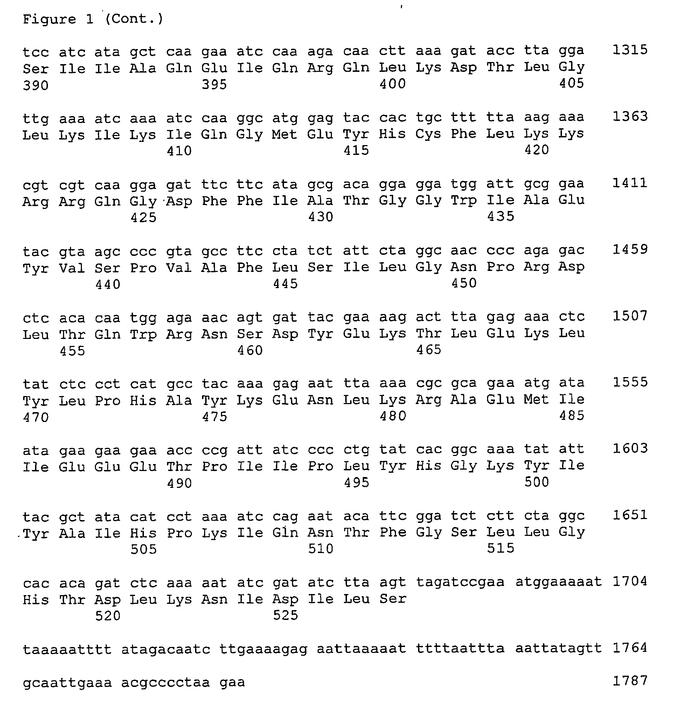

Figure 1 shows the nucleotide sequence of the gene encoding an ATP-binding cassette (SEQ ID No: 1) and the deduced amino acid sequence of the ATP-binding cassette from *Chlamydia pneumoniae* (SEQ ID No: 2) .

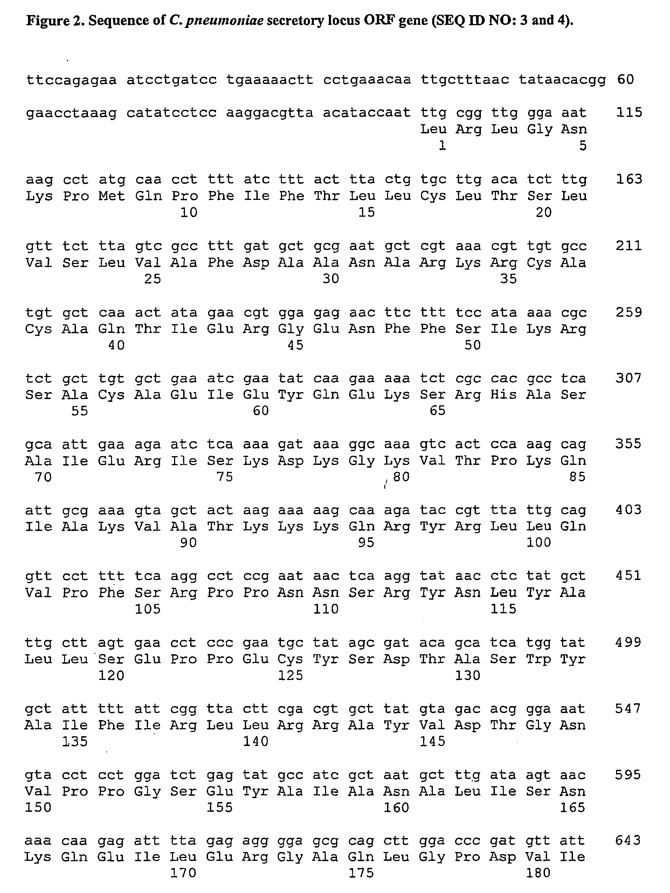

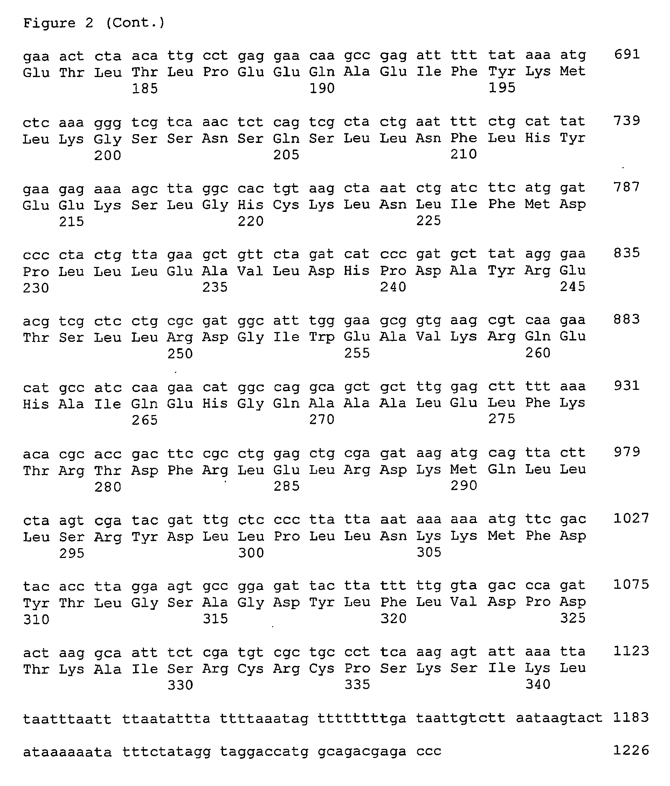

Figure 2 shows the nucleotide sequence of the gene encoding a secretory locus ORF (SEQID No: 3) and the deduced amino acid sequence of the secretory locus ORF from *Chlamydia pneumoniae* (SEQ ID No: 4).

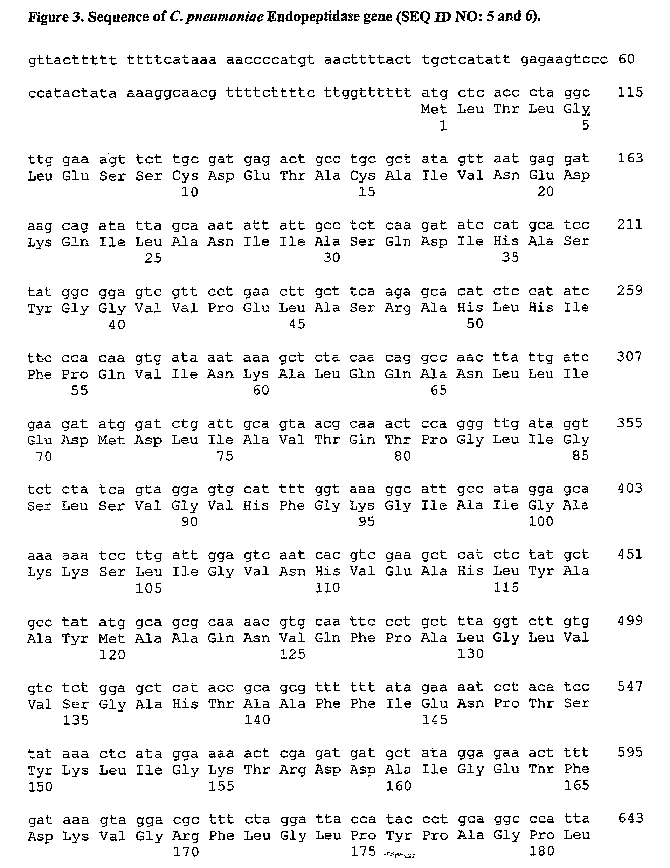

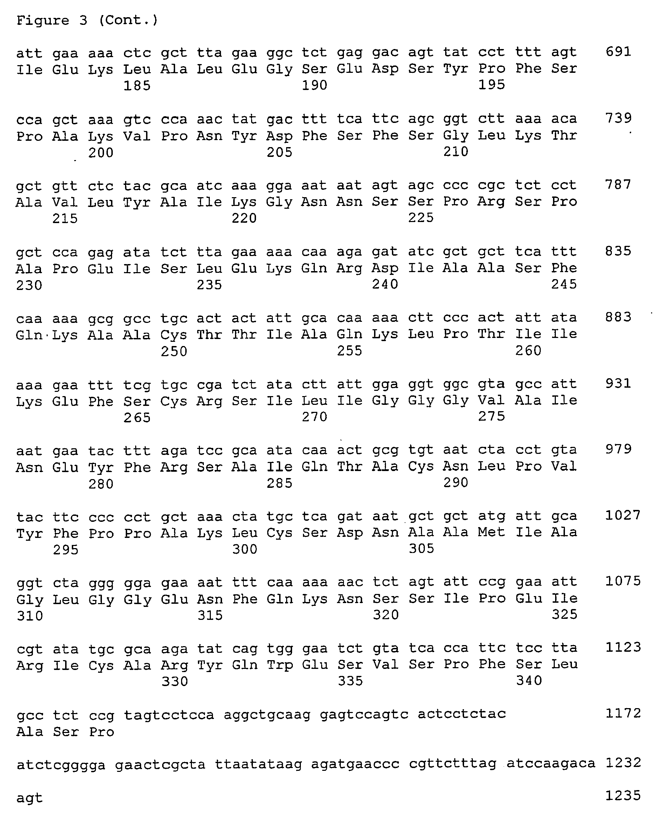

Figure 3 shows the nucleotide sequence of the gene encoding an endopeptidase (SEQ ID No: 5) and the deduced amino acid sequence of the endopeptidase from *Chlamydia pneumoniae* (SEQ ID No: 6).

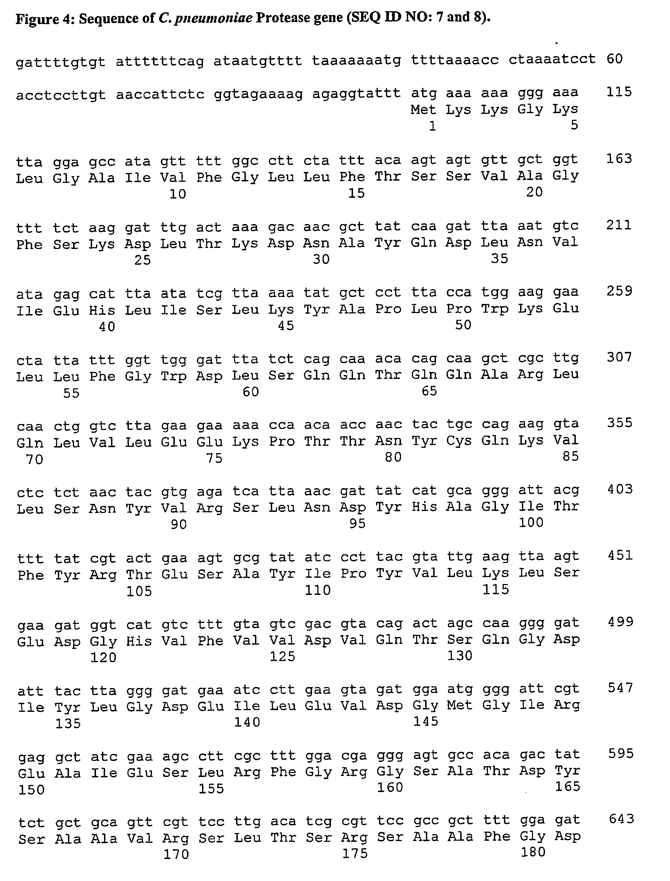

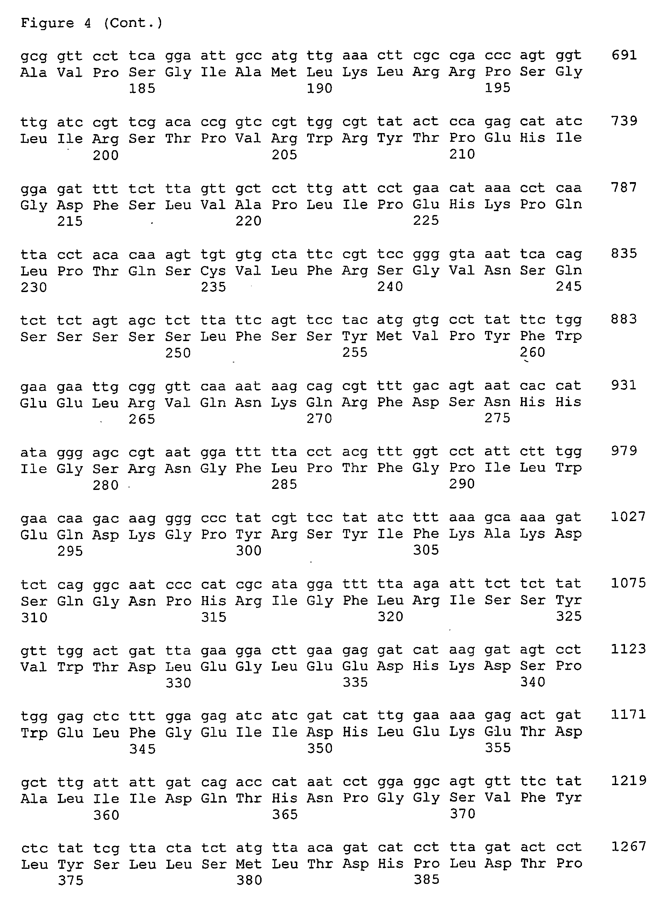

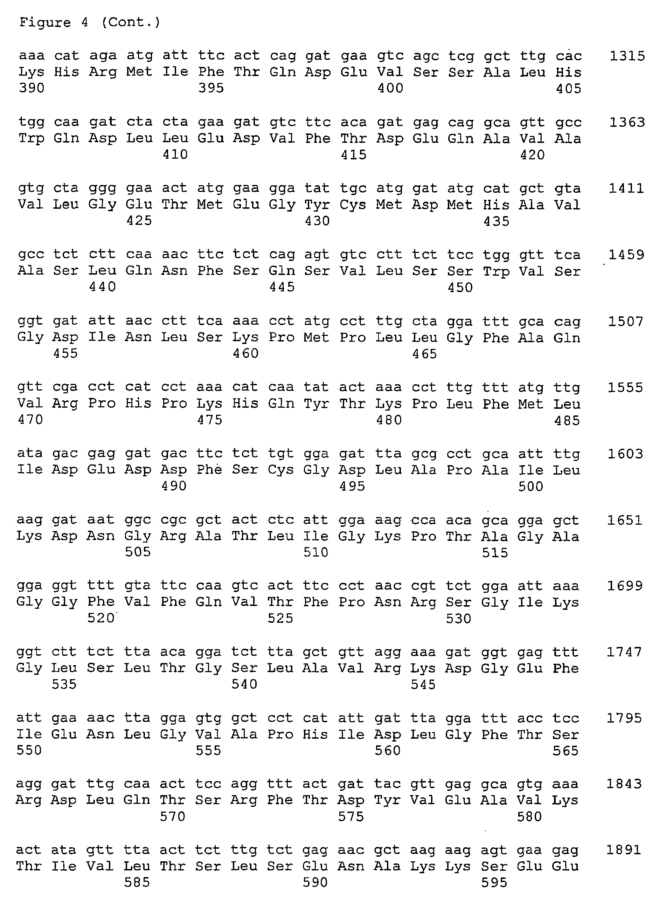

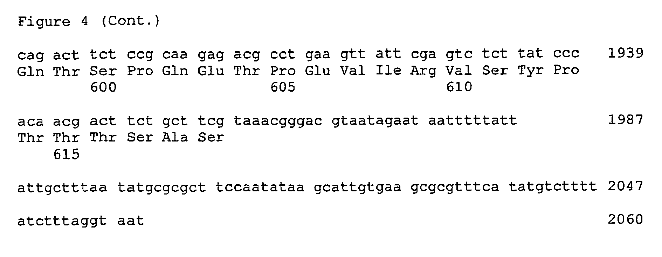

Figure 4 shows the nucleotide sequence of the gene encoding a protease (SEQ ID No: 7) and the deduced amino acid sequence of the protease from *Chlamydia pneumoniae* (SEQ ID No: 8).

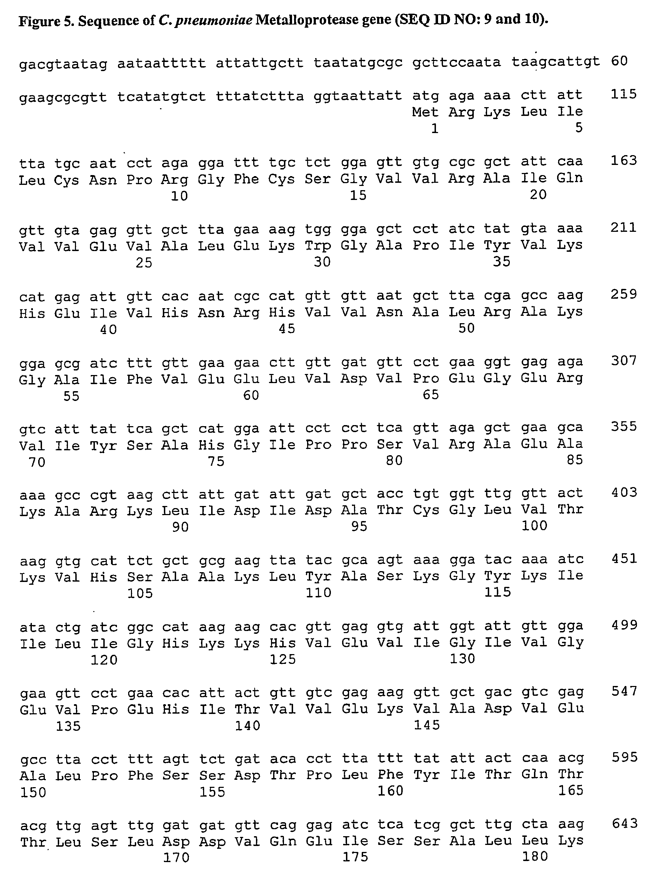

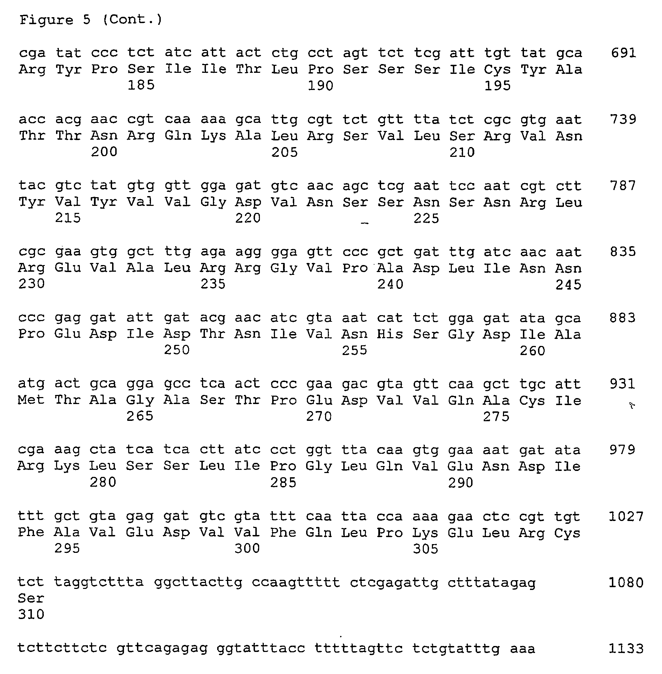

Figure 5 shows the nucleotide sequence of the gene encoding a metalloprotease (SEQ ID No: 9) and the deduced amino acid sequence of the metalloprotease from *Chlamydia pneumoniae* (SEQ ID No: 10).

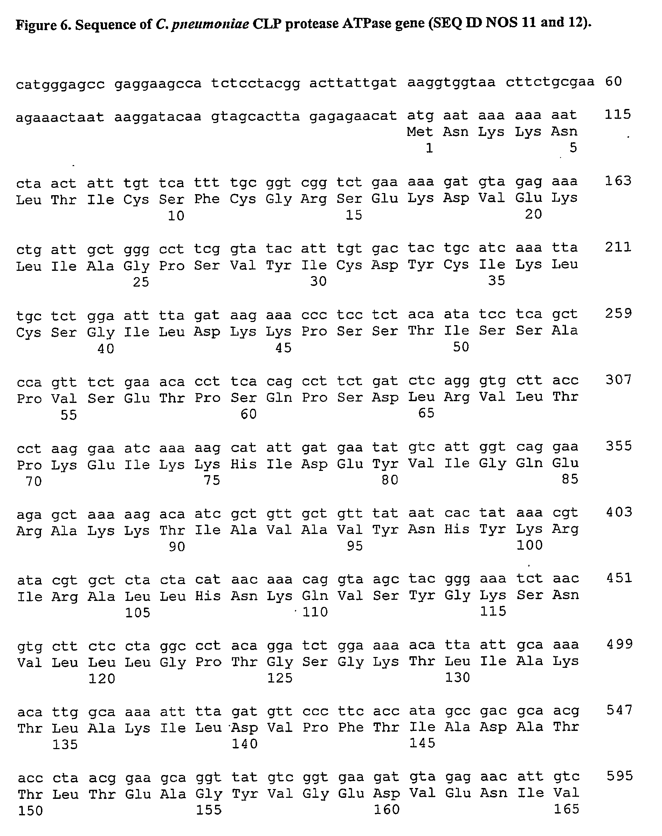

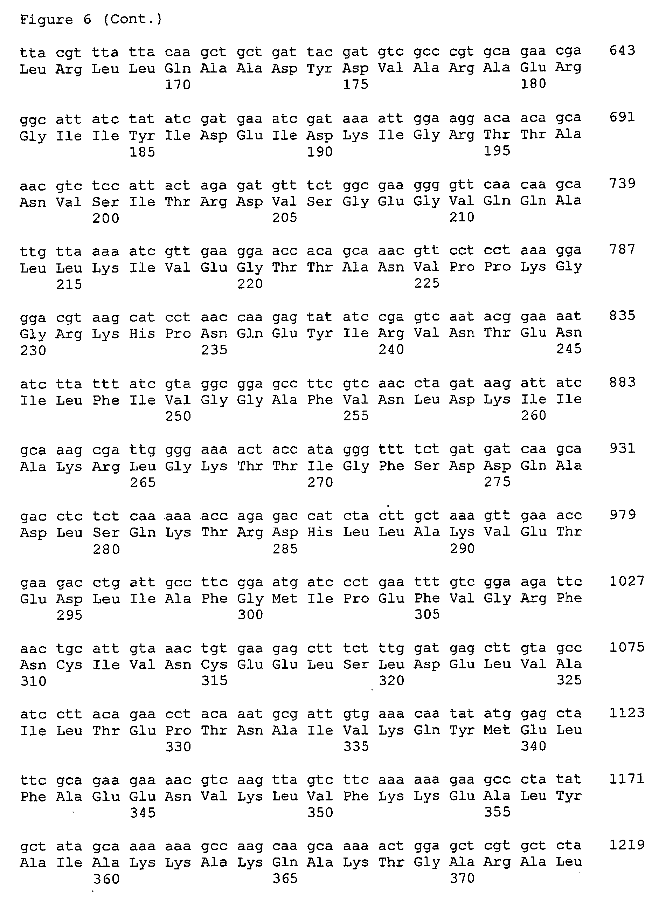

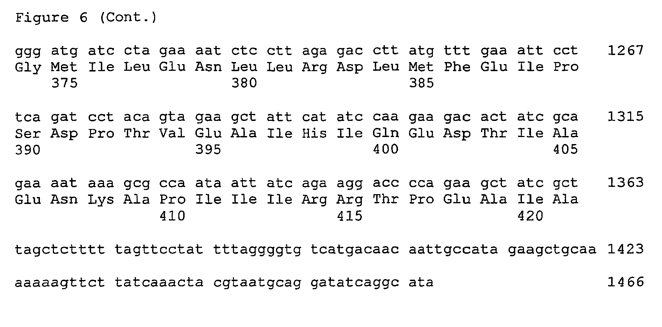

Figure 6 shows the nucleotide sequence of the gene encoding CLP protease ATPase (SEQ ID No: 11) and the deduced amino acid sequence of the CLP protease ATPase from *Chlamydia pneumoniae* (SEQ ID No: 12).

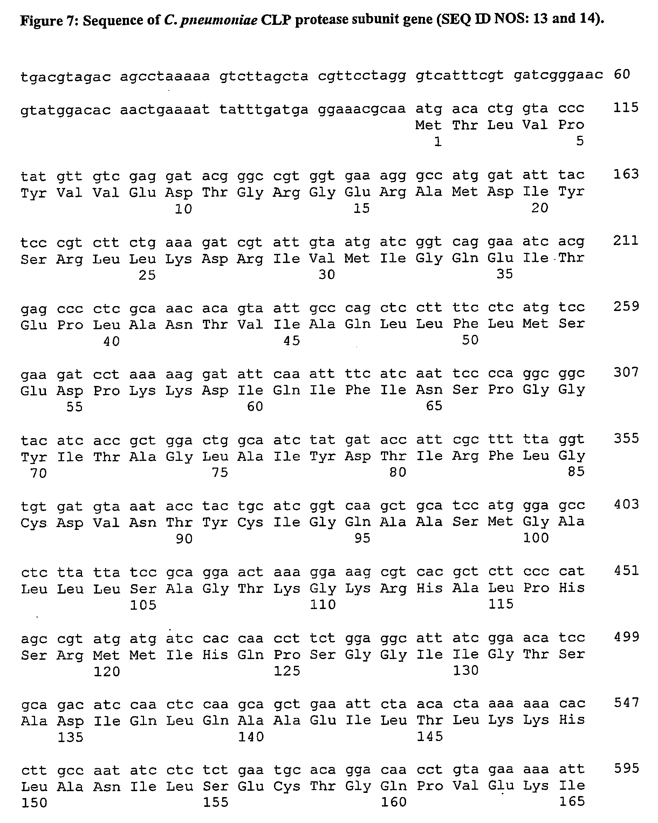

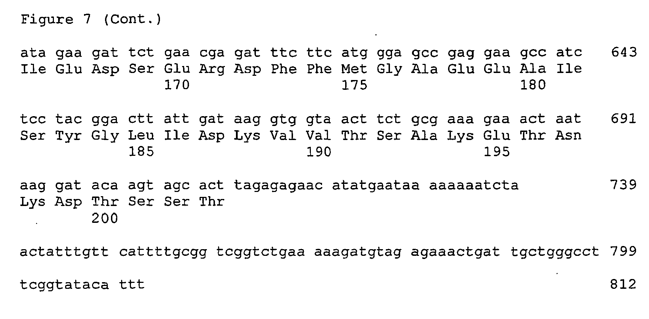

Figure 7 shows the nucleotide sequence of the gene encoding a CLP protease subunit (SEQ ID No: 13) and the deduced amino acid sequence of the CLP protease subunit from *Chlamydia pneumoniae* (SEQ ID No: 14).

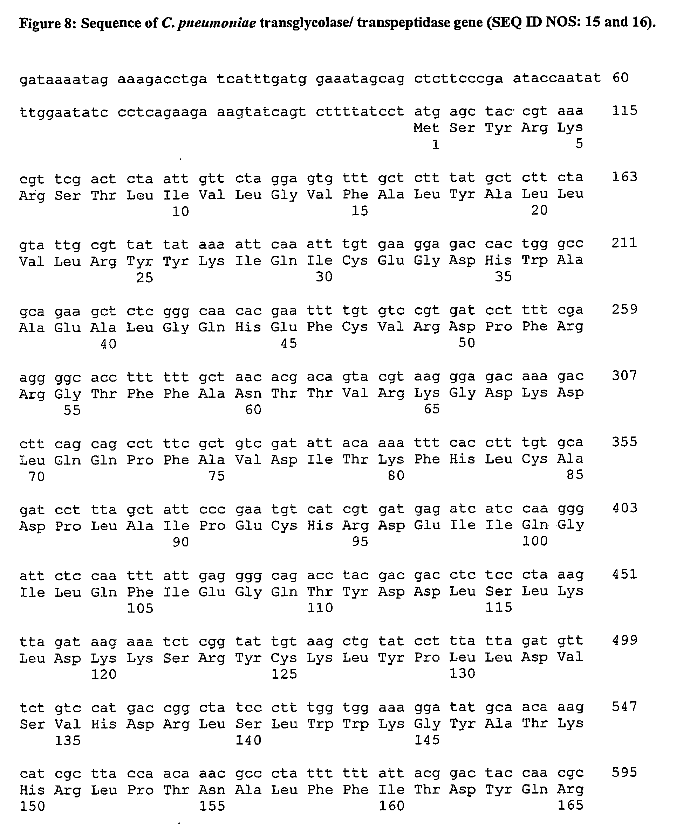

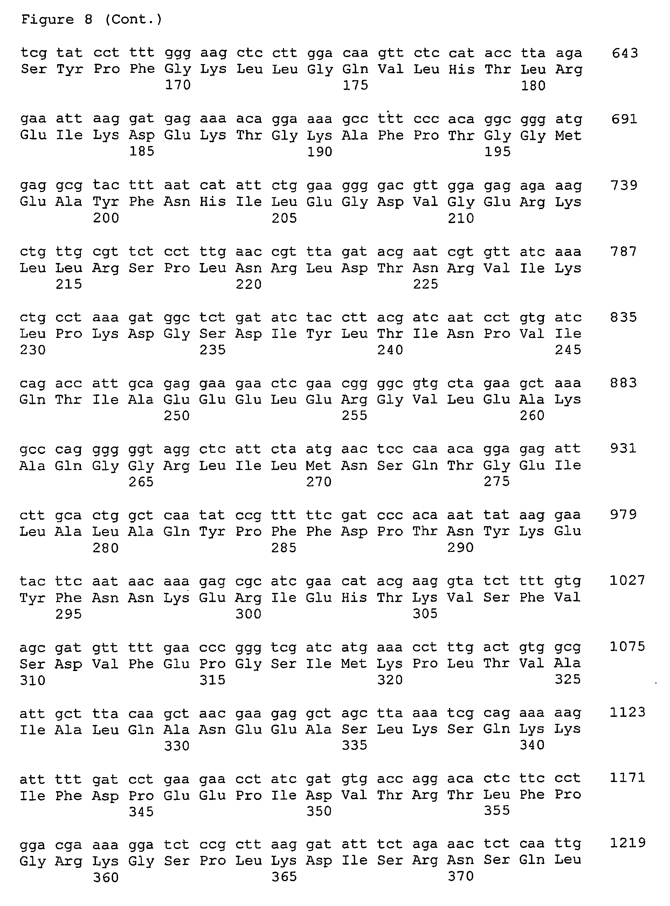

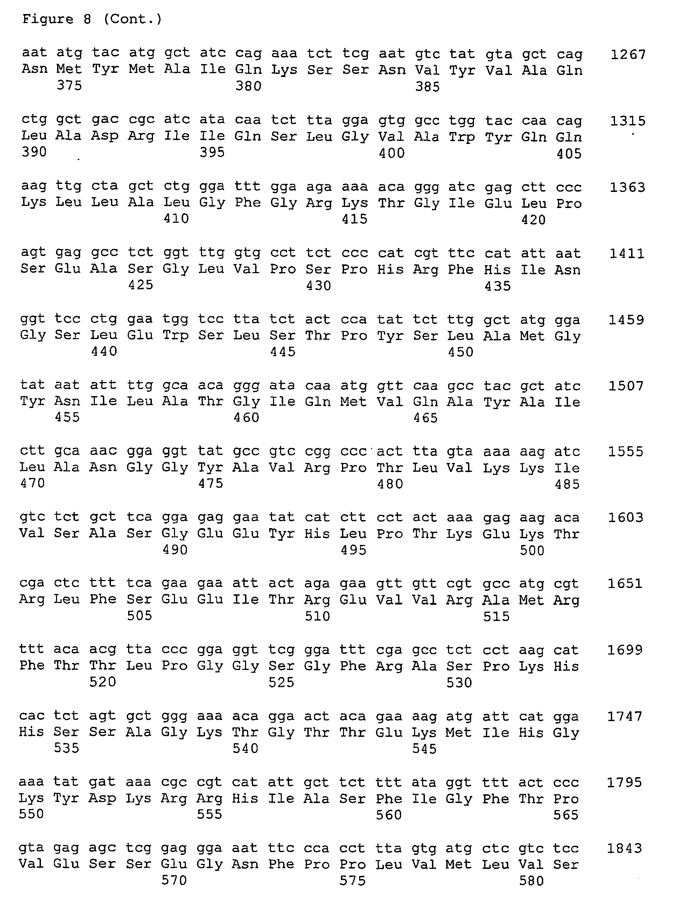

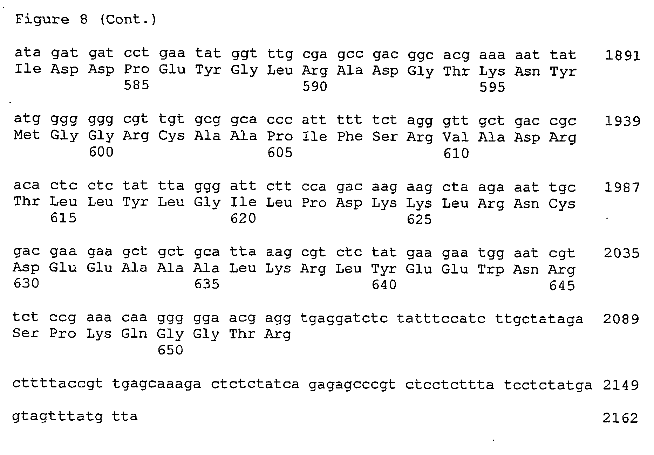

Figure 8 shows the nucleotide sequence of the gene encoding a translycolase / transpeptidase (SEQ ID No: 15) and the deduced amino acid sequence of the transglycolase / transpeptidase from *Chlamydia pneumoniae* (SEQ ID No: 16).

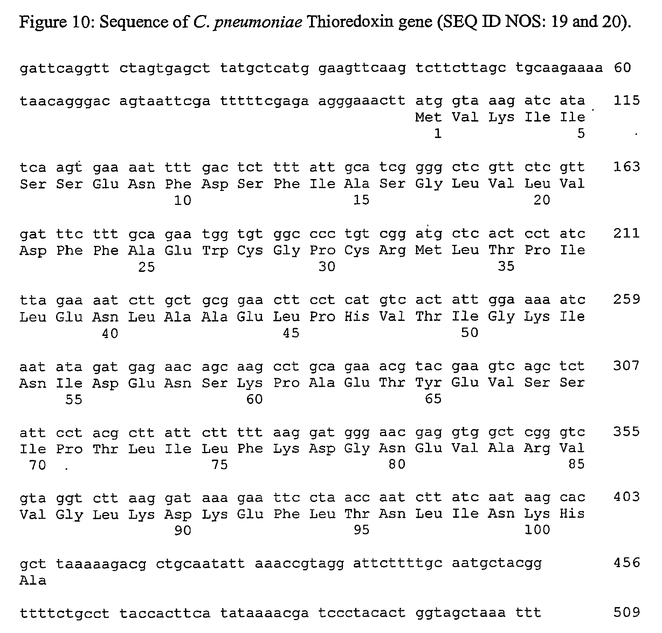

Figure 10 shows the nucleotide sequence of the gene encoding thioredoxin(SEQ ID No: 19) and the deduced amino acid sequence of thioredoxin from *Chlamydia pneumoniae* (SEQ ID No: 20).

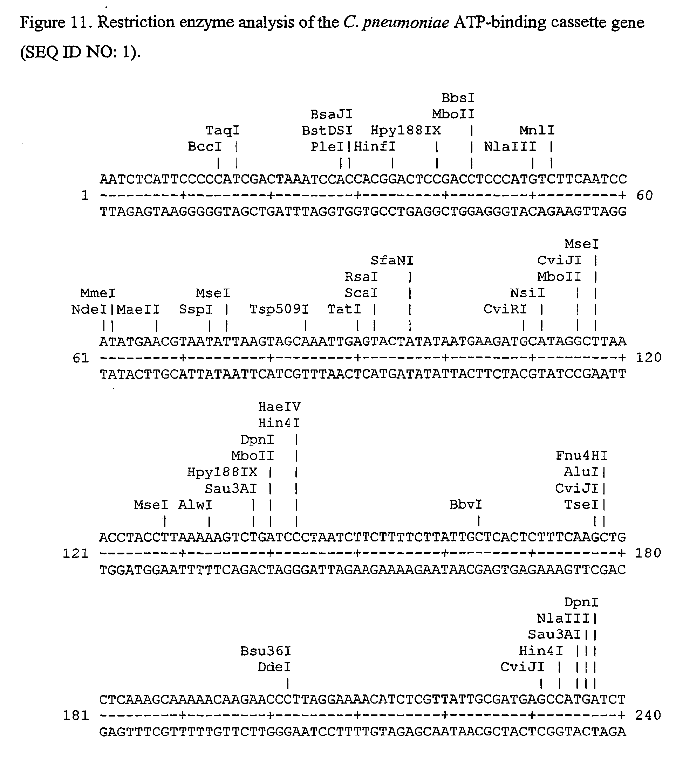

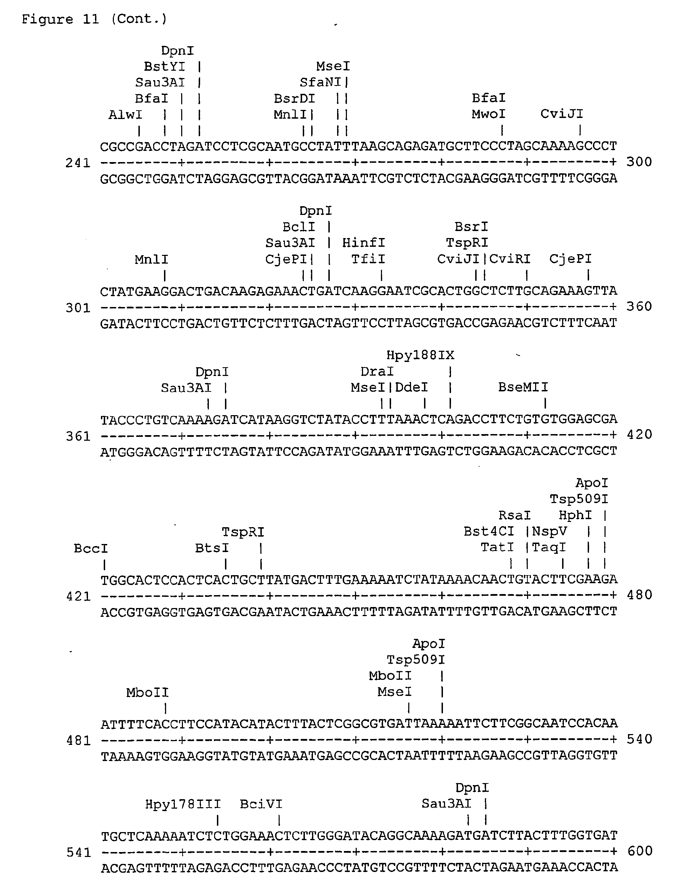

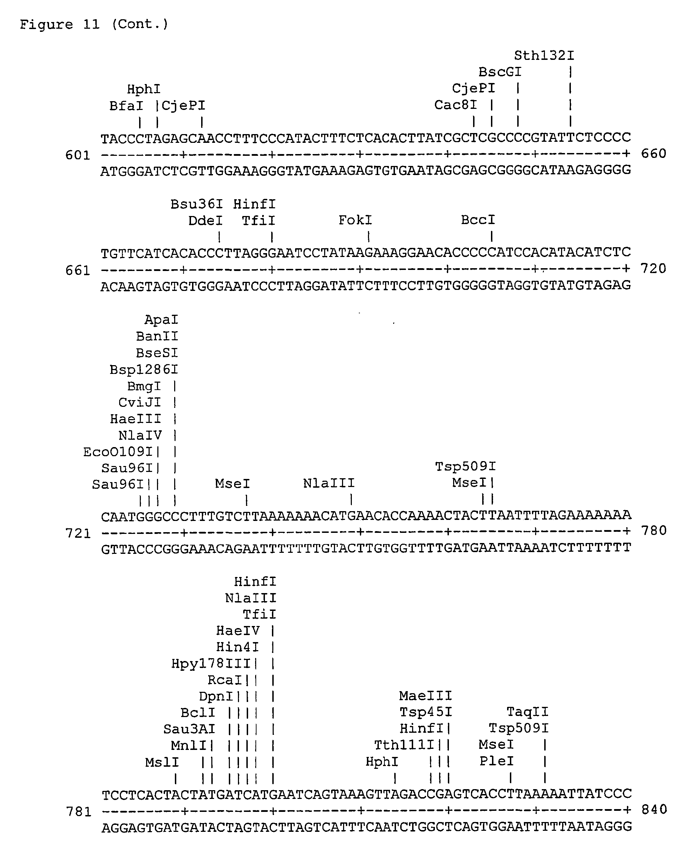

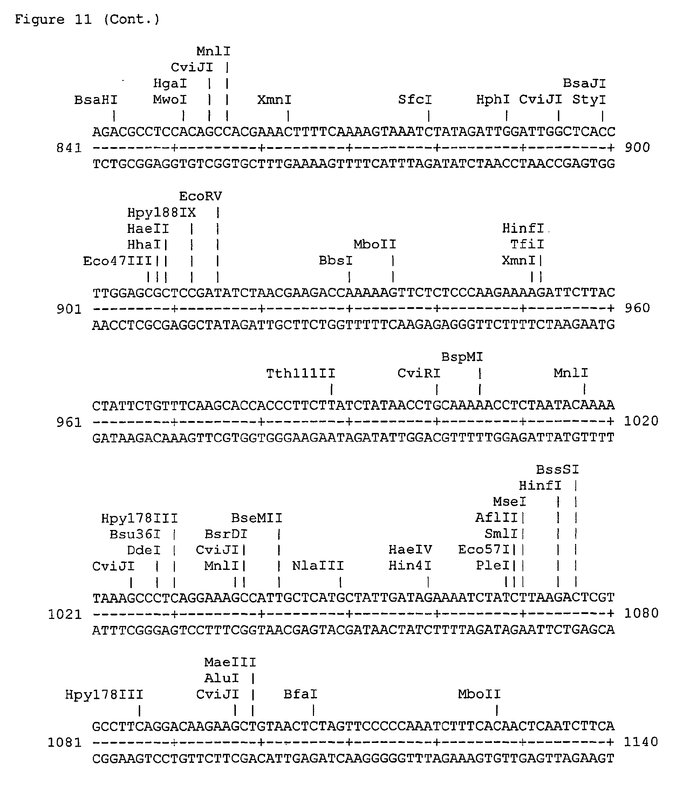

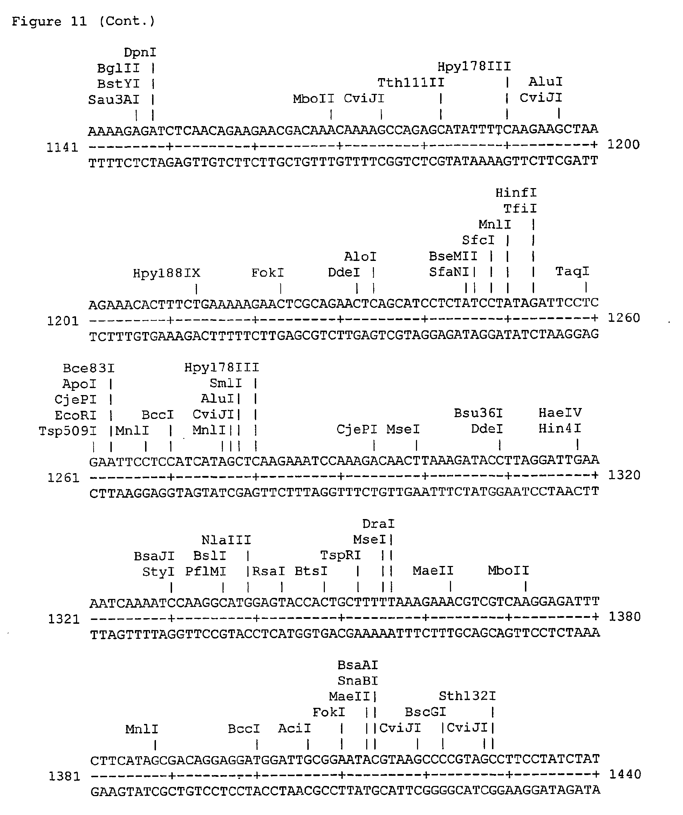

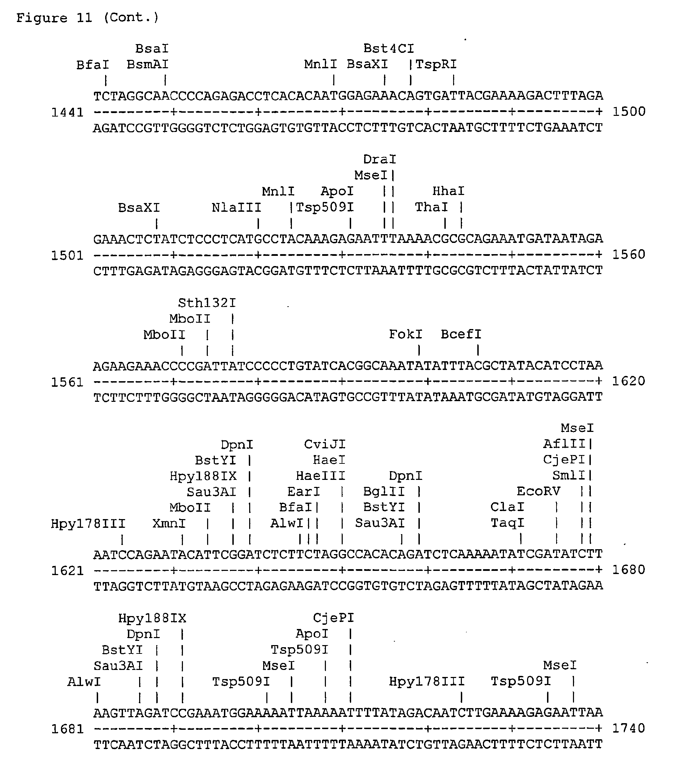

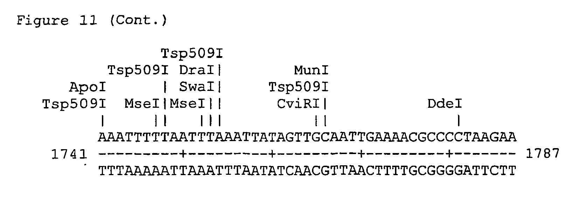

Figure 11 shows the restriction enzyme analysis of the *C. pneumoniae* gene encoding an ATP-binding cassette.

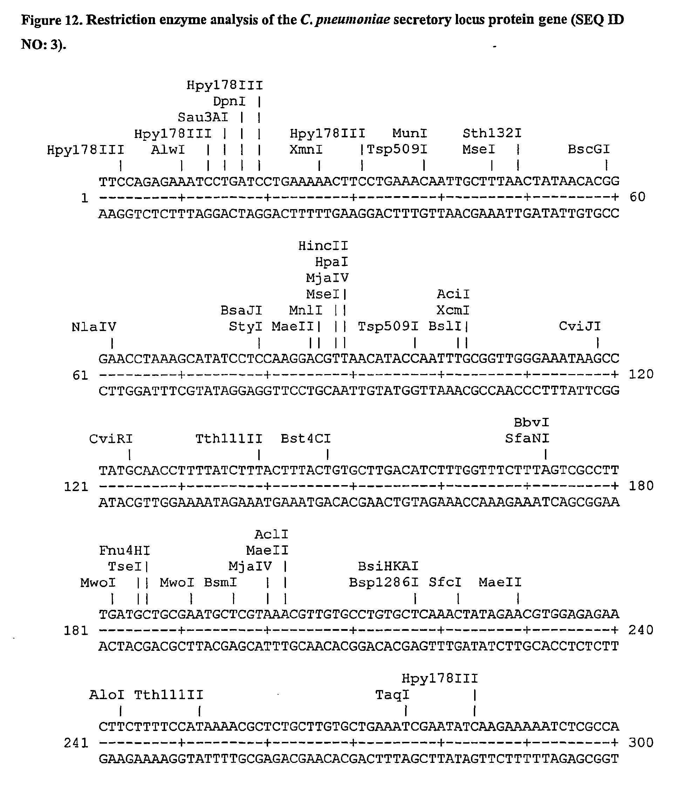

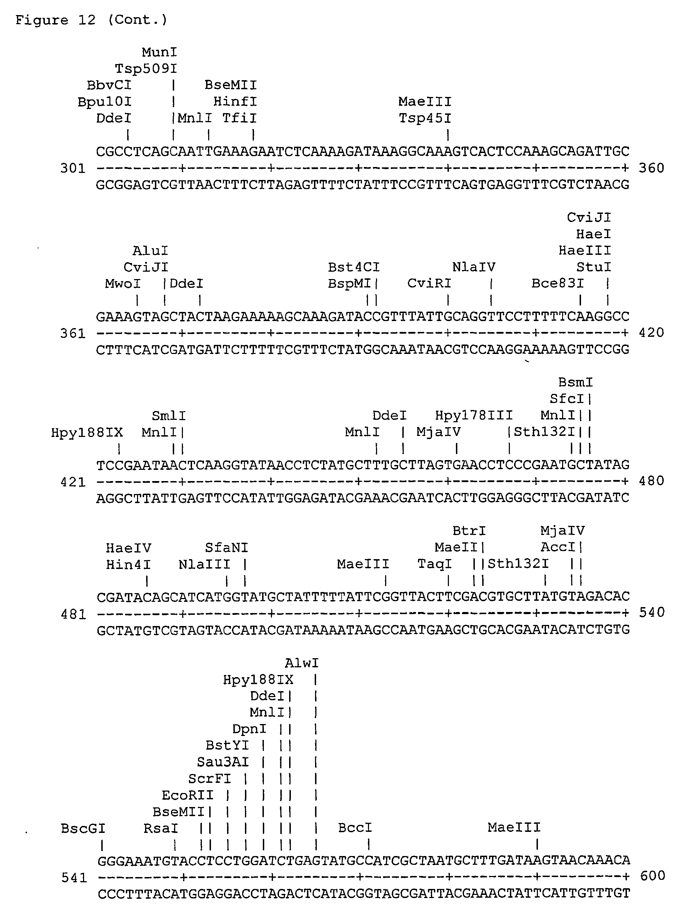

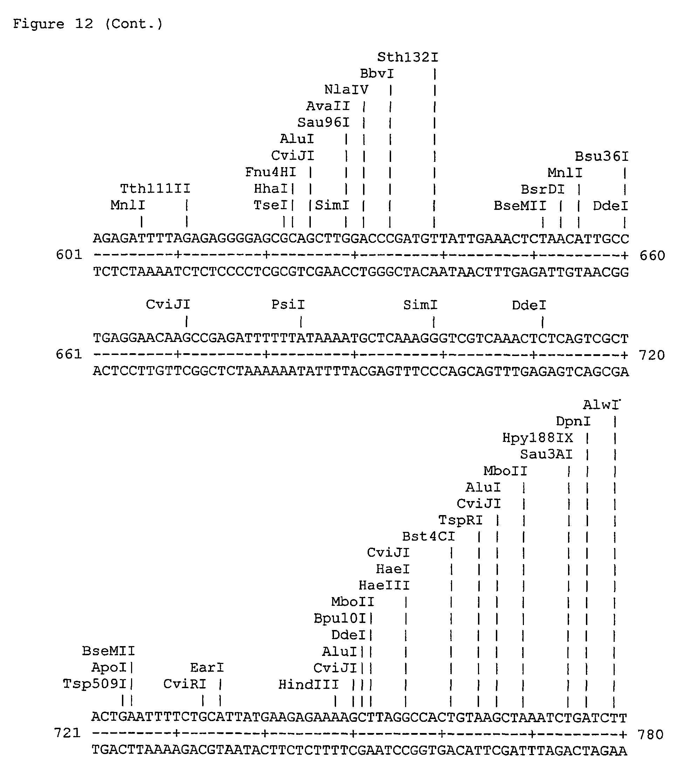

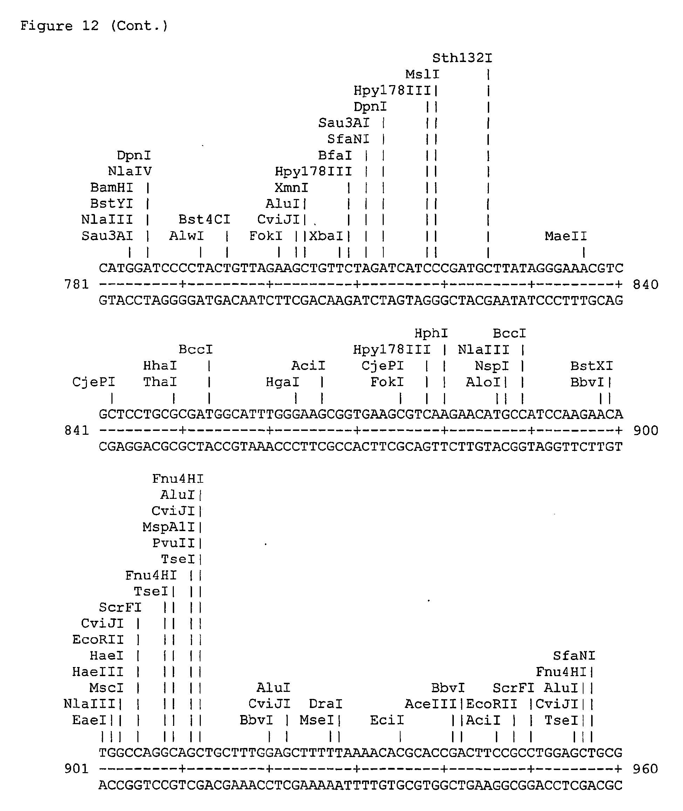

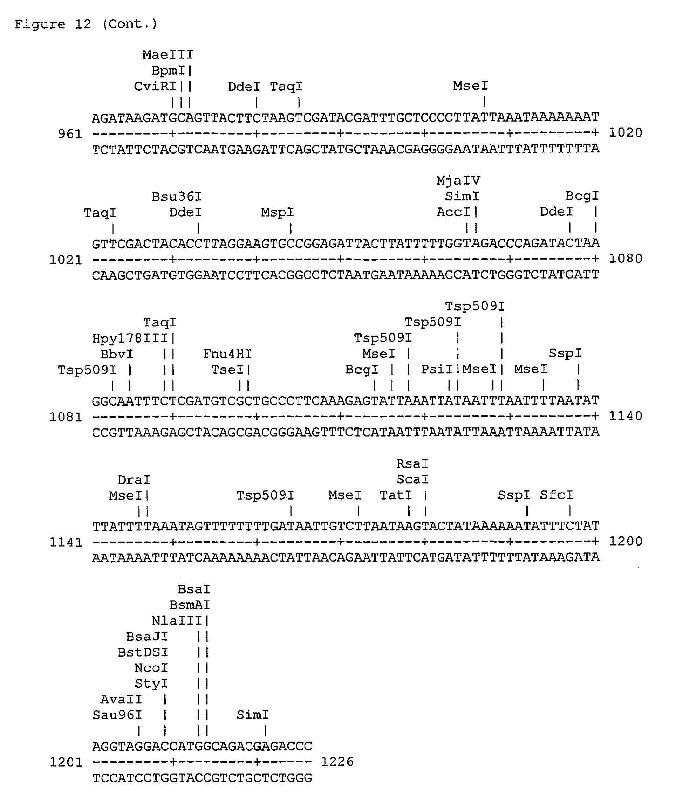

Figure 12 shows shows the restriction enzyme analysis of the *C. pneumoniae* gene encoding a secretory locus ORF.

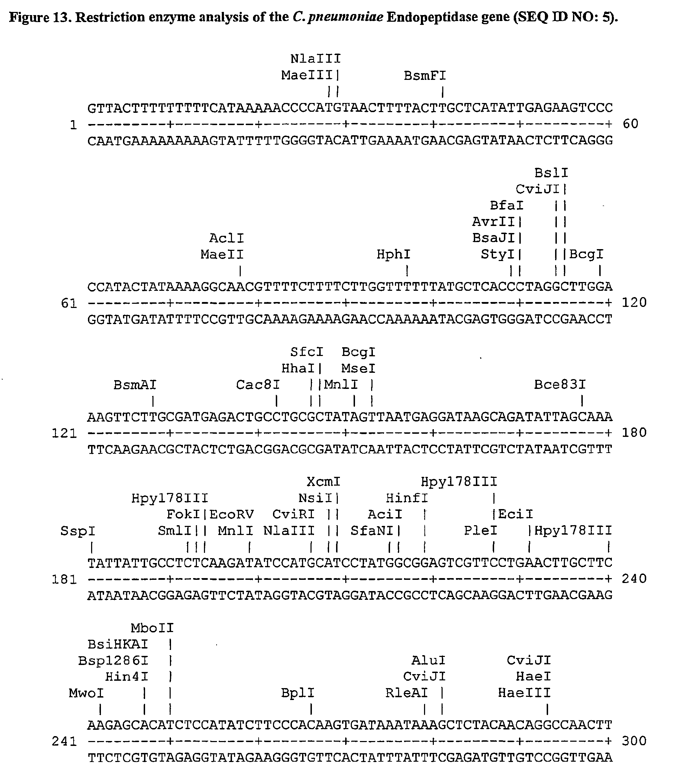

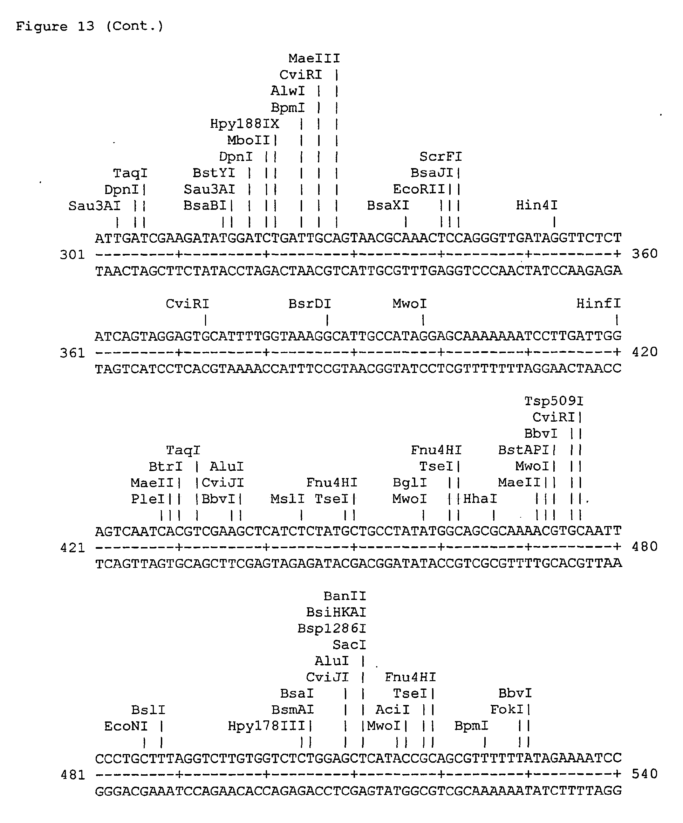

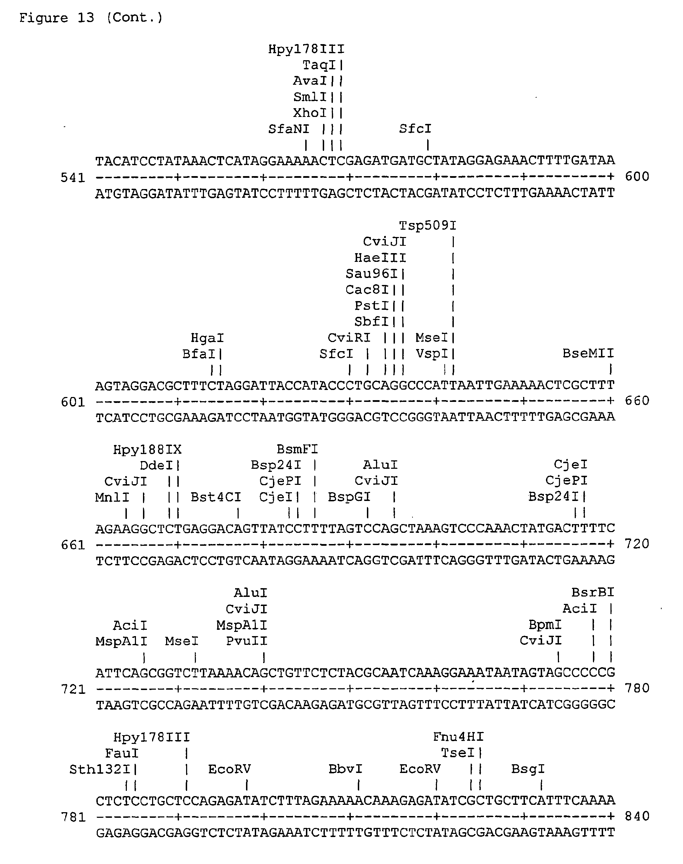

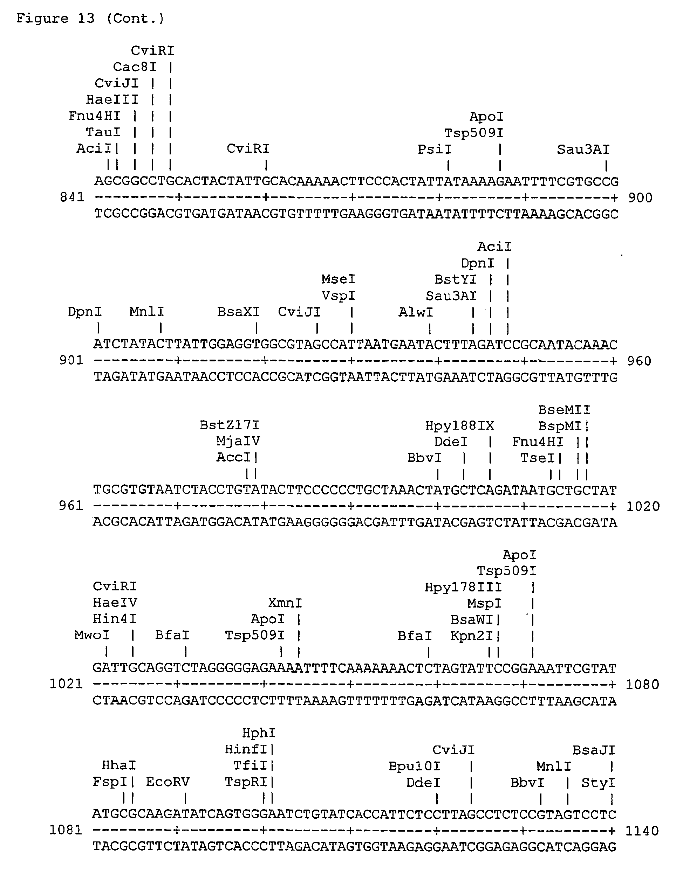

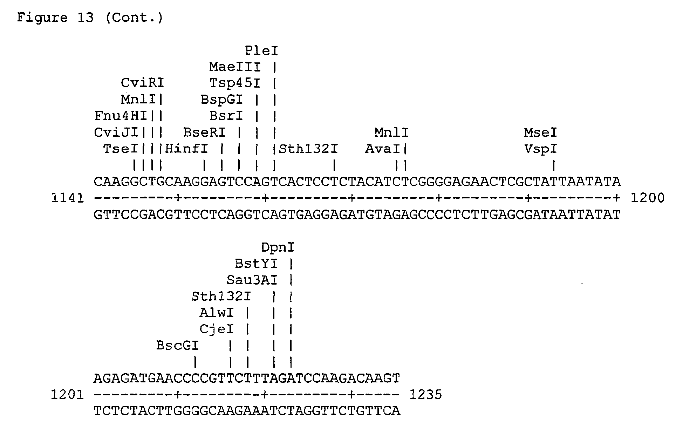

Figure 13 shows the restriction enzyme analysis of the *C. pneumoniae* gene encoding an endopeptidase.

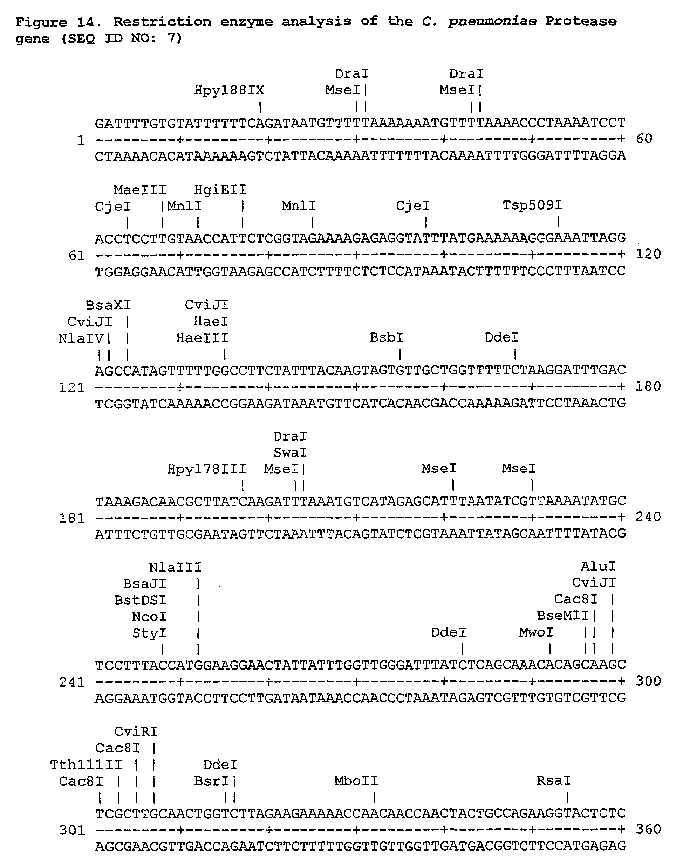

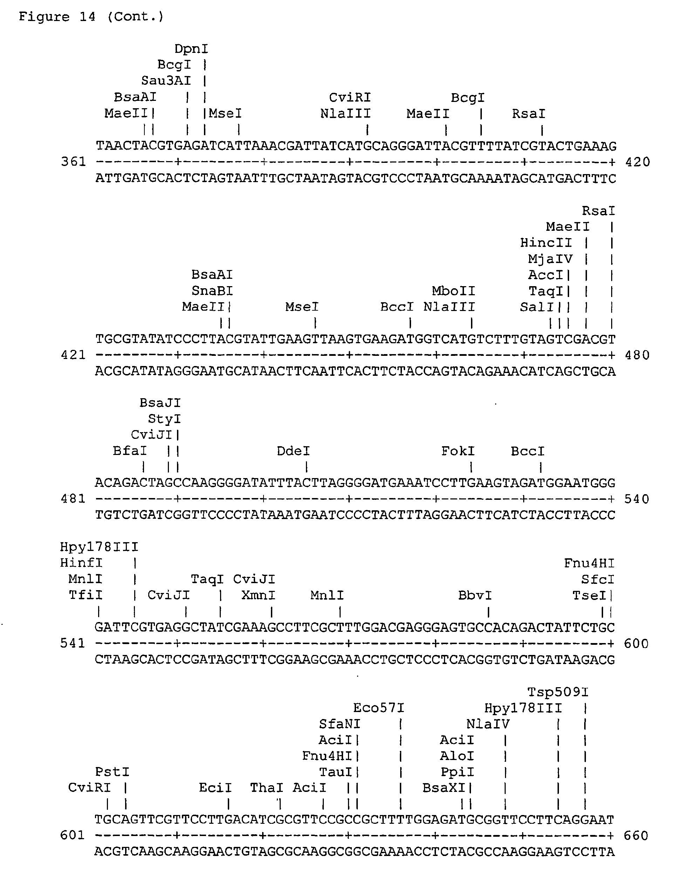

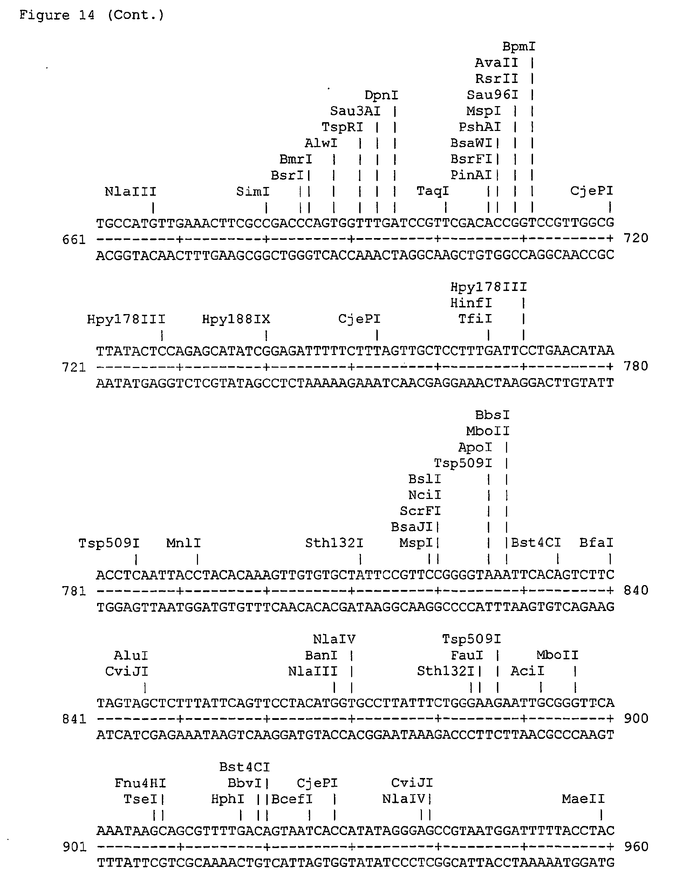

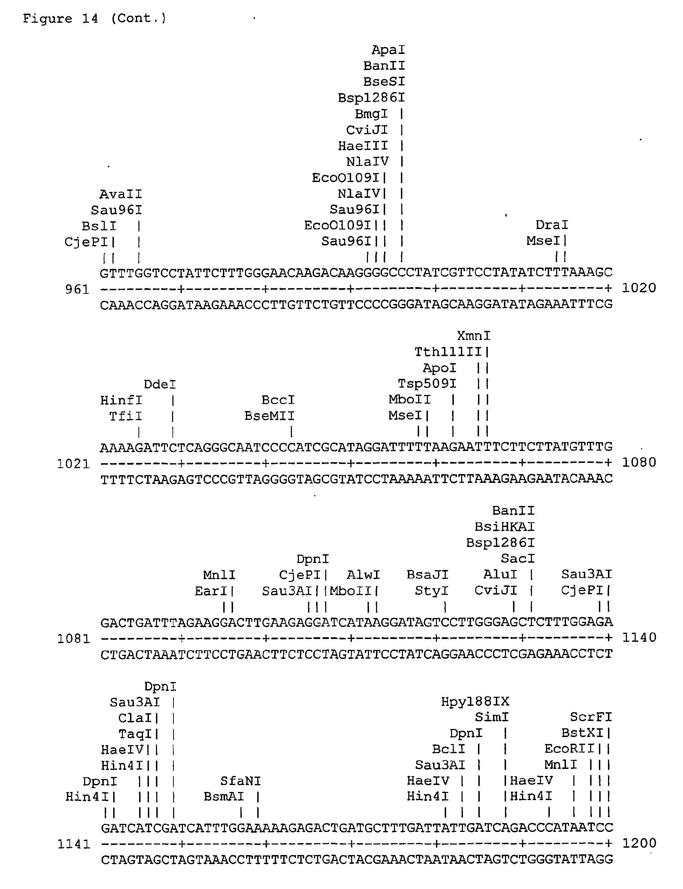

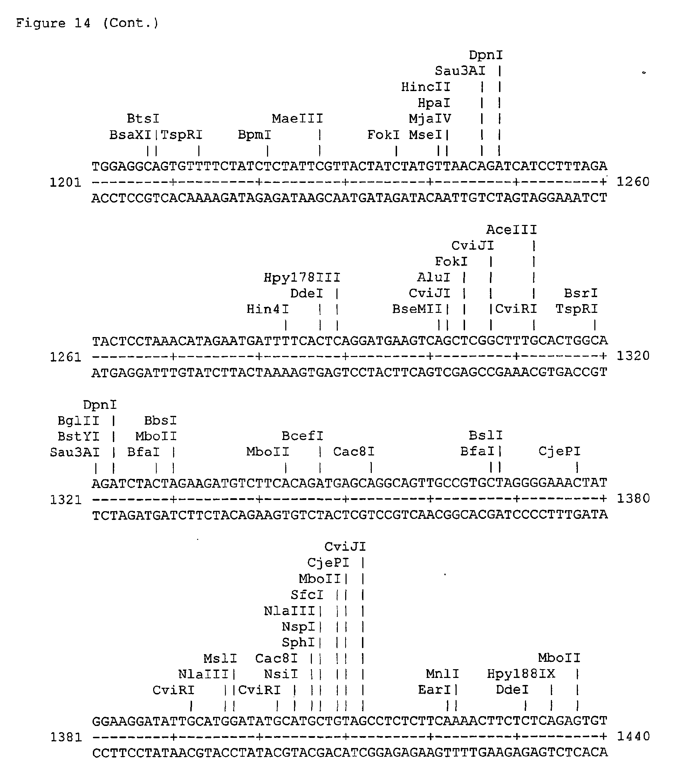

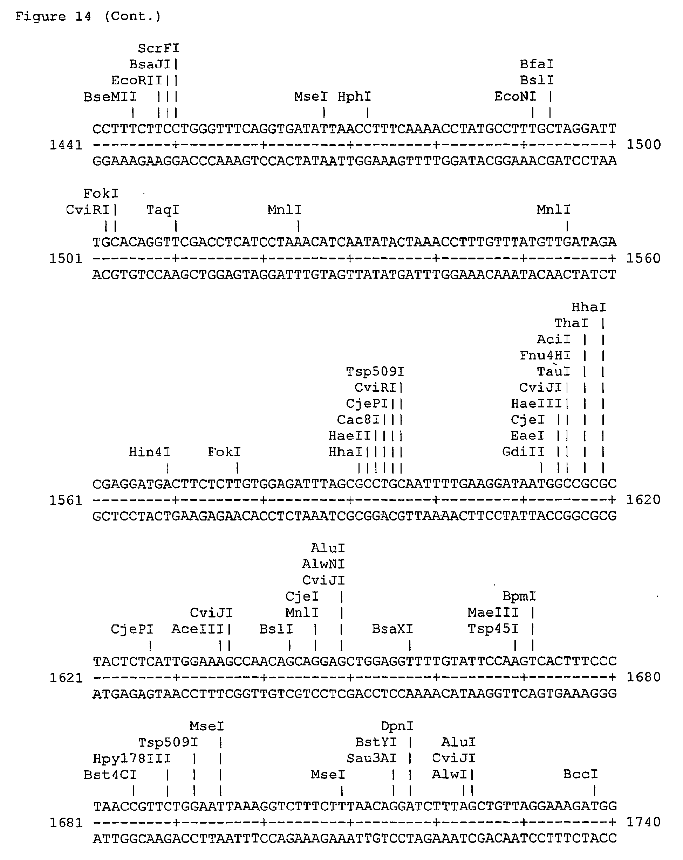

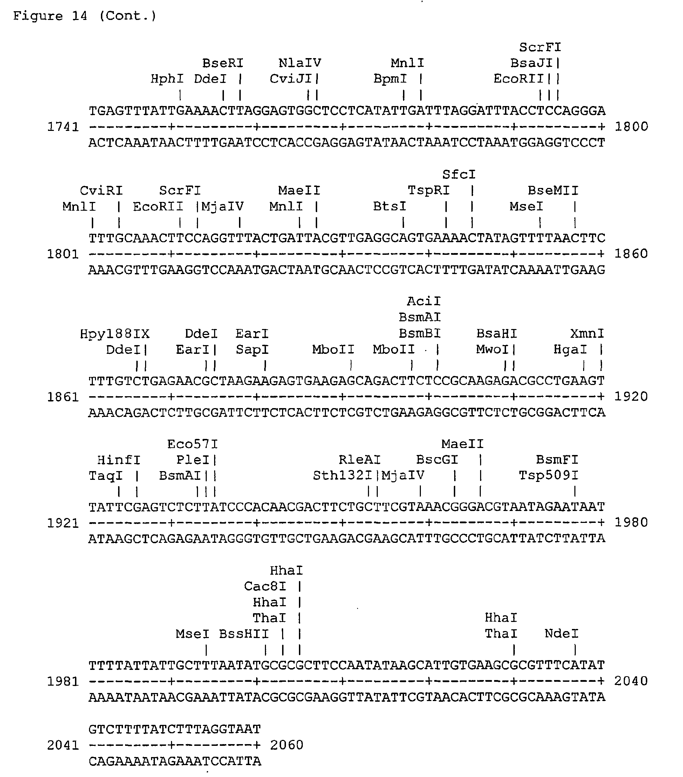

Figure 14 shows the restriction enzyme analysis of the *C. pneumoniae* gene encoding a protease.

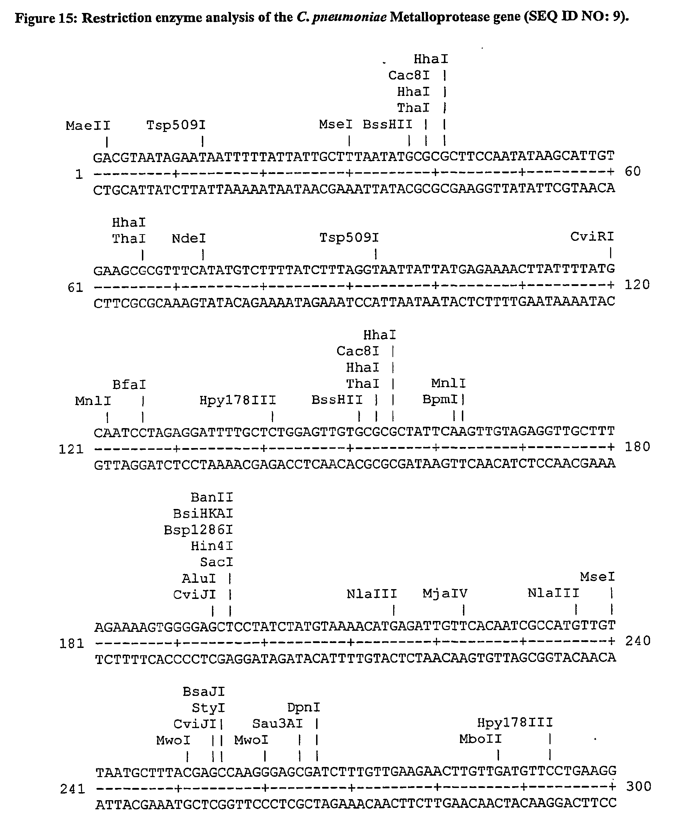

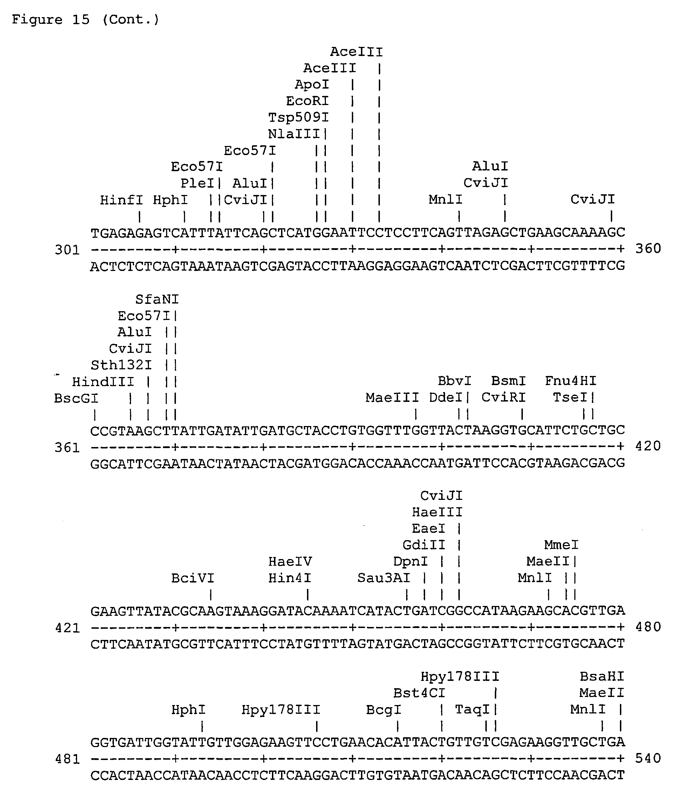

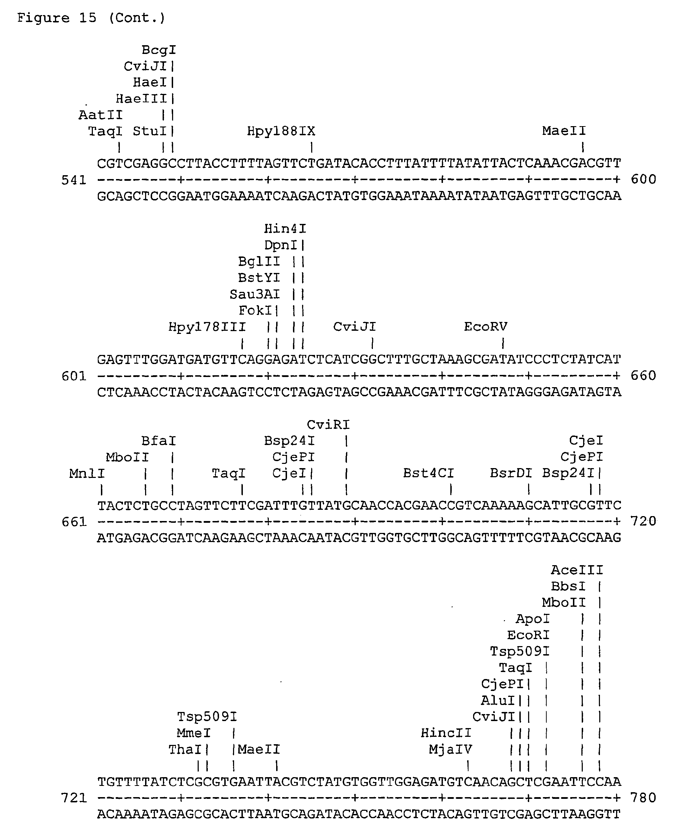

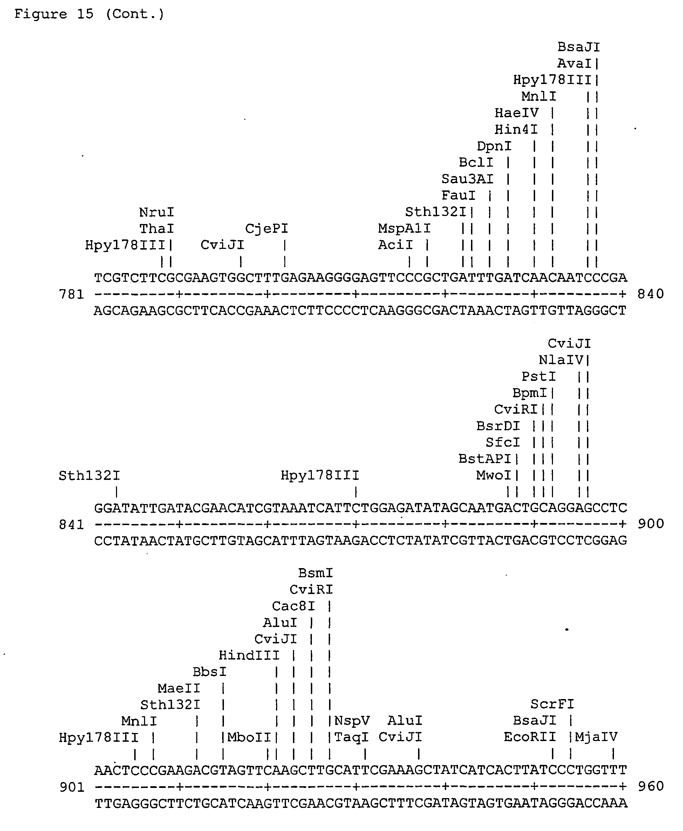

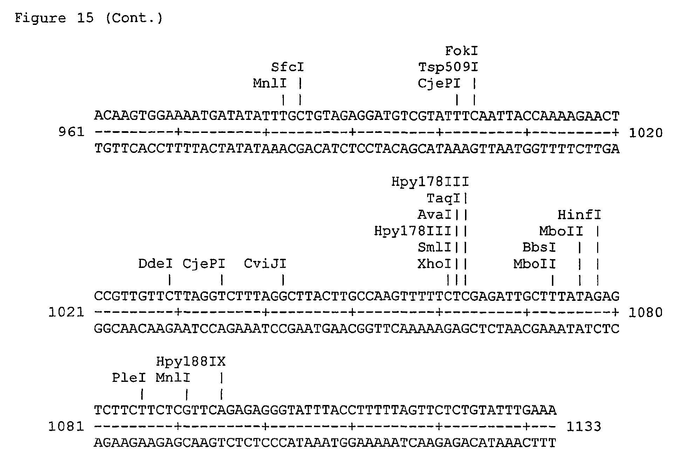

Figure 15 shows the restriction enzyme analysis of the *C. pneumoniae* gene encoding a metalloprotease.

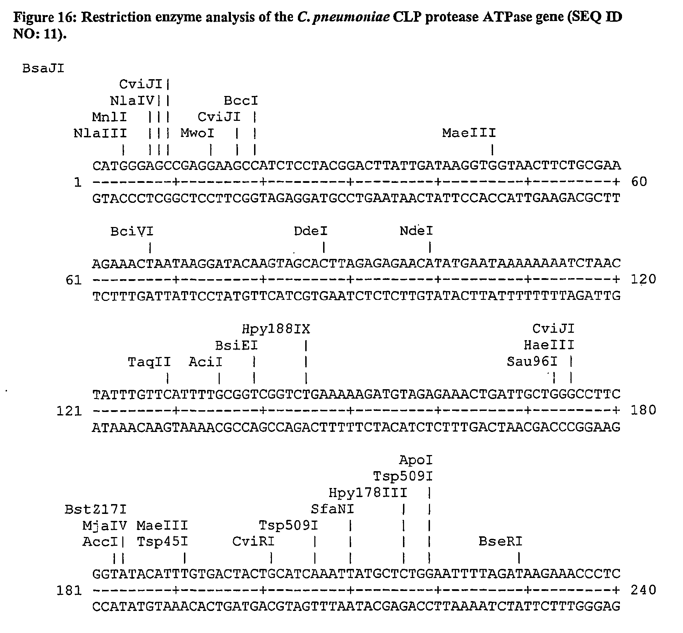

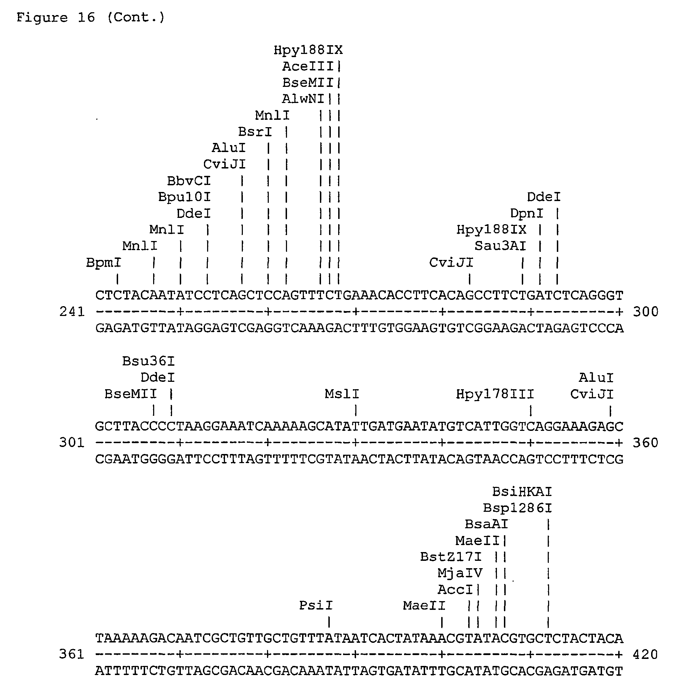

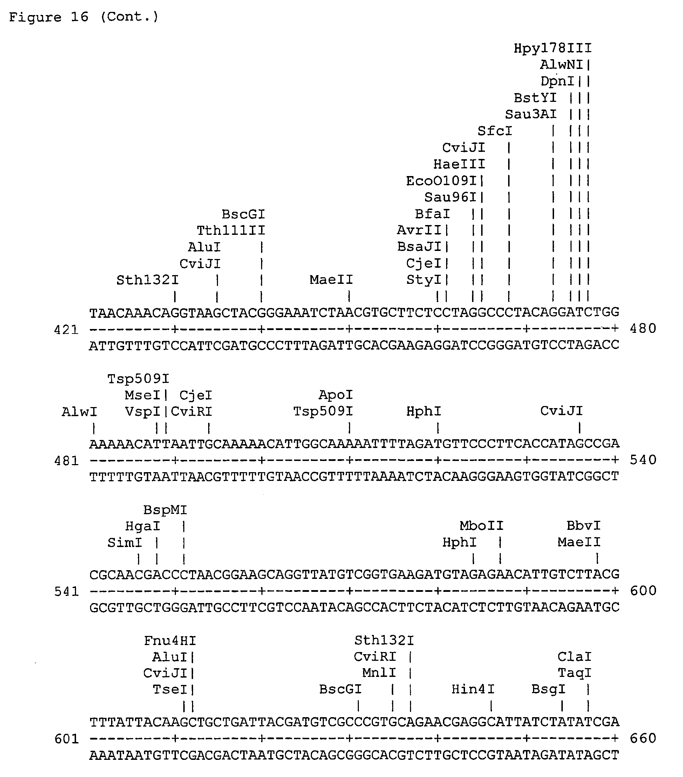

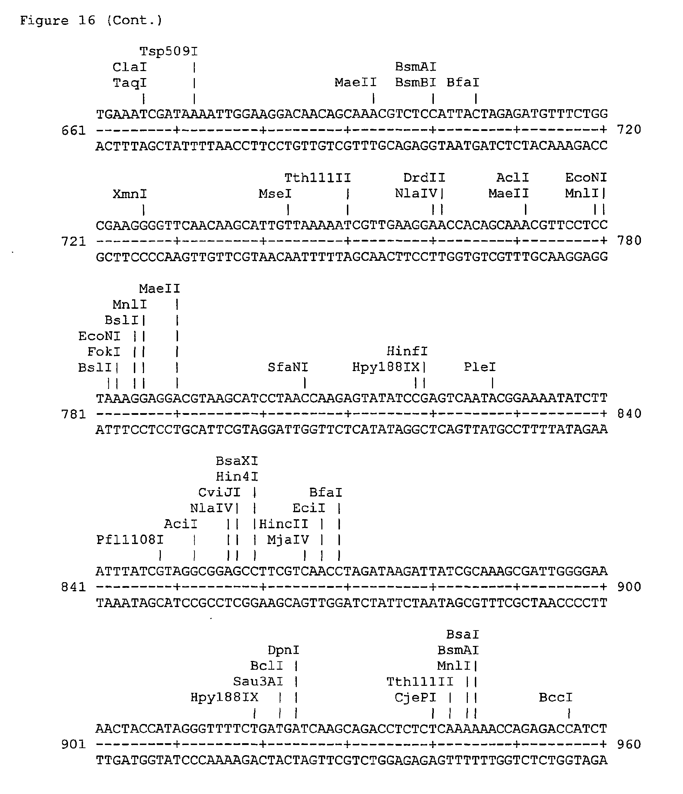

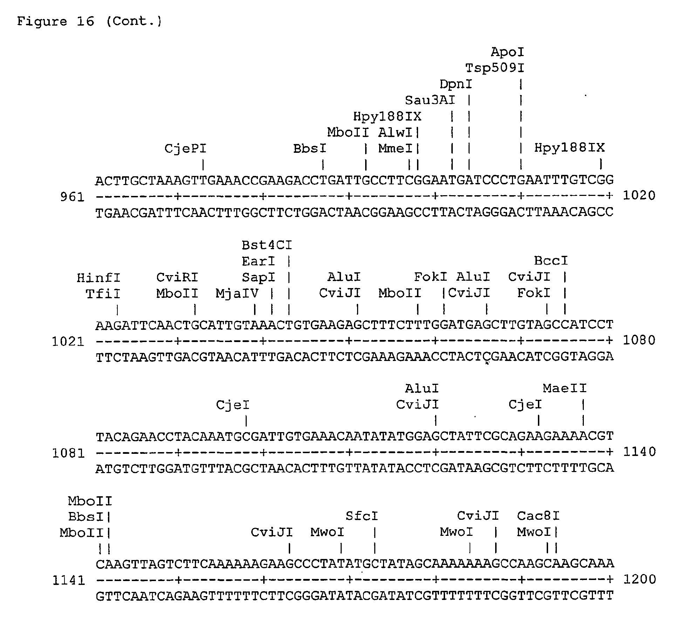

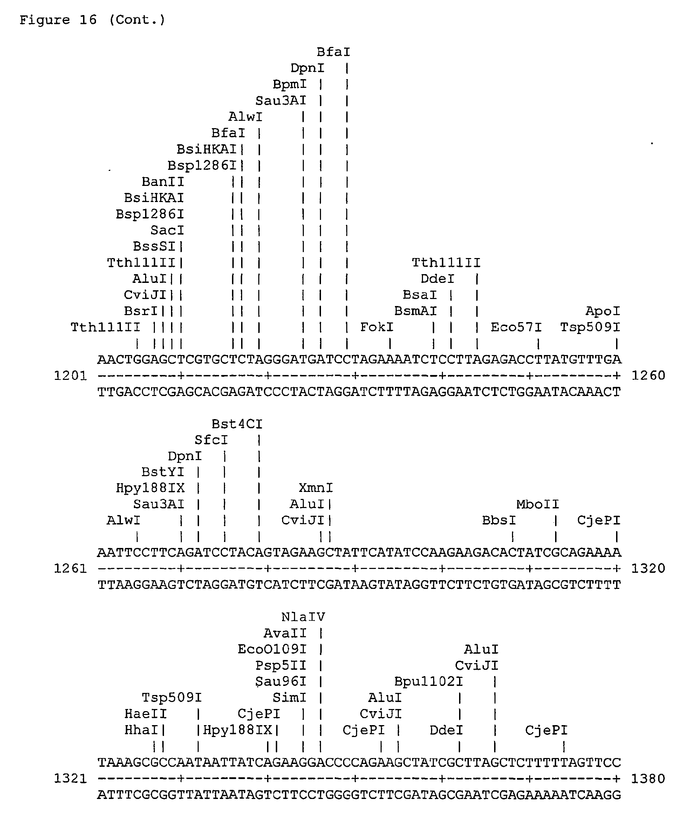

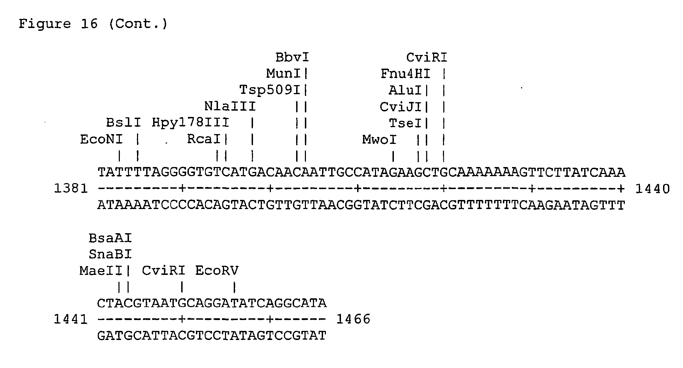

Figure 16 shows the restriction enzyme analysis of the *C. pneumoniae* gene encoding CLP protease ATPase.

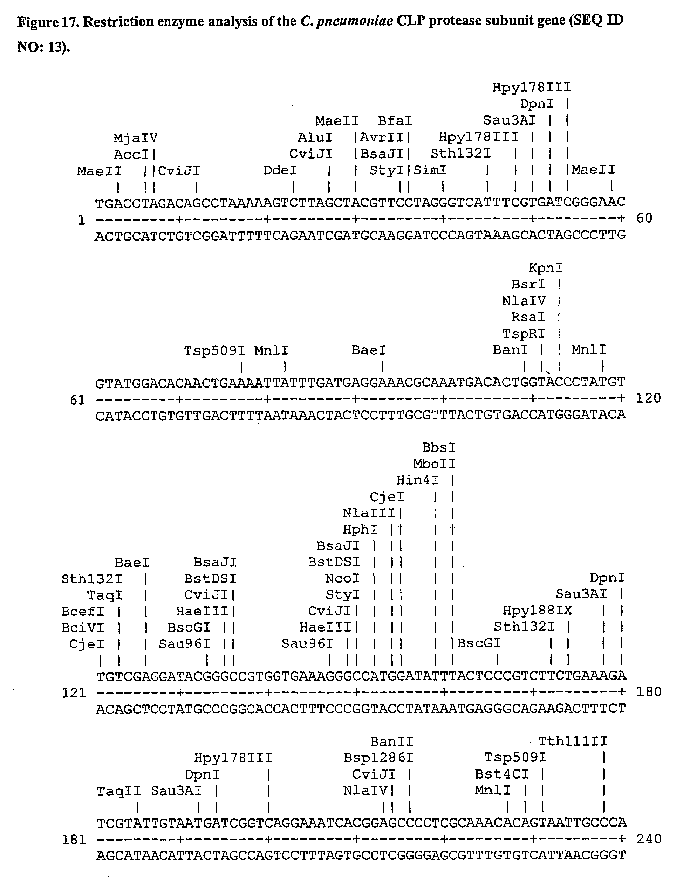

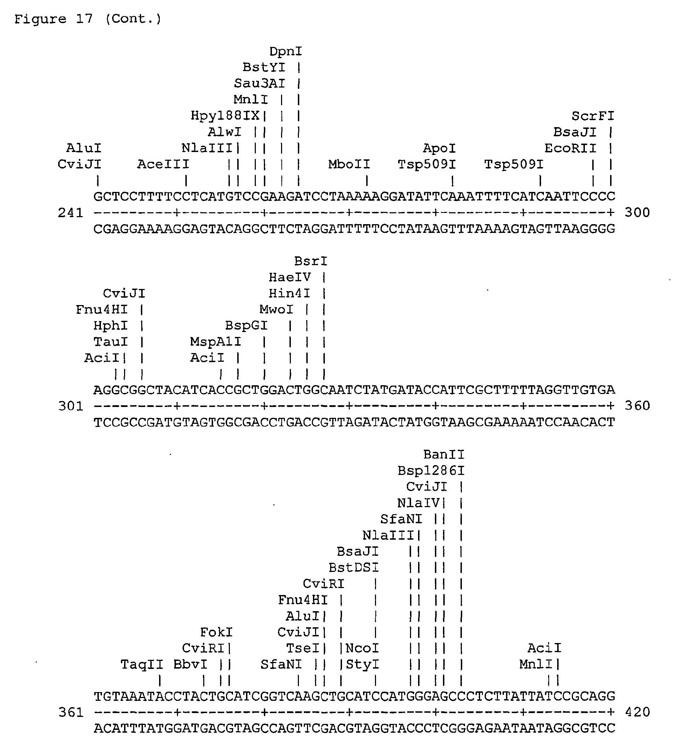

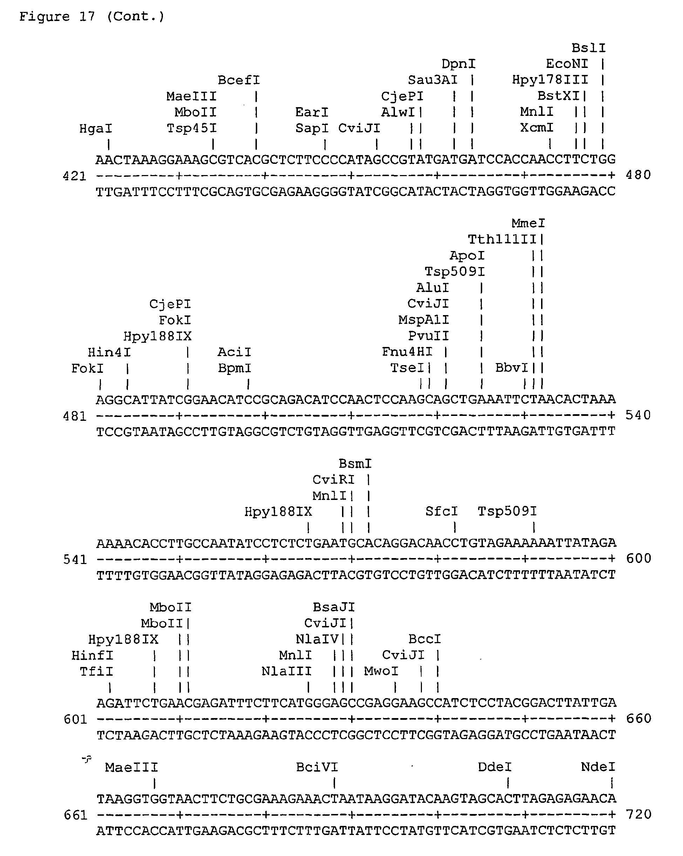

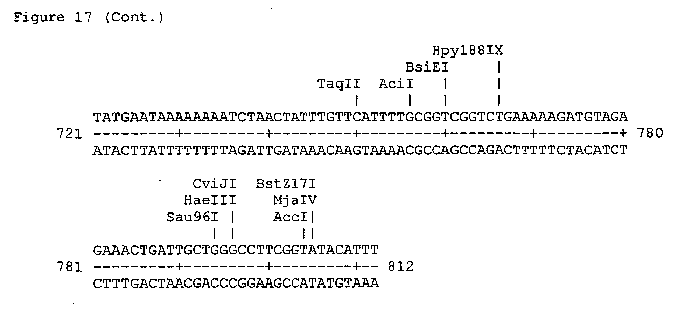

Figure 17 shows the restriction enzyme analysis of the *C. pneumoniae* gene encoding a CLP protease subunit.

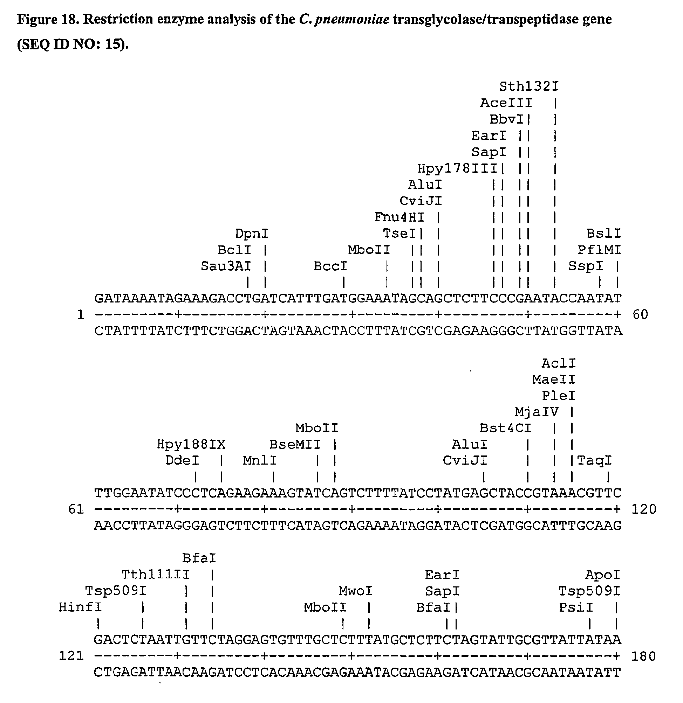

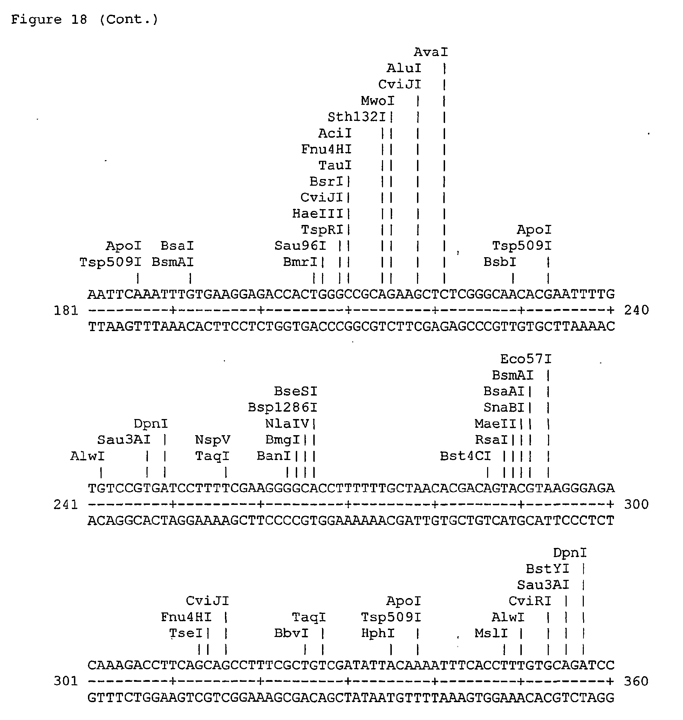

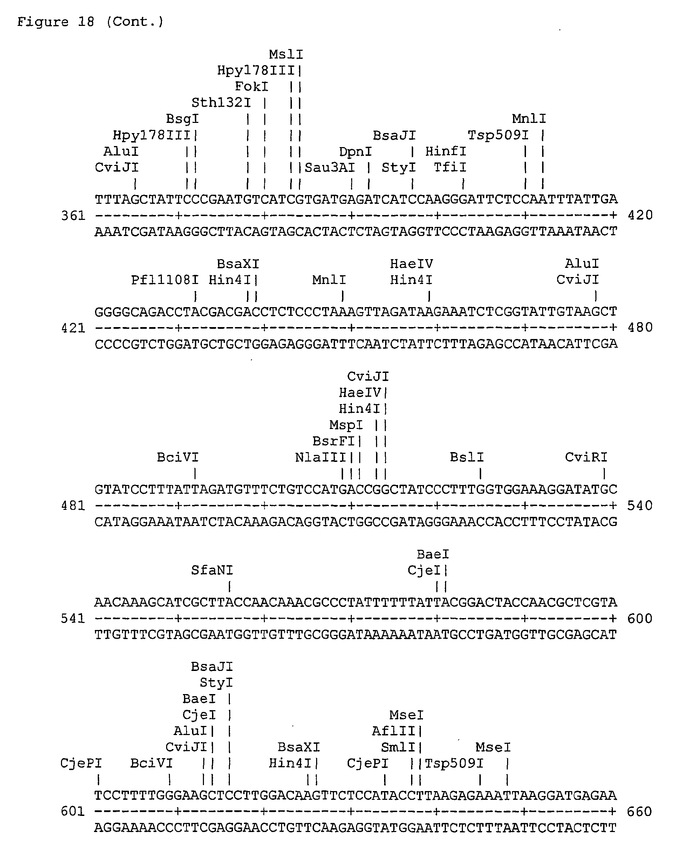

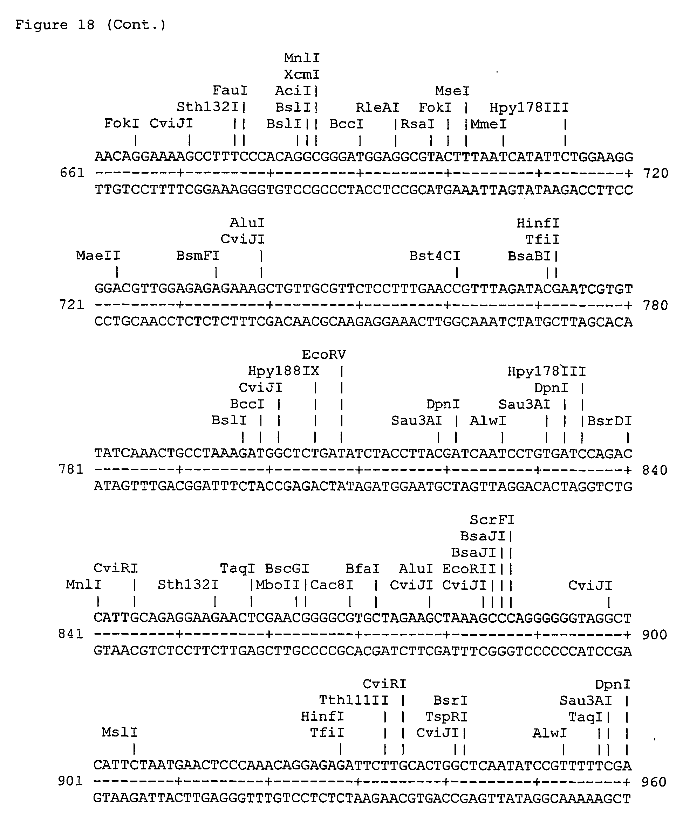

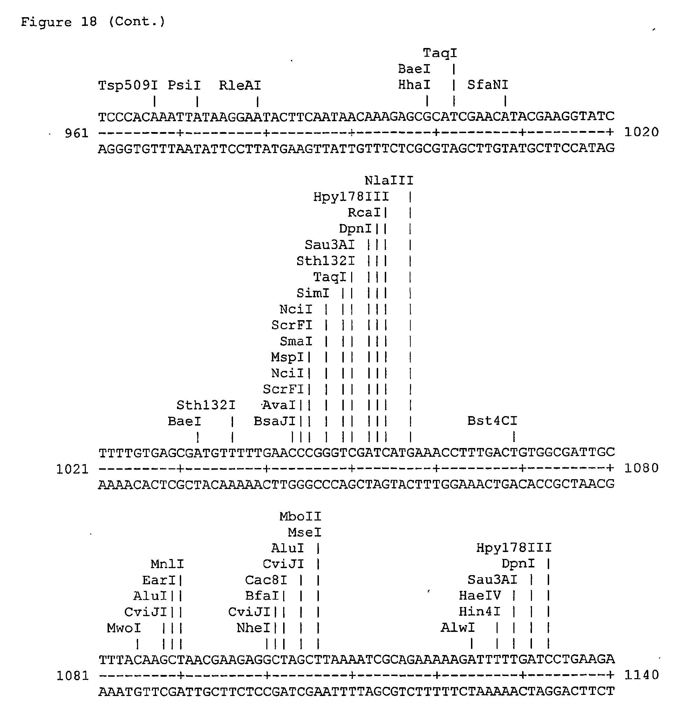

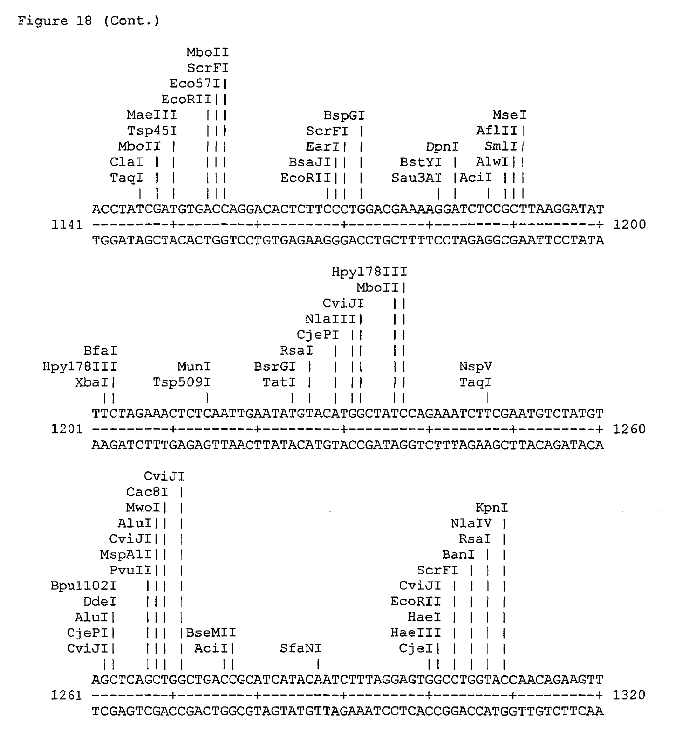

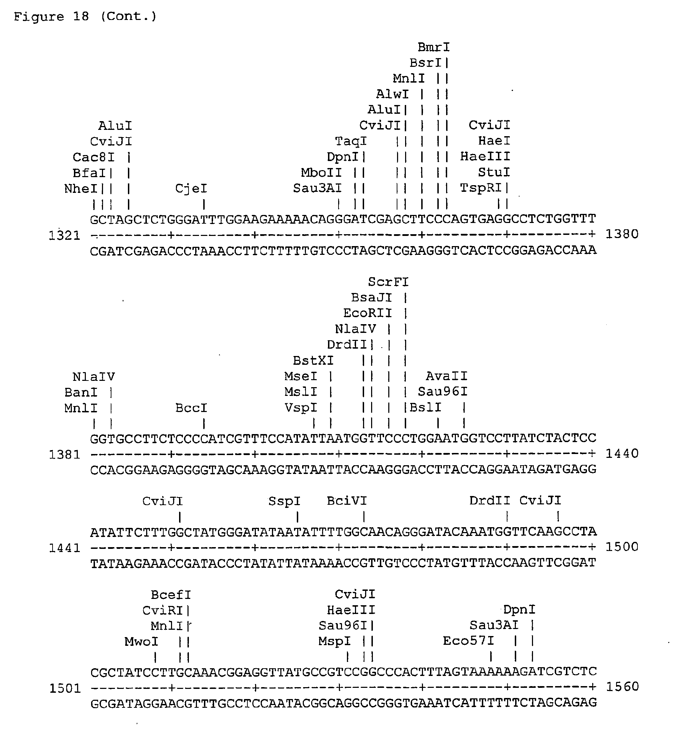

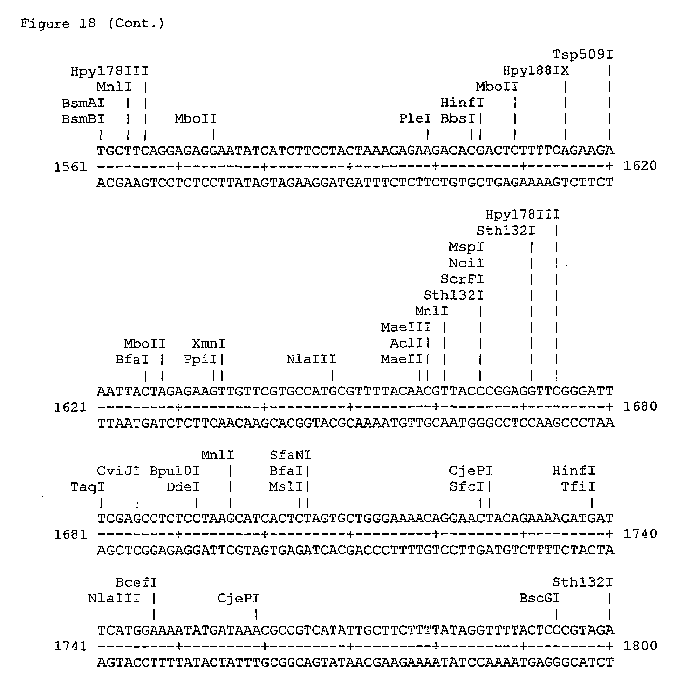

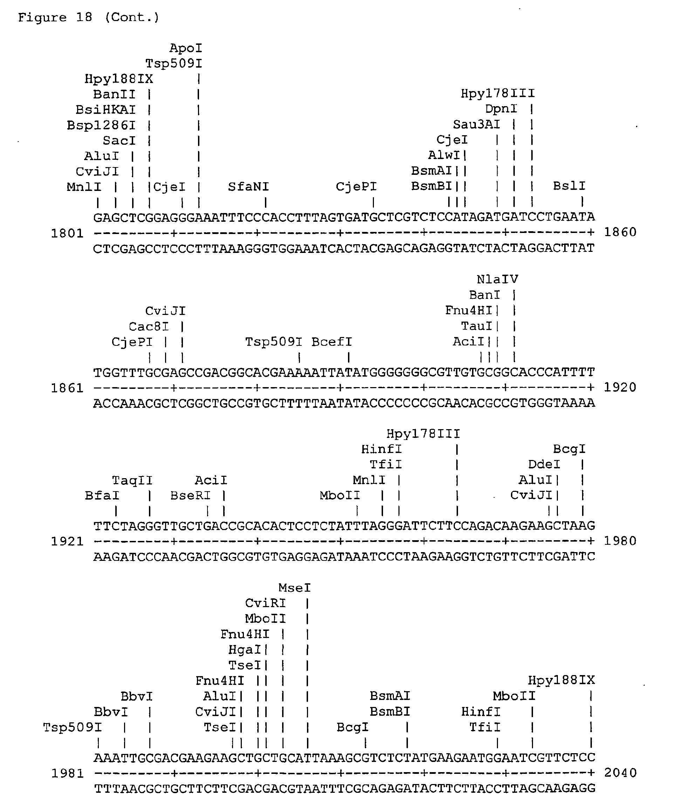

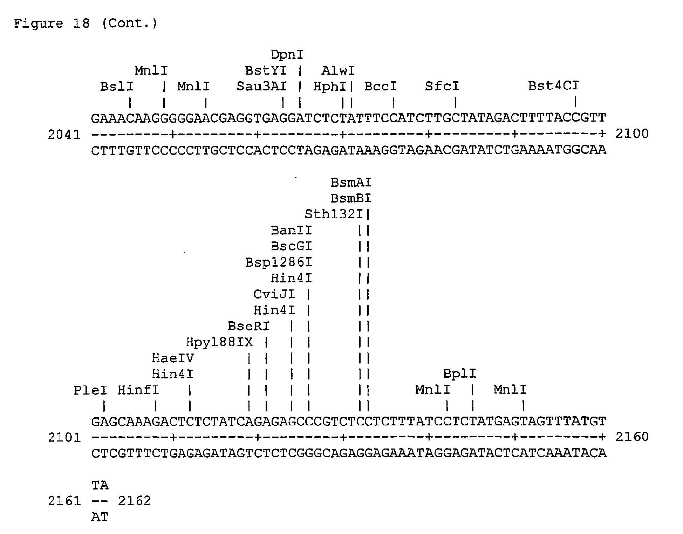

Figure 18 shows the restriction enzyme analysis of the *C. pneumoniae* gene encoding a translycolase / transpeptidase.

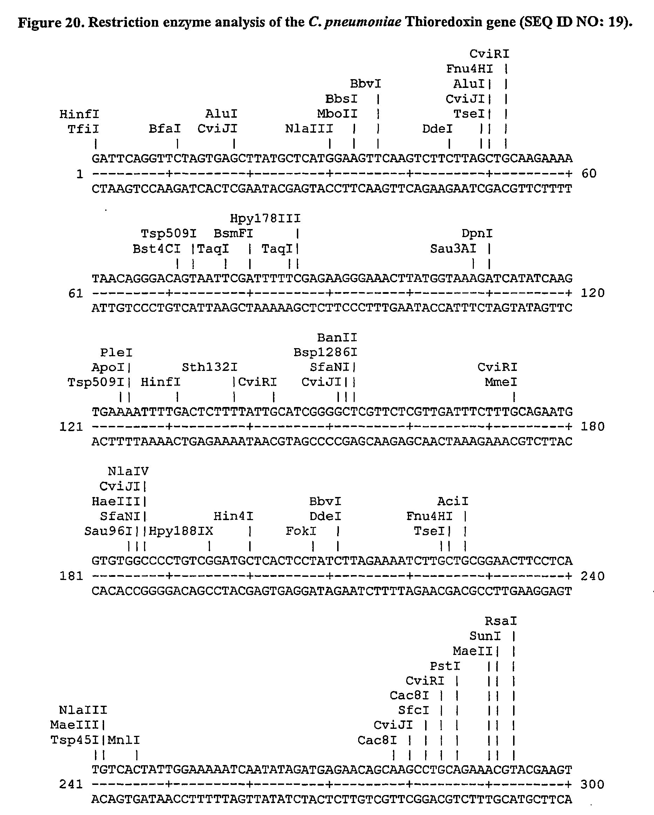

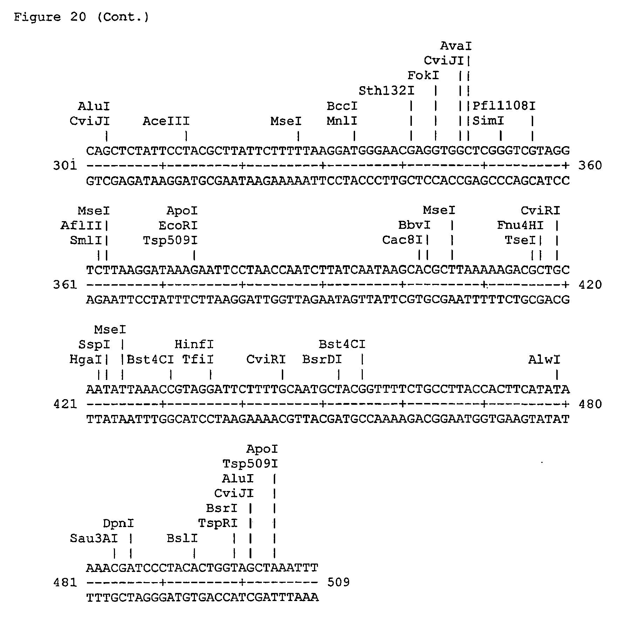

Figure 20 shows the restriction enzyme analysis of the *C. pneumoniae* gene encoding thioredoxin.

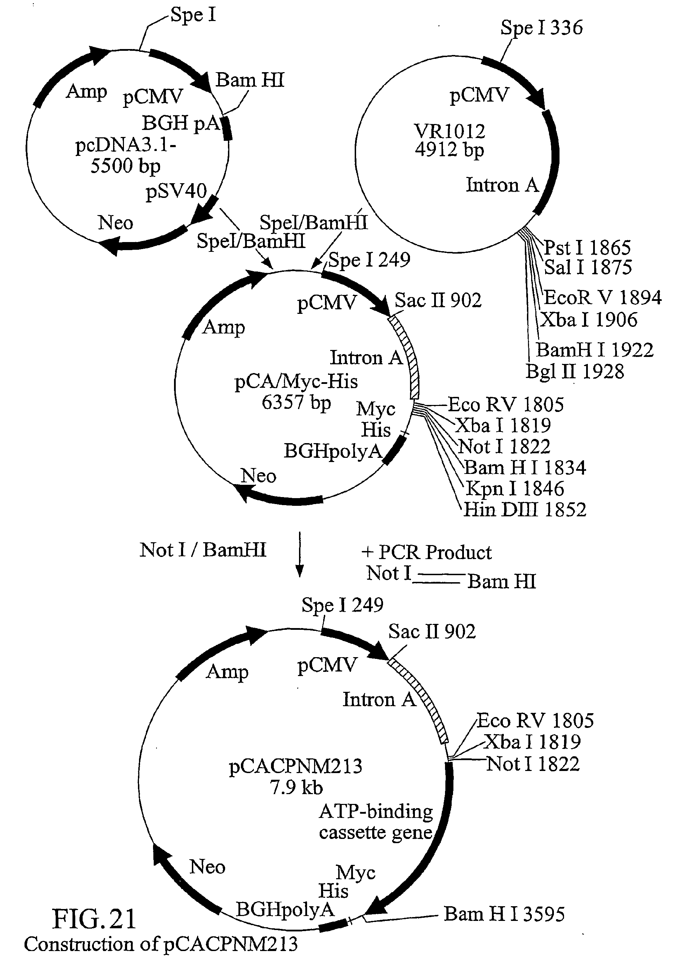

Figure 21 shows the construction and elements of plasmid pCACPNM213.

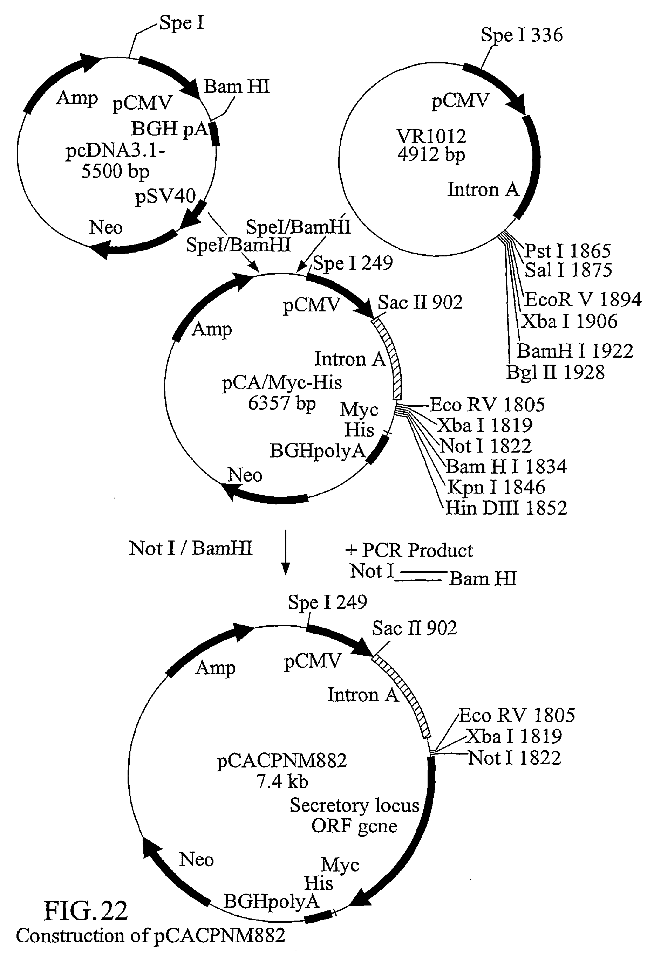

Figure 22 shows the construction and elements of plasmid pCACPNM882.

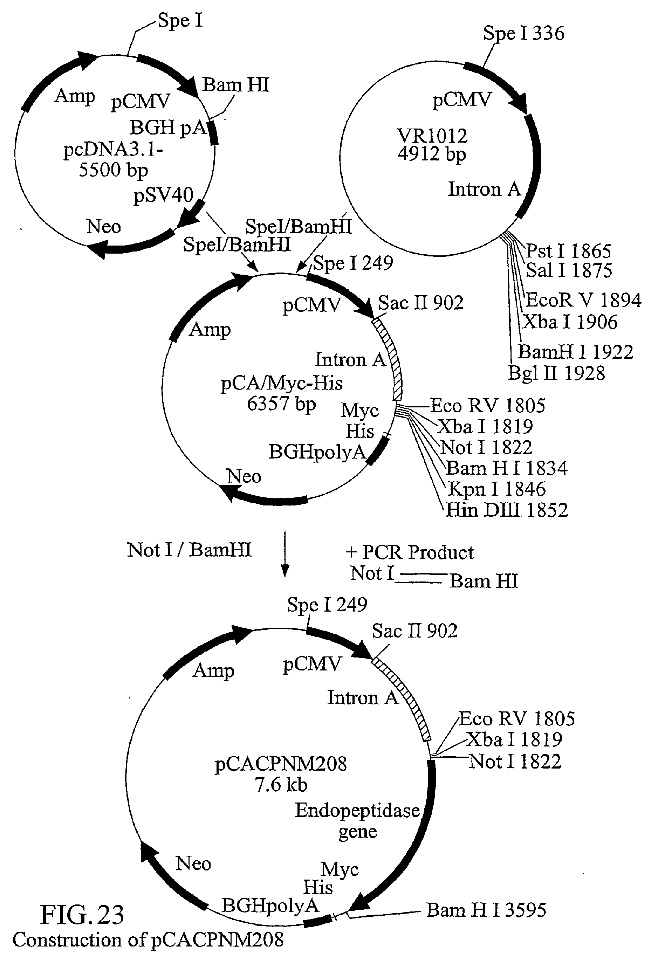

Figure 23 shows the construction and elements of plasmid pCACPNM208.

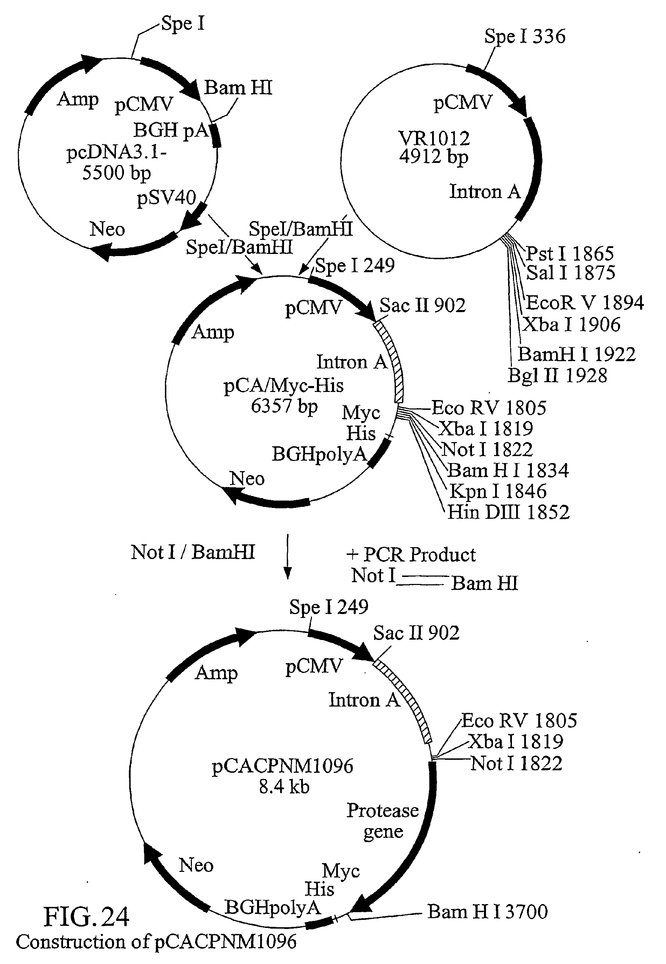

Figure 24 shows the construction and elements of plasmid pCACPNM1096.

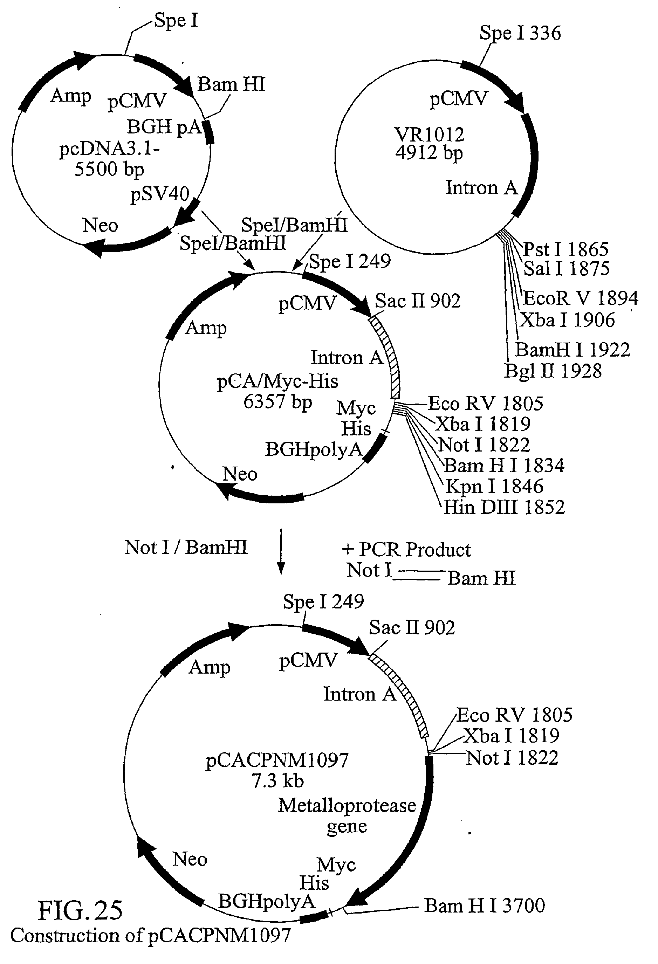

Figure 25 shows the construction and elements of plasmid pCACPNM1097.

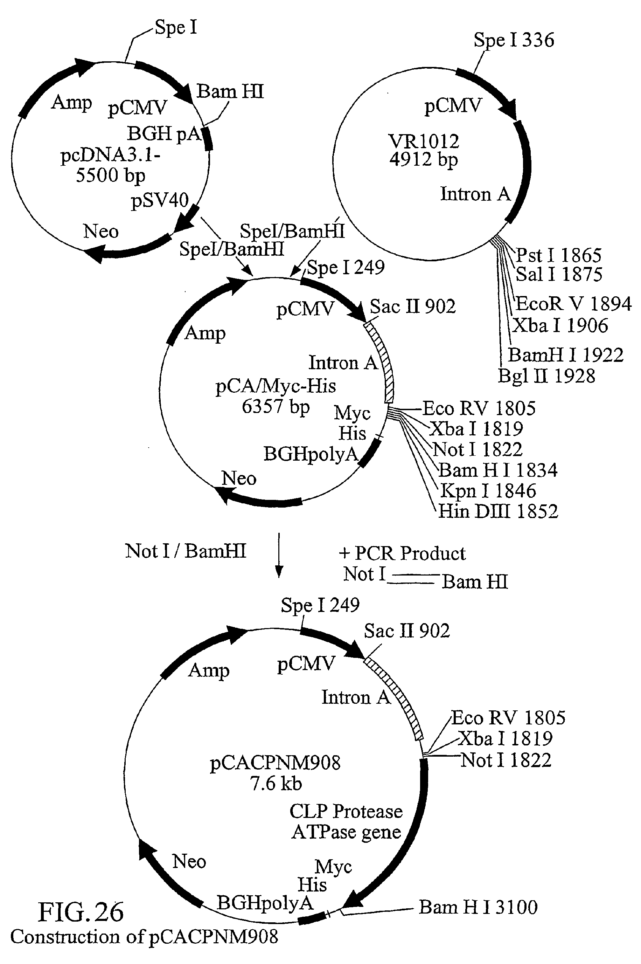

Figure 26 shows the construction and elements of plasmid pCACPNM908.

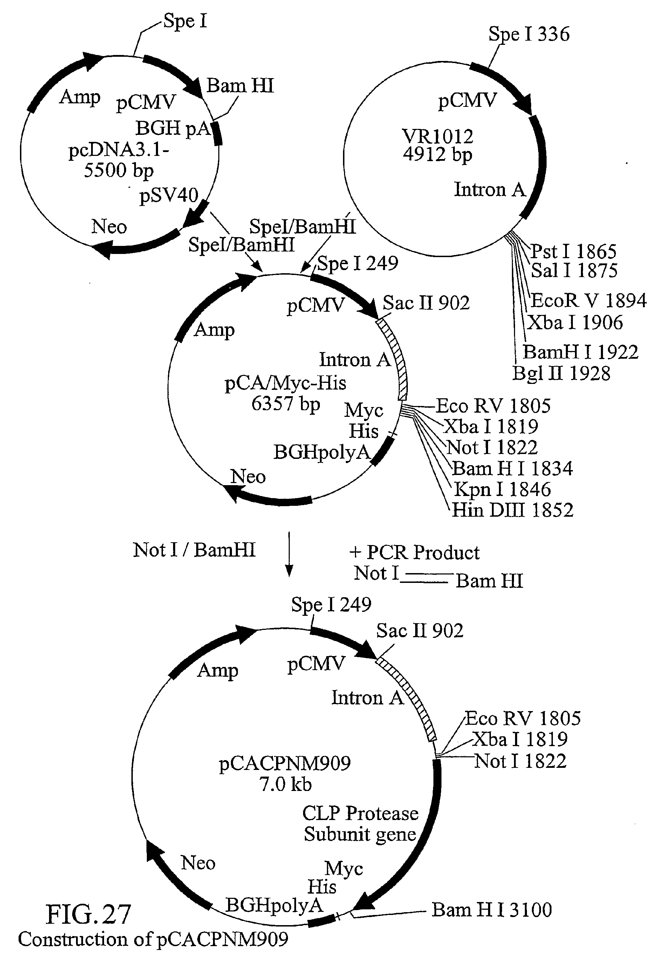

Figure 27 shows the construction and elements of plasmid pCACPNM909.

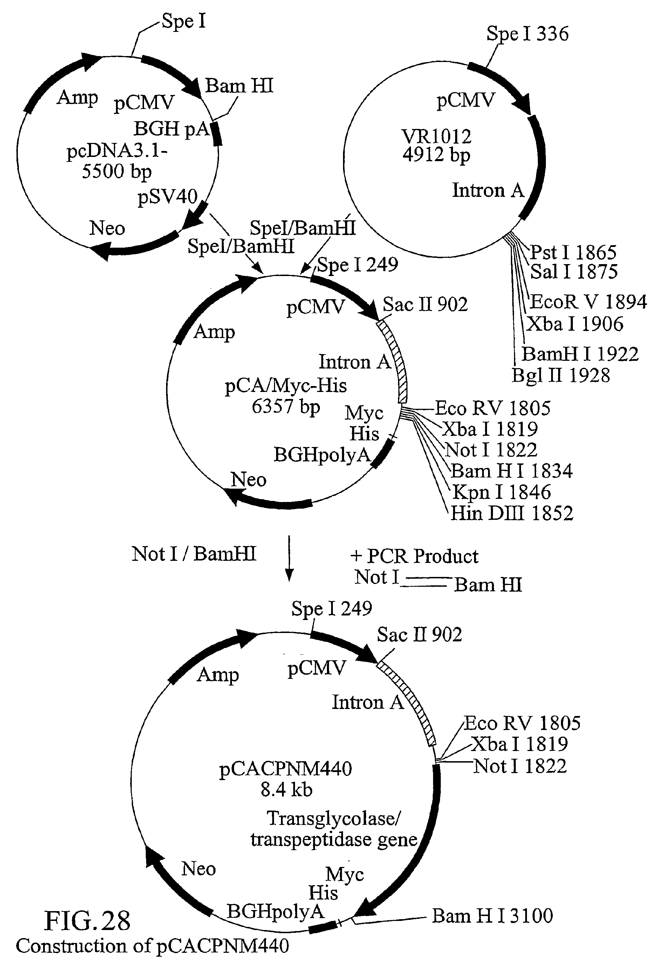

Figure 28 shows the construction and elements of plasmid pCACPNM440.

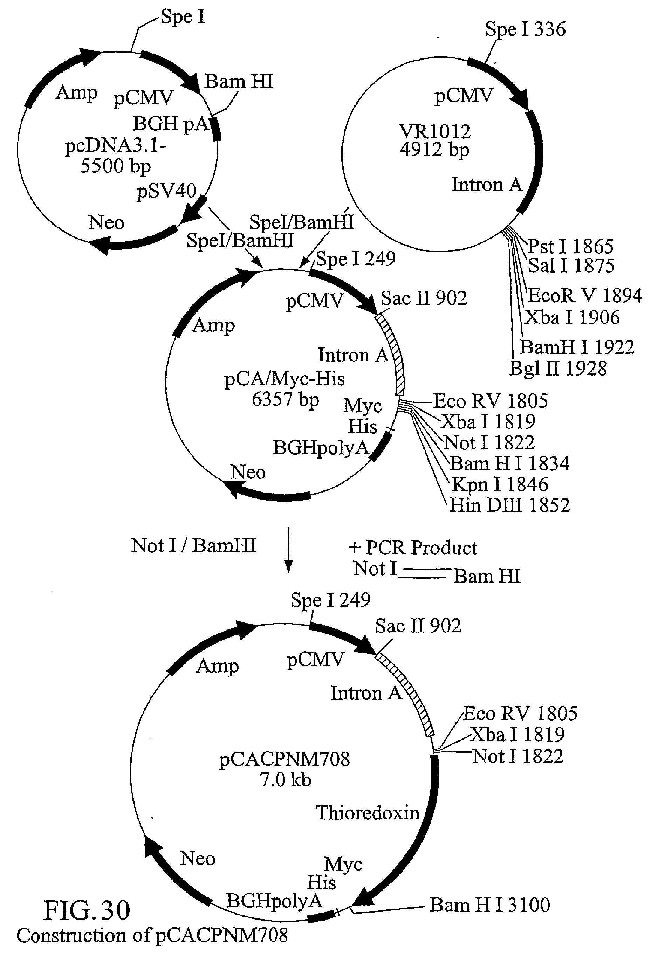

Figure 30 shows the construction and elements of plasmid pCACPNM708.

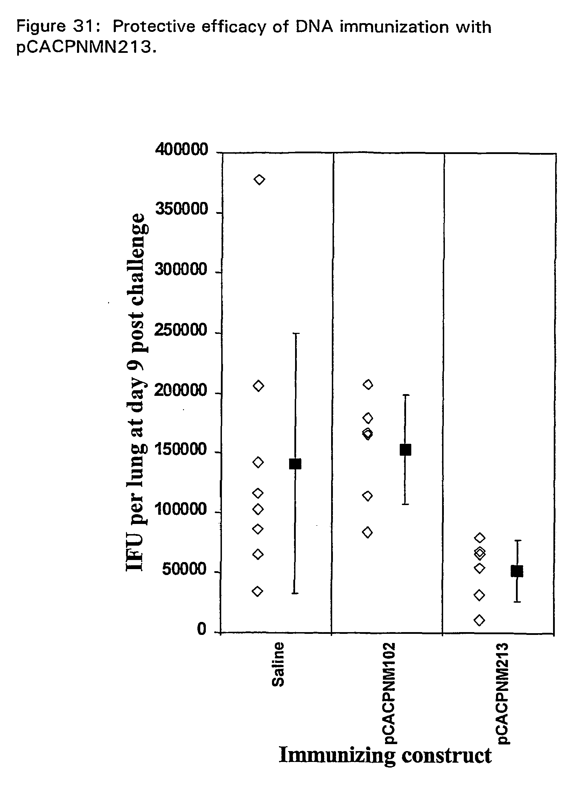

Figure 31 illustrates protection against *C. pneumoniae* infection by pCACPNM213 following DNA immunization.

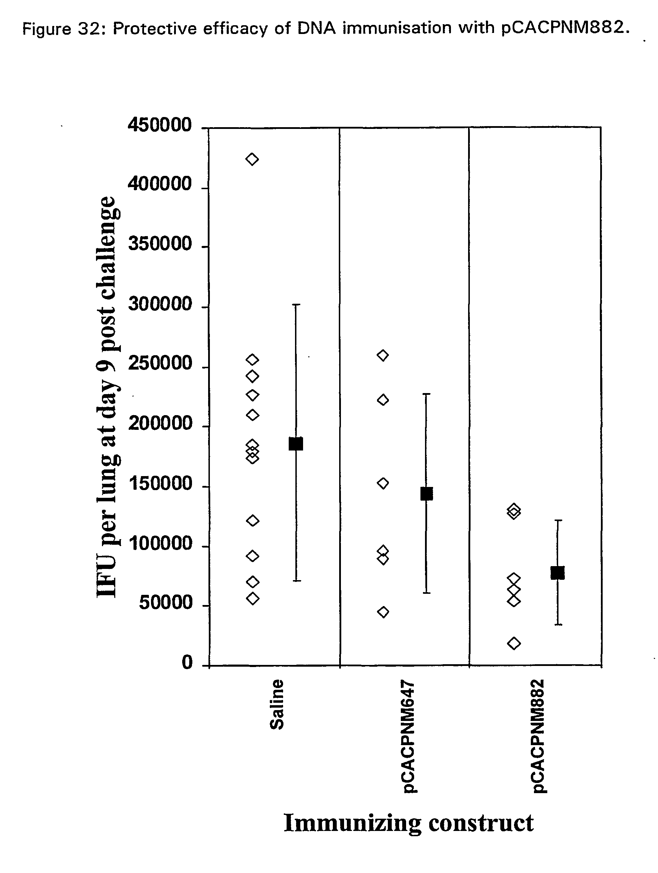

Figure 32 illustrates protection against *C. pneumoniae* infection by pCACPNM882 following DNA immunization.

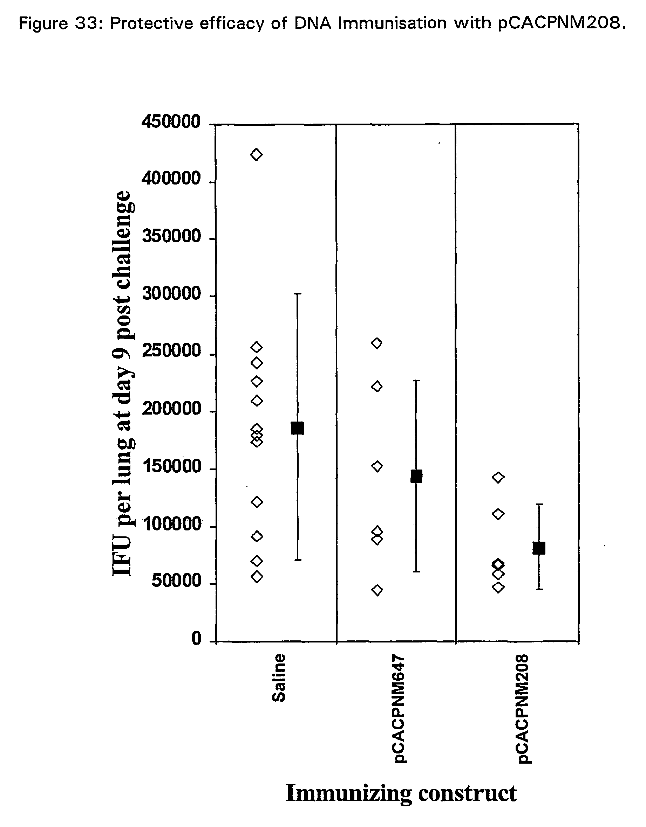

Figure 33 illustrates protection against *C. pneumoniae* infection by pCACPNM208 following DNA immunization.

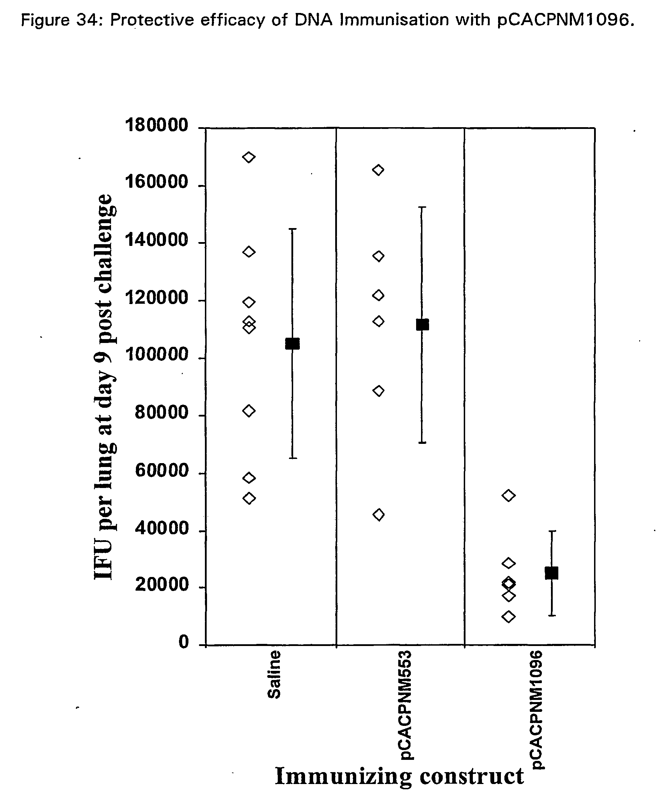

Figure 34 illustrates protection against *C. pneumoniae* infection by pCACPNM1096 following DNA immunization.

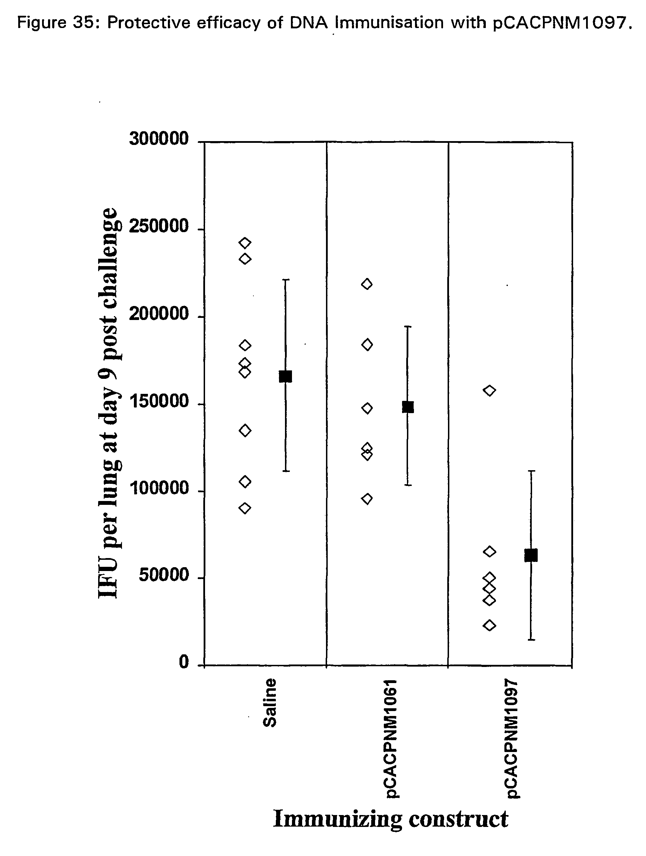

Figure 35 illustrates protection against *C. pneumoniae* infection by pCACPNM1097 following DNA immunization.

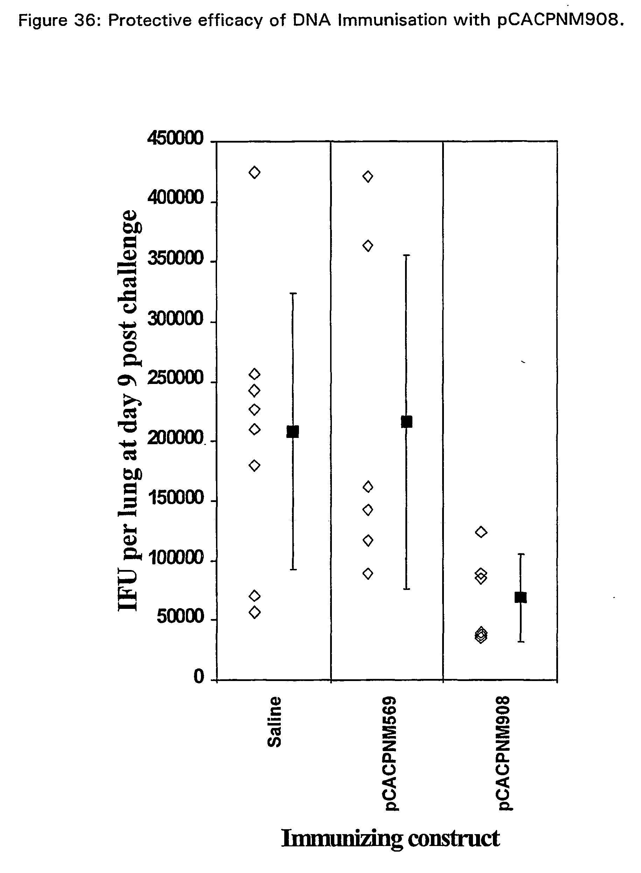

Figure 36 illustrates protection against *C. pneumoniae* infection by pCACPNM908 following DNA immunization.

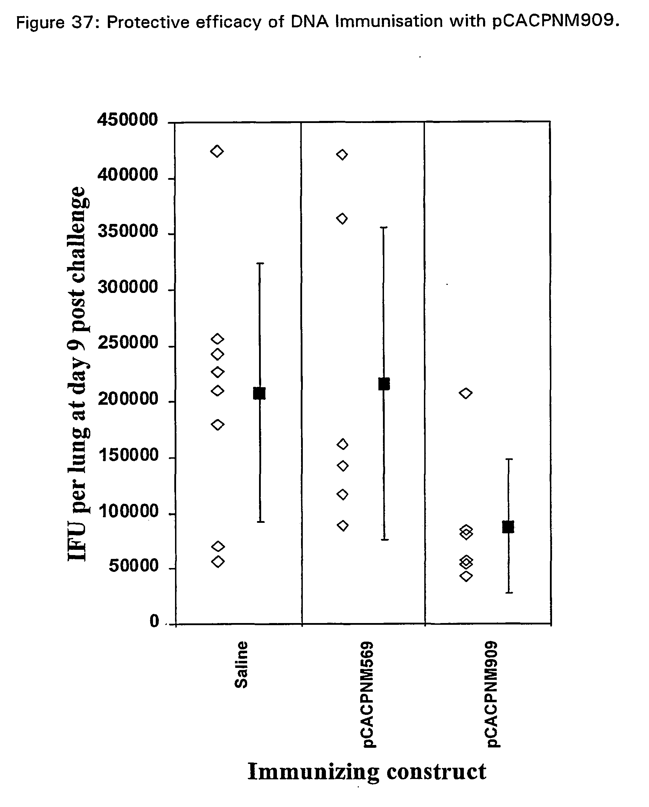

Figure 37 illustrates protection against *C. pneumoniae* infection by pCACPNM909 following DNA immunization.

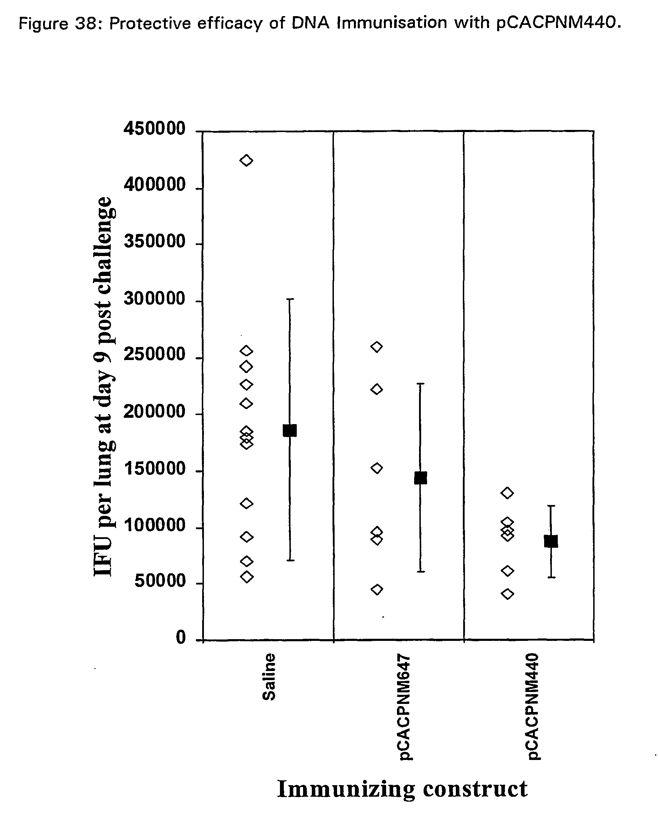

Figure 38 illustrates protection against *C. pneumoniae* infection by pCACPNM2440 following DNA immunization.

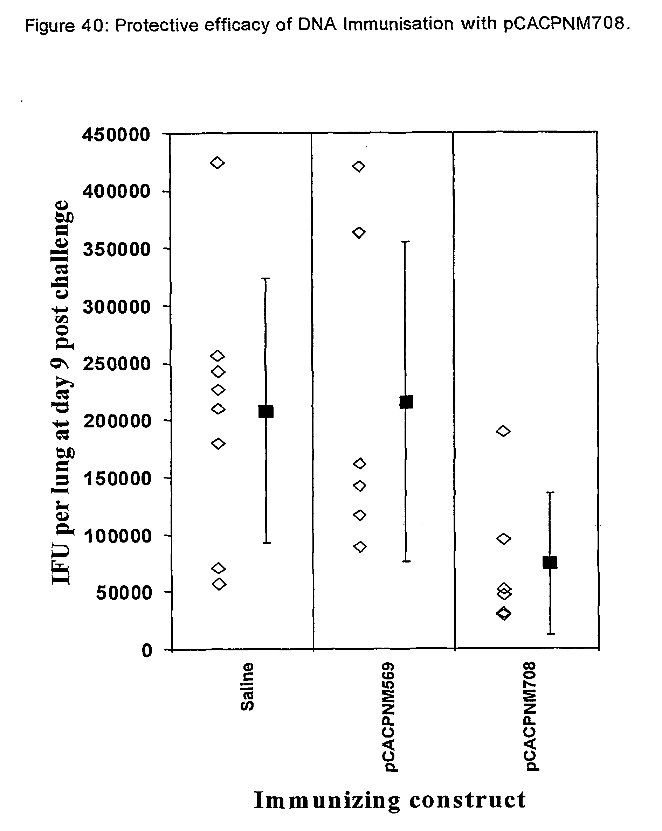

Figure 40 illustrates protection against *C. pneumoniae* infection by pCACPNM708 following DNA immunization.

### DETAILED DESCRIPTION OF INVENTION

Open reading frames (ORFs) encoding a number of Chlamydial proteins have been identified from the *C. pneumoniae* genome. These proteins include an ATP-binding cassette protein, a secretory locus ORF, an endopeptidase, a protease, a metalloprotease, CLP protease ATPase, a CLP protease subunit, a translycolase / transpeptidase, a CLPc protease and thioredoxin. The gene encoding each of these polypeptides has been inserted into an expression plasmid and shown to confer immune protection against chlamydial infection. Accordingly, any one of these and related polypeptides can be used to prevent and treat *Chlamydia* infection.

According to a first aspect disclosed in the application, isolated polynucleotides are described which encode *Chlamydia* polypeptides, whose amino acid sequences are shown in SEQ ID No: 2, 4, 6 ,8, 10, 12, 14, 16, 18 and 20.

The term "isolated polynucleotide" is defined as a polynucleotide removed from the environment in which it naturally occurs. For example, a naturally-occurring DNA molecule present in the genome of a living bacteria or as part of a gene bank is not isolated, but the same molecule separated from the remaining part of the bacterial genome, as a result of, *e.g.*, a cloning event (amplification), is isolated. Typically, an isolated DNA molecule is free from DNA regions (*e.g.*, coding regions) with which it is immediately contiguous at the 5' or 3' end, in the naturally occurring genome. Such isolated polynucleotides may be part of a vector or a composition and still be defined as isolated in that such a vector or composition is not part of the natural environment of such polynucleotide.

The polynucleotide disclosed in the application is either RNA or DNA (cDNA, genomic DNA, or synthetic DNA), or modifications, variants, homologs or fragments thereof. The DNA is either double-stranded or single-stranded, and, if single-stranded, is either the coding strand or the non-coding (anti-sense) strand. Any one of the sequences that encode the polypeptides disclosed in the application as shown in any one of SEQ ID Nos: 2, 4, 6 ,8, 10, 12, 14, 16, 18 and 20.is (a) a coding sequence, (b) a ribonucleotide sequence derived from transcription of (a), or (c) a coding sequence which uses the redundancy or degeneracy of the genetic code to encode the same polypeptides. By "polypeptide" or "protein" is meant any chain of amino acids, regardless of length or post-translational modification (*e.g.,* glycosylation or phosphorylation). Both terms are used interchangeably in the present application.

Consistent with an aspect disclosed in the application, amino acid sequences are provided which are homologous to any one of SEQ ID Nos: 2, 4, 6 ,8, 10, 12, 14, 16, 18 and 20. As used herein, "homologous amino acid sequence" is any polypeptide which is encoded, in whole or in part, by a nucleic acid sequence which hybridizes at 25-35°C below critical melting temperature (Tm), to any portion of the nucleic acid sequence of any one of SEQ ID Nos: 1, 3, 5, 7, 9, 11, 13, 15, 17 and 19. A homologous amino acid sequence is one that differs from an amino acid sequence shown in any one of SEQ ID Nos: 2, 4, 6 ,8, 10, 12, 14, 16, 18 and 20 by one or more conservative amino acid substitutions. Such a sequence also encompass serotypic variants (defined below) as well as sequences containing deletions or insertions which retain inherent characteristics of the polypeptide such as immunogenicity. Preferably, such a sequence is at least 75%, preferably at least 78%, more preferably at least 80%, even more preferably at least 85%, 88% or 90%, and most preferably at least 93%, 95% or 98% identical to any one of SEQ ID Nos: 2, 4, 6 8, 10, 12, 14, 16, 18 and 20.

Homologous amino acid sequences include sequences that are identical or substantially identical to any one of SEQ ID Nos: 2, 4, 6 ,8, 10, 12, 14, 16, 18 and 20. By "amino acid sequence substantially identical" is meant a sequence that is at least 90%, preferably 95%, more preferably 97%, and most preferably 99% identical to an amino acid sequence of reference and that preferably differs from the sequence of reference by a majority of conservative amino acid substitutions.

Conservative amino acid substitutions are substitutions among amino acids of the same class. These classes include, for example, amino acids having uncharged polar side chains, such as asparagine, glutamine, serine, threonine, and tyrosine; amino acids having basic side chains, such as lysine, arginine, and histidine; amino acids having acidic side chains, such as aspartic acid and glutamic acid; and amino acids having nonpolar side chains, such as glycine, alanine, valine, leucine, isoleucine, proline, phenylalanine, methionine, tryptophan, and cysteine.

Homology is measured using sequence analysis software such as Sequence Analysis Software Package of the Genetics Computer Group, University of Wisconsin Biotechnology Center, 1710 University Avenue, Madison, WI 53705. Amino acid sequences are aligned to maximize identity. Gaps may be artificially introduced into the sequence to attain proper alignment. Once the optimal alignment has been set up, the degree of homology is established by recording all of the positions in which the amino acids of both sequences are identical, relative to the total number of positions.

Homologous polynucleotide sequences are defined in a similar way. Preferably, a homologous sequence is one that is at least 45%, more preferably 50%, 55%, 60%, 65%, 70%, 75%, 80%, and even more preferably 85%, 87%, 90%, 93%, 96% and most preferably 99% identical to the coding sequence of any one of SEQ ID Nos: 1, 3, 5, 7, 9, 11, 13, 15, 17 and 19.

Consistent with an aspect disclosed in the application, polypeptides having a sequence homologous to any one of SEQ ID Nos: 2, 4, 6, 8, 10, 12, 14, 16, 18 and 20 include naturally-occurring allelic variants, as well as mutants or any other non-naturally occurring variants that retain the inherent characteristics of the polypeptide of any one of SEQ ID Nos: 2, 4, 6, 8, 10, 12, 14, 16, 18 and 20.

As is known in the art, an allelic variant is an alternate form of a polypeptide that is characterized as having a substitution, deletion, or addition of one or more amino acids that does not alter the biological function of the polypeptide. By "biological function" is meant the function of the polypeptide in the cells in which it naturally occurs, even if the function is not necessary for the growth or survival of the cells. For example, the biological function of a porin is to allow the entry into cells of compounds present in the extracellular medium. Biological function is distinct from antigenic property. A polypeptide can have more than one biological function.

Allelic variants are very common in nature. For example, a bacterial species such as *C. pneumoniae,* is usually represented by a variety of strains that differ from each other by minor allelic variations. Indeed, a polypeptide that fulfills the same biological function in different strains can have an amino acid sequence (and polynucleotide sequence) that is not identical in each of the strains. Despite this variation, an immune response directed generally against many allelic variants has been demonstrated. In studies of the *Chlamydial* MOMP antigen, cross-strain antibody binding plus neutralization of infectivity occurs despite amino acid sequence variation of MOMP from strain to strain, indicating that the MOMP, when used as an immunogen, is tolerant of amino acid variations.

Polynucleotides encoding homologous polypeptides or allelic variants are retrieved by polymerase chain reaction (PCR) amplification of genomic bacterial DNA extracted by conventional methods. This involves the use of synthetic oligonucleotide primers matching upstream and downstream of the 5' and 3' ends of the encoding domain. Suitable primers are designed according to the nucleotide sequence information provided in any one of SEQ ID Nos: 1, 3, 5, 7, 9, 11, 13, 15, 17 and 19. The procedure is as follows: a primer is selected which consists of 10 to 40, preferably 15 to 25 nucleotides. It is advantageous to select primers containing C and G nucleotides in a proportion sufficient to ensure efficient hybridization; *i.e.*, an amount of C and G nucleotides of at least 40%, preferably 50% of the total nucleotide content. A standard PCR reaction contains typically 0.5 to 5 Units of Taq DNA polymerase per 100 µL, 20 to 200 µM deoxynucleotide each, preferably at equivalent concentrations, 0.5 to 2.5 mM magnesium over the total deoxynucleotide concentration, 10⁵ to 10⁶ target molecules, and about 20 pmol of each primer. About 25 to 50 PCR cycles are performed, with an annealing temperature 15°C to 5°C below the true Tm of the primers. A more stringent annealing temperature improves discrimination against incorrectly annealed primers and reduces incorportion of incorrect nucleotides at the 3' end of primers. A denaturation temperature of 95°C to 97°C is typical, although higher temperatures may be appropriate for dematuration of G+C-rich targets. The number of cycles performed depends on the starting concentration of target molecules, though typically more than 40 cycles is not recommended as non-specific background products tend to accumulate.

An alternative method for retrieving polynucleotides encoding homologous polypeptides or allelic variants is by hybridization screening of a DNA or RNA library. Hybridization procedures are well-known in the art and are described in Ausubel *et al.,* (Ausubel *et al.,* Current Protocols in Molecular Biology, John Wiley & Sons Inc., 1994), Silhavy *et al.* (Silhavy *et al.* Experiments with Gene Fusions, Cold Spring Harbor Laboratory Press, 1984), and Davis *et al.* (Davis *et al.* A Manual for Genetic Engineering: Advanced Bacterial Genetics, Cold Spring Harbor Laboratory Press, 1980)). Important parameters for optimizing hybridization conditions are reflected in a formula used to obtain the critical melting temperature above which two complementary DNA strands separate from each other (Casey & Davidson, Nucl. Acid Res. (1977) 4:1539). For polynucleotides of about 600 nucleotides or larger, this formula is as follows: Tm = 81.5 + 0.41 x (% G+C) + 16.6 log (cation ion concentration) - 0.63 x (% formamide) - 600/base number. Under appropriate stringency conditions, hybridization temperature (Th) is approximately 20 to 40°C, 20 to 25°C, or, preferably 30 to 40°C below the calculated Tm. Those skilled in the art will understand that optimal temperature and salt conditions can be readily determined.

For the polynucleotides disclosed in the application, stringent conditions are achieved for both pre-hybridizing and hybridizing incubations (i) within 4-16 hours at 42°C, in 6 x SSC containing 50% formamide, or (ii) within 4-16 hours at 65°C in an aqueous 6 x SSC solution (1 M NaCl, 0.1 M sodium citrate (pH 7.0)). Typically, hybridization experiments are performed at a temperature from 60 to 68°C, e.g. 65°C. At such a temperature, stringent hybridization conditions can be achieved in 6xSSC, preferably in 2xSSC or 1xSSC, more preferably in 0.5xSSc, 0.3xSSC or 0.1xSSC (in the absence of formamide). 1xSSC contains 0.15 M NaCl and 0.015 M sodium citrate.

Useful homologs and fragments thereof that do not occur naturally are designed using known methods for identifying regions of an antigen that are likely to tolerate amino acid sequence changes and/or deletions. As an example, homologous polypeptides from different species are compared; conserved sequences are identified. The more divergent sequences are the most likely to tolerate sequence changes. Homology among sequences may be analyzed using, as an example, the BLAST homology searching algorithm of Altschul *et al.,* Nucleic Acids Res.; 25:3389-3402 (1997). Alternatively, sequences are modified such that they become more reactive to T- and/or B-cells, based on computer-assisted analysis of probable T- or B-cell epitopes Yet another alternative is to mutate a particular amino acid residue or sequence within the polypeptide in vitro, then screen the mutant polypeptides for their ability to prevent or treat *Chlamydia* infection according to the method outlined below.

A person skilled in the art will readily understand that by following the screening process disclosed in this application, it will be determined without undue experimentation whether a particular homolog of any one of SEQ ID Nos: 2, 4, 6, 8, 10, 12, 14, 16, 18 and 20 may be useful in the prevention or treatment of *Chlamydia* infection. The screening procedure comprises the steps:
(i) immunizing an animal, preferably mouse, with the test homolog or fragment;
(ii) inoculating the immunized animal with *Chlamydia*; and
(iii) selecting those homologs or fragments which confer protection against *Chlamydia.*

By "conferring protection" is meant that there is a reduction in severity of any of the effects of *Chlamydia* infection, in comparison with a control animal which was not immunized with the test homolog or fragment.

Consistent with the above mentioned aspect disclosed in the application, polypeptide derivatives are described that are partial sequences of any one of SEQ ID Nos: 2, 4, 6, 8, 10, 12, 14, 16, 18 and 20, partial sequences of polypeptide sequences homologous to any one of SEQ ID Nos: 2, 4, 6, 8, 10, 12, 14, 16, 18 and 20, polypeptides derived from full-length polypeptides by internal deletion, and fusion proteins.

It is an accepted practice in the field of immunology to use fragments and variants of protein immunogens as vaccines, as all that is required to induce an immune response to a protein is a small (*e.g.*, 8 to 10 amino acid) immunogenic region of the protein. Various short synthetic peptides corresponding to surface-exposed antigens of pathogens other than *Chlamydia* have been shown to be effective vaccine antigens against their respective pathogens, *e.g.* an 11 residue peptide of murine mammary tumor virus (Casey & Davidson, Nucl. Acid Res. (1977) 4:1539), a 16-residue peptide of Semliki Forest virus (Snijders *et al.,* 1991. J. Gen. Virol. 72:557-565), and two overlapping peptides of 15 residues each from canine parvovirus (Langeveld *et al.,* Vaccine 12(15):1473-1480, 1994)

Accordingly, it will be readily apparent to one skilled in the art, having read the present description, that partial sequences of any one of SEQ ID Nos: 2, 4, 6, 8, 10, 12, 14, 16, 18 and 20 or their homologous amino acid sequences are inherent to the full-length sequences and are disclosed by the present application. Such polypeptide fragments preferably are at least 12 amino acids in length. Advantageously, they are at least 15 amino acids, preferably at least 20, 25, 30, 35, 40, 45, 50 amino acids, more preferably at least 55, 60, 65, 70, 75 amino acids, and most preferably at least 80, 85, 90, 95, 100 amino acids in length.

Polynucleotides of 30 to 600 nucleotides encoding partial sequences of sequences homologous to any one of SEQ ID Nos: 2, 4, 6 ,8, 10, 12, 14, 16, 18 and 20 are retrieved by PCR amplification using the parameters outlined above and using primers matching the sequences upstream and downstream of the 5' and 3' ends of the fragment to be amplified. The template polynucleotide for such amplification is either the full length polynucleotide homologous to any one of SEQ ID Nos: 1, 3, 5, 7, 9, 11, 13, 15, 17 and 19, or a polynucleotide contained in a mixture of polynucleotides such as a DNA or RNA library. As an alternative method for retrieving the partial sequences, screening hybridization is carried out under conditions described above and using the formula for calculating Tm. Where fragments of 30 to 600 nucleotides are to be retrieved, the calculated Tm is corrected by subtracting (600/polynucleotide size in base pairs) and the stringency conditions are defined by a hybridization temperature that is 5 to 10°C below Tm. Where oligonucleotides shorter than 20-30 bases are to be obtained, the formula for calculating the Tm is as follows: Tm = 4 x (G+C) + 2 (A+T). For example, an 18 nucleotide fragment of 50% G+C would have an approximate Tm of 54°C. Short peptides that are fragments of any one of SEQ ID Nos: 2, 4, 6, 8, 10, 12, 14, 16, 18 and 20 or its homologous sequences, are obtained directly by chemical synthesis (E. Gross and H. J. Meinhofer, 4 The Peptides: Analysis, Synthesis, Biology; Modern Techniques of Peptide Synthesis, John Wiley & Sons (1981), and M. Bodanzki, Principles of Peptide Synthesis, Springer -Verlag (1984)).

Useful polypeptide derivatives, *e.g.*, polypeptide fragments, are designed using computer-assisted analysis of amino acid sequences. This would identify probable surface-exposed, antigenic regions (Hughes *et al.,* 1992. Infect. Immun. 60(9):3497). Analysis of 6 amino acid sequences contained in any one of SEQ ID Nos: 2, 4, 6, 8, 10, 12, 14, 16, 18 and 20, based on the product of flexibility and hydrophobicity propensities using the program SEQSEE (Wishart DS, *et al.* "SEQSEE: a comprehensive program suite for protein sequence analysis." *Comput Appl Biosci.* 1994 Apr;10(2):121-32), reveal potential B- and T-cell epitopes which may be used as a basis for selecting useful immunogenic fragments and variants. This analysis uses a reasonable combination of external surface features that is likely to be recognized by antibodies. Probable T-cell epitopes for HLA-A0201 MHC subclass may be revealed by an algorithms that emulate an approach developed at the NIH (Parker KC, *et al.* "Peptide binding to MHC class I molecules: implications for antigenic peptide prediction." *Immunol Res* 1995;14(1):34-57). The potential B-cell and T-cell epitopes are shown in Tables 2, 5, 7, 9, 11, 13, 15, 17 and 19 and SEQ ID NOs: 41 to 74. Sequences which are substantially identical to SEQ ID NOS: 41 to 74, or which are conservatively substituted variants of SEQ ID NOs: 41 to 74, are expected to be functional epitopes and are disclosed in the application.

Epitopes which induce a protective T cell-dependent immune response are present throughout the length of the polypeptide. However, some epitopes may be masked by secondary and tertiary structures of the polypeptide. To reveal such masked epitopes large internal deletions are created which remove much of the original protein structure and exposes the masked epitopes. Such internal deletions sometimes effect the additional advantage of removing immunodominant regions of high variability among strains.

Polynucleotides encoding polypeptide fragments and polypeptides having large internal deletions are constructed using standard methods (Ausubel *et al.,* Current Protocols in Molecular Biology, John Wiley & Sons Inc., 1994). Such methods include standard PCR, inverse PCR, restriction enzyme treatment of cloned DNA molecules, or the method of Kunkel *et al.* (Kunkel *et al.* Proc. Natl. Acad. Sci. USA (1985) 82:448). Components for these methods and instructions for their use are readily available from various commercial sources such as Stratagene. Once the deletion mutants have been constructed, they are tested for their ability to prevent or treat *Chlamydia* infection as described above.

As used herein, a fusion polypeptide is one that contains a polypeptide or a polypeptide derivative disclosed in the application fused at the N- or C-terminal end to any other polypeptide (hereinafter referred to as a peptide tail). A simple way to obtain such a fusion polypeptide is by translation of an in-frame fusion of the polynucleotide sequences, *i.e.*, a hybrid gene. The hybrid gene encoding the fusion polypeptide is inserted into an expression vector which is used to transform or transfect a host cell. Alternatively, the polynucleotide sequence encoding the polypeptide or polypeptide derivative is inserted into an expression vector in which the polynucleotide encoding the peptide tail is already present. Such vectors and instructions for their use are commercially available, *e.g.* the pMal-c2 or pMal-p2 system from New England Biolabs, in which the peptide tail is a maltose binding protein, the glutathione-S-transferase system of Pharmacia, or the His-Tag system available from Novagen. These and other expression systems provide convenient means for further purification of polypeptides and derivatives disclosed in the application.

An advantageous example of a fusion polypeptide is one where the polypeptide or homolog or fragment disclosed in the application is fused to a polypeptide having adjuvant activity, such as subunit B of either cholera toxin or *E. coli* heat-labile toxin. Another advantageous fusion is one where the polypeptide, homolog or fragment is fused to a strong T-cell epitope or B-cell epitope. Such an epitope may be one known in the art (*e.g.* the Hepatitis B virus core antigen, D.R. Millich *et al.,* "Antibody production to the nucleocapsid and envelope of the Hepatitis B virus primed by a single synthetic T cell site", Nature. 1987. 329:547-549), or one which has been identified in another polypeptide disclosed in the application based on computer-assisted analysis of probable T- or B-cell epitopes. Consistent with this aspect disclosed in the application is a fusion polypeptide comprising T- or B-cell epitopes from any one of SEQ ID Nos: 2, 4, 6, 8, 10, 12, 14, 16, 18 and 20 or its homolog or fragment, wherein the epitopes are derived from multiple variants of said polypeptide or homolog or fragment, each variant differing from another in the location and sequence of its epitope within the polypeptide. Such a fusion is effective in the prevention and treatment of *Chlamydia* infection since it optimizes the T- and B-cell response to the overall polypeptide, homolog or fragment.

To effect fusion, the polypeptide disclosed in the application is fused to the N-, or preferably, to the C-terminal end of the polypeptide having adjuvant activity or T- or B-cell epitope. Alternatively, a polypeptide fragment disclosed in the application is inserted internally within the amino acid sequence of the polypeptide having adjuvant activity. The T- or B-cell epitope may also be inserted internally within the amino acid sequence of the polypeptide disclosed in the application.

Consistent with the above described aspect, the polynucleotides disclosed in the application also encode hybrid precursor polypeptides containing heterologous signal peptides, which mature into polypeptides disclosed in the application. By "heterologous signal peptide" is meant a signal peptide that is not found in naturally-occurring precursors of polypeptides disclosed in the application.

Polynucleotide molecules disclosed in the application, including RNA, DNA, or modifications or combinations thereof, have various applications. A DNA molecule is used, for example, (i) in a process for producing the encoded polypeptide in a recombinant host system, (ii) in the construction of vaccine vectors such as poxviruses, which are further used in methods and compositions for preventing and/or treating *Chlamydia* infection, (iii) as a vaccine agent (as well as an RNA molecule), in a naked form or formulated with a delivery vehicle and, (iv) in the construction of attenuated *Chlamydia* strains that can over-express a polynucleotide disclosed in the application or express it in a non-toxic, mutated form.

Selected genes from pathogenic micro-organisms within an eukaryotic expression plasmid are useful as vaccines. Expression plasmids contain methylated CpG motifs that elicit innate cytokine responses that promote the canalization of CD4 T cell responses to a Th1 cytokine secretion pattern. The intracellular synthesis of the microbial protein, especially within transfected professional antigen-presenting cells, facilitates the presentation of antigen on class I and class II molecules and the induction of cell-mediated immunity. The use of one or a number of microbial protein-coding genes allows the presentation of protective antigens to the immune system to occur in the absence of microbe-directed immune evasion mechanisms and in the absence of competing or pathologic antigens. Immune responses primed by DNA vaccines are also readily amplified by protein-antigen immunization. Thus, immunization with DNA vaccines is particularly relevant to chlamydial vaccine design.

Accordingly, another aspect disclosed in the application encompasses (i) an expression cassette containing a DNA molecule described in the application placed under the control of the elements required for expression, in particular under the control of an appropriate promoter; (ii) an expression vector containing an expression cassette disclosed in the application; (iii) a procaryotic or eucaryotic cell transformed or transfected with an expression cassette and/or vector disclosed in the application, as well as (iv) a process for producing a polypeptide or polypeptide derivative encoded by a polynucleotide disclosed in the application, which involves culturing a procaryotic or eucaryotic cell transformed or transfected with an expression cassette and/or vector disclosed in the application, under conditions that allow expression of the DNA molecule described in the application and, recovering the encoded polypeptide or polypeptide derivative from the cell culture.

A recombinant expression system is selected from procaryotic and eucaryotic hosts. Eucaryotic hosts include yeast cells (*e.g.*, *Saccharomyces cerevisiae* or *Pichia pastoris*), mammalian cells (*e.g.*, COS1, NIH3T3, or JEG3 cells), arthropods cells (*e.g.*, *Spodoptera frugiperda* (SF9) cells), and plant cells. A preferred expression system is a procaryotic host such as *E. coli.* Bacterial and eucaryotic cells are available from a number of different sources including commercial sources to those skilled in the art, *e.g.*, the American Type Culture Collection (ATCC; Rockville, Maryland). Commercial sources of cells used for recombinant protein expression also provide instructions for usage of the cells.

The choice of the expression system depends on the features desired for the expressed polypeptide. For example, it may be useful to produce a polypeptide disclosed in the application in a particular lipidated form or any other form.

One skilled in the art would redily understand that not all vectors and expression control sequences and hosts would be expected to express equally well the polynucleotides disclosed in this application. With the guidelines described below, however, a selection of vectors, expression control sequences and hosts may be made without undue experimentation and without departing from the scope of this application.

In selecting a vector, the host must be chosen that is compatible with the vector which is to exist and possibly replicate in it. Considerations are made with respect to the vector copy number, the ability to control the copy number, expression of other proteins such as antibiotic resistance. In selecting an expression control sequence, a number of variables are considered. Among the important variable are the relative strength of the sequence (*e.g.* the ability to drive expression under various conditions), the ability to control the sequence's function, compatibility between the polynucleotide to be expressed and the control sequence (*e.g.* secondary structures are considered to avoid hairpin structures which prevent efficient transcription). In selecting the host, unicellular hosts are selected which are compatible with the selected vector, tolerant of any possible toxic effects of the expressed product, able to secrete the expressed product efficiently if such is desired, to be able to express the product in the desired conformation, to be easily scaled up, and to which ease of purification of the final product.

The choice of the expression cassette depends on the host system selected as well as the features desired for the expressed polypeptide. Typically, an expression cassette includes a promoter that is functional in the selected host system and can be constitutive or inducible; a ribosome binding site; a start codon (ATG) if necessary; a region encoding a signal peptide, *e.g.,* a lipidation signal peptide; a DNA molecule disclosed in the application; a stop codon; and optionally a 3' terminal region (translation and/or transcription terminator). The signal peptide encoding region is adjacent to the polynucleotide disclosed in the application and placed in proper reading frame. The signal peptide-encoding region is homologous or heterologous to the DNA molecule encoding the mature polypeptide and is compatible with the secretion apparatus of the host used for expression. The open reading frame constituted by the DNA molecule disclosed in the application, solely or together with the signal peptide, is placed under the control of the promoter so that transcription and translation occur in the host system. Promoters and signal peptide encoding regions are widely known and available to those skilled in the art and include, for example, the promoter of *Salmonella typhimurium* (and derivatives) that is inducible by arabinose (promoter araB) and is functional in Gram-negative bacteria such as *E. coli* (as described in U.S. Patent No. 5,028,530 and in Cagnon *et al.,* (Cagnon *et al.,* Protein Engineering (1991) 4(7):843)); the promoter of the gene of bacteriophage T7 encoding RNA polymerase, that is functional in a number of *E. coli* strains expressing T7 polymerase (described in U.S. Patent No. 4,952,496); OspA lipidation signal peptide ; and RlpB lipidation signal peptide (Takase *et al.,* J. Bact. (1987) 169:5692) .

The expression cassette is typically part of an expression vector, which is selected for its ability to replicate in the chosen expression system. Expression vectors (*e.g.*, plasmids or viral vectors) can be chosen, for example, from those described in Pouwels *et al.* (Cloning Vectors: A Laboratory Manual 1985, Supp. 1987). Suitable expression vectors can be purchased from various commercial sources.

Methods for transforming/transfecting host cells with expression vectors are well-known in the art and depend on the host system selected as described in Ausubel etal., (Ausubel etal., Current Protocols in Molecular Biology, John Wiley & Sons Inc., 1994).

Upon expression, a recombinant polypeptide disclosed in the application (or a polypeptide derivative) is produced and remains in the intracellular compartment, is secreted/excreted in the extracellular medium or in the periplasmic space, or is embedded in the cellular membrane. The polypeptide is recovered in a substantially purified form from the cell extract or from the supernatant after centrifugation of the recombinant cell culture. Typically, the recombinant polypeptide is purified by antibody-based affinity purification or by other well-known methods that can be readily adapted by a person skilled in the art, such as fusion of the polynucleotide encoding the polypeptide or its derivative to a small affinity binding domain. Antibodies useful for purifying by immunoaffinity the polypeptides disclosed in the application are obtained as described below.

A polynucleotide disclosed in the application can also be useful as a vaccine. There are two major routes, either using a viral or bacterial host as gene delivery vehicle (live vaccine vector) or administering the gene in a free form, e.g., inserted into a plasmid. Therapeutic or prophylactic efficacy of a polynucleotide disclosed in the application is evaluated as described below.

Accordingly, the invention provides a vaccine which comprises a physiologically acceptable diluent or carrier for use in a vaccine, and any one of(a) at least one first nucleic acid in a vaccine vector wherein the first nucleic acid encodes a first polypeptide;(b) the first polypeptide; (c) a fusion protein comprising the first polypeptide and a second polypeptide; or(d) a hybrid nucleic acid in a vaccine vector wherein the hybrid nucleic acid comprises the first nucleic acid fused in-frame with a nucleic acid encoding the second polypeptide; wherein each first nucleic acid is capable of being expressed and wherein the first polypeptide is selected from the group consisting of :(i) a polypeptide whose sequence is set forth in SEQ ID No:18;(ii) a polypeptide which is at least 75% identical in amino acid sequence to SEQ ID No: 18; and(iii) an immunogenic fragment comprising at least 12 consecutive amino acids from SEQ ID No: 18.

The invention also provides (i) a vaccine vector such as a poxvirus, containing a DNA molecule disclosed in the application, placed under the control of elements required for expression; (ii) a composition of matter comprising a vaccine vector of the invention, together with a diluent or carrier; specifically (iii) a pharmaceutical composition containing a therapeutically or prophylactically effective amount of a vaccine vector of the invention; (iv) a method for inducing an immune response against *Chlamydia* in a mammal (*e.g.*, a human; alternatively, the method can be used in veterinary applications for treating or preventing *Chlamydia* infection of animals, *e.g.*, cats or birds), which involves administering to the mammal an immunogenically effective amount of a vaccine vector of the invention to elicit a protective or therapeutic immune response to *Chlamydia* ; and particularly, (v) a method for preventing and/or treating a *Chlamydia* (*e.g., C. trachomatis, C. psittaci, C. pneumonia, C. pecorum*) infection, which involves administering a prophylactic or therapeutic amount of a vaccine vector of the invention to an infected individual. Additionally, the invention encompasses the use of a vaccine vector of the invention in the preparation of a medicament for preventing and/or treating *Chlamydia* infection.

As used herein, a vaccine vector expresses one or several polypeptides or derivatives disclosed in the application. The vaccine vector may express additionally a cytokine, such as interleukin-2 (IL-2) or interleukin-12 (IL-12), that enhances the immune response (adjuvant effect). It is understood that each of the components to be expressed is placed under the control of elements required for expression in a mammalian cell.

Consistent with the above described aspect of the invention is a composition comprising several vaccine vectors, each of them capable of expressing a polypeptide or derivative disclosed in the application. A composition may also comprise a vaccine vector capable of expressing an additional *Chlamydia* antigen, or a subunit, fragment, homolog, mutant, or derivative thereof; optionally together with or a cytokine such as IL-2 or IL-12.

A general principle is that recognition of a particular antigen is not in itself sufficient to produce an effective immune response. In some cases, a cell-mediated response is appropriate; in others, antibody.

Antigens of microorganisms vary considerably in their accessibility to cells of the immune system. Antigens which normally occur inside a pathogen may become accessible only when the pathogen or an infected cell is killed. Even antigens expressed at the cell surface may present only a limited range of their potential epitopes for antibody binding, depending on their orientation in the membrane. Protective structures, such as bacterial capsules, further limit the effective recognition of epitopes.

A distinction should be drawn between the overall composition of the immune response, those components of it which are important in the resolution of infection and the components which are responsible for the prevention of reinfection. In many cases, particular elements of the immune response are critically important; for example, cell-mediated immunity in leprosy. Even when considering a particular effector system, the response directed against some antigens is often much more effective than the responses to others. Immune responses to particular microbial antigens have different degrees of relevance to anti-microbial immunity, depending on the nature of the organism, it pathogenicity and the nature of the immune response it initiates.

The primary effectors against extracellular pathogens are antibody and complement. Binding of antibody to receptors on the pathogen can prevent it from attaching to its target cell. Antibody alone, or more effectively in association with complement, opsonizes pathogens for uptake by phagocytes expressing Fc receptors and complement receptors CR1 and CR3. Usually this will lead to intracellular destruction of the pathogen but if the phagocyte is unable to destroy it and is a facultative host cell, then antibody may actually promote the spread of infection. Such an eventuality, however, depends on the dynamic balance between the actions of the humoral and cell-mediated immune responses.

Sometimes effective antibodies must be of the right class to activate appropriate effectors. The important antigens are those involved in evasion of immune effector mechanisms; that is, pili, fimbriae and capsular antigens which constitute the major antigens of the outer layer of bacteria. Often epitope specificity is important, since it determines whether complement is deposited in a position to damage the outer membrane. There are also numerous protein antigens which can induce an antibody response; however, although the antibody response is partly species-specific and may be diagnostically useful, it is largely irrelevant to immunity. This is most obvious in lepromatous leprosy, where the patients have weak cell-mediated immunity, high levels of specific antibody and tissues heavily infected with bacteria.

In some cases, a particular type of antibody response is mandatory for clearance of the pathogen. This is true of many bacterial infections, where specific antibodies to surface antigens are necessary to neutralize the bacterial defences and opsonize the bacteria for phagocytes.

There are also cases where responses to individual antigens are essential for host immunity. The simplest examples are the toxins produced by the causative agents of diphtheria, tetanus and clostridial enteritis. The damage produced directly by the infectious agent in these diseases is slight by comparison with that produced by the secreted toxins. Consequently, protection against these conditions involves immunization to toxoids. Nevertheless, the immune system must still eradicate the primary site of the bacterial infection if the disease is to be resolved. The target antigens for bactericidal antibodies are extremely diverse and include LPS, capsular polysaccharides and other outer membrane proteins. Virulence factors can also provide good immunogens in a vaccine.

Tables 1, 3, 4, 6, 8, 10, 12, 14, 16 and 18, as well as corresponding Figures 31 to 40, demonstrate that the polypeptides disclosed herein are immunogenic. Furthermore, these Figures demonstrate that the polypeptides disclosed herein confer immunoprotection from *Chlamydia* infection, as evidenced by accelerated clearance of pulmonary infection. Such reduction in the severity of effects of Chlamydia infection is evidence that the polypeptides have generated an active functional immune response against the pathogen, rather than a mere antibody response against the antigen.

Animal models have been used to define the immunobiologic feature of *C. trachomatis* infection. The mouse model is particularly informative, largely because of the ready availability of immune reagents for murine studies and the development of transgenic and knockout (KO) mice. *C. trachomatis* mouse pneumonitis (MoPn) is the most widely tested biovar among the three *C. trachomatis* biovars (trachoma, lymphogranuloma venereum, and MoPn). Although human biovars have also been used in animal models, they normally require high inocula or pretreatment with progesterone. MoPn, which was originally isolated from mouse tissues, is thought to be a natural murine pathogen and thus offers an evolutionarily adapted pathogen for analysis of host-pathogen interactions.

The significant progress in chlamydial immunobiology based on murine models of MoPn infection has extended and clarified recent immunoepidemiologic studies in humans (Yang and Brunham (1998) Can J Infect Dis; 9:99-108). In particular, since the discovery of T helper (Th) 1 and 2 subsets, cytokine patterns have been shown to be critical in the regulation of immune responses to a variety of infectious agents including chlamydiae. Clinical investigation has shown that trachoma patients with severe conjunctival scarring have impaired cell-mediated immune responses to *C. trachomatis* and high IgG antibody titers (Yang and Brunham (1999) Curr Opin Infect Dis; 12:47-52). Cytokine analysis shows increased interleukin (IL)-4 and reduced interferon (IFN)-y production in subjects with scarring disease due to *C. trachomatis* infection compared with controls without scarring disease.

Vaccination methods for treating or preventing infection in a mammal comprises use of a vaccine vector disclosed in the application to be administered by any conventional route, particularly to a mucosal (*e.g.*, ocular, intranasal, oral, gastric, pulmonary, intestinal, rectal, vaginal, or urinary tract) surface or via the parenteral (*e.g.*, subcutaneous, intradermal, intramuscular, intravenous, or intraperitoneal) route. Preferred routes depend upon the choice of the vaccine vector. Treatment may be effected in a single dose or repeated at intervals. The appropriate dosage depends on various parameters understood by skilled artisans such as the vaccine vector itself, the route of administration or the condition of the mammal to be vaccinated (weight, age and the like).

Live vaccine vectors available in the art include viral vectors such as adenoviruses and poxviruses as well as bacterial vectors, *e.g., Shigella, Salmonella, Vibrio cholerae, Lactobacillus,* Bacille bilié de Calmette-Guérin (BCG), and *Streptococcus.*

An example of an adenovirus vector, as well as a method for constructing an adenovirus vector capable of expressing a DNA molecule disclosed in the application, are described in U.S. Patent No. 4,920,209. Poxvirus vectors include vaccinia and canary pox virus, described in U.S. Patent No. 4,722,848 and U.S. Patent No. 5,364,773, respectively. (Also see, *e.g.*, Tartaglia *et al.,* Virology (1992) 188:217) for a description of a vaccinia virus vector and Taylor *et al,* Vaccine (1995) 13:539 for a reference of a canary pox.) Poxvirus vectors capable of expressing a polynucleotide disclosed in the application are obtained by homologous recombination as described in Kieny *et al.,* Nature (1984) 312:163 so that the polynucleotide disclosed in the application is inserted in the viral genome under appropriate conditions for expression in mammalian cells. Generally, the dose of vaccine viral vector, for therapeutic or prophylactic use, can be of from about 1x10⁴ to about 1x10¹¹, advantageously from about 1x10⁷ to about 1x10¹⁰, preferably of from about 1x10⁷ to about 1x10⁹ plaque-forming units per kilogram. Preferably, viral vectors are administered parenterally; for example, in 3 doses, 4 weeks apart. It is preferable to avoid adding a chemical adjuvant to a composition containing a viral vector of the invention and thereby minimizing the immune response to the viral vector itself.

Non-toxicogenic *Vibrio cholerae* mutant strains that are useful as a live oral vaccine are known. Mekalanos *et al.,* Nature (1983) 306:551 and U.S. Patent No. 4,882,278 describe strains which have a substantial amount of the coding sequence of each of the two *ctxA* alleles deleted so that no functional *cholerae* toxin is produced. WO 92/11354 describes a strain in which the *irgA* locus is inactivated by mutation; this mutation can be combined in a single strain with *ctxA* mutations. WO 94/01533 describes a deletion mutant lacking functional *ctxA* and *attRS1* DNA sequences. These mutant strains are genetically engineered to express heterologous antigens, as described in WO 94/19482. An effective vaccine dose of a *Vibrio cholerae* strain capable of expressing a polypeptide or polypeptide derivative encoded by a DNA molecule disclosed in the application contains about 1x10⁵ to about 1x10⁹, preferably about 1x10⁶ to about 1x10⁸, viable bacteria in a volume appropriate for the selected route of administration. Preferred routes of administration include all mucosal routes; most preferably, these vectors are administered intranasally or orally.

Attenuated *Salmonella typhimurium* strains, genetically engineered for recombinant expression of heterologous antigens or not, and their use as oral vaccines are described in Nakayama *et al.* (Bio/Technology (1988) 6:693) and WO 92/11361. Preferred routes of administration include all mucosal routes; most preferably, these vectors are administered intranasally or orally.

Other bacterial strains used as vaccine vectors in the context of the present invention are described for *Shigella flexneri* in High *et al.,* EMBO (1992) 11:1991 and Sizemore *et al.,* Science (1995) 270:299; for *Streptococcus gordonii* in Medaglini *et al.,* Proc. Natl. Acad. Sci. USA (1995) 92:6868; and for Bacille Calmette Guerin in Flynn J.L., Cell. Mol. Biol. (1994) 40 (suppl. I):31, WO 88/06626, WO 90/00594, WO 91/13157, WO 92/01796, and WO 92/21376.

In bacterial vectors, the polynucleotide disclosed in the application is inserted into the bacterial genome or remains in a free state as part of a plasmid.

The composition comprising a vaccine bacterial vector disclosed in the application may further contain an adjuvant. A number of adjuvants are known to those skilled in the art. Preferred adjuvants are selected as provided below.

Accordingly, another aspect of the invention provides (i) a composition of matter comprising a polynucleotide disclosed in the application, together with a diluent or carrier; (ii) a pharmaceutical composition comprising a therapeutically or prophylactically effective amount of a polynucleotide disclosed in the application; (iii) a method for inducing an immune response against *Chlamydia* in a mammal by administration of an immunogenically effective amount of a polynucleotide disclosed in the application to elicit a protective immune response to *Chlamydia;* and particularly, (iv) a method for preventing and/or treating a *Chlamydia* (*e.g., C. trachomatis, C. psittaci, C. pneumoniae, or C. pecorum*) infection, by administering a prophylactic or therapeutic amount of a polynucleotide disclosed in the application to an infected individual. Additionally, the invention also encompasses the use of a polynucleotide disclosed in the application in the preparation of a medicament for preventing and/or treating *Chlamydia* infection. A preferred use includes the use of a DNA molecule placed under conditions for expression in a mammalian cell, especially in a plasmid that is unable to replicate in mammalian cells and to substantially integrate in a mammalian genome.

Use of the polynucleotides disclosed in the application include their administration to a mammal as a vaccine, for therapeutic or prophylactic purposes. Such polynucleotides are used in the form of DNA as part of a plasmid that is unable to replicate in a mammalian cell and unable to integrate into the mammalian genome. Typically, such a DNA molecule is placed under the control of a promoter suitable for expression in a mammalian cell. The promoter functions either ubiquitously or tissue-specifically. Examples of non-tissue specific promoters include the early Cytomegalovirus (CMV) promoter (described in U.S. Patent No. 4,168,062) and the Rous Sarcoma Virus promoter (described in Norton & Coffin, Molec. Cell Biol. (1985) 5:281). An example of a tissue-specific promoter is the desmin promoter which drives expression in muscle cells (Li *et al.,* Gene (1989) 78:243, Li & Paulin, J. Biol. Chem. (1991) 266:6562 and Li & Paulin, J. Biol. Chem. (1993) 268:10403). Use of promoters is well-known to those skilled in the art. Useful vectors are described in numerous publications, specifically WO 94/21797 and Hartikka *et al.,* Human Gene Therapy (1996) 7:1205.

Polynucleotides disclosed in the application which are used as vaccines encode either a precursor or a mature form of the corresponding polypeptide. In the precursor form, the signal peptide is either homologous or heterologous. In the latter case, a eucaryotic leader sequence such as the leader sequence of the tissue-type plasminogen factor (tPA) is preferred.

As used herein, a composition disclosed in the application contains one or several polynucleotides with optionally at least one additional polynucleotide encoding another *Chlamydia* antigen such as urease subunit A, B, or both, or a fragment, derivative, mutant, or analog thereof. The composition may also contain an additional polynucleotide encoding a cytokine, such as interleukin-2 (IL-2) or interleukin-12 (IL-12) so that the immune response is enhanced. These additional polynucleotides are placed under appropriate control for expression. Advantageously, DNA molecules disclosed in the application and/or additional DNA molecules to be included in the same composition, are present in the same plasmid.

Standard techniques of molecular biology for preparing and purifying polynucleotides are used in the preparation of polynucleotide therapeutics of the invention. For use as a vaccine, a polynucleotide disclosed in the application is formulated according to various methods outlined below.

One method utililizes the polynucleotide in a naked form, free of any delivery vehicles. Such a polynucleotide is simply diluted in a physiologically acceptable solution such as sterile saline or sterile buffered saline, with or without a carrier. When present, the carrier preferably is isotonic, hypotonic, or weakly hypertonic, and has a relatively low ionic strength, such as provided by a sucrose solution, *e.g.,* a solution containing 20% sucrose.

An alternative method utilizes the polynucleotide in association with agents that assist in cellular uptake. Examples of such agents are (i) chemicals that modify cellular permeability, such as bupivacaine (see, *e.g.*, WO 94/16737), (ii) liposomes for encapsulation of the polynucleotide, or (iii) cationic lipids or silica, gold, or tungsten microparticles which associate themselves with the polynucleotides.

Anionic and neutral liposomes are well-known in the art (see, *e.g.*, Liposomes: A Practical Approach, RPC New Ed, IRL press (1990), for a detailed description of methods for making liposomes) and are useful for delivering a large range of products, including polynucleotides.

Cationic lipids are also known in the art and are commonly used for gene delivery. Such lipids include Lipofectin^{™} also known as DOTMA (N-[1-(2,3-dioleyloxy)propyl]-N,N,N-trimethylammonium chloride), DOTAP (1,2-bis(oleyloxy)-3-(trimethylammonio)propane), DDAB (dimethyldioctadecylammonium bromide), DOGS (dioctadecylamidologlycyl spermine) and cholesterol derivatives such as DC-Chol (3 beta-(N-(N',N'-dimethyl aminomethane)-carbamoyl) cholesterol). A description of these cationic lipids can be found in EP 187,702, WO 90/11092, U.S. Patent No. 5,283,185, WO 91/15501, WO 95/26356, and U.S. Patent No. 5,527,928. Cationic lipids for gene delivery are preferably used in association with a neutral lipid such as DOPE (dioleyl phosphatidylethanolamine), as described in WO 90/11092 as an example.

Formulation containing cationic liposomes may optionally contain other transfection-facilitating compounds. A number of them are described in WO 93/18759, WO 93/19768, WO 94/25608, and WO 95/02397. They include spermine derivatives useful for facilitating the transport of DNA through the nuclear membrane (see, for example, WO 93/18759) and membrane-permeabilizing compounds such as GALA, Gramicidine S, and cationic bile salts (see, for example, WO 93/19768).

Gold or tungsten microparticles are used for gene delivery, as described in WO 91/00359, WO 93/17706, and *Tang et al.* Nature (1992) 356:152. The microparticle-coated polynucleotide is injected via intradermal or intraepidermal routes using a needleless injection device ("gene gun"), such as those described in U.S. Patent No. 4,945,050, U.S. Patent No. 5,015,580, and WO 94/24263.

The amount of DNA to be used in a vaccine recipient depends, *e.g.*, on the strength of the promoter used in the DNA construct, the immunogenicity of the expressed gene product, the condition of the mammal intended for administration (*e.g.*, the weight, age, and general health of the mammal), the mode of administration, and the type of formulation. In general, a therapeutically or prophylactically effective dose from about 1 µg to about 1 mg, preferably, from about 10 µg to about 800 µg and, more preferably, from about 25 µg to about 250 µg, can be administered to human adults. The administration can be achieved in a single dose or repeated at intervals.

The route of administration is any conventional route used in the vaccine field. As general guidance, a polynucleotide disclosed in the application is administered via a mucosal surface, *e.g.*, an ocular, intranasal, pulmonary, oral, intestinal, rectal, vaginal, and urinary tract surface; or via a parenteral route, *e.g.*, by an intravenous, subcutaneous, intraperitoneal, intradermal, intraepidermal, or intramuscular route. The choice of administration route depends on the formulation that is selected. A polynucleotide formulated in association with bupivacaine is advantageously administered into muscles. When a neutral or anionic liposome or a cationic lipid, such as DOTMA or DC-Chol, is used, the formulation can be advantageously injected via intravenous, intranasal (aerosolization), intramuscular, intradermal, and subcutaneous routes. A polynucleotide in a naked form can advantageously be administered via the intramuscular, intradermal, or subcutaneous routes.

Although not absolutely required, such a composition can also contain an adjuvant. If so, a systemic adjuvant that does not require concomitant administration in order to exhibit an adjuvant effect is preferable such as, *e.g.*, QS21, which is described in U.S. Patent No. 5,057,546.

The sequence information provided in the present application discloses the design of specific nucleotide probes and primers that could be used for diagnostic purposes. Accordingly, the application discloses a nucleotide probe or primer having a sequence found in or derived by degeneracy of the genetic code from a sequence shown in any one of SEQ ID Nos: 1, 3, 5, 7, 9, 11, 13, 15, 17 and 19.

The term "probe" as used in the present application refers to DNA (preferably single stranded) or RNA molecules (or modifications or combinations thereof) that hybridize under the stringent conditions, as defined above, to nucleic acid molecules having any one of SEQ ID Nos: 1, 3, 5, 7, 9, 11, 13, 15, 17 and 19 or to a sequence homologous to any one of SEQ ID Nos: 1, 3, 5, 7, 9, 11, 13, 15, 17 and 19, or to its complementary or anti-sense sequence. Generally, probes are significantly shorter than full-length sequences. Such probes contain from about 5 to about 100, preferably from about 10 to about 80, nucleotides. In particular, probes have sequences that are at least 75%, preferably at least 80% or 85%, more preferably 90% or 95% homologous to a portion of any one of SEQ ID Nos: 1, 3, 5, 7, 9, 11, 13, 15, 17 and 19 or that are complementary to such sequences. Probes may contain modified bases such as inosine, methyl-5-deoxycytidine, deoxyuridine, dimethylamino-5-deoxyuridine, or diamino-2, 6-purine. Sugar or phosphate residues may also be modified or substituted. For example, a deoxyribose residue may be replaced by a polyamide (Nielsen *et al.,* Science (1991) 254:1497) and phosphate residues may be replaced by ester groups such as diphosphate, alkyl, arylphosphonate and phosphorothioate esters. In addition, the 2'-hydroxyl group on ribonucleotides may be modified by including such groups as alkyl groups.

The Probes disclosed in the application can be used in diagnostic tests, as capture or detection probes. Such capture probes are conventionally immobilized on a solid support, directly or indirectly, by covalent means or by passive adsorption. A detection probe is labelled by a detection marker selected from: radioactive isotopes, enzymes such as peroxidase, alkaline phosphatase, and enzymes able to hydrolyze a chromogenic, fluorogenic, or luminescent substrate, compounds that are chromogenic, fluorogenic, or luminescent, nucleotide base analogs, and biotin.

The Probes disclosed in the application can be used in any conventional hybridization technique, such as dot blot (Maniatis *et al.,* Molecular Cloning: A Laboratory Manual (1982) Cold Spring Harbor Laboratory Press, Cold Spring Harbor, New York), Southern blot (Southern, J. Mol. Biol. (1975) 98:503), northern blot (identical to Southern blot with the exception that RNA is used as a target), or the sandwich technique (Dunn *et al.,* Cell (1977) 12:23). The latter technique involves the use of a specific capture probe and/or a specific detection probe with nucleotide sequences that at least partially differ from each other.

A primer is a probe of usually about 10 to about 40 nucleotides that is used to initiate enzymatic polymerization of DNA in an amplification process (*e.g.*, PCR), in an elongation process, or in a reverse transcription method. Primers used in diagnostic methods involving PCR are labeled by methods known in the art.

As described herein, the application also discloses (i) a reagent comprising a probe described in the application for detecting and/or identifying the presence of *Chlamydia* in a biological material; (ii) a method for detecting and/or identifying the presence of *Chlamydia* in a biological material, in which (a) a sample is recovered or derived from the biological material, (b) DNA or RNA is extracted from the material and denatured, and (c) exposed to a probe disclosed in the application, for example, a capture, detection probe or both, under stringent hybridization conditions, such that hybridization is detected; and (iii) a method for detecting and/or identifying the presence of *Chlamydia* in a biological material, in which (a) a sample is recovered or derived from the biological material, (b) DNA is extracted therefrom, (c) the extracted DNA is primed with at least one, and preferably two, primers described in the application and amplified by polymerase chain reaction, and (d) the amplified DNA fragment is produced.

It is apparent that disclosure of a polynucleotide sequence of any one of SEQ ID Nos: 1, 3, 5, 7, 9, 11, 13, 15, 17 and 19, its homologs and partial sequences enable their corresponding amino acid sequences. Accordingly, another aspect disclosed in the application features a substantially purified polypeptide or polypeptide derivative having an amino acid sequence encoded by a polynucleotide described in the application.

A "substantially purified polypeptide" as used herein is defined as a polypeptide that is separated from the environment in which it naturally occurs and/or that is free of the majority of the polypeptides that are present in the environment in which it was synthesized. For example, a substantially purified polypeptide is free from cytoplasmic polypeptides. Those skilled in the art would readily understand that the polypeptides disclosed in the application may be purified from a natural source, *i.e.*, a *Chlamydia* strain, or produced by recombinant means.

Consistent with the above aspect disclosed in the application are polypeptides, homologs or fragments which are modified or treated to enhance their immunogenicity in the target animal, in whom the polypeptide, homolog or fragments are intended to confer protection against *Chlamydia.* Such modifications or treatments include: amino acid substitutions with an amino acid derivative such as 3-methyhistidine, 4-hydroxyproline, 5-hydroxylysine etc., modifications or deletions which are carried out after preparation of the polypeptide, homolog or fragment, such as the modification of free amino, carboxyl or hydroxyl side groups of the amino acids.

Identification of homologous polypeptides or polypeptide derivatives encoded by polynucleotides disclosed in the application which have specific antigenicity is achieved by screening for cross-reactivity with an antiserum raised against the polypeptide of reference having an amino acid sequence of any one of SEQ ID Nos: 2, 4, 6, 8, 10, 12, 14, 16, 18 and 20. The procedure is as follows: a monospecific hyperimmune antiserum is raised against a purified reference polypeptide, a fusion polypeptide (for example, an expression product of MBP, GST, or His-tag systems, the description and instructions for use of which are contained in Invitrogen product manuals for pcDNA3.1/Myc-His(+) A, B, and C and for the Xpress^{™} System Protein Purification), or a synthetic peptide predicted to be antigenic. Where an antiserum is raised against a fusion polypeptide, two different fusion systems are employed. Specific antigenicity can be determined according to a number of methods, including Western blot (Towbin *et al.,* Proc. Natl. Acad. Sci. USA (1979) 76:4350), dot blot, and ELISA, as described below.

In a Western blot assay, the product to be screened, either as a purified preparation or a total *E. coli* extract, is submitted to SDS-Page electrophoresis as described by Laemmli (Nature (1970) 227:680). After transfer to a nitrocellulose membrane, the material is further incubated with the monospecific hyperimmune antiserum diluted in the range of dilutions from about 1:5 to about 1:5000, preferably from about 1:100 to about 1:500. Specific antigenicity is shown once a band corresponding to the product exhibits reactivity at any of the dilutions in the above range.

In an ELISA assay, the product to be screened is preferably used as the coating antigen. A purified preparation is preferred, although a whole cell extract can also be used. Briefly, about 100 µl of a preparation at about 10 µg protein/ml are distributed into wells of a 96-well polycarbonate ELISA plate. The plate is incubated for 2 hours at 37°C then overnight at 4°C. The plate is washed with phosphate buffer saline (PBS) containing 0.05% Tween 20 (PBS/Tween buffer). The wells are saturated with 250 µl PBS containing 1% bovine serum albumin (BSA) to prevent non-specific antibody binding. After 1 hour incubation at 37°C, the plate is washed with PBS/Tween buffer. The antiserum is serially diluted in PBS/Tween buffer containing 0.5% BSA. 100 µl of dilutions are added per well. The plate is incubated for 90 minutes at 37°C, washed and evaluated according to standard procedures. For example, a goat anti-rabbit peroxidase conjugate is added to the wells when specific antibodies were raised in rabbits. Incubation is carried out for 90 minutes at 37°C and the plate is washed. The reaction is developed with the appropriate substrate and the reaction is measured by colorimetry (absorbance measured spectrophotometrically). Under the above experimental conditions, a positive reaction is shown by O.D. values greater than a non immune control serum.

In a dot blot assay, a purified product is preferred, although a whole cell extract can also be used. Briefly, a solution of the product at about 100 µg/ml is serially two-fold diluted in 50 mM Tris-HCl (pH 7.5). 100 µl of each dilution are applied to a nitrocellulose membrane 0.45 µm set in a 96-well dot blot apparatus (Biorad). The buffer is removed by applying vacuum to the system. Wells are washed by addition of 50 mM Tris-HCl (pH 7.5) and the membrane is air-dried. The membrane is saturated in blocking buffer (50 mM Tris-HCl (pH 7.5) 0.15 M NaCl, 10 g/L skim milk) and incubated with an antiserum dilution from about 1:50 to about 1:5000, preferably about 1:500. The reaction is revealed according to standard procedures. For example, a goat anti-rabbit peroxidase conjugate is added to the wells when rabbit antibodies are used. Incubation is carried out 90 minutes at 37°C and the blot is washed. The reaction is developed with the appropriate substrate and stopped. The reaction is measured visually by the appearance of a colored spot, *e.g.*, by colorimetry. Under the above experimental conditions, a positive reaction is shown once a colored spot is associated with a dilution of at least about 1:5, preferably of at least about 1:500.

Therapeutic or prophylactic efficacy of a polypeptide or derivative disclosed in the application can be evaluated as described below. Another aspect of the invention provides (i) a composition of matter comprising a polypeptide disclosed in the application together with a diluent or carrier; specifically (ii) a pharmaceutical composition containing a therapeutically or prophylactically effective amount of a polypeptide disclosed in the application; (iii) a method for inducing an immune response against *Chlamydia* in a mammal, by administering to the mammal an immunogenically effective amount of a polypeptide disclosed in the application to elicit a protective immune response to *Chlamydia;* and particularly, (iv) a method for preventing and/or treating a *Chlamydia* (*e.g., C. trachomatis. C. psittaci, C. pneumoniae.* or *C. pecorum*) infection, by administering a prophylactic or therapeutic amount of a polypeptide disclosed in the application to an infected individual. Additionally, the above aspect of the invention encompasses the use of a polypeptide disclosed in the application in the preparation of a medicament for preventing and/or treating *Chlamydia* infection.

As used herein, the immunogenic compositions disclosed in the application, are administered by conventional routes known the vaccine field, in particular to a mucosal (*e.g.*, ocular, intranasal, pulmonary, oral, gastric, intestinal, rectal, vaginal, or urinary tract) surface or via the parenteral (*e.g.,* subcutaneous, intradermal, intramuscular, intravenous, or intraperitoneal) route. The choice of administration route depends upon a number of parameters, such as the adjuvant associated with the polypeptide. If a mucosal adjuvant is used, the intranasal or oral route is preferred. If a lipid formulation or an aluminum compound is used, the parenteral route is preferred with the sub-cutaneous or intramuscular route being most preferred. The choice also depends upon the nature of the vaccine agent. For example, a polypeptide disclosed in the application fused to CTB or LTB is best administered to a mucosal surface.

As used herein, the composition disclosed in the application contains one or several polypeptides or derivatives described in the application. The composition optionally contains at least one additional *Chlamydia* antigen, or a subunit, fragment, homolog, mutant, or derivative thereof.

For use in a composition disclosed in the application, a polypeptide or derivative thereof is formulated into or with liposomes, preferably neutral or anionic liposomes, microspheres, ISCOMS, or virus-like-particles (VLPs) to facilitate delivery and/or enhance the immune response. These compounds are readily available to one skilled in the art; for example, see Liposomes: A Practical Approach, RCP New Ed, IRL press (1990).

Adjuvants other than liposomes and the like are also used and are known in the art. Adjuvants may protect the antigen from rapid dispersal by sequestering it in a local deposit, or they may contain substances that stimulate the host to secrete factors that are chemotactic for macrophages and other components of the immune system. An appropriate selection can conventionally be made by those skilled in the art, for example, from those described below (under a further aspect disclosed in the application).

Treatment is achieved in a single dose or repeated as necessary at intervals, as can be determined readily by one skilled in the art. For example, a priming dose is followed by three booster doses at weekly or monthly intervals. An appropriate dose depends on various parameters including the recipient (*e.g.*, adult or infant), the particular vaccine antigen, the route and frequency of administration, the presence/absence or type of adjuvant, and the desired effect (*e.g.,* protection and/or treatment), as can be determined by one skilled in the art. In general, a vaccine antigen disclosed in the application is administered by a mucosal route in an amount from about 10 µg to about 500 mg, preferably from about 1 mg to about 200 mg. For the parenteral route of administration, the dose usually does not exceed about 1 mg, preferably about 100 µg.

When used as vaccine agents, polynucleotides and polypeptides disclosed in the application may be used sequentially as part of a multistep immunization process. For example, a mammal is initially primed with a vaccine vector disclosed in the application such as a pox virus, *e.g., via* the parenteral route, and then boosted twice with the polypeptide encoded by the vaccine vector, *e.g., via* the mucosal route. In another example, liposomes associated with a polypeptide or derivative disclosed in the application is also used for priming, with boosting being carried out mucosally using a soluble polypeptide or derivative disclosed in the application in combination with a mucosal adjuvant (*e.g.*, LT).

A polypeptide derivative disclosed in the application is also used in accordance with the above described aspect as a diagnostic reagent for detecting the presence of anti-*Chlamydia* antibodies, *e.g.*, in a blood sample. Such polypeptides are about 5 to about 80, preferably about 10 to about 50 amino acids in length. They are either labeled or unlabeled, depending upon the diagnostic method. Diagnostic methods involving such a reagent are described below.

Upon expression of a DNA molecule disclosed in the application, a polypeptide or polypeptide derivative is produced and purified using known laboratory techniques. As described above, the polypeptide or polypeptide derivative may be produced as a fusion protein containing a fused tail that facilitates purification. The fusion product is used to immunize a small mammal, *e.g.*, a mouse or a rabbit, in order to raise antibodies against the polypeptide or polypeptide derivative (monospecific antibodies). Accordingly, a further aspect disclosed in the application provides a monospecific antibody that binds to a polypeptide or polypeptide derivative described in the application.

By "monospecific antibody" is meant an antibody that is capable of reacting with a unique naturally-occurring *Chlamydia* polypeptide. An antibody disclosed in the application is either polyclonal or monoclonal. Monospecific antibodies may be recombinant, *e.g.*, chimeric (*e.g.*, constituted by a variable region of murine origin associated with a human constant region), humanized (a human immunoglobulin constant backbone together with hypervariable region of animal, *e.g.*, murine, origin), and/or single chain. Both polyclonal and monospecific antibodies may also be in the form of immunoglobulin fragments, *e.g.*, F(ab)'2 or Fab fragments. The antibodies disclosed in the application are of any isotype, *e.g.,* IgG or IgA, and polyclonal antibodies are of a single isotype or a mixture of isotypes.

Antibodies against the polypeptides, homologs or fragments disclosed in the application are generated by immunization of a mammal with a composition comprising said polypeptide, homolog or fragment. Such antibodies may be polyclonal or monoclonal. Methods to produce polyclonal or monoclonal antibodies are well known in the art. For a review, see "Antibodies, A Laboratory Manual, Cold Spring Harbor Laboratory, Eds. E. Harlow and D. Lane (1988), and D.E. Yelton *et al.,* 1981. Ann. Rev. Biochem. 50:657-680. For monoclonal antibodies, see Kohler & Milstein (1975) Nature 256:495-497.

The antibodies disclosed in the application, which are raised to a polypeptide or polypeptide derivative described in the application, are produced and identified using standard immunological assays, *e.g.*, Western blot analysis, dot blot assay, or ELISA (see, *e.g.,* Coligan *et al.,* Current Protocols in Immunology (1994) John Wiley & Sons, Inc., New York, NY). The antibodies are used in diagnostic methods to detect the presence of a *Chlamydia* antigen in a sample, such as a biological sample. The antibodies are also used in affinity chromatography for purifying a polypeptide or polypeptide derivative disclosed in the application. As is discussed further below, such antibodies may be used in prophylactic and therapeutic passive immunization methods.

Accordingly, another aspect disclosed in the application provides (i) a reagent for detecting the presence of *Chlamydia* in a biological sample that contains an antibody, polypeptide, or polypeptide derivative disclosed in the application; and (ii) a diagnostic method for detecting the presence of *Chlamydia* in a biological sample, by contacting the biological sample with an antibody, a polypeptide, or a polypeptide derivative disclosed in the application, such that an immune complex is formed, and by detecting such complex to indicate the presence of *Chlamydia* in the sample or the organism from which the sample is derived.

Those skilled in the art will readily understand that the immune complex is formed between a component of the sample and the antibody, polypeptide, or polypeptide derivative, whichever is used, and that any unbound material is removed prior to detecting the complex. It is understood that a polypeptide reagent is useful for detecting the presence of anti-*Chlamydia* antibodies in a sample, *e.g.,* a blood sample, while an antibody disclosed in the application is used for screening a sample, such as a gastric extract or biopsy, for the presence of *Chlamydia* polypeptides.

For diagnostic applications, the reagent (*i.e.,* the antibody, polypeptide, or polypeptide derivative disclosed in the application) is either in a free state or immobilized on a solid support, such as a tube, a bead, or any other conventional support used in the field. Immobilization is achieved using direct or indirect means. Direct means include passive adsorption (non-covalent binding) or covalent binding between the support and the reagent. By "indirect means" is meant that an anti-reagent compound that interacts with a reagent is first attached to the solid support. For example, if a polypeptide reagent is used, an antibody that binds to it can serve as an anti-reagent, provided that it binds to an epitope that is not involved in the recognition of antibodies in biological samples. Indirect means may also employ a ligand-receptor system, for example, where a molecule such as a vitamin is grafted onto the polypeptide reagent and the corresponding receptor immobilized on the solid phase. This is illustrated by the biotin-streptavidin system. Alternatively, a peptide tail is added chemically or by genetic engineering to the reagent and the grafted or fused product immobilized by passive adsorption or covalent linkage of the peptide tail.

Such diagnostic agents may be included in a kit which also comprises instructions for use. The reagent is labeled with a detection means which allows for the detection of the reagent when it is bound to its target. The detection means may be a fluorescent agent such as fluorescein isocyanate or fluorescein isothiocyanate, or an enzyme such as horse radish peroxidase or luciferase or alkaline phosphatase, or a radioactive element such as ¹²⁵I or ⁵¹Cr.

Accordingly, another aspect disclosed in the application concerns a process for purifying, from a biological sample, a polypeptide or polypeptide derivative disclosed in the application, which involves carrying out antibody-based affinity chromatography with the biological sample, wherein the antibody is a monospecific antibody disclosed in the application.

For use in a purification process disclosed in the application, the antibody is either polyclonal or monospecific, and preferably is of the IgG type. Purified IgGs is prepared from an antiserum using standard methods (see, *e.g.,* Coligan *et al.,* Current Protocols in Immunology (1994) John Wiley & Sons, Inc., New York, NY.). Conventional chromatography supports, as well as standard methods for grafting antibodies, are described in, *e.g.*, Antibodies: A Laboratory Manual, D. Lane, E. Harlow, Eds. (1988) and outlined below.

Briefly, a biological sample, such as an *C. pneumoniae* extract preferably in a buffer solution, is applied to a chromatography material, preferably equilibrated with the buffer used to dilute the biological sample so that the polypeptide or polypeptide derivative disclosed in the application (*i.e.*, the antigen) is allowed to adsorb onto the material. The chromatography material, such as a gel or a resin coupled to an antibody disclosed in the application, is in either a batch form or a column. The unbound components are washed off and the antigen is then eluted with an appropriate elution buffer, such as a glycine buffer or a buffer containing a chaotropic agent, *e.g.*, guanidine HCl, or high salt concentration (*e.g.*, 3 M MgCl₂). Eluted fractions are recovered and the presence of the antigen is detected, *e.g.*, by measuring the absorbance at 280 nm.

Another aspect disclosed in the application provides (i) a composition of matter comprising a monospecific antibody disclosed in the application, together with a diluent or carrier; (ii) a pharmaceutical composition comprising a therapeutically or disclosed in prophylactically effective amount of a monospecific antibody disclosed in the application, and (iii) a method for treating or preventing a *Chlamydia* (*e.g., C. trachomatis, C. psittaci, C. pneumoniae or C. pecorum*) infection, by administering a therapeutic or prophylactic amount of a monospecific antibody disclosed in the application to an infected individual. Additionally, the above mentioned aspect of the application encompasses the use of a monospecific antibody disclosed in the application in the preparation of a medicament for treating or preventing *Chlamydia* infection.

The monospecific antibody is either polyclonal or monoclonal, preferably of the IgA isotype (predominantly). In passive immunization, the antibody is administered to a mucosal surface of a mammal, *e.g.*, the gastric mucosa, *e.g.*, orally or intragastrically, advantageously, in the presence of a bicarbonate buffer. Alternatively, systemic administration, not requiring a bicarbonate buffer, is carried out. A monospecific antibody disclosed in the application is administered as a single active component or as a mixture with at least one monospecific antibody specific for a different *Chlamydia* polypeptide. The amount of antibody and the particular regimen used are readily determined by one skilled in the art. For example, daily administration of about 100 to 1,000 mg of antibodies over one week, or three doses per day of about 100 to 1,000 mg of antibodies over two or three days, are effective regimens for most purposes.

Therapeutic or prophylactic efficacy are evaluated using standard methods in the art, *e.g.*, by measuring induction of a mucosal immune response or induction of protective and/or therapeutic immunity, using, *e.g.*, the *C. pneumoniae* mouse model. Those skilled in the art will readily recognize that the *C. pneumoniae* strain of the model may be replaced with another *Chlamydia* strain. For example, the efficacy of DNA molecules and polypeptides from *C. pneumoniae* is preferably evaluated in a mouse model using *C. pneumoniae* strain. Protection is determined by comparing the degree of *Chlamydia* infection to that of a control group. Protection is shown when infection is reduced by comparison to the control group. Such an evaluation is made for polynucleotides, vaccine vectors, polypeptides and derivatives thereof, as well as antibodies disclosed in the application.

Adjuvants useful in any of the vaccine compositions described above are as follows.

Adjuvants for parenteral administration include aluminum compounds, such as aluminum hydroxide, aluminum phosphate, and aluminum hydroxy phosphate. The antigen is precipitated with, or adsorbed onto, the aluminum compound according to standard protocols. Other adjuvants, such as RIBI (ImmunoChem, Hamilton, MT), are used in parenteral administration.

Adjuvants for mucosal administration include bacterial toxins, *e.g.*, the cholera toxin (CT), the *E. coli* heat-labile toxin (LT), the *Clostridium difficile* toxin A and the *pertussis* toxin (PT), or combinations, subunits, toxoids, or mutants thereof such as a purified preparation of native cholera toxin subunit B (CTB). Fragments, homologs, derivatives, and fusions to any of these toxins are also suitable, provided that they retain adjuvant activity. Preferably, a mutant having reduced toxicity is used. Suitable mutants are described, *e.g.*, in WO 95/17211 (Arg-7-Lys CT mutant), WO 96/06627 (Arg-192-Gly LT mutant), and WO 95/34323 (Arg-9-Lys and Glu-129-Gly PT mutant). Additional LT mutants that are used in the methods and compositions disclosed in the application include, *e.g.,* Ser-63-Lys, Ala-69Gly, Glu-110-Asp, and Glu-112-Asp mutants. Other adjuvants, such as a bacterial monophosphoryl lipid A (MPLA) of, *e.g., E. coli, Salmonella minnesota, Salmonella typhimurium,* or *Shigella flexneri;* saponins, or polylactide glycolide (PLGA) microspheres, is also be used in mucosal administration.

Adjuvants useful for both mucosal and parenteral administrations include polyphosphazene (WO 95/02415), DC-chol (3 b-(N-(N',N'-dimethyl aminomethane)-carbamoyl) cholesterol; U.S. Patent No. 5,283,185 and WO 96/14831) and QS-21 (WO 88/09336).

Any pharmaceutical composition of the invention containing a polynucleotide, a polypeptide, a polypeptide derivative, or an antibody disclosed in the application, is manufactured in a conventional manner. In particular, it is formulated with a pharmaceutically acceptable diluent or carrier, *e.g.*, water or a saline solution such as phosphate buffer saline. In general, a diluent or carrier is selected on the basis of the mode and route of administration, and standard pharmaceutical practice. Suitable pharmaceutical carriers or diluents, as well as pharmaceutical necessities for their use in pharmaceutical formulations, are described in *Remington's Pharmaceutical Sciences,* a standard reference text in this field and in the USP/NF.

The invention also includes methods in which *Chlamydia* infection are treated by oral administration of a *Chlamydia* polypeptide disclosed in the application and a mucosal adjuvant, in combination with an antibiotic, an antacid, sucralfate, or a combination thereof. Examples of such compounds that can be administered with the vaccine antigen and the adjuvant are antibiotics, including, *e.g.*, macrolides, tetracyclines, and derivatives thereof (specific examples of antibiotics that can be used include azithromycin or doxicyclin or immunomodulators such as cytokines or steroids). In addition, compounds containing more than one of the above-listed components coupled together, are used. The application also discloses compositions for carrying out these methods, *i.e.,* compositions containing a *Chlamydia* antigen (or antigens) disclosed in the application, an adjuvant, and one or more of the above-listed compounds, in a pharmaceutically acceptable carrier or diluent.

It has recently been shown that the 60kDa cysteine rich membrane protein contains a sequence cross-reactive with the murine alpha-myosin heavy chain epitope M7A-alpha, an epitope conserved in humans (Bachmaier *et al.,* Science (1999) 283:1335). This cross-reactivity is proposed to contribute to the development of cardiovascular disease, so it may be beneficial to remove this epitope, and any other epitopes cross-reactive with human antigens, from the protein if it is to be used as a vaccine. Accordingly, another aspect disclosed in the present application includes the modification of the coding sequence, for example, by deletion or substitution of the nucleotides encoding the epitope from polynucleotides encoding the protein, as to improve the efficacy and safety of the protein as a vaccine. A similar approach may be appropriate for any protective antigen found to have unwanted homologies or cross-reactivities with human antigens.

Amounts of the above-listed compounds used in the methods and compositions disclosed in the application are readily determined by one skilled in the art. Treatment/immunization schedules are also known and readily designed by one skilled in the art. For example, the non-vaccine components can be administered on days 1-14, and the vaccine antigen + adjuvant can be administered on days 7, 14, 21, and 28.

### EXAMPLES

The above disclosure generally desc bes the present invention. A more complete understanding ca be obtained by reference to the following specific examples and, in particular, in examples related to the pCACPNM459 plasmid. These examples are described solely for purposes of illustration and are not intended to limit the scope of the invention.

### Example 1:

These examples illustrate the preparation of plasmid vectors used in immunoprotection studies.

### A. Preparation of plasmid vector pCACPNM213

The ATP-binding cassette gene was amplified from *Chlamydia pneumoniae* genomic DNA strain CWLO29 by polymerase chain reaction (PCR) using a 5' primer (5' ATAAGAATGCGGCCGCCACC**ATGAAGATGCATAGGCTTAAACC** 3'; SEQ ID No:21) and a 3' primer (5' GCGCCGGATCCC**ACTTAAGATATCGATATTTTTGAG** 3'; SEQ ID No:22). The 5' primer contains a NotI restriction site, a ribosome binding site, an initiation codon and a sequence at the 5' end of the ATP-binding cassette protein coding sequence. The 3' primer includes the sequence encoding the C-terminal sequence of the ATP-binding cassette protein gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 21) with transcription under control of the human CMV promoter.

### B. Preparation of plasmid vector pCACPNM882

The secretory locus ORF gene was amplified from *Chlamydia pneumoniae* genomic DNA strain CWLO29 by polymerase chain reaction (PCR) using a 5' primer (5' ATAAGAATGCGGCCGCCACC**ATGCGGTTGGGAAATAAGCCTATGC** 3'; SEQ ID No:23) and a 3' primer (5' GCGCCGGTACCG**TAATTTAATACTCTTTGAAGGGC** 3'; SEQ ID No:24). The 5' primer contains a NotI restriction site, a ribosome binding site, an initiation codon and a sequence at the 5' end of the secretory locus ORF coding sequence. The 3' primer includes the sequence encoding the C-terminal sequence of the secretory locus ORF protein and a KpnI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and KpnI and cloned into the pCA-Myc-His eukaryotic expression vector described in Example 2 (Figure 22) with transcription under control of the human CMV promoter.

### C. Preparation of plasmid vector pCACPNM208

The endopeptidase gene was amplified from *Chlamydia pneumoniae* genomic DNA strain CWLO29 by polymerase chain reaction (PCR) using a 5' primer
(5' ATAAGAATGCGGCCGCCACCATGCTCACCCTAGGCTTGGAAAGTTCTTG 3'; SEQ ID No:25) and a 3' primer
(5' GCTTTGGAGGATCCCCGGAGAGGCTAAGGAGAATGG 3'; SEQ ID No:26).
The 5' primer contains a NotI restriction site, a ribosome binding site, an initiation codon and a sequence at the 5' end of the endopeptidase protein coding sequence. The 3' primer includes the sequence encoding the C-terminal sequence of the endopeptidase protein gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 23) with transcription under control of the human CMV promoter.

### D. Preparation of plasmid vector pCACPNM1096

The protease gene was amplified from *Chlamydia pneumoniae* genomic DNA strain CWLO29 by polymerase chain reaction (PCR) using a 5' primer (5' ATAAGAATGCGGCCGCCACC**ATGAAAAAAGGGAAATTAGGAGCC** 3'; SEQ ID No:27) and a 3' primer (5' GCGCCGGATCCC**CGAAGCAGAAGTCGTTGTGGG** 3'; SEQ ID No:28). The 5' primer contains a NotI restriction site, a ribosome binding site, an initiation codon and a sequence at the 5' end of the protease protein coding sequence. The 3' primer includes the sequence encoding the C-terminal sequence of the protease protein gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 24) with transcription under control of the human CMV promoter.

### E. Preparation of plasmid vector pCACPNM1097

The metalloprotease gene was amplified from *Chlamydia pneumoniae* genomic DNA strain CWLO29 by polymerase chain reaction (PCR) using a 5' primer (5' ATAAGAATGCGGCCGCCACC**ATGAGAAAACTTATTTTATGCAATCCTA** 3'; SEQ ID No:29) and a 3' primer (5' GCGCCGGATCCC**AGAACAACGGAGTTCTTTTGG** 3'; SEQ ID No:30). The 5' primer contains a NotI restriction site, a ribosome binding site, an initiation codon and a sequence at the 5' end of the metalloprotease protein coding sequence. The 3' primer includes the sequence encoding the C-terminal sequence of the metalloprotease protein gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 25) with transcription under control of the human CMV promoter.

### F. Preparation of plasmid vector pCACPNM908

The CLP protease ATPase gene was amplified from *Chlamydia pneumoniae* genomic DNA strain CWLO29 by polymerase chain reaction (PCR) using a 5' primer (5' ATAAGAATGCGGCCGCCACC**ATGAATAAAAAAAATCTAACTATTTG** 3'; SEQ ID No:31) and a 3' primer (5' GCGCCGGATCCC**AGCGATAGCTTCTGGGGTCC** 3'; SEQ ID No:32). The 5' primer contains a NotI restriction site, a ribosome binding site, an initiation codon and a sequence at the 5' end of the CLP protease ATPase protein coding sequence. The 3' primer includes the sequence encoding the C-terminal sequence of the CLP protease ATPase gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 26) with transcription under control of the human CMV promoter.

### G. Preparation of plasmid vector pCACPNM909

The gene encoding CLP protease subunit was amplified from *Chlamydia pneumoniae* genomic DNA strain CWLO29 by polymerase chain reaction (PCR) using a 5' primer (5' ATAAGAATGCGGCCGCCACC**ATGACACTGGTACCCTATGTTG** 3'; SEQ ID No:33) and a 3' primer (5' GCGCCGGATCCC**AGTGCTACTTGTATCCTTATTAG** 3'; SEQ ID No:34). The 5' primer contains a NotI restriction site, a ribosome binding site, an initiation codon and a sequence at the 5' end of the CLP protease subunit coding sequence. The 3' primer includes the sequence encoding the C-terminal sequence of the CLP protease subunit gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 27) with transcription under control of the human CMV promoter.

### H. Preparation of plasmid vector pCACPNM440

The translycolase / transpeptidase gene was amplified from *Chlamydia pneumoniae* genomic DNA strain CWLO29 by polymerase chain reaction (PCR) using a 5' primer (5' ATAAGAATGCGGCCGCCACC**ATGAGCTACCGTAAACGTTCGACTC** 3'; SEQ ID No:35) and a 3' primer (5' GCGCCGGATCCC**CCTCGTTCCCCCTTGTTTCGGAG** 3'; SEQ ID No:36). The 5' primer contains a NotI restriction site, a ribosome binding site, an initiation codon and a sequence at the 5' end of the translycolase / transpeptidase coding sequence. The 3' primer includes the sequence encoding the C-terminal sequence of the translycolase / transpeptidase gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 28) with transcription under control of the human CMV promoter.

### I. Preparation of plasmid vector pCACPNM459

The gene encoding CLPc protease was amplified from *Chlamydia pneumoniae* genomic DNA strain CWLO29 by polymerase chain reaction (PCR) using a 5' primer (5' ATAAGAATGCGGCCGCCACC**ATGTTTGAGAAGTTCACTAATAGAGC** 3'; SEQ ID No:37) and a 3' primer (5' GCGCCGGTACCG**TGATTCCAAGTGAGGGCTAGGG** 3'; SEQ ID No:38). The 5' primer contains a NotI restriction site, a ribosome binding site, an initiation codon and a sequence at the 5' end of the CLPc protease coding sequence. The 3' primer includes the sequence encoding the C-terminal sequence of the CLPc protease gene and a KpnI restriction site. The stop codon was excluded and an additional nucleotide was inserted to obtain an in-frame fusion with the Histidine tag.

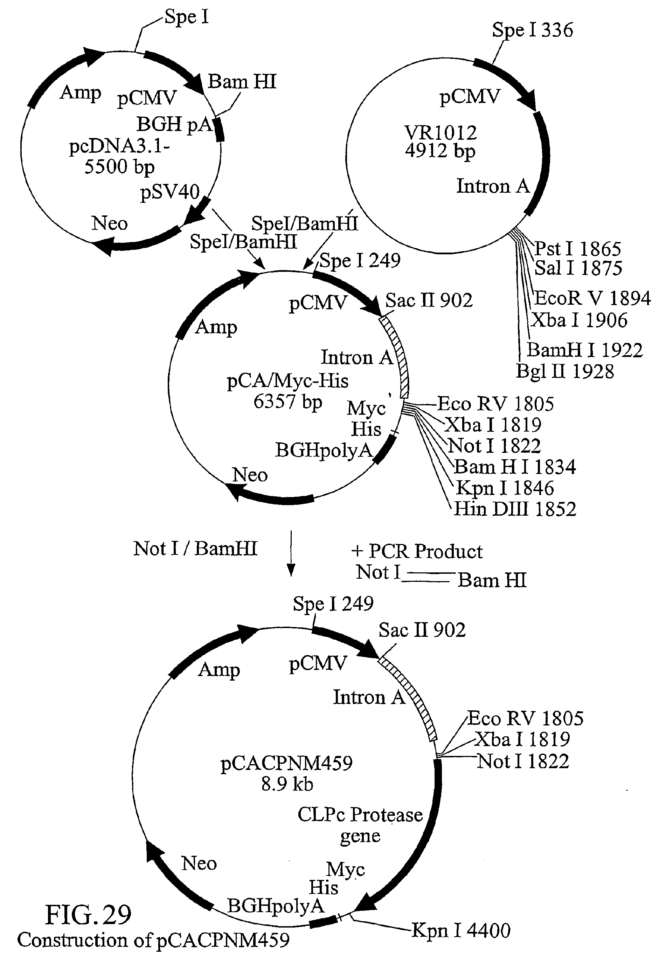

After amplification, the PCR fragment was purified using QIAquick^{™} PCR purification kit (Qiagen), digested with NotI and KpnI and cloned into the pCA-Myc-His eukaryotic expression vector described in Example 2 (Figure 29) with transcription under control of the human CMV promoter.

### J. Preparation of plasmid vector pCACPNM708

The thioredoxin gene was amplified from *Chlamydia pneumoniae* genomic DNA strain CWLO29 by polymerase chain reaction (PCR) using a 5' primer (5' ATAAGAATGCGGCCGCCACC**ATGGTAAAGATCATATCAAGTG** 3'; SEQ ID No:39) and a 3' primer (5' GCGCCGGATCCC**AGCGTGCTTATTGATAAG** 3'; SEQ ID No:40). The 5' primer contains a NotI restriction site, a ribosome binding site, an initiation codon and a sequence at the 5' end of the thioredoxin coding sequence. The 3' primer includes the sequence encoding the C-terminal sequence of the thioredoxin gene and a BamHI restriction site. The stop codon was excluded and an additional nucleotide was inserted to obtain an in-frame fusion with the Histidine tag.

After amplification, the PCR fragment was purified using QIAquick^{™} PCR purification kit (Qiagen), digested with NotI and BamHI and cloned into the pCA-Myc-His eukaryotic expression vector described in Example 2 (Figure 30) with transcription under control of the human CMV promoter.

### Example 2:

Plasmid pcDNA3.1(-)Myc-His C (Invitrogen) was restricted with SpeI and BamHI to remove the CMV promoter and the remaining vector fragment was isolated. The CMV promoter and intron A from plasmid VR-1012 (Vical) was isolated on a SpeI / BamHI fragment. The fragments were ligated together to produce plasmid pCA/Myc-His.

The NotI/BamHI restricted PCR fragment containing the ATP-binding cassette gene was ligated into the NotI and BamHI restricted plasmid pCA/Myc-His to produce plasmid pCACPNM213 (Figure 21).

The NotI/KpnI restricted PCR fragment containing the Secretory locus ORF gene was ligated into the NotI and KpnI restricted plasmid pCA/Myc-His to produce plasmid pCACPNM882 (Figure 22).

The NotI/BamHI restricted PCR fragment containing the endopeptidase gene was ligated into the NotI and BamHI restricted plasmid pCA/Myc-His to produce plasmid pCACPNM208 (Figure 23).

The NotI/BamHI restricted PCR fragment containing the Protease gene was ligated into the NotI and BamHI restricted plasmid pCA/Myc-His to produce plasmid pCACPNM1096 (Figure 24).

The NotI/BamHI restricted PCR fragment containing the Metalloprotease gene was ligated into the NotI and BamHI restricted plasmid pCA/Myc-His to produce plasmid pCACPNM1097 (Figure 25).

The NotI/BamHI restricted PCR fragment containing the CLP protease ATPase gene was ligated into the NotI and BamHI restricted plasmid pCA/Myc-His to produce plasmid pCACPNM908 (Figure 26).

The NotI/BamHI restricted PCR fragment containing the CLP protease subunit gene was ligated into the NotI and BamHI restricted plasmid pCA/Myc-His to produce plasmid pCACPNM909 (Figure 27).

The NotI/BamHI restricted PCR fragment containing the transglycolase/transpeptidase gene was ligated into the NotI and BamHI restricted plasmid pCA/Myc-His to produce plasmid pCACPNM440 (Figure 28).

The NotI/KpnI restricted PCR fragment containing the CLPc protease gene was ligated into the NotI and KpnI restricted plasmid pCA/Myc-His to produce plasmid pCACPNM459 (Figure 29).

The NotI/BamHI restricted PCR fragment containing the Thioredoxin gene was ligated into the NotI and BamHI restricted plasmid pCA/Myc-His to produce plasmid pCACPNM708 (Figure 30).

Each of the resulting plasmids pCACPNM213, pCACPNM882, pCACPNM208, pCACPNM1096, pCACPNM1097, pCACPNM909, pCACPNM440, pCACPNM459 and pCACPNM708, was transferred by electroporation into *E. coli* XL-1 blue (Stratagene) which was grown in LB broth containing 50 µg/ml carbenicillin. The plasmid was isolated by the Endo Free Plasmid Giga Kit^{™} (Qiagen) large scale DNA purification system. DNA concentration was determined by absorbance at 260 nm and the plasmid was verified after gel electrophoresis and ethidium bromide staining by comparison to molecular weight standards. The 5' and 3' ends of the gene were verified by sequencing using a LiCor model 4000 L DNA sequencer and IRD-800 labelled primers.

### Example 3:

This example illustrates the immunization of mice to achieve protection against an intranasal challenge of *C. pneumoniae.*

It has been previously demonstrated (Yang *et al.* Infect. Immun. May 1993. 61(5):2037-40) that mice are susceptible to intranasal infection with different isolates of *C. pneumoniae.* Strain AR-39 (Grayston *et al* (1990) Journal of Infectious Diseases 161:618-625) was used in Balb/c mice as a challenge infection model to examine the capacity of *Chlamydia* gene products delivered as naked DNA to elicit a protective response against a sublethal *C. pneumoniae* lung infection. Protective immunity is defined as an accelerated clearance of pulmonary infection.

Groups of 7 to 9 week old male Balb/c mice (8 to 10 per group) were immunized intramuscularly (i.m.) plus intranasally (i.n.) with plasmid DNA containing each of the *C. pneumoniae* protein gene as described in Examples 1 and 2. Saline or the plasmid vector lacking an inserted Chlamydial gene was given to groups of control animals.

For i.m. immunization, alternate left and right quadriceps were injected with 100µg of DNA in 50µl of PBS on three occasions at 0, 3 and 6 weeks. For i.n. immunization, anaesthetized mice were aspirated 50µl of PBS containing 50 µg DNA on three occasions at 0, 3 and 6 weeks. At week 8, immunized mice were inoculated i.n. with 5 x 10⁵ IFU of *C. pneumoniae,* strain AR39 in 100µl of SPG buffer to test their ability to limit the growth of a sublethal *C. pneumoniae* challenge.

Lungs were taken from mice at day 9 post-challenge and immediately homogenised in SPG buffer (7.5% sucrose, 5mM glutamate, 12.5mM phosphate pH7.5). The homogenate was stored frozen at -70°C until assay. Dilutions of the homogenate were assayed for the presence of infectious *Chlamydia* by inoculation onto monolayers of susceptible cells. The inoculum was centrifuged onto the cells at 3000rpm for 1 hour, then the cells were incubated for three days at 35°C in the presence of 1µg/ml cycloheximide. After incubation the monolayers were fixed with formalin and methanol then immunoperoxidase stained for the presence of Chlamydial inclusions using convalescent sera from rabbits infected with *C. pneumoniae* and metal-enhanced DAB as a peroxidase substrate.

### A. Immunization with pCACPNM213

Figure 31 and Table 1 show that mice immunized i.n. and i.m. with pCACPNM213 had chlamydial lung titers less than 60,000 in 3 of 6 cases at day 9 (mean 51,833) whereas the range of values for control mice sham immunized with saline was 34,200-377,800 IFU/lung (mean 141,450) at day 9. DNA immunisation *per se* was not responsible for the observed protective effect since another plasmid DNA construct, pCACPNM102, failed to protect, with lung titers in immunised mice similar to those obtained for saline-immunized control mice (mean 153,283). The construct pCACPNM102 is identical to pCACPNM213 except that the nucleotide sequence encoding the putative ATP-binding cassette is replaced with a *C. pneumoniae* nucleotide sequence encoding an unrelated ATP Synthase Subunit I protein.

### B. Immunization with pCACPNM882

Figure 32 and Table 3 show that mice immunized i.n. and i.m. with pCACPNM882 had chlamydial lung titers less than 73,000 in 4 of 6 cases at day 9 (mean 77,500) whereas the range of values for control mice sham immunized with saline was 56,000-424,000 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM882 except that the nucleotide sequence encoding the putative Secretory locus ORF is replaced with a *C. pneumoniae* nucleotide sequence encoding an unrelated substrate binding protein.

### C. Immunization with pCACPNM208

Figure 33 and Table 4 show that mice immunized i.n. and i.m. with pCACPNM208 had chlamydial lung titers less than 67,000 in 4 of 6 cases at day 9 (mean 81,766) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM208 except that the nucleotide sequence encoding the putative Endopeptidase is replaced with a *C. pneumoniae* nucleotide sequence encoding an unrelated protein.

### D Immunization with pCACPNM1096

Figure 34 and Table 6 show that mice immunized i.n. and i.m. with pCACPNM1096 had chlamydial lung titers less than 30,000 in 5 of 6 cases at day 9 (mean 25,000) whereas.the range of values for control mice sham immunized with saline was 51,300-170,000 IFU/lung (mean 105,150) at day 9. DNA immunisation *per se* was not responsible for the observed protective effect since another plasmid DNA construct, pCACPNM553, failed to protect, with lung titers in immunised mice similar to those obtained for saline-immunized control mice (mean 111,583). The construct pCACPNM553 is identical to pCACPNM1096 except that the nucleotide sequence encoding the putative Protease is replaced with a *C. pneumoniae* nucleotide sequence encoding an unrelated protease.

### E. Immunization with pCACPNMI097

Figure 35 and Table 8 show that mice immunized i.n. and i.m. with pCACPNM1097 had chlamydial lung titers less than 51,000 in 4 of 6 cases at day 9 (mean 62,883) whereas the range of values for control mice sham immunized with saline was 90,000-242,100 IFU/lung (mean 166,287) at day 9. DNA immunisation *per se* was not responsible for the observed protective effect since another plasmid DNA construct, pCACPNM1061, failed to protect, with lung titers in immunised mice similar to those obtained for saline-immunized control mice (mean 148,566). The construct pCACPNM1061 is identical to pCACPNM1097 except that the nucleotide sequence encoding the putative Metalloprotease is replaced with a *C. pneumoniae* nucleotide sequence encoding an unrelated zinc Metalloprotease.

### F. Immunization with pCACPNM908

Figure 36 and Table 10 show that mice immunized i.n. and i.m. with pCACPNM908 had chlamydial lung titers less than 40,000 in 3 of 6 cases at day 9 (mean 68,333) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM908 except that the nucleotide sequence encoding the putative CLP protease ATPase is replaced with a *C. pneumoniae* nucleotide sequence encoding an unrelated signal peptidase.

### G. Immunization with pCACPNM909

Figure 37 and Table 12 show that mice immunized i.n. and i.m. with pCACPNM909 had chlamydial lung titers less than 85,000 in 5 of 6 cases at day 9 (mean 87,683) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM909 except that the nucleotide sequence encoding the putative CLP protease subunit is replaced with a *C. pneumoniae* nucleotide sequence encoding an unrelated signal peptidase.

### H. Immunization with pCACPNM440

Figure 38 and Table 14 show that mice immunized i.n. and i.m. with pCACPNM440 had chlamydial lung titers less than 98,000 in 4 of 6 cases at day 9 (mean 87,616) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647 failed to protect, with lung titers in immunised mice similar to those obtained for saline-immunized control mice (mean 143,883). The construct pCACPNM647 is identical to pCACPNM440 except that the nucleotide sequence encoding the putative transglycolase /transpeptidase gene is replaced with a *C. pneumoniae* nucleotide sequence encoding an unrelated gene.

### I. Immunization with pCACPNM459

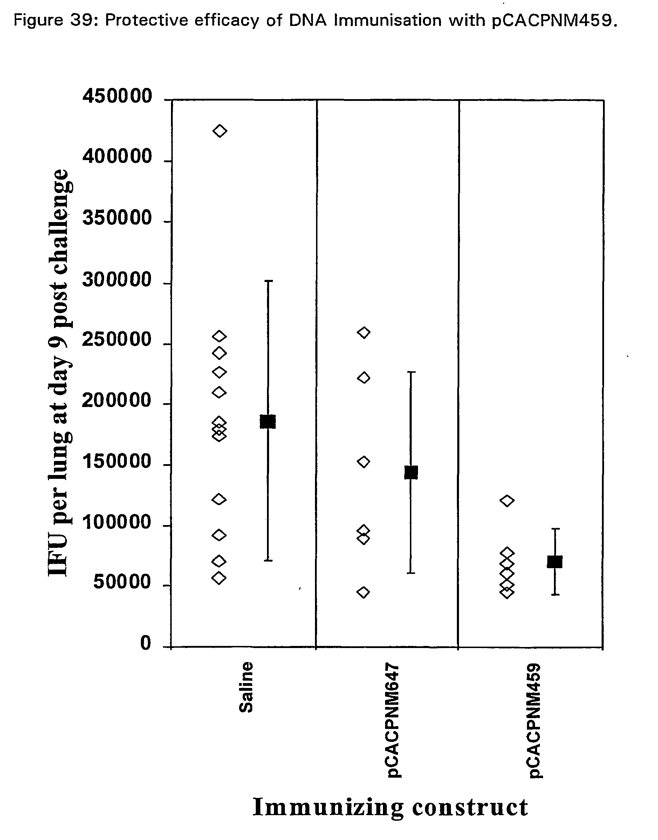

Figure 39 and Table 16 show that mice immunized i.n. and i.m. with pCACPNM459 had chlamydial lung titers less than 70,000 in 4 of 6 cases at day 9 (mean 70,516) whereas the range of values for control mice sham immunized with saline was 56,000-424,100 IFU/lung (mean 186,291) at day 9. DNA immunisation *per se* was not responsible for the observed protective effect since another plasmid DNA construct, pCACPNM647, failed to protect, with lung titers in immunised mice similar to those obtained for saline-immunized control mice (mean 143,883). The construct pCACPNM647 is identical to pCACPNM459 except that the nucleotide sequence encoding the putative CLPc protease whose sequence is set forth in SEQ ID No: 18 is replaced with a *C. pneumoniae* nucleotide sequence encoding an unrelated gene.

### J. Immunization with pCACPNM708

Figure 40 and Table 18 show that mice immunized i.n. and i.m. with pCACPNM708 had chlamydial lung titers less than 52,000 in 4 of 6 cases at day 9 (mean 73,916) whereas the range of values for control mice sham immunized with saline was 56,000-424,100 IFU/lung (mean 207,962) at day 9. DNA immunisation *per se* was not responsible for the observed protective effect since another plasmid DNA construct, pCACPNM569, failed to protect, with lung titers in immunised mice similar to those obtained for saline-immunized control mice (mean 215,600). The construct pCACPNM569 is identical to pCACPNM708 except that the nucleotide sequence encoding the putative thioredoxin is replaced with a *C. pneumoniae* nucleotide sequence encoding an unrelated *C. pneumoniae* gene.

### Example 4:

This example illustrates the identification of B- and T-cell epitopes in proteins as expressed from each of pCACPNM213, pCACPNM882, pCACPNM208, pCACPNM1096, pCACPNM1097, pCACPNM909, pCACPNM440, pCACPNM459 and pCACPNM708.

B-cell epitopes were identified based on the product of flexibilty and hydrophobicity propensities using the program SEQSEE (Wishart DS, et al. "SEQSEE: a comprehensive program suite for protein sequence analysis." *Comput Appl Biosci.* 1994 Apr;10(2):121-32) to identify external surface features (epitopes). T-cell epitopes for HLA-A0201 MHC subclass were identified based on the algorithm of Parker et al. 1995 (Parker KC, et al. "Peptide binding to MHC class I molecules: implications for antigenic peptide prediction." *Immunol Res* 1995; 14 (1) :34-57). These epitopes are shown in Tables 2, 5, 7, 9, 11, 13, 15, 17 and 19 and SEQ ID NOs: 41 to 74.

**Table 1**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM102 | pCACPNM213 |
| | Day 9 | Day 9 | Day 9 |
| | | | |
| 1 | 64900 | 207500 | 54200 |
| 2 | 116500 | 166500 | 10600 |
| 3 | 34200 | 114700 | 67400 |
| 4 | 377800 | 167400 | 32000 |
| 5 | 86200 | 179700 | 66900 |
| 6 | 206200 | 83900 | 79900 |
| 7 | 142600 | | |
| 8 | 103200 | | |
| | | | |
| | | | |
| | | | |
| MEAN | 141450 | 153283.333 | 51833.3333 |
| SD | 108598.7 | 45417.99 | 25908.35 |
| | | | |
| Wilcoxon p | | 1.655 | 0.0293 |

**Table 2: Identified B- T-cell epitopes from CPNM213**

| **B cell epitope** | **T cell epitope** |
|---|---|
| 188 VHHTLRESYKKGTPPST | 434 WIAEYVSPV (SEQ ID No: 43) |
| (SEQ ID No: 41) | |
| 345 NLQKEISTEERQTKAR | |
| (SEQ ID No: 42) | |

**Table 3**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM647 | pCACPNM882 |
| | Day 9 | Day 9 | Day 9 |
| | | | |
| 1 | 209800 | 45100 | 18100 |
| 2 | 70000 | 222000 | 130300 |
| 3 | 226700 | 152500 | 72900 |
| 4 | 178900 | 89000 | 53500 |
| 5 | 424100 | 95500 | 63400 |
| 6 | 242200 | 259200 | 126800 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| | | | |
| MEAN | 186291.667 | 143883.333 | 77500 |
| SD | 100263.3 | 83169.31 | 43686.75 |
| | | | |
| Wilcoxon p | | 0.4936 | 0.0182 |

**Table 4**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM647 | pCACPNM208 |
| | Day 9 | Day 9 | Day 9 |
| | | | |
| 1 | 209800 | 45100 | 142500 |
| 2 | 70000 | 222000 | 66900 |
| 3 | 226700 | 152500 | 58200 |
| 4 | 178900 | 89000 | 46500 |
| 5 | 424100 | 95500 | 110900 |
| 6 | 242200 | 259200 | 65600 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| MEAN | 186291.667 | 143883.333 | 81766.6667 |
| SD | 100263.3 | 83169.31 | 36929.10 |
| | | | |
| Wilcoxon p | | 0.4936 | 0.0135 |

**Table 5 Identified B- T-cell epitopes from CPNM208**

| **B cell epitope** | **T cell epitope** |
|---|---|
| 220 KGNNSSPRSPAP | 67 LLIEDMDLI |
| (SEQ ID No: 44) | (SEQ ID No: 46) |
| 313 GENFQKNSS | 66 NLLIEDMDL |
| (SEQ ID No: 45) | (SEQ ID No:47) |

**Table 6**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM553 | pCACPNM1096 |
| | Day 9 | Day 9 | Day 9 |
| | | | |
| 1 | 136900 | 135600 | 21000 |
| 2 | 81700 | 112600 | 9700 |
| 3 | 119400 | 88600 | 28500 |
| 4 | 58500 | 121700 | 52000 |
| 5 | 110600 | 165300 | 17200 |
| 6 | 51300 | 45700 | 21600 |
| 7 | 170000 | | |
| 8 | 112800 | | |
| | | | |
| | | | |
| MEAN | 105150 | 111583.333 | 25000 |
| SD | 39876.3 | 41071.91 | 14585.88 |
| | | | |
| Wilcoxon p | | 1.245 | 0.0013 |

**Table 7 Identified B- T-cell epitopes from CPNM1096**

| **B cell epitope** | **T cell epitope** |
|---|---|
| 328 TDLEGLEEDHKDSPWE | 135 YLGDEILEV |
| (SEQ ID No: 48) | (SEQ ID No: 50) |
| 589 SENAKKSEEQTSPQETPE | 373 YLYSLLSML |
| (SEQ ID No: 49) | (SEQ ID No: 51) |

**Table 8**

| MOUSE | BACTERIAL LOAD (INCLUSION FORMING | | |
|---|---|---|---|
| | UNITS PER LUNG) IN THE LUNGS OF | | |
| | BALB/C MICE IMMUNIZED WITH | | |
| | VARIOUS DNA IMMUNIZATION | | |
| | CONSTRUCTS | | |
| | IMMUNIZING CONSTRUCT | | |
| | Saline | pCACPNM106 1 | pCACPNM1097 |
| | Day 9 | Day 9 | Day 9 |
| | | | |
| 1 | 232900 | 120800 | 50300 |
| 2 | 168100 | 184100 | 43900 |
| 3 | 105500 | 95600 | 65200 |
| 4 | 173100 | 147500 | 157900 |
| 5 | 90000 | 218700 | 22800 |
| 6 | 242100 | 124700 | 37200 |
| 7 | 183700 | | |
| 8 | 134900 | | |
| | | | |
| MEAN | 166287.5 | 148566.667 | 62883.3333 |
| SD | 54821.4 | 45450.00 | 48618.00 |
| | | | |
| Wilcoxon p | | 0.662 | 0.0047 |

**Table 9 Identified B- T-cell epitopes from CPNM1097**

| **B cell epitope** | **T cell epitope** |
|---|---|
| 198 TTNRQKAL | 207 SVLSRVNYV |
| (SEQ ID No: 52) | (SEQ ID No: 54) |
| 221 VNSSNSNRLRE | 279 KLSSLIPGL |
| (SEQ ID No: 53) | (SEQ ID No: 55) |
| | 118 ILIGHKKHV |
| | (SEQ ID No: 56) |

**Table 10**

| MOUSE | BACTERIAL LOAD (INCLUSION FORMING | | |
|---|---|---|---|
| | UNITS PER LUNG) IN THE LUNGS OF | | |
| | BALB/C MICE IMMUNIZED WITH | | |
| | VARIOUS DNA IMMUNIZATION | | |
| | CONSTRUCTS | | |
| | IMMUNIZING CONSTRUCT | | |
| | Saline | pCACPNM569 | pCACPNM908 |
| | Day 9 | Day 9 | Day 9 |
| | | | |
| 1 | 209800 | 142800 | 37300 |
| 2 | 70000 | 420700 | 85000 |
| 3 | 226700 | 116600 | 35700 |
| 4 | 178900 | 161300 | 39700 |
| 5 | 424100 | 89200 | 123400 |
| 6 | 242200 | 363000 | 88900 |
| 7 | 256000 | | |
| 8 | 56000 | | |
| | | | |
| MEAN | 207962.5 | 215600 | 68333.3333 |
| SD | 115585.8 | 139870.70 | 36279.40 |
| | | | |
| Wilcoxon p | | 0.8518 | 0.02 |

**Table 11 Identified B- T-cell epitopes from CPNM908**

| **B cell epitope** | **T cell epitope** |
|---|---|
| 226 PPKGGRKHPNQEYI | 137 KILDVPFTI |
| (SEQ ID No: 57) | (SEQ ID No: 59) |
| 273 SDDQADLSQKTRDH | 168 LLQAADYDV- |
| (SEQ ID No: 58) | (SEQ ID No: 60) |

**Table 12**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM569 | PCACPNM909 |
| | Day 9 | Day 9 | Day 9 |
| | | | |
| 1 | 209800 | 142800 | 206700 |
| 2 | 70000 | 420700 | 84700 |
| 3 | 226700 | 116600 | 81100 |
| 4 | 178900 | 161300 | 56700 |
| 5 | 424100 | 89200 | 53900 |
| 6 | 242200 | 363000 | 43000 |
| 7 | 256000 | | |
| 8 | 56000 | | |
| | | | |
| MEAN | 207962.5 | 215600 | 87683.3333 |
| SD | 115585.8 | 139870.70 | 60522.87 |
| | | | |
| Wilcoxon p | | 0.8518 | 0.0426 |
| | | | |
| | | | |

**Table 13 Identified B- T-cell epitopes from CPNM909**

| **B cell epitope** | **T cell epitope** |
|---|---|
| 107 GTKGKRHAL | 76 AIYDTIRFL |
| (SEQ ID No: 61) | (SEQ ID No: 63) |
| 193 AKETNKDTSST | |
| (SEQ ID No: 62) | |

**Table 14**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM647 | pCACPNM440 |
| | Day 9 | Day 9 | Day 9 |
| | | | |
| 1 | 209800 | 45100 | 97200 |
| 2 | 70000 | 222000 | 92500 |
| 3 | 226700 | 152500 | 104400 |
| 4 | 178900 | 89000 | 60900 |
| 5 | 424100 | 95500 | 40400 |
| 6 | 242200 | 259200 | 130300 |
| 7 | 256000 | | |
| 8 | 56000 | | |
| 9 | 173600 | | |
| 10 | 185000 | | |
| 11 | 121400 | | |
| 12 | 91800 | | |
| | | | |
| | | | |
| | | | |
| MEAN | 186291.667 | 143883.333 | 87616.6667 |
| SD | 100263.3 | 83169.31 | 32132.31 |
| | | | |
| Wilcoxon p | | 0.4936 | 0.0415 |

**Table 15 Identified B- T-cell epitopes from CPNM440**

| **B cell epitope** | **T cell epitope** |
|---|---|
| 287 DPTNYKEYFNNKERIEHTK | 40 ALGQHEFCV |
| (SEQ ID No: 64) | (SEQ ID No: 66) |
| 637 KRLYEEWNRSPKQGGTR | 456 ILATGIQMV |
| (SEQ ID No: 65) | (SEQ ID No: 67) |

**Table 16**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM647 | pCACPNM459 |
| | Day 9 | Day 9 | Day 9 |
| | | | |
| 1 | 209800 | 45100 | 77400 |
| 2 | 70000 | 222000 | 60700 |
| 3 | 226700 | 152500 | 121000 |
| 4 | 178900 | 89000 | 68500 |
| 5 | 424100 | 95500 | 44800 |
| 6 | 242200 | 259200 | 50700 |
| 7 | 256000 | | |
| 8 | 56000 | | |
| 9 | 173600 | | |
| 10 | 185000 | | |
| 11 | 121400 | | |
| 12 | 91800 | | |
| | | | |
| MEAN | 186291.667 | 143883.333 | 70516.6667 |
| SD | 100263.3 | 83169.31 | 27387.69 |
| | | | |
| Wilcoxon p | | 0.4936 | 0.0047 |

**Table 17 Identified B- T-cell epitopes from CPNM459**

| **B cell epitope** | **T cell epitope** |
|---|---|
| 467 DEEKKLRERLQSMKQEWENHKEEHQ | 565 FLFLGPTGV |
| (SEQ ID No: 68) | (SEQ ID No: 70) |
| 548 IRRSRTGIKDPNRPTG | 410 FLPDKAIDL |
| (SEQ ID No: 69) | (SEQ ID No: 71) |

**Table 18**

| MOUSE | BACTERIAL LOAD (INCLUSION | | |
|---|---|---|---|
| | FORMING UNITS PER LUNG) IN THE | | |
| | LUNGS OF BALB/C MICE IMMUNIZED | | |
| | WITH VARIOUS DNA IMMUNIZATION | | |
| | CONSTRUCTS | | |
| | IMMUNIZING CONSTRUCT | | |
| | Saline | pCACPNM569 | pCACPNM708 |
| | Day 9 | Day 9 | Day 9 |
| | | | |
| 1 | 209800 | 142800 | 95100 |
| 2 | 70000 | 420700 | 189600 |
| 3 | 226700 | 116600 | 29000 |
| 4 | 178900 | 161300 | 51400 |
| 5 | 424100 | 89200 | 31500 |
| 6 | 242200 | 363000 | 46900 |
| 7 | 256000 | | |
| 8 | 56000 | | |
| | | | |
| MEAN | 207962.5 | 215600 | 73916.6667 |
| SD | 115585.8 | 139870.70 | 61457.22 |
| | | | |
| Wilcoxon p | | 0.8518 | 0.0127 |

**Table 19 Identified B- T-cell epitopes from pCPNM708**

| **B cell epitope** | **T cell epitope** |
|---|---|
| 54 NIDENSKPAETYE | 40 NLAAELPHV |
| (SEQ ID No: 72) | (SEQ ID No: 73) |
| | 74 ILFKDGNEV |
| | (SEQ ID No: 74) |

## Claims

1. A vaccine, which comprises a physiologically acceptable diluent or carrier for use in a vaccine, and any one of :
(a) at least one first nucleic acid in a vaccine vector wherein the first nucleic acid encodes a first polypeptide;
(b) the first polypeptide;
(c) a fusion protein comprising the first polypeptide and a second polypeptide; or
(d) a hybrid nucleic acid in a vaccine vector wherein the hybrid nucleic acid comprises the first nucleic acid fused in-frame with a nucleic acid encoding the second polypeptide;
wherein each first nucleic acid is capable of being expressed and wherein the first polypeptide is selected from the group consisting of :
(i) a polypeptide whose sequence is set forth in SEQ ID No:18;
(ii) a polypeptide which is at least 75% identical in amino acid sequence to SEQ ID No: 18; and
(iii) an immunogenic fragment comprising at least 12 consecutive amino acids from SEQ ID No: 18.

2. The vaccine of claim 1, wherein the vaccine comprises the at least one first nucleic acid in a vaccine vector, and wherein the first nucleic acid is selected from the group consisting of:
(i) a nucleic acid which encodes a polypeptide wherein the polypeptide is at least 75% identical in amino acid sequence to the sequence set forth in SEQ ID No: 18;
(ii) a nucleic acid which encodes a polypeptide whose amino acid sequence is set forth in SEQ ID No:18; and
(iii) a nucleic acid which encodes an immunogenic fragment wherein the fragment comprises at least 12 consecutive amino acids from SEQ ID No: 18.

3. The vaccine according to claim 1, wherein the vaccine comprises the hybrid nucleic acid in a vaccine vector, and wherein the hybrid nucleic acid comprises a first nucleic acid selected from the group consisting of:
(i) a nucleic acid which encodes a polypeptide wherein the polypeptide is at least 75% identical in amino acid sequence to the sequence set forth in SEQ ID No: 18;
(ii) a nucleic acid which encodes a polypeptide whose amino acid sequence is set forth in SEQ ID No: 18; and
(iii) a nucleic acid which encodes an immunogenic fragment wherein the fragment comprises at least 12 consecutive amino acids from SEQ ID No: 18.

4. The vaccine according to claim 2 or 3 wherein the nucleic acid defined in (ii) comprises the nucleic acid set forth in SEQ ID No: 17; and wherein the nucleic acid sequence defined in (iii) comprises at least 36 consecutive nucleotides from the nucleic acid sequence set forth in SEQ ID No: 17.

5. The vaccine of any one of claims 1 to 4 wherein the second polypeptide is a heterologous signal peptide.

6. The vaccine of any one of claims 1 to 4 wherein the second polypeptide has adjuvant activity.

7. The vaccine of any one of claims 1 to 6 wherein each first nucleic acid is operably linked to one or more expression control sequences.

8. The vaccine according to any one of claims 1 to 7, wherein the vaccine comprises the first nucleic acid in a vaccine vector or the hybrid nucleic acid in a vaccine vector, and wherein the vaccine further comprises a second nucleic acid encoding an additional polypeptide which enhances the immune response to the first polypeptide.

9. The vaccine of claim 8 wherein the second nucleic acid encodes an additional *Chlamydia* polypeptide.

10. The vaccine according to claim 1, wherein the vaccine comprises the first polypeptide.

11. The vaccine according to claim 1, wherein the vaccine comprises the fusion protein.

12. The vaccine of claim 11 wherein the second polypeptide is a heterologous signal peptide.

13. The vaccine of claim 11 wherein the second polypeptide has adjuvant activity.

14. A vaccine of any one of claims 10 to 13, further comprising an additional polypeptide which enhances the immune response to the first polypeptide.

15. The vaccine of claim 14 wherein the additional polypeptide comprises a Chlamydia polypeptide.

16. The vaccine of any one of claims 1 to 15 wherein the immunogenic fragment comprising at least 12 consecutive amino acids from SEQ ID No: 18 comprises SEQ ID No: 68 or 69.

17. The vaccine of claim 2 which comprises the first nucleic acid, wherein the first nucleic acid encodes the polypeptide of SEQ ID No: 18.

18. The vaccine of claim 17 wherein the first nucleic acid comprises SEQ ID No:17.

19. A pharmaceutical composition comprising the vaccine according to any one of claims 10 to 18 and a pharmaceutically acceptable carrier.

20. Use of an effective amount of the vaccine of any one of claims 1 to 18 in preparing a medicament for preventing or treating *Chlamydia* infection.

21. Use of an effective amount of the composition of claim 19 in preparing a medicament for preventing or treating *Chlamydia* infection.

22. A commercial package comprising the vaccine defined in any one of claims 1 to 18, and instructions for use of the vaccine in eliciting an immunoprotective response in a mammal.

23. A commercial package comprising the vaccine defined in any one of claims 10 to 15, and instructions for use of the vaccine in eliciting an immunoprotective response in a mammal.

24. The vaccine of claim 1 comprising an expression plasmid **characterized in that** it is plasmid pCACPNM459 as shown in figure 29 and **in that** it comprises:
(a) the Ampicilin resistance gene;
(b) the Neomycin resistance gene;
(c) the Cytomegalovirus promoter (pCMV);
(d) the intron A sequence;
(e) the coding sequence of the polynucleotide molecule of SEQ ID No:1 encoding the polypeptide designated CPLc protease;
(f) the BGH poly A sequence; and
(g) the Myc-His sequence.

## Patentansprüche

1. Impfstoff, der ein physiologisch akzeptables Verdünnungsmittel oder einen Träger zur Verwendung in einem Impfstoff umfasst, und jedes von:
(a) mindestens einer erste Nukleinsäure in einem Impfvektor, wobei die erste Nukleinsäure für ein erstes Polypeptid kodiert.
(b) dem ersten Polypeptid;
(c) einem Fusionsprotein, umfassend das erste Polypeptid and ein zweites Polypeptid; oder
(d) einem Hybrid-Nukleinsäure in einem Impfvektor, wobei die Hybrid-Nukleinsäure die erste Nukleinsäure fusioniert in-frame mit einer Nukleinsäure umfasst, die für das zweite Polypeptid kodiert;
wobei jede erste Nukleinsäure in der Lage ist, exprimiert zu werden, und wobei das erste Polypeptid ausgewählt ist aus der Gruppe bestehend aus:
(i) einem Polypeptid, dessen Sequenz in SEQ ID No: 18 angegeben ist;
(ii) einem Polypeptid, das in seiner Aminosäuresequenz zu mindestens 75% identisch zu SEQ ID No: 18 ist; und
(iii) einem immunogenen Fragment umfassend mindestens 12 aufeinander folgende Aminosäuren von SEQ ID No: 18.

2. Impfstoff nach Anspruch 1, wobei der Impfstoff die mindestens erste Nukleinsäure in einem Impfvektor umfasst, und wobei die erste Nukleinsäure ausgewählt ist aus der Gruppe bestehend aus:
(i) einer Nukleinsäure, die für ein Polypeptid kodiert, wobei das Polypeptid in seiner Aminosäuresequenz zu mindestens 75% identisch zu der in SEQ ID No: 18 angegebenen Sequenz ist;
(ii) einer Nukleinsäure, die für ein Polypeptid kodiert, dessen Aminosäuresequenz in SEQ ID No: 18 angegeben ist; und
(iii) einer Nukleinsäure, die für ein immunogenes Fragment kodiert, wobei das Fragment mindestens 12 aufeinander folgende Aminosäuren aus SEQ ID No: 18 umfasst.

3. Impfstoff nach Anspruch 1, wobei der Impfstoff die Hybrid Nukleinsäure in einem Impfvektor umfasst, und wobei die Hybrid-Nukleinsäure eine erste Nukleinsäure umfasst, die ausgewählt ist aus der Gruppe bestehend aus:
(i) einer Nukleinsäure, die für ein Polypeptid kodiert, wobei das Polypeptid in seiner Aminosäuresequenz zu mindestens 75% identisch zu der in SEQ ID No: 18 angegebenen Sequenz ist;
(ii) einer Nukleinsäure, die für ein Polypeptid kodiert, dessen Aminosäuresequenz in SEQ ID No: 18 angegeben ist; und
(iii) einer Nukleinsäure, die für ein immunogenes Fragment kodiert, wobei das Fragment mindestens 12 aufeinander folgende Aminosäuren aus SEQ ID No: 18 umfasst.

4. Impfstoff nach Anspruch 2 oder 3, wobei die in (ii) definierte Nukleinsäure die in SEQ ID No: 17 angegebene Sequenz umfasst, und wobei die Nukleinsäuresequenz der in (iii) definierten Sequenz mindestens 36 aufeinander folgende Nukleotide von der in SEQ ID No: 17 angegebenen Sequenz umfasst.

5. Impfstoff nach einem der Ansprüche 1 bis 4, wobei das zweite Polypeptid ein heterologes Signalpeptid ist.

6. Impfstoff nach einem der Ansprüche 1 bis 4, wobei das zweite Polypeptid eine Adjuvant-Aktivität aufweist.

7. Impfstoff nach einem der Ansprüche 1 bis 6, wobei jede erste Nukleinsäure mit einer oder mehreren Expressions-Kontrollsequenzen operativ verbunden ist.

8. Impfstoff nach einem der Ansprüche 1 bis 7, wobei der Impfstoff die erste Nukleinsäure in einem Impfvektor oder die Hybrid-Nukleinsäure in einem Impfvektor umfasst, und wobei der Impfstoff weiter eine zweite Nukleinsäure umfasst, die für ein zusätzliches Polypeptid ko-diert, das die Immunantwort gegen das erste Polypeptid verstärkt.

9. Impfstoff nach Anspruch 8, wobei die zweite Nukleinsäure für ein zusätzliches *Chlamydia* Polypeptid kodiert.

10. Impfstoff nach Anspruch 1, wobei der Impfstoff das erste Polypeptid umfasst.

11. Impfstoff nach Anspruch 1, wobei der Impfstoff das Fusionsprotein umfasst.

12. Impfstoff nach Anspruch 11, wobei das zweite Polypeptid ein heterologes Signalpeptid ist..

13. Impfstoff nach Anspruch 11, wobei das zweite Polypeptid eine Adjuvant-Aktivität aufweist.

14. Impfstoff nach einem der Ansprüche 10 bis 13, weiter umfassend ein zusätzliches Polypeptid, das die Immunantwort gegen das erste Polypeptid verstärkt.

15. Impfstoff nach Anspruch 14, wobei das zusätzliche Polypeptid ein *Chlamydia* Polypeptid umfasst.

16. Impfstoff nach einem der Ansprüche 1 bis 15, wobei das immunogene Fragment, das mindestens 12 aufeinander folgende Aminosäuren aus SEQ ID No; 18 umfasst, SEQ ID No: 68 oder 69 umfasst.

17. Impfstoff nach Anspruch 2, der die erste Nukleinsäure umfasst, wobei die Nukleinsäure für das Polypeptid von SEQ ID No: 18 kodiert.

18. Impfstoff nach Anspruch 17, wobei die erste Nukleinsäure SEQ ID No: 17 umfasst.

19. Pharmazeutische Zusammensetzung, umfassend den Impfstoff nach einem der Ansprüche 10 bis 18 und einen pharmazeutisch akzeptablen Träger.

20. Verwendung einer effektiven Menge des Impfstoffs nach einem der Ansprüche 1 bis 18 zur Herstellung eines Medikaments zur Verhinderung oder Behandlung von *Chlamydia-*Infektion.

21. Verwendung einer effektiven Menge der Zusammensetzung nach Anspruch 19 zur Herstellung eines Medikaments zur Verhinderung oder Behandlung von Chlamydia-Infektion.

22. Kommerzielle Packung, umfassend den Impfstoff wie definiert in einem der Ansprüche 1 bis 18, und Anweisungen zur Verwendung des Impfstoffs zur Hervorrufung einer immunprotektiven Antwort in einem Säuger.

23. Kommerzielle Packung, umfassend den Impfstoff wie definiert in einem der Ansprüche 10 bis 15, und Anweisungen zur Verwendung des Impfstoffs zur Hervorrufung einer immunprotektiven Antwort in einem Säuger.

24. Impfstoff nach Anspruch 1, umfassend ein Expressionsplasmid, **dadurch gekennzeichnet, dass** dieses Plasmid pCACPNM459 ist, wie in Figur 29 gezeigt, und dass dieses umfasst:
(a) das Ampicillin Resistenzgen;
(b) das Neomycin Resistenzgen:
(c) den Cytomegalovirus Promotor (pCMV);
(d) die Intron A Sequenz;
(e) die kodierende Sequenz des Polynukleotid-Moleküls der SEQ ID No: 1, das für das als CPLc-Protease bezeichnete Polypeptid kodiert;
(f) die BGH poly A-Sequenz; und
(g) die Myc-His-Sequenz.

## Revendications

1. Vaccin, qui comprend un diluant ou véhicule physiologiquement acceptable pour utilisation dans un vaccin, et l'un quelconque de :
(a) au moins un premier acide nucléique dans un vecteur vaccinal dans lequel le premier acide nucléique code pour un premier polypeptide ;
(b) le premier polypeptide ;
(c) une protéine de fusion comprenant le premier polypeptide et un second polypeptide ; ou
(d) un acide nucléique hybride dans un vecteur vaccinal dans lequel l'acide nucléique hybride comprend le premier acide nucléique fusionné dans le cadre avec un acide nucléique codant pour le second polypeptide ;
dans lequel chaque premier acide nucléique est susceptible d'être exprimé et
dans lequel le premier polypeptide est choisi dans le groupe constitué par :
(i) un polypeptide dont la séquence est représentée par SEQ ID N° : 18 ;
(ii) un polypeptide qui est identique à au moins 75 % en séquence d'acides aminés à SEQ ID N° : 18 ; et
(iii) un fragment immunogène comprenant au moins 12 acides aminés consécutifs de SEQ ID N° : 18.

2. Vaccin selon la revendication 1, le vaccin comprenant le au moins un premier acide nucléique dans un vecteur vaccinal, et dans lequel le premier acide nucléique est choisi dans le groupe constitué par :
(i) un acide nucléique qui code pour un polypeptide dans lequel le polypeptide est identique à au moins 75 % en séquence d'acides aminés à la séquence représentée par SEQ ID N° : 18 ;
(ii) un acide nucléique qui code pour un polypeptide dont la séquence d'acides aminés est représentée par SEQ ID N° : 18 ; et
(iii) un acide nucléique qui code pour un fragment immunogène dans lequel le fragment comprend au moins 12 acides aminés consécutifs de SEQ ID N° : 18.

3. Vaccin selon la revendication 1, le vaccin comprenant l'acide nucléique hybride dans un vecteur vaccinal, et dans lequel l'acide nucléique hybride comprend un premier acide nucléique choisi dans le groupe constitué par :
(i) un acide nucléique qui code pour un polypeptide dans lequel le polypeptide est identique à au moins 75 % en séquence d'acides aminés à la séquence représentée par SEQ ID N° : 18 ;
(ii) un acide nucléique qui code pour un polypeptide dont la séquence d'acides aminés est représentée par SEQ ID N° : 18 ; et
(iii) un acide nucléique qui code pour un fragment immunogène dans lequel le fragment comprend au moins 12 acides aminés consécutifs de SEQ ID N° : 18.

4. Vaccin selon la revendication 2 ou 3 dans lequel l'acide nucléique défini en (ii) comprend l'acide nucléique représenté par SEQ ID N° : 17 ; et dans lequel la séquence d'acide nucléique définie en (iii) comprend au moins 36 nucléotides consécutifs de la séquence d'acide nucléique représentée par SEQ ID N° : 17.

5. Vaccin selon l'une quelconque des revendications 1 à 4 dans lequel le second polypeptide est un peptide signal hétérologue.

6. Vaccin selon l'une quelconque des revendications 1 à 4 dans lequel le second polypeptide possède une activité adjuvante.

7. Vaccin selon l'une quelconque des revendications 1 à 6 dans lequel chaque premier acide nucléique est fonctionnellement lié à une ou plusieurs séquences de contrôle de l'expression.

8. Vaccin selon l'une quelconque des revendications 1 à 7, le vaccin comprenant le premier acide nucléique dans un vecteur vaccinal ou l'acide nucléique hybride dans un vecteur vaccinal, et le vaccin comprenant en outre un second acide nucléique codant pour un polypeptide supplémentaire qui améliore la réponse immunitaire au premier polypeptide.

9. Vaccin selon la revendication 8 dans lequel le second acide nucléique code pour un polypeptide supplémentaire de *Chlamydia.*

10. Vaccin selon la revendication 1, le vaccin comprenant le premier polypeptide.

11. Vaccin selon la revendication 1, le vaccin comprenant la protéine de fusion.

12. Vaccin selon la revendication 11 dans lequel le second polypeptide est un peptide signal hétérologue.

13. Vaccin selon la revendication 11 dans lequel le second polypeptide possède une activité adjuvante.

14. Vaccin selon l'une quelconque des revendications 10 à 13, comprenant en outre un polypeptide supplémentaire qui améliore la réponse immunitaire au premier polypeptide.

15. Vaccin selon la revendication 14 dans lequel le polypeptide supplémentaire comprend un polypeptide de *Chlamydia.*

16. Vaccin selon l'une quelconque des revendications 1 à 15 dans lequel le fragment immunogène comprenant au moins 12 acides aminés consécutifs de SEQ ID N° : 18 comprend SEQ ID N° : 68 ou 69.

17. Vaccin selon la revendication 2 qui comprend le premier acide nucléique, dans lequel le premier acide nucléique code pour le polypeptide de SEQ ID N° : 18.

18. Vaccin selon la revendication 17 dans lequel le premier acide nucléique comprend SEQ ID N° : 17.

19. Composition pharmaceutique comprenant le vaccin selon l'une quelconque des revendications 10 à 18 et un véhicule pharmaceutiquement acceptable.

20. Utilisation d'une quantité efficace du vaccin selon l'une quelconque des revendications 1 à 18 pour la préparation d'un médicament destiné à prévenir ou traiter une infection à *Chlamydia.*

21. Utilisation d'une quantité efficace de la composition selon la revendication 19 pour la préparation d'un médicament destiné à prévenir ou traiter une infection à *Chlamydia.*

22. Conditionnement commercial comprenant le vaccin défini dans l'une quelconque des revendications 1 à 18, et des instructions pour utilisation du vaccin dans l'induction d'une réponse immunoprotectrice chez un mammifère.

23. Conditionnement commercial comprenant le vaccin défini dans l'une quelconque des revendications 10 à 15, et des instructions pour utilisation du vaccin dans l'induction d'une réponse immunoprotectrice chez un mammifère.

24. Vaccin selon la revendication 1 comprenant un plasmide d'expression **caractérisé en ce qu'**il s'agit du plasmide pCACPNM459 tel que représenté sur la figure 29 et **en ce qu'**il comprend :
(a) le gène de résistance à l'ampicilline ;
(b) le gène de résistance à la néomycine ;
(c) le promoteur du cytomégalovirus (pCMV) ;
(d) la séquence de l'intron A ;
(e) la séquence codante de la molécule polynucléotidique de SEQ ID N° : 1 codant pour le polypeptide désigné CPLc protéase ;
(f) la séquence poly A de la BGH ; et
(g) la séquence Myc-His.


### SEQUENCE LISTING

<110> Aventis Pasteur Limited
<120> Chlamydia antigens and corresponding DNA fragments and uses thereof
<130> 77813-57
<140> to be assigned
   <141>
<150> US 60/194,475
   <151> 2000-04-04
<160> 74
<170> PatentIn Ver. 2.0
<210> 1
   <211> 1787
   <212> DNA
   <213> Chlamydia pneumoniae
<220>
   <221> CDS
   <222> (101) .. (1684)
<400> 1
   aatctcattc ccccatcgac taaatccacc acggactccg acctcccatg tcttcaatcc 60
   atatgaacgt aatattaagt agcaaattga gtactatata atg aag atg cat agg   115
                                               Met Lys Met His Arg
                                                1               5
   ctt aaa cct acc tta aaa agt ctg atc cct aat ctt ctt ttc tta ttg   163
   Leu Lys Pro Thr Leu Lys Ser Leu Ile Pro Asn Leu Leu Phe Leu Leu
                       10                  15                  20
   ctc act ctt tca agc tgc tca aag caa aaa caa gaa ccc tta gga aaa   211
   Leu Thr Leu Ser Ser Cys Ser Lys Gln Lys Gln Glu Pro Leu Gly Lys
                   25                  30                  35
   cat ctc gtt att gcg atg agc cat gat ctc gcc gac cta gat cct cgc   259
   His Leu Val Ile Ala Met Ser His Asp Leu Ala Asp Leu Asp Pro Arg
               40                  45                  50
   aat gcc tat tta agc aga gat gct tcc cta gca aaa gcc ctc tat gaa   307
   Asn Ala Tyr Leu Ser Arg Asp Ala Ser Leu Ala Lys Ala Leu Tyr Glu
           55                  60                  65
   gga ctg aca aga gaa act gat caa gga atc gca ctg gct ctt gca gaa   355
   Gly Leu Thr Arg Glu Thr Asp Gln Gly Ile Ala Leu Ala Leu Ala Glu
    70                  75                  80                  85
   agt tat acc ctg tca aaa gat cat aag gtc tat acc ttt aaa ctc aga   403
   Ser Tyr Thr Leu Ser Lys Asp His Lys Val Tyr Thr Phe Lys Leu Arg
                       90                  95                 100
   cct tct gtg tgg agc gat ggc act cca ctc act gct tat gac ttt gaa    451
   Pro Ser Val Trp Ser Asp Gly Thr Pro Leu Thr Ala Tyr Asp Phe Glu
               105                 110                 115
   aaa tct ata aaa caa ctg tac ttc gaa gaa ttt tca cct tcc ata cat    499
   Lys Ser Ile Lys Gln Leu Tyr Phe Glu Glu Phe Ser Pro Ser Ile His
           120                 125                 130
   act tta ctc ggc gtg att aaa aat tct tcg gca atc cac aat gct caa    547
   Thr Leu Leu Gly Val Ile Lys Asn Ser Ser Ala Ile His Asn Ala Gln
       135                 140                 145
   aaa tct ctg gaa act ctt ggg ata cag gca aaa gat gat ctt act ttg    595
   Lys Ser Leu Glu Thr Leu Gly Ile Gln Ala Lys Asp Asp Leu Thr Leu
   150                 155                 160                 165
   gtg att acc cta gag caa cct ttc cca tac ttt ctc aca ctt atc gct    643
   Val Ile Thr Leu Glu Gln Pro Phe Pro Tyr Phe Leu Thr Leu Ile Ala
                   170                 175                 180
   cgc ccc gta ttc tcc cct gtt cat cac acc ctt agg gaa tcc tat aag    691
   Arg Pro Val Phe Ser Pro Val His His Thr Leu Arg Glu Ser Tyr Lys
               185                 190                 195
   aaa gga aca ccc cca tcc aca tac atc tcc aat ggg ccc ttt gtc tta    739
   Lys Gly Thr Pro Pro Ser Thr Tyr Ile Ser Asn Gly Pro Phe Val Leu
           200                 205                 210
   aaa aaa cat gaa cac caa aac tac tta att tta gaa aaa aat cct cac    787
   Lys Lys His Glu His Gln Asn Tyr Leu Ile Leu Glu Lys Asn Pro His
       215                 220                 225
   tac tat gat cat gaa tca gta aag tta gac cga gtc acc tta aaa att    835
   Tyr Tyr Asp His Glu Ser Val Lys Leu Asp Arg Val Thr Leu Lys Ile
   230                 235                 240                 245
   atc cca gac gcc tcc aca gcc acg aaa ctt ttc aaa agt aaa tct ata    883
   Ile Pro Asp Ala Ser Thr Ala Thr Lys Leu Phe Lys Ser Lys Ser Ile
                   250                 255                 260
   gat tgg att ggc tca cct tgg agc gct ccg ata tct aac gaa gac caa    931
   Asp Trp Ile Gly Ser Pro Trp Ser Ala Pro Ile Ser Asn Glu Asp Gln
               265                 270                 275
   aaa gtt ctc tcc caa gaa aag att ctt acc tat tct gtt tca agc acc    979
   Lys Val Leu Ser Gln Glu Lys Ile Leu Thr Tyr Ser Val Ser Ser Thr
           280                 285                 290
   acc ctt ctt atc tat aac ctg caa aaa cct cta ata caa aat aaa gcc    1027
   Thr Leu Leu Ile Tyr Asn Leu Gln Lys Pro Leu Ile Gln Asn Lys Ala
       295                 300                 305
   ctc agg aaa gcc att gct cat gct att gat aga aaa tct atc tta aga    1075
   Leu Arg Lys Ala Ile Ala His Ala Ile Asp Arg Lys Ser Ile Leu Arg
   310                 315                 320                 325
   ctc gtg cct tca gga caa gaa gct gta act cta gtt ccc cca aat ctt    1123
   Leu Val Pro Ser Gly Gln Glu Ala Val Thr Leu Val Pro Pro Asn Leu
                   330                 335                 340
   tca caa ctc aat ctt caa aaa gag atc tca aca gaa gaa cga caa aca    1171
   Ser Gln Leu Asn Leu Gln Lys Glu Ile Ser Thr Glu Glu Arg Gln Thr
               345                 350                 355
   aaa gcc aga gca tat ttt caa gaa gct aaa gaa aca ctt tct gaa aaa    1219
   Lys Ala Arg Ala Tyr Phe Gln Glu Ala Lys Glu Thr Leu Ser Glu Lys
           360                 365                 370
   gaa ctc gca gaa ctc agc atc ctc tat cct ata gat tcc tcg aat tcc    1267
   Glu Leu Ala Glu Leu Ser Ile Leu Tyr Pro Ile Asp Ser Ser Asn Ser
       375                 380                 385
   tcc atc ata gct caa gaa atc caa aga caa ctt aaa gat acc tta gga    1315
   Ser Ile Ile Ala Gln Glu Ile Gln Arg Gln Leu Lys Asp Thr Leu Gly
   390                 395                 400                 405
   ttg aaa atc aaa atc caa ggc atg gag tac cac tgc ttt tta aag aaa    1363
   Leu Lys Ile Lys Ile Gln Gly Met Glu Tyr His Cys Phe Leu Lys Lys
                   410                 415                 420
   cgt cgt caa gga gat ttc ttc ata gcg aca gga gga tgg att gcg gaa    1411
   Arg Arg Gln Gly Asp Phe Phe Ile Ala Thr Gly Gly Trp Ile Ala Glu
               425                 430                 435
   tac gta agc ccc gta gcc ttc cta tct att cta ggc aac ccc aga gac    1459
   Tyr Val Ser Pro Val Ala Phe Leu Ser Ile Leu Gly Asn Pro Arg Asp
           440                 445                 450
   ctc aca caa tgg aga aac agt gat tac gaa aag act tta gag aaa ctc    1507
   Leu Thr Gln Trp Arg Asn Ser Asp Tyr Glu Lys Thr Leu Glu Lys Leu
       455                 460                 465
   tat ctc cct cat gcc tac aaa gag aat tta aaa cgc gca gaa atg ata    1555
   Tyr Leu Pro His Ala Tyr Lys Glu Asn Leu Lys Arg Ala Glu Met Ile
   470                 475                 480                 485
   ata gaa gaa gaa acc ccg att atc ccc ctg tat cac ggc aaa tat att    1603
   Ile Glu Glu Glu Thr Pro Ile Ile Pro Leu Tyr His Gly Lys Tyr Ile
                   490                 495                 500
   tac gct ata cat cct aaa atc cag aat aca ttc gga tct ctt cta ggc    1651
   Tyr Ala Ile His Pro Lys Ile Gln Asn Thr Phe Gly Ser Leu Leu Gly
               505                 510                 515
   cac aca gat ctc aaa aat atc gat atc tta agt tagatccgaa atggaaaaat 1704
   His Thr Asp Leu Lys Asn Ile Asp Ile Leu Ser
           520                 525
   taaaaatttt atagacaatc ttgaaaagag aattaaaaat ttttaattta aattatagtt 1764
   gcaattgaaa acgcccctaa gaa                                         1787
<210> 2
   <211> 528
   <212> PRT
   <213> Chlamydia pneumoniae
<220>
   <221> SITE
   <222> (188)...(61)
   <223> B-cell epitope
<220>
   <221> SITE
   <222> (345)...(355)
   <223> B-cell epitope
<220>
   <221> SITE
   <222> (434)...(442)
   <223> T-cell epitope
<400> 2
   Met Lys Met His Arg Leu Lys Pro Thr Leu Lys Ser Leu Ile Pro Asn
    1               5                  10                  15
   Leu Leu Phe Leu Leu Leu Thr Leu Ser Ser Cys Ser Lys Gln Lys Gln
               20                  25                  30
   Glu Pro Leu Gly Lys His Leu Val Ile Ala Met Ser His Asp Leu Ala
           35                  40                  45
   Asp Leu Asp Pro Arg Asn Ala Tyr Leu Ser Arg Asp Ala Ser Leu Ala
       50                  55                  60
   Lys Ala Leu Tyr Glu Gly Leu Thr Arg Glu Thr Asp Gln Gly Ile Ala
   65                  70                  75                  80
   Leu Ala Leu Ala Glu Ser Tyr Thr Leu Ser Lys Asp His Lys Val Tyr
                   85                  90                  95
   Thr Phe Lys Leu Arg Pro Ser Val Trp Ser Asp Gly Thr Pro Leu Thr
               100                 105                 110
   Ala Tyr Asp Phe Glu Lys Ser Ile Lys Gln Leu Tyr Phe Glu Glu Phe
           115                 120                 125
   Ser Pro Ser Ile His Thr Leu Leu Gly Val Ile Lys Asn Ser Ser Ala
       130                 135                 140
   Ile His Asn Ala Gln Lys Ser Leu Glu Thr Leu Gly Ile Gln Ala Lys
   145                 150                 155                 160
   Asp Asp Leu Thr Leu Val Ile Thr Leu Glu Gln Pro Phe Pro Tyr Phe
                   165                 170                 175
   Leu Thr Leu Ile Ala Arg Pro Val Phe Ser Pro Val His His Thr Leu
               180                 185                 190
   Arg Glu Ser Tyr Lys Lys Gly Thr Pro Pro Ser Thr Tyr Ile Ser Asn
           195                 200                 205
   Gly Pro Phe Val Leu Lys Lys His Glu His Gln Asn Tyr Leu Ile Leu
       210                 215                 220
   Glu Lys Asn Pro His Tyr Tyr Asp His Glu Ser Val Lys Leu Asp Arg
   225                 230                 235                 240
   Val Thr Leu Lys Ile Ile Pro Asp Ala Ser Thr Ala Thr Lys Leu Phe
   245 250 255
   Lys Ser Lys Ser Ile Asp Trp Ile Gly Ser Pro Trp Ser Ala Pro Ile
   260 265 270
   Ser Asn Glu Asp Gln Lys Val Leu Ser Gln Glu Lys Ile Leu Thr Tyr
   275 280 285
   Ser Val Ser Ser Thr Thr Leu Leu Ile Tyr Asn Leu Gln Lys Pro Leu
   290 295 300
   Ile Gln Asn Lys Ala Leu Arg Lys Ala Ile Ala His Ala Ile Asp Arg
   305 310 315 320
   Lys Ser Ile Leu Arg Leu Val Pro Ser Gly Gln Glu Ala Val Thr Leu
   325 330 335
   Val Pro Pro Asn Leu Ser Gln Leu Asn Leu Gln Lys Glu Ile Ser Thr
   340 345 350
   Glu Glu Arg Gln Thr Lys Ala Arg Ala Tyr Phe Gln Glu Ala Lys Glu
   355 360 365
   Thr Leu Ser Glu Lys Glu Leu Ala Glu Leu Ser Ile Leu Tyr Pro Ile
   370 375 380
   Asp Ser Ser Asn Ser Ser Ile Ile Ala Gln Glu Ile Gln Arg Gln Leu
   385 390 395 400
   Lys Asp Thr Leu Gly Leu Lys Ile Lys Ile Gln Gly Met Glu Tyr His
   405 410 415
   Cys Phe Leu Lys Lys Arg Arg Gln Gly Asp Phe Phe Ile Ala Thr Gly
   420 425 430
   Gly Trp Ile Ala Glu Tyr Val Ser Pro Val Ala Phe Leu Ser Ile Leu
   435 440 445
   Gly Asn Pro Arg Asp Leu Thr Gln Trp Arg Asn Ser Asp Tyr Glu Lys
   450 455 460
   Thr Leu Glu Lys Leu Tyr Leu Pro His Ala Tyr Lys Glu Asn Leu Lys
   465 470 475 480
   Arg Ala Glu Met Ile Ile Glu Glu Glu Thr Pro Ile Ile Pro Leu Tyr
   485 490 495
   His Gly Lys Tyr Ile Tyr Ala Ile His Pro Lys Ile Gln Asn Thr Phe
   500 505 510
   Gly Ser Leu Leu Gly His Thr Asp Leu Lys Asn Ile Asp Ile Leu Ser
   515 520 525
<210> 3
   <211> 1226
   <212> DNA
   <213> Chlamydia pneumoniae
<220>
   <221> CDS
   <222> (101)..(1123)
<400> 3
   ttccagagaa atcctgatcc tgaaaaactt cctgaaacaa ttgctttaac tataacacgg 60
   gaacctaaag catatcctcc aaggacgtta acataccaat ttg cgg ttg gga aat   115
                                              Leu Arg Leu Gly Asn
                                              1               5
   aag cct atg caa cct ttt atc ttt act tta ctg tgc ttg aca tct ttg   163
   Lys Pro Met Gln Pro Phe Ile Phe Thr Leu Leu Cys Leu Thr Ser Leu
                   10                  15                  20
   gtt tct tta gtc gcc ttt gat gct gcg aat gct cgt aaa cgt tgt gcc   211
   Val Ser Leu Val Ala Phe Asp Ala Ala Asn Ala Arg Lys Arg Cys Ala
               25                  30                  35
   tgt gct caa act ata gaa cgt gga gag aac ttc ttt tcc ata aaa cgc   259
   Cys Ala Gln Thr Ile Glu Arg Gly Glu Asn Phe Phe Ser Ile Lys Arg
           40                  45                  50
   tct gct tgt gct gaa atc gaa tat caa gaa aaa tct cgc cac gcc tca   307
   Ser Ala Cys Ala Glu Ile Glu Tyr Gln Glu Lys Ser Arg His Ala Ser
       55                  60                  65
   gca att gaa aga atc tca aaa gat aaa ggc aaa gtc act cca aag cag   355
   Ala Ile Glu Arg Ile Ser Lys Asp Lys Gly Lys Val Thr Pro Lys Gln
   70                  75                  80                  85
   att gcg aaa gta gct act aag aaa aag caa aga tac cgt tta ttg cag   403
   Ile Ala Lys Val Ala Thr Lys Lys Lys Gln Arg Tyr Arg Leu Leu Gln
                   90                  95                  100
   gtt cct ttt tca agg cct ccg aat aac tca agg tat aac ctc tat gct   451
   Val Pro Phe Ser Arg Pro Pro Asn Asn Ser Arg Tyr Asn Leu Tyr Ala
               105                 110                 115
   ttg ctt agt gaa cct ccc gaa tgc tat agc gat aca gca tca tgg tat   499
   Leu Leu Ser Glu Pro Pro Glu Cys Tyr Ser Asp Thr Ala Ser Trp Tyr
           120                 125                 130
   gct att ttt att cgg tta ctt cga cgt gct tat gta gac acg gga aat   547
   Ala Ile Phe Ile Arg Leu Leu Arg Arg Ala Tyr Val Asp Thr Gly Asn
       135                 140                 145
   gta cct cct gga tct gag tat gcc atc gct aat gct ttg ata agt aac   595
   Val Pro Pro Gly Ser Glu Tyr Ala Ile Ala Asn Ala Leu Ile Ser Asn
   150                 155                 160                 165
   aaa caa gag att tta gag agg gga gcg cag ctt gga ccc gat gtt att   643
   Lys Gln Glu Ile Leu Glu Arg Gly Ala Gln Leu Gly Pro Asp Val Ile
                   170                 175                 180
   gaa act cta aca ttg cct gag gaa caa gcc gag att ttt tat aaa atg   691
   Glu Thr Leu Thr Leu Pro Glu Glu Gln Ala Glu Ile Phe Tyr Lys Met
               185                 190                 195
   ctc aaa ggg tcg tca aac tct cag tcg cta ctg aat ttt ctg cat tat     739
   Leu Lys Gly Ser Ser Asn Ser Gln Ser Leu Leu Asn Phe Leu His Tyr
           200                 205                 210
   gaa gag aaa agc tta ggc cac tgt aag cta aat ctg atc ttc atg gat     787
   Glu Glu Lys Ser Leu Gly His Cys Lys Leu Asn Leu Ile Phe Met Asp
       215                 220                 225
   ccc cta ctg tta gaa gct gtt cta gat cat ccc gat gct tat agg gaa     835
   Pro Leu Leu Leu Glu Ala Val Leu Asp His Pro Asp Ala Tyr Arg Glu
   230                 235                 240                 245
   acg tcg ctc ctg cgc gat ggc att tgg gaa gcg gtg aag cgt caa gaa     883
   Thr Ser Leu Leu Arg Asp Gly Ile Trp Glu Ala Val Lys Arg Gln Glu
                   250                 255                 260
   cat gcc atc caa gaa cat ggc cag gca gct gct ttg gag ctt ttt aaa     931
   His Ala Ile Gln Glu His Gly Gln Ala Ala Ala Leu Glu Leu Phe Lys
               265                 270                 275
   aca cgc acc gac ttc cgc ctg gag ctg cga gat aag atg cag tta ctt     979
   Thr Arg Thr Asp Phe Arg Leu Glu Leu Arg Asp Lys Met Gln Leu Leu
           280                 285                 290
   cta agt cga tac gat ttg ctc ccc tta tta aat aaa aaa atg ttc gac    1027
   Leu Ser Arg Tyr Asp Leu Leu Pro Leu Leu Asn Lys Lys Met Phe Asp
       295                 300                 305
   tac acc tta gga agt gcc gga gat tac tta ttt ttg gta gac cca gat    1075
   Tyr Thr Leu Gly Ser Ala Gly Asp Tyr Leu Phe Leu Val Asp Pro Asp
   310                 315                 320                 325
   act aag gca att tct cga tgt cgc tgc cct tca aag agt att aaa tta    1123
   Thr Lys Ala Ile Ser Arg Cys Arg Cys Pro Ser Lys Ser Ile Lys Leu
                   330                 335                 340
   taatttaatt ttaatattta ttttaaatag ttttttttga taattgtctt aataagtact 1183
   ataaaaaata tttctatagg taggaccatg gcagacgaga ccc                  1226
<210> 4
   <211> 341
   <212 PRT
   <213> Chlamydia pneumoniae
<400> 4
   Leu Arg Leu Gly Asn Lys Pro Met Gln Pro Phe Ile Phe Thr Leu Leu
   1 5 10 15
   Cys Leu Thr Ser Leu Val Ser Leu Val Ala Phe Asp Ala Ala Asn Ala
   20 25 30
   Arg Lys Arg Cys Ala Cys Ala Gln Thr Ile Glu Arg Gly Glu Asn Phe
   35 40 45
   Phe Ser Ile Lys Arg Ser Ala Cys Ala Glu Ile Glu Tyr Gln Glu Lys
   50 55 60
   Ser Arg His Ala Ser Ala Ile Glu Arg Ile Ser Lys Asp Lys Gly Lys
   65 70 75 80
   Val Thr Pro Lys Gln Ile Ala Lys Val Ala Thr Lys Lys Lys Gln Arg
   85 90 95
   Tyr Arg Leu Leu Gln Val Pro Phe Ser Arg Pro Pro Asn Asn Ser Arg
   100 105 110
   Tyr Asn Leu Tyr Ala Leu Leu Ser Glu Pro Pro Glu Cys Tyr Ser Asp
   115 120 125
   Thr Ala Ser Trp Tyr Ala Ile Phe Ile Arg Leu Leu Arg Arg Ala Tyr
   130 135 140
   Val Asp Thr Gly Asn Val Pro Pro Gly Ser Glu Tyr Ala Ile Ala Asn
   145 150 155 160
   Ala Leu Ile Ser Asn Lys Gln Glu Ile Leu Glu Arg Gly Ala Gln Leu
   165 170 175
   Gly Pro Asp Val Ile Glu Thr Leu Thr Leu Pro Glu Glu Gln Ala Glu
   180 185 190
   Ile Phe Tyr Lys Met Leu Lys Gly Ser Ser Asn Ser Gln Ser Leu Leu
   195 200 205
   Asn Phe Leu His Tyr Glu Glu Lys Ser Leu Gly His Cys Lys Leu Asn
   210 215 220
   Leu Ile Phe Met Asp Pro Leu Leu Leu Glu Ala Val Leu Asp His Pro
   225 230 235 240
   Asp Ala Tyr Arg Glu Thr Ser Leu Leu Arg Asp Gly Ile Trp Glu Ala
   245 250 255
   Val Lys Arg Gln Glu His Ala Ile Gln Glu His Gly Gln Ala Ala Ala
   260 265 270
   Leu Glu Leu Phe Lys Thr Arg Thr Asp Phe Arg Leu Glu Leu Arg Asp
   275 280 285
   Lys Met Gln Leu Leu Leu Ser Arg Tyr Asp Leu Leu Pro Leu Leu Asn
   290 295 300
   Lys Lys Met Phe Asp Tyr Thr Leu Gly Ser Ala Gly Asp Tyr Leu Phe
   305 310 315 320
   Leu Val Asp Pro Asp Thr Lys Ala Ile Ser Arg Cys Arg Cys Pro Ser
   325 330 335
   Lys Ser Ile Lys Leu
   340
<210> 5
   <211> 1235
   <212> DNA
   <213> Chlamydia pneumoniae
<220>
   <221> CDS
   <222> (101)..(1132)
<400> 5
   gttacttttt ttttcataaa aaccccatgt aacttttact tgctcatatt gagaagtccc 60
   ccatactata aaaggcaacg ttttcttttc ttggtttttt atg ctc acc cta ggc   115
                                               Met Leu Thr Leu Gly
                                                1               5
   ttg gaa agt tct tgc gat gag act gcc tgc gct ata gtt aat gag gat   163
   Leu Glu Ser Ser Cys Asp Glu Thr Ala Cys Ala Ile Val Asn Glu Asp
                   10                  15                  20
   aag cag ata tta gca aat att att gcc tct caa gat atc cat gca tcc   211
   Lys Gln Ile Leu Ala Asn Ile Ile Ala Ser Gln Asp Ile His Ala Ser
               25                  30                  35
   tat ggc gga gtc gtt cct gaa ctt gct tca aga gca cat ctc cat atc   259
   Tyr Gly Gly Val Val Pro Glu Leu Ala Ser Arg Ala His Leu His Ile
           40                  45                  50
   ttc cca caa gtg ata aat aaa gct cta caa cag gcc aac tta ttg atc   307
   Phe Pro Gln Val Ile Asn Lys Ala Leu Gln Gln Ala Asn Leu Leu Ile
       55                  60                  65
   gaa gat atg gat ctg att gca gta acg caa act cca ggg ttg ata ggt   355
   Glu Asp Met Asp Leu Ile Ala Val Thr Gln Thr Pro Gly Leu Ile Gly
   70                  75                  80                  85
   tct cta tca gta gga gtg cat ttt ggt aaa ggc att gcc ata gga gca   403
   Ser Leu Ser Val Gly Val His Phe Gly Lys Gly Ile Ala Ile Gly Ala
                   90                  95                  100
   aaa aaa tcc ttg att gga gtc aat cac gtc gaa gct cat ctc tat gct   451
   Lys Lys Ser Leu Ile Gly Val Asn His Val Glu Ala His Leu Tyr Ala
               105                 110                 115
   gcc tat atg gca gcg caa aac gtg caa ttc cct gct tta ggt ctt gtg   499
   Ala Tyr Met Ala Ala Gln Asn Val Gln Phe Pro Ala Leu Gly Leu Val
           120                 125                 130
   gtc tct gga gct cat acc gca gcg ttt ttt ata gaa aat cct aca tcc   547
   Val Ser Gly Ala His Thr Ala Ala Phe Phe Ile Glu Asn Pro Thr Ser
       135                 140                 145
   tat aaa ctc ata gga aaa act cga gat gat gct ata gga gaa act ttt   595
   Tyr Lys Leu Ile Gly Lys Thr Arg Asp Asp Ala Ile Gly Glu Thr Phe
   150                 155                 160                 165
   gat aaa gta gga cgc ttt cta gga tta cca tac cct gca ggc cca tta   643
   Asp Lys Val Gly Arg Phe Leu Gly Leu Pro Tyr Pro Ala Gly Pro Leu
                   170                 175                 180
   att gaa aaa ctc gct tta gaa ggc tct gag gac agt tat cct ttt agt   691
   Ile Glu Lys Leu Ala Leu Glu Gly Ser Glu Asp Ser Tyr Pro Phe Ser
               185                 190                 195
   cca gct aaa gtc cca aac tat gac ttt tca ttc agc ggt ctt aaa aca    739
   Pro Ala Lys Val Pro Asn Tyr Asp Phe Ser Phe Ser Gly Leu Lys Thr
           200                 205                 210
   gct gtt ctc tac gca atc aaa gga aat aat agt agc ccc cgc tct cct    787
   Ala Val Leu Tyr Ala Ile Lys Gly Asn Asn Ser Ser Pro Arg Ser Pro
       215                 220                 225
   gct cca gag ata tct tta gaa aaa caa aga gat atc gct gct tca ttt    835
   Ala Pro Glu Ile Ser Leu Glu Lys Gln Arg Asp Ile Ala Ala Ser Phe
   230                 235                 240                 245
   caa aaa gcg gcc tgc act act att gca caa aaa ctt ccc act att ata    883
   Gln Lys Ala Ala Cys Thr Thr Ile Ala Gln Lys Leu Pro Thr Ile Ile
                   250                 255                 260
   aaa gaa ttt tcg tgc cga tct ata ctt att gga ggt ggc gta gcc att    931
   Lys Glu Phe Ser Cys Arg Ser Ile Leu Ile Gly Gly Gly Val Ala Ile
               265                 270                 275
   aat gaa tac ttt aga tcc gca ata caa act gcg tgt aat cta cct gta    979
   Asn Glu Tyr Phe Arg Ser Ala Ile Gln Thr Ala Cys Asn Leu Pro Val
           280                 285                 290
   tac ttc ccc cct gct aaa cta tgc tca gat aat gct gct atg att gca    1027
   Tyr Phe Pro Pro Ala Lys Leu Cys Ser Asp Asn Ala Ala Met Ile Ala
       295                 300                 305
   ggt cta ggg gga gaa aat ttt caa aaa aac tct agt att ccg gaa att    1075
   Gly Leu Gly Gly Glu Asn Phe Gln Lys Asn Ser Ser Ile Pro Glu Ile
   310                 315                 320                 325
   cgt ata tgc gca aga tat cag tgg gaa tct gta tca cca ttc tcc tta    1123
   Arg Ile Cys Ala Arg Tyr Gln Trp Glu Ser Val Ser Pro Phe Ser Leu
                   330                 335                 340
   gcc tct ccg tagtcctcca aggctgcaag gagtccagtc actcctctac            1172
   Ala Ser Pro
   atctcgggga gaactcgcta ttaatataag agatgaaccc cgttctttag atccaagaca 1232
   agt                                                               1235
<210> 6
   <211> 344
   <212> PRT
   <213> Chlamydia pneumoniae
<220>
   <221> SITE
   <222> (220)...(231)
   <223> B-cell epitope
<220>
   <221> SITE
   <222> (313)...(321)
   <223> B-cell epitope
<220>
   <221> SITE
   <222> (67)...(76)
   <223> T-cell epitope
<220>
   <221> SITE
   <222> (66)... (75)
   <223> T-cell epitope
<400> 6
   Met Leu Thr Leu Gly Leu Glu Ser Ser Cys Asp Glu Thr Ala Cys Ala
    1               5                  10                  15
   Ile Val Asn Glu Asp Lys Gln Ile Leu Ala Asn Ile Ile Ala Ser Gln
               20                  25                  30
   Asp Ile His Ala Ser Tyr Gly Gly Val Val Pro Glu Leu Ala Ser Arg
           35                  40                  45
   Ala His Leu His Ile Phe Pro Gln Val Ile Asn Lys Ala Leu Gln Gln
       50                  55                  60
   Ala Asn Leu Leu Ile Glu Asp Met Asp Leu Ile Ala Val Thr Gln Thr
   65                  70                  75                  80
   Pro Gly Leu Ile Gly Ser Leu Ser Val Gly Val His Phe Gly Lys Gly
                   85                  90                  95
   Ile Ala Ile Gly Ala Lys Lys Ser Leu Ile Gly Val Asn His Val Glu
               100                 105                 110
   Ala His Leu Tyr Ala Ala Tyr Met Ala Ala Gln Asn Val Gln Phe Pro
           115                 120                 125
   Ala Leu Gly Leu Val Val Ser Gly Ala His Thr Ala Ala Phe Phe Ile
       130                 135                 140
   Glu Asn Pro Thr Ser Tyr Lys Leu Ile Gly Lys Thr Arg Asp Asp Ala
   145                 150                 155                 160
   Ile Gly Glu Thr Phe Asp Lys Val Gly Arg Phe Leu Gly Leu Pro Tyr
                   165                 170                 175
   Pro Ala Gly Pro Leu Ile Glu Lys Leu Ala Leu Glu Gly Ser Glu Asp
               180                 185                 190
   Ser Tyr Pro Phe Ser Pro Ala Lys Val Pro Asn Tyr Asp Phe Ser Phe
           195                 200                 205
   Ser Gly Leu Lys Thr Ala Val Leu Tyr Ala Ile Lys Gly Asn Asn Ser
       210                 215                 220
   Ser Pro Arg Ser Pro Ala Pro Glu Ile Ser Leu Glu Lys Gln Arg Asp
   225                 230                 235                 240
   Ile Ala Ala Ser Phe Gln Lys Ala Ala Cys Thr Thr Ile Ala Gln Lys
                   245                 250                 255
   Leu Pro Thr Ile Ile Lys Glu Phe Ser Cys Arg Ser Ile Leu Ile Gly
               260                 265                 270
   Gly Gly Val Ala Ile Asn Glu Tyr Phe Arg Ser Ala Ile Gln Thr Ala
           275                 280                 285
   Cys Asn Leu Pro Val Tyr Phe Pro Pro Ala Lys Leu Cys Ser Asp Asn
       290                 295                 300
   Ala Ala Met Ile Ala Gly Leu Gly Gly Glu Asn Phe Gln Lys Asn Ser
   305                 310                 315                 320
   Ser Ile Pro Glu Ile Arg Ile Cys Ala Arg Tyr Gln Trp Glu Ser Val
                   325                 330                 335
   Ser Pro Phe Ser Leu Ala Ser Pro
               340
<210> 7
   <211> 2060
   <212> DNA
   <213> Chlamydia pneumoniae
<220>
   <221> CDS
   <222> (101)..(1957)
<400> 7
   gattttgtgt attttttcag ataatgtttt taaaaaaatg ttttaaaacc ctaaaatcct 60
   acctccttgt aaccattctc ggtagaaaag agaggtattt atg aaa aaa ggg aaa   115
                                            Met Lys Lys Gly Lys
                                             1               5
   tta gga gcc ata gtt ttt ggc ctt cta ttt aca agt agt gtt gct ggt   163
   Leu Gly Ala Ile Val Phe Gly Leu Leu Phe Thr Ser Ser Val Ala Gly
                   10                  15                  20
   ttt tct aag gat ttg act aaa gac aac gct tat caa gat tta aat gtc   211
   Phe Ser Lys Asp Leu Thr Lys Asp Asn Ala Tyr Gln Asp Leu Asn Val
               25                  30                  35
   ata gag cat tta ata tcg tta aaa tat gct cct tta cca tgg aag gaa   259
   Ile Glu His Leu Ile Ser Leu Lys Tyr Ala Pro Leu Pro Trp Lys Glu
           40                  45                  50
   cta tta ttt ggt tgg gat tta tct cag caa aca cag caa gct cgc ttg   307
   Leu Leu Phe Gly Trp Asp Leu Ser Gln Gln Thr Gln Gln Ala Arg Leu
       55                  60                  65
   caa ctg gtc tta gaa gaa aaa cca aca acc aac tac tgc cag aag gta   355
   Gln Leu Val Leu Glu Glu Lys Pro Thr Thr Asn Tyr Cys Gln Lys Val
   70                  75                  80                  85
   ctc tct aac tac gtg aga tca tta aac gat tat cat gca ggg att acg   403
   Leu Ser Asn Tyr Val Arg Ser Leu Asn Asp Tyr His Ala Gly Ile Thr
                   90                  95                  100
   ttt tat cgt act gaa agt gcg tat atc cct tac gta ttg aag tta agt    451
   Phe Tyr Arg Thr Glu Ser Ala Tyr Ile Pro Tyr Val Leu Lys Leu Ser
               105                 110                 115
   gaa gat ggt cat gtc ttt gta gtc gac gta cag act agc caa ggg gat    499
   Glu Asp Gly His Val Phe Val Val Asp Val Gln Thr Ser Gln Gly Asp
           120                 125                 130
   att tac tta ggg gat gaa atc ctt gaa gta gat gga atg ggg att cgt    547
   Ile Tyr Leu Gly Asp Glu Ile Leu Glu Val Asp Gly Met Gly Ile Arg
       135                 140                 145
   gag gct atc gaa agc ctt cgc ttt gga cga ggg agt gcc aca gac tat    595
   Glu Ala Ile Glu Ser Leu Arg Phe Gly Arg Gly Ser Ala Thr Asp Tyr
   150                 155                 160                 165
   tct gct gca gtt cgt tcc ttg aca tcg cgt tcc gcc gct ttt gga gat    643
   Ser Ala Ala Val Arg Ser Leu Thr Ser Arg Ser Ala Ala Phe Gly Asp
                   170                 175                 180
   gcg gtt cct tca gga att gcc atg ttg aaa ctt cgc cga ccc agt ggt    691
   Ala Val Pro Ser Gly Ile Ala Met Leu Lys Leu Arg Arg Pro Ser Gly
               185                 190                 195
   ttg atc cgt tcg aca ccg gtc cgt tgg cgt tat act cca gag cat atc    739
   Leu Ile Arg Ser Thr Pro Val Arg Trp Arg Tyr Thr Pro Glu His Ile
           200                 205                 210
   gga gat ttt tct tta gtt gct cct ttg att cct gaa cat aaa cct caa    787
   Gly Asp Phe Ser Leu Val Ala Pro Leu Ile Pro Glu His Lys Pro Gln
       215                 220                 225
   tta cct aca caa agt tgt gtg cta ttc cgt tcc ggg gta aat tca cag    835
   Leu Pro Thr Gln Ser Cys Val Leu Phe Arg Ser Gly Val Asn Ser Gln
   230                 235                 240                 245
   tct tct agt agc tct tta ttc agt tcc tac atg gtg cct tat ttc tgg    883
   Ser Ser Ser Ser Ser Leu Phe Ser Ser Tyr Met Val Pro Tyr Phe Trp
                   250                 255                 260
   gaa gaa ttg cgg gtt caa aat aag cag cgt ttt gac agt aat cac cat    931
   Glu Glu Leu Arg Val Gln Asn Lys Gln Arg Phe Asp Ser Asn His His
               265                 270                 275
   ata ggg agc cgt aat gga ttt tta cct acg ttt ggt cct att ctt tgg    979
   Ile Gly Ser Arg Asn Gly Phe Leu Pro Thr Phe Gly Pro Ile Leu Trp
           280                 285                 290
   gaa caa gac aag ggg ccc tat cgt tcc tat atc ttt aaa gca aaa gat    1027
   Glu Gln Asp Lys Gly Pro Tyr Arg Ser Tyr Ile Phe Lys Ala Lys Asp
       295                 300                 305
   tct cag ggc aat ccc cat cgc ata gga ttt tta aga att tct tct tat    1075
   Ser Gln Gly Asn Pro His Arg Ile Gly Phe Leu Arg Ile Ser Ser Tyr
   310                 315                 320                 325
   gtt tgg act gat tta gaa gga ctt gaa gag gat cat aag gat agt cct    1123
   Val Trp Thr Asp Leu Glu Gly Leu Glu Glu Asp His Lys Asp Ser Pro
                   330                 335                 340
   tgg gag ctc ttt gga gag atc atc gat cat ttg gaa aaa gag act gat    1171
   Trp Glu Leu Phe Gly Glu Ile Ile Asp His Leu Glu Lys Glu Thr Asp
               345                 350                 355
   gct ttg att att gat cag acc cat aat cct gga ggc agt gtt ttc tat    1219
   Ala Leu Ile Ile Asp Gln Thr His Asn Pro Gly Gly Ser Val Phe Tyr
           360                 365                 370
   ctc tat tcg tta cta tct atg tta aca gat cat cct tta gat act cct    1267
   Leu Tyr Ser Leu Leu Ser Met Leu Thr Asp His Pro Leu Asp Thr Pro
       375                 380                 385
   aaa cat aga atg att ttc act cag gat gaa gtc agc tcg gct ttg cac    1315
   Lys His Arg Met Ile Phe Thr Gln Asp Glu Val Ser Ser Ala Leu His
   390                 395                 400                 405
   tgg caa gat cta cta gaa gat gtc ttc aca gat gag cag gca gtt gcc    1363
   Trp Gln Asp Leu Leu Glu Asp Val Phe Thr Asp Glu Gln Ala Val Ala
                   410                 415                 420
   gtg cta ggg gaa act atg gaa gga tat tgc atg gat atg cat gct gta    1411
   Val Leu Gly Glu Thr Met Glu Gly Tyr Cys Met Asp Met His Ala Val
               425                 430                 435
   gcc tct ctt caa aac ttc tct cag agt gtc ctt tct tcc tgg gtt tca    1459
   Ala Ser Leu Gln Asn Phe Ser Gln Ser Val Leu Ser Ser Trp Val Ser
           440                 445                 450
   ggt gat att aac ctt tca aaa cct atg cct ttg cta gga ttt gca cag    1507
   Gly Asp Ile Asn Leu Ser Lys Pro Met Pro Leu Leu Gly Phe Ala Gln
       455                 460                 465
   gtt cga cct cat cct aaa cat caa tat act aaa cct ttg ttt atg ttg    1555
   Val Arg Pro His Pro Lys His Gln Tyr Thr Lys Pro Leu Phe Met Leu
   470                 475                 480                 485
   ata gac gag gat gac ttc tct tgt gga gat tta gcg cct gca att ttg    1603
   Ile Asp Glu Asp Asp Phe Ser Cys Gly Asp Leu Ala Pro Ala Ile Leu
                   490                 495                 500
   aag gat aat ggc cgc gct act ctc att gga aag cca aca gca gga gct    1651
   Lys Asp Asn Gly Arg Ala Thr Leu Ile Gly Lys Pro Thr Ala Gly Ala
               505                 510                 515
   gga ggt ttt gta ttc caa gtc act ttc cct aac cgt tct gga att aaa    1699
   Gly Gly Phe Val Phe Gln Val Thr Phe Pro Asn Arg Ser Gly Ile Lys
           520                 525                 530
   ggt ctt tct tta aca gga tct tta gct gtt agg aaa gat ggt gag ttt    1747
   Gly Leu Ser Leu Thr Gly Ser Leu Ala Val Arg Lys Asp Gly Glu Phe
       535                 540                 545
   att gaa aac tta gga gtg gct cct cat att gat tta gga ttt acc tcc    1795
   Ile Glu Asn Leu Gly Val Ala Pro His Ile Asp Leu Gly Phe Thr Ser
   550                 555                 560                 565
   agg gat ttg caa act tcc agg ttt act gat tac gtt gag gca gtg aaa    1843
   Arg Asp Leu Gln Thr Ser Arg Phe Thr Asp Tyr Val Glu Ala Val Lys
                   570                 575                 580
   act ata gtt tta act tct ttg tct gag aac gct aag aag agt gaa gag   1891
   Thr Ile Val Leu Thr Ser Leu Ser Glu Asn Ala Lys Lys Ser Glu Glu
               585                 590                 595
   cag act tct ccg caa gag acg cct gaa gtt att cga gtc tct tat ccc   1939
   Gln Thr Ser Pro Gln Glu Thr Pro Glu Val Ile Arg Val Ser Tyr Pro
           600                 605                 610
   aca acg act tct gct tcg taaacgggac gtaatagaat aatttttatt          1987
   Thr Thr Thr Ser Ala Ser
       615
   attgctttaa tatgcgcgct tccaatataa gcattgtgaa gcgcgtttca tatgtctttt 2047
   atctttaggt aat                                                    2060
<210> 8
   <211> 619
   <212> PRT
   <213> Chlamydia pneumoniae
<220>
   <221> SITE
   <222> (328)...(343)
   <223> B-cell epitope
<220>
   <221> SITE
   <222> (589)...(606)
   <223> B-cell epitope
<220>
   <221> SITE
   <222> (135)...(144)
   <223> T-cell epitope
<220>
   <221> SITE
   <222> (373)...(382)
   <223> T-cell epitope
<400> 8
   Met Lys Lys Gly Lys Leu Gly Ala Ile Val Phe Gly Leu Leu Phe Thr
   1               5                   10                  15
   Ser Ser Val Ala Gly Phe Ser Lys Asp Leu Thr Lys Asp Asn Ala Tyr
               20                  25                  30
   Gln Asp Leu Asn Val Ile Glu His Leu Ile Ser Leu Lys Tyr Ala Pro
           35                  40                  45
   Leu Pro Trp Lys Glu Leu Leu Phe Gly Trp Asp Leu Ser Gln Gln Thr
       50                  55                  60
   Gln Gln Ala Arg Leu Gln Leu Val Leu Glu Glu Lys Pro Thr Thr Asn
   65                  70                  75                  80
   Tyr Cys Gln Lys Val Leu Ser Asn Tyr Val Arg Ser Leu Asn Asp Tyr
                   85                  90                  95
   His Ala Gly Ile Thr Phe Tyr Arg Thr Glu Ser Ala Tyr Ile Pro Tyr
   100 105 110
   Val Leu Lys Leu Ser Glu Asp Gly His Val Phe Val Val Asp Val Gln
   115 120 125
   Thr Ser Gln Gly Asp Ile Tyr Leu Gly Asp Glu Ile Leu Glu Val Asp
   130 135 140
   Gly Met Gly Ile Arg Glu Ala Ile Glu Ser Leu Arg Phe Gly Arg Gly
   145 150 155 160
   Ser Ala Thr Asp Tyr Ser Ala Ala Val Arg Ser Leu Thr Ser Arg Ser
   165 170 175
   Ala Ala Phe Gly Asp Ala Val Pro Ser Gly Ile Ala Met Leu Lys Leu
   180 185 190
   Arg Arg Pro Ser Gly Leu Ile Arg Ser Thr Pro Val Arg Trp Arg Tyr
   195 200 205
   Thr Pro Glu His Ile Gly Asp Phe Ser Leu Val Ala Pro Leu Ile Pro
   210 215 220
   Glu His Lys Pro Gln Leu Pro Thr Gln Ser Cys Val Leu Phe Arg Ser
   225 230 235 240
   Gly Val Asn Ser Gln Ser Ser Ser Ser Ser Leu Phe Ser Ser Tyr Met
   245 250 255
   Val Pro Tyr Phe Trp Glu Glu Leu Arg Val Gln Asn Lys Gln Arg Phe
   260 265 270
   Asp Ser Asn His His Ile Gly Ser Arg Asn Gly Phe Leu Pro Thr Phe
   275 280 285
   Gly Pro Ile Leu Trp Glu Gln Asp Lys Gly Pro Tyr Arg Ser Tyr Ile
   290 295 300
   Phe Lys Ala Lys Asp Ser Gln Gly Asn Pro His Arg Ile Gly Phe Leu
   305 310 315 320
   Arg Ile Ser Ser Tyr Val Trp Thr Asp Leu Glu Gly Leu Glu Glu Asp
   325 330 335
   His Lys Asp Ser Pro Trp Glu Leu Phe Gly Glu Ile Ile Asp His Leu
   340 345 350
   Glu Lys Glu Thr Asp Ala Leu Ile Ile Asp Gln Thr His Asn Pro Gly
   355 360 365
   Gly Ser Val Phe Tyr Leu Tyr Ser Leu Leu Ser Met Leu Thr Asp His
   370 375 380
   Pro Leu Asp Thr Pro Lys His Arg Met Ile Phe Thr Gln Asp Glu Val
   385 390 395 400
   Ser Ser Ala Leu His Trp Gln Asp Leu Leu Glu Asp Val Phe Thr Asp
   405 410 415
   Glu Gln Ala Val Ala Val Leu Gly Glu Thr Met Glu Gly Tyr Cys Met
               420                 425                 430
   Asp Met His Ala Val Ala Ser Leu Gln Asn Phe Ser Gln Ser Val Leu
           435                 440                 445
   Ser Ser Trp Val Ser Gly Asp Ile Asn Leu Ser Lys Pro Met Pro Leu
       450                 455                 460
   Leu Gly Phe Ala Gln Val Arg Pro His Pro Lys His Gln Tyr Thr Lys
   465                 470                 475                 480
   Pro Leu Phe Met Leu Ile Asp Glu Asp Asp Phe Ser Cys Gly Asp Leu
                   485                 490                 495
   Ala Pro Ala Ile Leu Lys Asp Asn Gly Arg Ala Thr Leu Ile Gly Lys
               500                 505                 510
   Pro Thr Ala Gly Ala Gly Gly Phe Val Phe Gln Val Thr Phe Pro Asn
           515                 520                 525
   Arg Ser Gly Ile Lys Gly Leu Ser Leu Thr Gly Ser Leu Ala Val Arg
       530                 535                 540
   Lys Asp Gly Glu Phe Ile Glu Asn Leu Gly Val Ala Pro His Ile Asp
   545                 550                 555                 560
   Leu Gly Phe Thr Ser Arg Asp Leu Gln Thr Ser Arg Phe Thr Asp Tyr
                   565                 570                 575
   Val Glu Ala Val Lys Thr Ile Val Leu Thr Ser Leu Ser Glu Asn Ala
               580                 585                 590
   Lys Lys Ser Glu Glu Gln Thr Ser Pro Gln Glu Thr Pro Glu Val Ile
           595                 600                 605
   Arg Val Ser Tyr Pro Thr Thr Thr Ser Ala Ser
       610                 615
<210> 9
   <211> 1133
   <212> DNA
   <213> Chlamydia pneumoniae
<220>
   <221> CDS
   <222> (101)..(1030)
<400> 9
   gacgtaatag aataattttt attattgctt taatatgcgc gcttccaata taagcattgt 60
   gaagcgcgtt tcatatgtct tttatcttta ggtaattatt atg aga aaa ctt att   115
                                               Met Arg Lys Leu Ile
                                                1               5
   tta tgc aat cct aga gga ttt tgc tct gga gtt gtg cgc gct att caa   163
   Leu Cys Asn Pro Arg Gly Phe Cys Ser Gly Val Val Arg Ala Ile Gln
                   10                  15                  20
   gtt gta gag gtt gct tta gaa aag tgg gga gct cct atc tat gta aaa    211
   Val Val Glu Val Ala Leu Glu Lys Trp Gly Ala Pro Ile Tyr Val Lys
               25                  30                  35
   cat gag att gtt cac aat cgc cat gtt gtt aat gct tta cga gcc aag    259
   His Glu Ile Val His Asn Arg His Val Val Asn Ala Leu Arg Ala Lys
           40                  45                  50
   gga gcg atc ttt gtt gaa gaa ctt gtt gat gtt cct gaa ggt gag aga    307
   Gly Ala Ile Phe Val Glu Glu Leu Val Asp Val Pro Glu Gly Glu Arg
       55                  60                  65
   gtc att tat tca gct cat gga att cct cct tca gtt aga gct gaa gca    355
   Val Ile Tyr Ser Ala His Gly Ile Pro Pro Ser Val Arg Ala Glu Ala
   70                  75                  80                  85
   aaa gcc cgt aag ctt att gat att gat gct acc tgt ggt ttg gtt act    403
   Lys Ala Arg Lys Leu Ile Asp Ile Asp Ala Thr Cys Gly Leu Val Thr
                   90                  95                  100
   aag gtg cat tct gct gcg aag tta tac gca agt aaa gga tac aaa atc    451
   Lys Val His Ser Ala Ala Lys Leu Tyr Ala Ser Lys Gly Tyr Lys Ile
               105                 110                 115
   ata ctg atc ggc cat aag aag cac gtt gag gtg att ggt att gtt gga    499
   Ile Leu Ile Gly His Lys Lys His Val Glu Val Ile Gly Ile Val Gly
           120                 125                 130
   gaa gtt cct gaa cac att act gtt gtc gag aag gtt gct gac gtc gag    547
   Glu Val Pro Glu His Ile Thr Val Val Glu Lys Val Ala Asp Val Glu
       135                 140                 145
   gcc tta cct ttt agt tct gat aca cct tta ttt tat att act caa acg    595
   Ala Leu Pro Phe Ser Ser Asp Thr Pro Leu Phe Tyr Ile Thr Gln Thr
   150                 155                 160                 165
   acg ttg agt ttg gat gat gtt cag gag atc tca tcg gct ttg cta aag    643
   Thr Leu Ser Leu Asp Asp Val Gln Glu Ile Ser Ser Ala Leu Leu Lys
                   170                 175                 180
   cga tat ccc tct atc att act ctg cct agt tct tcg att tgt tat gca    691
   Arg Tyr Pro Ser Ile Ile Thr Leu Pro Ser Ser Ser Ile Cys Tyr Ala
               185                 190                 195
   acc acg aac cgt caa aaa gca ttg cgt tct gtt tta tct cgc gtg aat    739
   Thr Thr Asn Arg Gln Lys Ala Leu Arg Ser Val Leu Ser Arg Val Asn
           200                 205                 210
   tac gtc tat gtg gtt gga gat gtc aac agc tcg aat tcc aat cgt ctt    787
   Tyr Val Tyr Val Val Gly Asp Val Asn Ser Ser Asn Ser Asn Arg Leu
       215                 220                 225
   cgc gaa gtg gct ttg aga agg gga gtt ccc gct gat ttg atc aac aat    835
   Arg Glu Val Ala Leu Arg Arg Gly Val Pro Ala Asp Leu Ile Asn Asn
   230                 235                 240                 245
   ccc gag gat att gat acg aac atc gta aat cat tct gga gat ata gca    883
   Pro Glu Asp Ile Asp Thr Asn Ile Val Asn His Ser Gly Asp Ile Ala
                   250                 255                 260
   atg act gca gga gcc tca act ccc gaa gac gta gtt caa gct tgc att     931
   Met Thr Ala Gly Ala Ser Thr Pro Glu Asp Val Val Gln Ala Cys Ile
               265                 270                 275
   cga aag cta tca tca ctt atc cct ggt tta caa gtg gaa aat gat ata     979
   Arg Lys Leu Ser Ser Leu Ile Pro Gly Leu Gln Val Glu Asn Asp Ile
           280                 285                 290
   ttt gct gta gag gat gtc gta ttt caa tta cca aaa gaa ctc cgt tgt     1027
   Phe Ala Val Glu Asp Val Val Phe Gln Leu Pro Lys Glu Leu Arg Cys
       295                 300                 305
   tct taggtcttta ggcttacttg ccaagttttt ctcgagattg ctttatagag          1080
   Ser
   310
   tcttcttctc gttcagagag ggtatttacc tttttagttc tctgtatttg aaa          1133
<210> 10
   <211> 310
   <212> PRT
   <213> Chlamydia pneumoniae
<220>
   <221> SITE
   <222> (198)...(205)
   <223> B-cell epitope
<220>
   <221> SITE
   <222> (221)...(231)
   <223> B-cell epitope
<220>
   <221> SITE
   <222> (207)...(216)
   <223> T-cell epitope
<220>
   <221> SITE
   <222> (279)...(288)
   <223> T-cell epitope
<220>
   <221> SITE
   <222> (118)...(127)
   <223> T-cell epitope
<400> 10
   Met Arg Lys Leu Ile Leu Cys Asn Pro Arg Gly Phe Cys Ser Gly Val
    1               5                  10                  15
   Val Arg Ala Ile Gln Val Val Glu Val Ala Leu Glu Lys Trp Gly Ala
               20                  25                  30
   Pro Ile Tyr Val Lys His Glu Ile Val His Asn Arg His Val Val Asn
           35                  40                  45
   Ala Leu Arg Ala Lys Gly Ala Ile Phe Val Glu Glu Leu Val Asp Val
   50 55 60
   Pro Glu Gly Glu Arg Val Ile Tyr Ser Ala His Gly Ile Pro Pro Ser
   65 70 75 80
   Val Arg Ala Glu Ala Lys Ala Arg Lys Leu Ile Asp Ile Asp Ala Thr
   85 90 95
   Cys Gly Leu Val Thr Lys Val His Ser Ala Ala Lys Leu Tyr Ala Ser
   100 105 110
   Lys Gly Tyr Lys Ile Ile Leu Ile Gly His Lys Lys His Val Glu Val
   115 120 125
   Ile Gly Ile Val Gly Glu Val Pro Glu His Ile Thr Val Val Glu Lys
   130 135 140
   Val Ala Asp Val Glu Ala Leu Pro Phe Ser Ser Asp Thr Pro Leu Phe
   145 150 155 160
   Tyr Ile Thr Gln Thr Thr Leu Ser Leu Asp Asp Val Gln Glu Ile Ser
   165 170 175
   Ser Ala Leu Leu Lys Arg Tyr Pro Ser Ile Ile Thr Leu Pro Ser Ser
   180 185 190
   Ser Ile Cys Tyr Ala Thr Thr Asn Arg Gln Lys Ala Leu Arg Ser Val
   195 200 205
   Leu Ser Arg Val Asn Tyr Val Tyr Val Val Gly Asp Val Asn Ser Ser
   210 215 220
   Asn Ser Asn Arg Leu Arg Glu Val Ala Leu Arg Arg Gly Val Pro Ala
   225 230 235 240
   Asp Leu Ile Asn Asn Pro Glu Asp Ile Asp Thr Asn Ile Val Asn His
   245 250 255
   Ser Gly Asp Ile Ala Met Thr Ala Gly Ala Ser Thr Pro Glu Asp Val
   260 265 270
   Val Gln Ala Cys Ile Arg Lys Leu Ser Ser Leu Ile Pro Gly Leu Gln
   275 280 285
   Val Glu Asn Asp Ile Phe Ala Val Glu Asp Val Val Phe Gln Leu Pro
   290 295 300
   Lys Glu Leu Arg Cys Ser
   305 310
<210> 11
   <211> 1466
   <212> DNA
   <213> Chlamydia pneumoniae
<220>
   <221> CDS
   <222> (101)..(1363)
<400> 11
   catgggagcc gaggaagcca tctcctacgg acttattgat aaggtggtaa cttctgcgaa 60
   agaaactaat aaggatacaa gtagcactta gagagaacat atg aat aaa aaa aat   115
                                               Met Asn Lys Lys Asn
                                                1               5
   cta act att tgt tca ttt tgc ggt cgg tct gaa aaa gat gta gag aaa   163
   Leu Thr Ile Cys Ser Phe Cys Gly Arg Ser Glu Lys Asp Val Glu Lys
                   10                  15                  20
   ctg att gct ggg cct tcg gta tac att tgt gac tac tgc atc aaa tta   211
   Leu Ile Ala Gly Pro Ser Val Tyr Ile Cys Asp Tyr Cys Ile Lys Leu
               25                  30                  35
   tgc tct gga att tta gat aag aaa ccc tcc tct aca ata tcc tca gct   259
   Cys Ser Gly Ile Leu Asp Lys Lys Pro Ser Ser Thr Ile Ser Ser Ala
           40                  45                  50
   cca gtt tct gaa aca cct tca cag cct tct gat ctc agg gtg ctt acc   307
   Pro Val Ser Glu Thr Pro Ser Gln Pro Ser Asp Leu Arg Val Leu Thr
       55                  60                  65
   cct aag gaa atc aaa aag cat att gat gaa tat gtc att ggt cag gaa   355
   Pro Lys Glu Ile Lys Lys His Ile Asp Glu Tyr Val Ile Gly Gln Glu
   70                  75                  80                  85
   aga gct aaa aag aca atc gct gtt gct gtt tat aat cac tat aaa cgt   403
   Arg Ala Lys Lys Thr Ile Ala Val Ala Val Tyr Asn His Tyr Lys Arg
                   90                  95                  100
   ata cgt gct cta cta cat aac aaa cag gta agc tac ggg aaa tct aac   451
   Ile Arg Ala Leu Leu His Asn Lys Gln Val Ser Tyr Gly Lys Ser Asn
               105                 110                 115
   gtg ctt ctc cta ggc cct aca gga tct gga aaa aca tta att gca aaa   499
   Val Leu Leu Leu Gly Pro Thr Gly Ser Gly Lys Thr Leu Ile Ala Lys
           120                 125                 130
   aca ttg gca aaa att tta gat gtt ccc ttc acc ata gcc gac gca acg   547
   Thr Leu Ala Lys Ile Leu Asp Val Pro Phe Thr Ile Ala Asp Ala Thr
       135                 140                 145
   acc cta acg gaa gca ggt tat gtc ggt gaa gat gta gag aac att gtc   595
   Thr Leu Thr Glu Ala Gly Tyr Val Gly Glu Asp Val Glu Asn Ile Val
   150                 155                 160                 165
   tta cgt tta tta caa gct gct gat tac gat gtc gcc cgt gca gaa cga   643
   Leu Arg Leu Leu Gln Ala Ala Asp Tyr Asp Val Ala Arg Ala Glu Arg
                   170                 175                 180
   ggc att atc tat atc gat gaa atc gat aaa att gga agg aca aca gca   691
   Gly Ile Ile Tyr Ile Asp Glu Ile Asp Lys Ile Gly Arg Thr Thr Ala
               185                 190                 195
   aac gtc tcc att act aga gat gtt tct ggc gaa ggg gtt caa caa gca   739
   Asn Val Ser Ile Thr Arg Asp Val Ser Gly Glu Gly Val Gln Gln Ala
           200                 205                 210
   ttg tta aaa atc gtt gaa gga acc aca gca aac gtt cct cct aaa gga    787
   Leu Leu Lys Ile Val Glu Gly Thr Thr Ala Asn Val Pro Pro Lys Gly
       215                 220                 225
   gga cgt aag cat cct aac caa gag tat atc cga gtc aat acg gaa aat    835
   Gly Arg Lys His Pro Asn Gln Glu Tyr Ile Arg Val Asn Thr Glu Asn
   230                 235                 240                 245
   atc tta ttt atc gta ggc gga gcc ttc gtc aac cta gat aag att atc    883
   Ile Leu Phe Ile Val Gly Gly Ala Phe Val Asn Leu Asp Lys Ile Ile
                   250                 255                 260
   gca aag cga ttg ggg aaa act acc ata ggg ttt tct gat gat caa gca    931
   Ala Lys Arg Leu Gly Lys Thr Thr Ile Gly Phe Ser Asp Asp Gln Ala
               265                 270                 275
   gac ctc tct caa aaa acc aga gac cat cta ctt gct aaa gtt gaa acc    979
   Asp Leu Ser Gln Lys Thr Arg Asp His Leu Leu Ala Lys Val Glu Thr
           280                 285                 290
   gaa gac ctg att gcc ttc gga atg atc cct gaa ttt gtc gga aga ttc    1027
   Glu Asp Leu Ile Ala Phe Gly Met Ile Pro Glu Phe Val Gly Arg Phe
       295                 300                 305
   aac tgc att gta aac tgt gaa gag ctt tct ttg gat gag ctt gta gcc    1075
   Asn Cys Ile Val Asn Cys Glu Glu Leu Ser Leu Asp Glu Leu Val Ala
   310                 315                 320                 325
   atc ctt aca gaa cct aca aat gcg att gtg aaa caa tat atg gag cta    1123
   Ile Leu Thr Glu Pro Thr Asn Ala Ile Val Lys Gln Tyr Met Glu Leu
                   330                 335                 340
   ttc gca gaa gaa aac gtc aag tta gtc ttc aaa aaa gaa gcc cta tat    1171
   Phe Ala Glu Glu Asn Val Lys Leu Val Phe Lys Lys Glu Ala Leu Tyr
               345                 350                 355
   gct ata gca aaa aaa gcc aag caa gca aaa act gga gct cgt gct cta    1219
   Ala Ile Ala Lys Lys Ala Lys Gln Ala Lys Thr Gly Ala Arg Ala Leu
           360                 365                 370
   ggg atg atc cta gaa aat ctc ctt aga gac ctt atg ttt gaa att cct    1267
   Gly Met Ile Leu Glu Asn Leu Leu Arg Asp Leu Met Phe Glu Ile Pro
       375                 380                 385
   tca gat cct aca gta gaa gct att cat atc caa gaa gac act atc gca    1315
   Ser Asp Pro Thr Val Glu Ala Ile His Ile Gln Glu Asp Thr Ile Ala
   390                 395                 400                 405
   gaa aat aaa gcg cca ata att atc aga agg acc cca gaa gct atc gct    1363
   Glu Asn Lys Ala Pro Ile Ile Ile Arg Arg Thr Pro Glu Ala Ile Ala
                   410                 415                 420
   tagctctttt tagttcctat tttaggggtg tcatgacaac aattgccata gaagctgcaa 1423
   aaaaagttct tatcaaacta cgtaatgcag gatatcaggc ata                  1466
<210> 12
   <211> 421
   <212> PRT
   <213> Chlamydia pneumoniae
<220>
   <221> SITE
   <222> (226)...(239)
   <223> B-cell epitope
<220>
   <221> SITE
   <222> (273)...(286)
   <223> B-cell epitope
<220>
   <221> SITE
   <222> (137)...(146)
   <223> T-cell epitope
<220>
   <221> SITE
   <222> (168)...(177)
   <223> T-cell epitope
<400> 12
   Met Asn Lys Lys Asn Leu Thr Ile Cys Ser Phe Cys Gly Arg Ser Glu
    1               5                  10                  15
   Lys Asp Val Glu Lys Leu Ile Ala Gly Pro Ser Val Tyr Ile Cys Asp
               20                  25                  30
   Tyr Cys Ile Lys Leu Cys Ser Gly Ile Leu Asp Lys Lys Pro Ser Ser
           35                  40                  45
   Thr Ile Ser Ser Ala Pro Val Ser Glu Thr Pro Ser Gln Pro Ser Asp
       50                  55                  60
   Leu Arg Val Leu Thr Pro Lys Glu Ile Lys Lys His Ile Asp Glu Tyr
   65                  70                  75                  80
   Val Ile Gly Gln Glu Arg Ala Lys Lys Thr Ile Ala Val Ala Val Tyr
                   85                  90                  95
   Asn His Tyr Lys Arg Ile Arg Ala Leu Leu His Asn Lys Gln Val Ser
               100                 105                 110
   Tyr Gly Lys Ser Asn Val Leu Leu Leu Gly Pro Thr Gly Ser Gly Lys
           115                 120                 125
   Thr Leu Ile Ala Lys Thr Leu Ala Lys Ile Leu Asp Val Pro Phe Thr
       130                 135                 140
   Ile Ala Asp Ala Thr Thr Leu Thr Glu Ala Gly Tyr Val Gly Glu Asp
   145                 150                 155                 160
   Val Glu Asn Ile Val Leu Arg Leu Leu Gln Ala Ala Asp Tyr Asp Val
                   165                 170                 175
   Ala Arg Ala Glu Arg Gly Ile Ile Tyr Ile Asp Glu Ile Asp Lys Ile
               180                 185                 190
   Gly Arg Thr Thr Ala Asn Val Ser Ile Thr Arg Asp Val Ser Gly Glu
   195 200 205
   Gly Val Gln Gln Ala Leu Leu Lys Ile Val Glu Gly Thr Thr Ala Asn
   210 215 220
   Val Pro Pro Lys Gly Gly Arg Lys His Pro Asn Gln Glu Tyr Ile Arg
   225 230 235 240
   Val Asn Thr Glu Asn Ile Leu Phe Ile Val Gly Gly Ala Phe Val Asn
   245 250 255
   Leu Asp Lys Ile Ile Ala Lys Arg Leu Gly Lys Thr Thr Ile Gly Phe
   260 265 270
   Ser Asp Asp Gln Ala Asp Leu Ser Gln Lys Thr Arg Asp His Leu Leu
   275 280 285
   Ala Lys Val Glu Thr Glu Asp Leu Ile Ala Phe Gly Met Ile Pro Glu
   290 295 300
   Phe Val Gly Arg Phe Asn Cys Ile Val Asn Cys Glu Glu Leu Ser Leu
   305 310 315 320
   Asp Glu Leu Val Ala Ile Leu Thr Glu Pro Thr Asn Ala Ile Val Lys
   325 330 335
   Gln Tyr Met Glu Leu Phe Ala Glu Glu Asn Val Lys Leu Val Phe Lys
   340 345 350
   Lys Glu Ala Leu Tyr Ala Ile Ala Lys Lys Ala Lys Gln Ala Lys Thr
   355 360 365
   Gly Ala Arg Ala Leu Gly Met Ile Leu Glu Asn Leu Leu Arg Asp Leu
   370 375 380
   Met Phe Glu Ile Pro Ser Asp Pro Thr Val Glu Ala Ile His Ile Gln
   385 390 395 400
   Glu Asp Thr Ile Ala Glu Asn Lys Ala Pro Ile Ile Ile Arg Arg Thr
   405 410 415
   Pro Glu Ala Ile Ala
   420
<210> 13
   <211> 812
   <212> DNA
   <213> Chlamydia pneumoniae
<220>
   <221> CDS
   <222> (101)..(709)
<400> 13
   tgacgtagac agcctaaaaa gtcttagcta cgttcctagg gtcatttcgt gatcgggaac 60
   gtatggacac aactgaaaat tatttgatga ggaaacgcaa atg aca ctg gta ccc    115
                                              Met Thr Leu Val Pro
                                               1               5
   tat gtt gtc gag gat acg ggc cgt ggt gaa agg gcc atg gat att tac    163
   Tyr Val Val Glu Asp Thr Gly Arg Gly Glu Arg Ala Met Asp Ile Tyr
                   10                  15                  20
   tcc cgt ctt ctg aaa gat cgt att gta atg atc ggt cag gaa atc acg    211
   Ser Arg Leu Leu Lys Asp Arg Ile Val Met Ile Gly Gln Glu Ile Thr
               25                  30                  35
   gag ccc ctc gca aac aca gta att gcc cag ctc ctt ttc ctc atg tcc    259
   Glu Pro Leu Ala Asn Thr Val Ile Ala Gln Leu Leu Phe Leu Met Ser
           40                  45                  50
   gaa gat cct aaa aag gat att caa att ttc atc aat tcc cca ggc ggc    307
   Glu Asp Pro Lys Lys Asp Ile Gln Ile Phe Ile Asn Ser Pro Gly Gly
       55                  60                  65
   tac atc acc gct gga ctg gca atc tat gat acc att cgc ttt tta ggt    355
   Tyr Ile Thr Ala Gly Leu Ala Ile Tyr Asp Thr Ile Arg Phe Leu Gly
   70                  75                  80                  85
   tgt gat gta aat acc tac tgc atc ggt caa gct gca tcc atg gga gcc    403
   Cys Asp Val Asn Thr Tyr Cys Ile Gly Gln Ala Ala Ser Met Gly Ala
                   90                  95                  100
   ctc tta tta tcc gca gga act aaa gga aag cgt cac gct ctt ccc cat    451
   Leu Leu Leu Ser Ala Gly Thr Lys Gly Lys Arg His Ala Leu Pro His
               105                 110                 115
   agc cgt atg atg atc cac caa cct tct gga ggc att atc gga aca tcc    499
   Ser Arg Met Met Ile His Gln Pro Ser Gly Gly Ile Ile Gly Thr Ser
           120                 125                 130
   gca gac atc caa ctc caa gca gct gaa att cta aca cta aaa aaa cac    547
   Ala Asp Ile Gln Leu Gln Ala Ala Glu Ile Leu Thr Leu Lys Lys His
       135                 140                 145
   ctt gcc aat atc ctc tct gaa tgc aca gga caa cct gta gaa aaa att    595
   Leu Ala Asn Ile Leu Ser Glu Cys Thr Gly Gln Pro Val Glu Lys Ile
   150                 155                 160                 165
   ata gaa gat tct gaa cga gat ttc ttc atg gga gcc gag gaa gcc atc    643
   Ile Glu Asp Ser Glu Arg Asp Phe Phe Met Gly Ala Glu Glu Ala Ile
                   170                 175                 180
   tcc tac gga ctt att gat aag gtg gta act tct gcg aaa gaa act aat    691
   Ser Tyr Gly Leu Ile Asp Lys Val Val Thr Ser Ala Lys Glu Thr Asn
               185                 190                 195
   aag gat aca agt agc act tagagagaac atatgaataa aaaaaatcta           739
   Lys Asp Thr Ser Ser Thr
           200
   actatttgtt cattttgcgg tcggtctgaa aaagatgtag agaaactgat tgctgggcct 799
   tcggtataca ttt                                                    812
<210> 14
   <211> 203
   <212> PRT
   <213> Chlamydia pneumoniae
<220>
   <221> SITE
   <222> (107)...(116)
   <223> B-cell epitope
<220>
   <221> SITE
   <222> (193)...(203)
   <223> B-cell epitope
<220>
   <221> SITE
   <222> (76)...(85)
   <223> T-cell epitope
<400> 14
   Met Thr Leu Val Pro Tyr Val Val Glu Asp Thr Gly Arg Gly Glu Arg
   1               5                   10                  15
   Ala Met Asp Ile Tyr Ser Arg Leu Leu Lys Asp Arg Ile Val Met Ile
               20                  25                  30
   Gly Gln Glu Ile Thr Glu Pro Leu Ala Asn Thr Val Ile Ala Gln Leu
           35                  40                  45
   Leu Phe Leu Met Ser Glu Asp Pro Lys Lys Asp Ile Gln Ile Phe Ile
       50                  55                  60
   Asn Ser Pro Gly Gly Tyr Ile Thr Ala Gly Leu Ala Ile Tyr Asp Thr
   65                  70                  75                  80
   Ile Arg Phe Leu Gly Cys Asp Val Asn Thr Tyr Cys Ile Gly Gln Ala
                   85                  90                  95
   Ala Ser Met Gly Ala Leu Leu Leu Ser Ala Gly Thr Lys Gly Lys Arg
               100                 105                 110
   His Ala Leu Pro His Ser Arg Met Met Ile His Gln Pro Ser Gly Gly
           115                 120                 125
   Ile Ile Gly Thr Ser Ala Asp Ile Gln Leu Gln Ala Ala Glu Ile Leu
       130                 135                 140
   Thr Leu Lys Lys His Leu Ala Asn Ile Leu Ser Glu Cys Thr Gly Gln
   145                 150                 155                 160
   Pro Val Glu Lys Ile Ile Glu Asp Ser Glu Arg Asp Phe Phe Met Gly
                   165                 170                 175
   Ala Glu Glu Ala Ile Ser Tyr Gly Leu Ile Asp Lys Val Val Thr Ser
               180                 185                 190
   Ala Lys Glu Thr Asn Lys Asp Thr Ser Ser Thr
           195                 200
<210> 15
   <211> 2162
   <212> DNA
   <213> Chlamydia pneumoniae
<220>
   <221> CDS
   <222> (101) .. (2059)
<400> 15
   gataaaatag aaagacctga tcatttgatg gaaatagcag ctcttcccga ataccaatat 60
   ttggaatatc cctcagaaga aagtatcagt cttttatcct atg agc tac cgt aaa   115
                                               Met Ser Tyr Arg Lys
                                                1               5
   cgt tcg act cta att gtt cta gga gtg ttt gct ctt tat gct ctt cta   163
   Arg Ser Thr Leu Ile Val Leu Gly Val Phe Ala Leu Tyr Ala Leu Leu
                    10                  15                  20
   gta ttg cgt tat tat aaa att caa att tgt gaa gga gac cac tgg gcc   211
   Val Leu Arg Tyr Tyr Lys Ile Gln Ile Cys Glu Gly Asp His Trp Ala
                25                  30                  35
   gca gaa gct ctc ggg caa cac gaa ttt tgt gtc cgt gat cct ttt cga   259
   Ala Glu Ala Leu Gly Gln His Glu Phe Cys Val Arg Asp Pro Phe Arg
            40                  45                  50
   agg ggc acc ttt ttt gct aac acg aca gta cgt aag gga gac aaa gac   307
   Arg Gly Thr Phe Phe Ala Asn Thr Thr Val Arg Lys Gly Asp Lys Asp
        55                  60                  65
   ctt cag cag cct ttc gct gtc gat att aca aaa ttt cac ctt tgt gca   355
   Leu Gln Gln Pro Phe Ala Val Asp Ile Thr Lys Phe His Leu Cys Ala
    70                  75                  80                  85
   gat cct tta gct att ccc gaa tgt cat cgt gat gag atc atc caa ggg   403
   Asp Pro Leu Ala Ile Pro Glu Cys His Arg Asp Glu Ile Ile Gln Gly
                    90                  95                  100
   att ctc caa ttt att gag ggg cag acc tac gac gac ctc tcc cta aag   451
   Ile Leu Gln Phe Ile Glu Gly Gln Thr Tyr Asp Asp Leu Ser Leu Lys
                105                 110                 115
   tta gat aag aaa tct cgg tat tgt aag ctg tat cct tta tta gat gtt   499
   Leu Asp Lys Lys Ser Arg Tyr Cys Lys Leu Tyr Pro Leu Leu Asp Val
            120                 125                 130
   tct gtc cat gac cgg cta tcc ctt tgg tgg aaa gga tat gca aca aag   547
   Ser Val His Asp Arg Leu Ser Leu Trp Trp Lys Gly Tyr Ala Thr Lys
        135                 140                 145
   cat cgc tta cca aca aac gcc cta ttt ttt att acg gac tac caa cgc   595
   His Arg Leu Pro Thr Asn Ala Leu Phe Phe Ile Thr Asp Tyr Gln Arg
   150                 155                 160                 165
   tcg tat cct ttt ggg aag ctc ctt gga caa gtt ctc cat acc tta aga   643
   Ser Tyr Pro Phe Gly Lys Leu Leu Gly Gln Val Leu His Thr Leu Arg
                   170                 175                 180
   gaa att aag gat gag aaa aca gga aaa gcc ttt ccc aca ggc ggg atg     691
   Glu Ile Lys Asp Glu Lys Thr Gly Lys Ala Phe Pro Thr Gly Gly Met
               185                 190                 195
   gag gcg tac ttt aat cat att ctg gaa ggg gac gtt gga gag aga aag     739
   Glu Ala Tyr Phe Asn His Ile Leu Glu Gly Asp Val Gly Glu Arg Lys
           200                 205                 210
   ctg ttg cgt tct cct ttg aac cgt tta gat acg aat cgt gtt atc aaa     787
   Leu Leu Arg Ser Pro Leu Asn Arg Leu Asp Thr Asn Arg Val Ile Lys
       215                 220                 225
   ctg cct aaa gat ggc tct gat atc tac ctt acg atc aat cct gtg atc     835
   Leu Pro Lys Asp Gly Ser Asp Ile Tyr Leu Thr Ile Asn Pro Val Ile
   230                 235                 240                 245
   cag acc att gca gag gaa gaa ctc gaa cgg ggc gtg cta gaa gct aaa     883
   Gln Thr Ile Ala Glu Glu Glu Leu Glu Arg Gly Val Leu Glu Ala Lys
                   250                 255                 260
   gcc cag ggg ggt agg ctc att cta atg aac tcc caa aca gga gag att     931
   Ala Gln Gly Gly Arg Leu Ile Leu Met Asn Ser Gln Thr Gly Glu Ile
               265                 270                 275
   ctt gca ctg gct caa tat ccg ttt ttc gat ccc aca aat tat aag gaa     979
   Leu Ala Leu Ala Gln Tyr Pro Phe Phe Asp Pro Thr Asn Tyr Lys Glu
           280                 285                 290
   tac ttc aat aac aaa gag cgc atc gaa cat acg aag gta tct ttt gtg     1027
   Tyr Phe Asn Asn Lys Glu Arg Ile Glu His Thr Lys Val Ser Phe Val
       295                 300                 305
   agc gat gtt ttt gaa ccc ggg tcg atc atg aaa cct ttg act gtg gcg     1075
   Ser Asp Val Phe Glu Pro Gly Ser Ile Met Lys Pro Leu Thr Val Ala
   310                 315                 320                 325
   att gct tta caa gct aac gaa gag gct agc tta aaa tcg cag aaa aag     1123
   Ile Ala Leu Gln Ala Asn Glu Glu Ala Ser Leu Lys Ser Gln Lys Lys
                   330                 335                 340
   att ttt gat cct gaa gaa cct atc gat gtg acc agg aca ctc ttc cct     1171
   Ile Phe Asp Pro Glu Glu Pro Ile Asp Val Thr Arg Thr Leu Phe Pro
               345                 350                 355
   gga cga aaa gga tct ccg ctt aag gat att tct aga aac tct caa ttg     1219
   Gly Arg Lys Gly Ser Pro Leu Lys Asp Ile Ser Arg Asn Ser Gln Leu
           360                 365                 370
   aat atg tac atg gct atc cag aaa tct tcg aat gtc tat gta gct cag     1267
   Asn Met Tyr Met Ala Ile Gln Lys Ser Ser Asn Val Tyr Val Ala Gln
       375                 380                 385
   ctg gct gac cgc atc ata caa tct tta gga gtg gcc tgg tac caa cag     1315
   Leu Ala Asp Arg Ile Ile Gln Ser Leu Gly Val Ala Trp Tyr Gln Gln
   390                 395                 400                 405
   aag ttg cta gct ctg gga ttt gga aga aaa aca ggg atc gag ctt ccc     1363
   Lys Leu Leu Ala Leu Gly Phe Gly Arg Lys Thr Gly Ile Glu Leu Pro
                   410                 415                 420
   agt gag gcc tct ggt ttg gtg cct tct ccc cat cgt ttc cat att aat   1411
   Ser Glu Ala Ser Gly Leu Val Pro Ser Pro His Arg Phe His Ile Asn
               425                 430                 435
   ggt tcc ctg gaa tgg tcc tta tct act cca tat tct ttg gct atg gga   1459
   Gly Ser Leu Glu Trp Ser Leu Ser Thr Pro Tyr Ser Leu Ala Met Gly
           440                 445                 450
   tat aat att ttg gca aca ggg ata caa atg gtt caa gcc tac gct atc   1507
   Tyr Asn Ile Leu Ala Thr Gly Ile Gln Met Val Gln Ala Tyr Ala Ile
       455                 460                 465
   ctt gca aac gga ggt tat gcc gtc cgg ccc act tta gta aaa aag atc   1555
   Leu Ala Asn Gly Gly Tyr Ala Val Arg Pro Thr Leu Val Lys Lys Ile
   470                 475                 480                 485
   gtc tct gct tca gga gag gaa tat cat ctt cct act aaa gag aag aca   1603
   Val Ser Ala Ser Gly Glu Glu Tyr His Leu Pro Thr Lys Glu Lys Thr
                   490                 495                 500
   cga ctc ttt tca gaa gaa att act aga gaa gtt gtt cgt gcc atg cgt   1651
   Arg Leu Phe Ser Glu Glu Ile Thr Arg Glu Val Val Arg Ala Met Arg
               505                 510                 515
   ttt aca acg tta ccc gga ggt tcg gga ttt cga gcc tct cct aag cat   1699
   Phe Thr Thr Leu Pro Gly Gly Ser Gly Phe Arg Ala Ser Pro Lys His
           520                 525                 530
   cac tct agt gct ggg aaa aca gga act aca gaa aag atg att cat gga   1747
   His Ser Ser Ala Gly Lys Thr Gly Thr Thr Glu Lys Met Ile His Gly
       535                 540                 545
   aaa tat gat aaa cgc cgt cat att gct tct ttt ata ggt ttt act ccc   1795
   Lys Tyr Asp Lys Arg Arg His Ile Ala Ser Phe Ile Gly Phe Thr Pro
   550                 555                 560                 565
   gta gag agc tcg gag gga aat ttc cca cct tta gtg atg ctc gtc tcc   1843
   Val Glu Ser Ser Glu Gly Asn Phe Pro Pro Leu Val Met Leu Val Ser
                   570                 575                 580
   ata gat gat cct gaa tat ggt ttg cga gcc gac ggc acg aaa aat tat   1891
   Ile Asp Asp Pro Glu Tyr Gly Leu Arg Ala Asp Gly Thr Lys Asn Tyr
               585                 590                 595
   atg ggg ggg cgt tgt gcg gca ccc att ttt tct agg gtt gct gac cgc   1939
   Met Gly Gly Arg Cys Ala Ala Pro Ile Phe Ser Arg Val Ala Asp Arg
           600                 605                 610
   aca ctc ctc tat tta ggg att ctt cca gac aag aag cta aga aat tgc   1987
   Thr Leu Leu Tyr Leu Gly Ile Leu Pro Asp Lys Lys Leu Arg Asn Cys
       615                 620                 625
   gac gaa gaa gct gct gca tta aag cgt ctc tat gaa gaa tgg aat cgt   2035
   Asp Glu Glu Ala Ala Ala Leu Lys Arg Leu Tyr Glu Glu Trp Asn Arg
   630                 635                 640                 645
   tct ccg aaa caa ggg gga acg agg tgaggatctc tatttccatc ttgctataga   2089
   Ser Pro Lys Gln Gly Gly Thr Arg
                   650
   cttttaccgt tgagcaaaga ctctctatca gagagcccgt ctcctcttta tcctctatga 2149
   gtagtttatg tta                                                    2162
<210> 16
   <211> 653
   <212> PRT
   <213> Chlamydia pneumoniae
<220>
   <221> SITE
   <222> (287)...(306)
   <223> B-cell epitope
<220>
   <221> SITE
   <222> (637)...(653)
   <223> B-cell epitope
<220>
   <221> SITE
   <222> (40) ... (48)
   <223> T-cell epitope
<220>
   <221> SITE
   <222> (456) ... (465)
   <223> T-cell epitope
<400> 16
   Met Ser Tyr Arg Lys Arg Ser Thr Leu Ile Val Leu Gly Val Phe Ala
    1               5                  10                  15
   Leu Tyr Ala Leu Leu Val Leu Arg Tyr Tyr Lys Ile Gln Ile Cys Glu
               20                  25                  30
   Gly Asp His Trp Ala Ala Glu Ala Leu Gly Gln His Glu Phe Cys Val
           35                  40                  45
   Arg Asp Pro Phe Arg Arg Gly Thr Phe Phe Ala Asn Thr Thr Val Arg
       50                  55                  60
   Lys Gly Asp Lys Asp Leu Gln Gln Pro Phe Ala Val Asp Ile Thr Lys
   65                  70                  75                  80
   Phe His Leu Cys Ala Asp Pro Leu Ala Ile Pro Glu Cys His Arg Asp
                   85                  90                  95
   Glu Ile Ile Gln Gly Ile Leu Gln Phe Ile Glu Gly Gln Thr Tyr Asp
               100                 105                 110
   Asp Leu Ser Leu Lys Leu Asp Lys Lys Ser Arg Tyr Cys Lys Leu Tyr
           115                 120                 125
   Pro Leu Leu Asp Val Ser Val His Asp Arg Leu Ser Leu Trp Trp Lys
       130                 135                 140
   Gly Tyr Ala Thr Lys His Arg Leu Pro Thr Asn Ala Leu Phe Phe Ile
   145                 150                 155                 160
   Thr Asp Tyr Gln Arg Ser Tyr Pro Phe Gly Lys Leu Leu Gly Gln Val
   165 170 175
   Leu His Thr Leu Arg Glu Ile Lys Asp Glu Lys Thr Gly Lys Ala Phe
   180 185 190
   Pro Thr Gly Gly Met Glu Ala Tyr Phe Asn His Ile Leu Glu Gly Asp
   195 200 205
   Val Gly Glu Arg Lys Leu Leu Arg Ser Pro Leu Asn Arg Leu Asp Thr
   210 215 220
   Asn Arg Val Ile Lys Leu Pro Lys Asp Gly Ser Asp Ile Tyr Leu Thr
   225 230 235 240
   Ile Asn Pro Val Ile Gln Thr Ile Ala Glu Glu Glu Leu Glu Arg Gly
   245 250 255
   Val Leu Glu Ala Lys Ala Gln Gly Gly Arg Leu Ile Leu Met Asn Ser
   260 265 270
   Gln Thr Gly Glu Ile Leu Ala Leu Ala Gln Tyr Pro Phe Phe Asp Pro
   275 280 285
   Thr Asn Tyr Lys Glu Tyr Phe Asn Asn Lys Glu Arg Ile Glu His Thr
   290 295 300
   Lys Val Ser Phe Val Ser Asp Val Phe Glu Pro Gly Ser Ile Met Lys
   305 310 315 320
   Pro Leu Thr Val Ala Ile Ala Leu Gln Ala Asn Glu Glu Ala Ser Leu
   325 330 335
   Lys Ser Gln Lys Lys Ile Phe Asp Pro Glu Glu Pro Ile Asp Val Thr
   340 345 350
   Arg Thr Leu Phe Pro Gly Arg Lys Gly Ser Pro Leu Lys Asp Ile Ser
   355 360 365
   Arg Asn Ser Gln Leu Asn Met Tyr Met Ala Ile Gln Lys Ser Ser Asn
   370 375 380
   Val Tyr Val Ala Gln Leu Ala Asp Arg Ile Ile Gln Ser Leu Gly Val
   385 390 395 400
   Ala Trp Tyr Gln Gln Lys Leu Leu Ala Leu Gly Phe Gly Arg Lys Thr
   405 410 415
   Gly Ile Glu Leu Pro Ser Glu Ala Ser Gly Leu Val Pro Ser Pro His
   420 425 430
   Arg Phe His Ile Asn Gly Ser Leu Glu Trp Ser Leu Ser Thr Pro Tyr
   435 440 445
   Ser Leu Ala Met Gly Tyr Asn Ile Leu Ala Thr Gly Ile Gln Met Val
   450 455 460
   Gln Ala Tyr Ala Ile Leu Ala Asn Gly Gly Tyr Ala Val Arg Pro Thr
   465 470 475 480
   Leu Val Lys Lys Ile Val Ser Ala Ser Gly Glu Glu Tyr His Leu Pro
   485 490 495
   Thr Lys Glu Lys Thr Arg Leu Phe Ser Glu Glu Ile Thr Arg Glu Val
   500 505 510
   Val Arg Ala Met Arg Phe Thr Thr Leu Pro Gly Gly Ser Gly Phe Arg
   515 520 525
   Ala Ser Pro Lys His His Ser Ser Ala Gly Lys Thr Gly Thr Thr Glu
   530 535 540
   Lys Met Ile His Gly Lys Tyr Asp Lys Arg Arg His Ile Ala Ser Phe
   545 550 555 560
   Ile Gly Phe Thr Pro Val Glu Ser Ser Glu Gly Asn Phe Pro Pro Leu
   565 570 575
   Val Met Leu Val Ser Ile Asp Asp Pro Glu Tyr Gly Leu Arg Ala Asp
   580 585 590
   Gly Thr Lys Asn Tyr Met Gly Gly Arg Cys Ala Ala Pro Ile Phe Ser
   595 600 605
   Arg Val Ala Asp Arg Thr Leu Leu Tyr Leu Gly Ile Leu Pro Asp Lys
   610 615 620
   Lys Leu Arg Asn Cys Asp Glu Glu Ala Ala Ala Leu Lys Arg Leu Tyr
   625 630 635 640
   Glu Glu Trp Asn Arg Ser Pro Lys Gln Gly Gly Thr Arg
   645 650
<210> 17
   <211> 2738
   <212> DNA
   <213> Chlamydia pneumoniae
<220>
   <221> CDS
   <222> (101)..(2635)
<400> 17
   gaattttacc aaatttgctg gtttagagcg aagagttgca tcattatttt aaatttcgta 60
   tatgcttaag gaaagttcta cccctgtctt ttaggttttt atg ttt gag aag ttc   115
                                               Met Phe Glu Lys Phe
                                                1               5
   act aat aga gca aaa caa gtc att aaa ctg gcg aaa aag gag gct cag   163
   Thr Asn Arg Ala Lys Gln Val Ile Lys Leu Ala Lys Lys Glu Ala Gln
                   10                  15                  20
   cgt tta aat cat aac tac ctg ggt act gag cac atc ctg ctt ggt ctt   211
   Arg Leu Asn His Asn Tyr Leu Gly Thr Glu His Ile Leu Leu Gly Leu
               25                  30                  35
   ctc aaa ctt ggt caa ggg gta gct gtt aat gta tta cgc aac ctc ggt      259
   Leu Lys Leu Gly Gln Gly Val Ala Val Asn Val Leu Arg Asn Leu Gly
           40                  45                  50
   ata gat ttt gat acg gca cgg caa gag gtg gaa cgc ctg att ggt tat      307
   Ile Asp Phe Asp Thr Ala Arg Gln Glu Val Glu Arg Leu Ile Gly Tyr
       55                  60                  65
   ggt cca gaa att caa gtc tac gga gac cct gcc ctt aca gga aga gta      355
   Gly Pro Glu Ile Gln Val Tyr Gly Asp Pro Ala Leu Thr Gly Arg Val
   70                  75                  80                  85
   aaa aaa tct ttt gaa tca gca aat gaa gag gcc agc ctt tta gag cac      403
   Lys Lys Ser Phe Glu Ser Ala Asn Glu Glu Ala Ser Leu Leu Glu His
                   90                  95                  100
   aat tat gtc ggg acg gag cat tta ctc tta ggg atc cta cat caa tca      451
   Asn Tyr Val Gly Thr Glu His Leu Leu Leu Gly Ile Leu His Gln Ser
               105                 110                 115
   gat agt gtc gct ctt cag gta tta gaa aac tta cat atc gat cca aga      499
   Asp Ser Val Ala Leu Gln Val Leu Glu Asn Leu His Ile Asp Pro Arg
           120                 125                 130
   gag gtt cgt aag gaa att ctt aga gaa tta gag acc ttc aat cta caa      547
   Glu Val Arg Lys Glu Ile Leu Arg Glu Leu Glu Thr Phe Asn Leu Gln
       135                 140                 145
   ctt cct cct tcg tcg tcg tct tct tcc tca tcc tct cga agc aac cct      595
   Leu Pro Pro Ser Ser Ser Ser Ser Ser Ser Ser Ser Arg Ser Asn Pro
   150                 155                 160                 165
   tca tct tca aaa tct cct tta ggt cat agc tta ggt tct gac aaa aac      643
   Ser Ser Ser Lys Ser Pro Leu Gly His Ser Leu Gly Ser Asp Lys Asn
                   170                 175                 180
   gaa aag ctt tct gct ctg aaa gca tat ggt tat gat tta acg gag atg      691
   Glu Lys Leu Ser Ala Leu Lys Ala Tyr Gly Tyr Asp Leu Thr Glu Met
               185                 190                 195
   gtc cga gag tct aag ctc gat cct gtc att ggt cgt tct tca gaa gtc      739
   Val Arg Glu Ser Lys Leu Asp Pro Val Ile Gly Arg Ser Ser Glu Val
           200                 205                 210
   gaa cgg ttg att ttg att ctt tgc cga aga aga aaa aac aat cct gta      787
   Glu Arg Leu Ile Leu Ile Leu Cys Arg Arg Arg Lys Asn Asn Pro Val
       215                 220                 225
   ctt att gga gaa gct gga gtt ggt aag act gca att gtt gag ggt ctg      835
   Leu Ile Gly Glu Ala Gly Val Gly Lys Thr Ala Ile Val Glu Gly Leu
   230                 235                 240                 245
   gct caa aaa atc att ctg aat gag gtt cct gat gcc tta cgg aaa aag      883
   Ala Gln Lys Ile Ile Leu Asn Glu Val Pro Asp Ala Leu Arg Lys Lys
                   250                 255                 260
   cga ctg att act cta gat cta gca tta atg att gct gga aca aaa tat      931
   Arg Leu Ile Thr Leu Asp Leu Ala Leu Met Ile Ala Gly Thr Lys Tyr
               265                 270                 275
   cga ggg caa ttt gag gaa cgg atc aaa gct gtc atg gat gaa gtt cgc     979
   Arg Gly Gln Phe Glu Glu Arg Ile Lys Ala Val Met Asp Glu Val Arg
           280                 285                 290
   aag cat gga aac atc ttg ctc ttc att gac gag ctc cac acg att gta    1027
   Lys His Gly Asn Ile Leu Leu Phe Ile Asp Glu Leu His Thr Ile Val
       295                 300                 305
   gga gca gga gca gct gaa ggt gct atc gat gct tca aac att tta aaa    1075
   Gly Ala Gly Ala Ala Glu Gly Ala Ile Asp Ala Ser Asn Ile Leu Lys
   310                 315                 320                 325
   cct gcg tta gcg cga ggt gaa att cag tgt att gga gca act acg ata    1123
   Pro Ala Leu Ala Arg Gly Glu Ile Gln Cys Ile Gly Ala Thr Thr Ile
                   330                 335                 340
   gat gag tat cgc aag cac ata gaa aaa gac gca gct tta gaa cgt cgt    1171
   Asp Glu Tyr Arg Lys His Ile Glu Lys Asp Ala Ala Leu Glu Arg Arg
               345                 350                 355
   ttc caa aaa atc gtg gtt cac cct cct agt gta gat gag act att gag    1219
   Phe Gln Lys Ile Val Val His Pro Pro Ser Val Asp Glu Thr Ile Glu
           360                 365                 370
   att tta cgt ggc ctc aag aaa aag tat gaa gaa cat cac aat gtc ttc    1267
   Ile Leu Arg Gly Leu Lys Lys Lys Tyr Glu Glu His His Asn Val Phe
       375                 380                 385
   att act gaa gaa gct tta aaa gca gct gcg act ctt tct gat caa tat    1315
   Ile Thr Glu Glu Ala Leu Lys Ala Ala Ala Thr Leu Ser Asp Gln Tyr
   390                 395                 400                 405
   gtt cat gga cgt ttc ctc cct gat aaa gca ata gat ctt tta gat gaa    1363
   Val His Gly Arg Phe Leu Pro Asp Lys Ala Ile Asp Leu Leu Asp Glu
                   410                 415                 420
   gct ggg gct cgt gtc cgt gtg aat aca atg ggt cag cct aca gat tta    1411
   Ala Gly Ala Arg Val Arg Val Asn Thr Met Gly Gln Pro Thr Asp Leu
               425                 430                 435
   atg aag cta gag gct gaa atc gaa aat aca aaa ttg gcc aaa gag cag    1459
   Met Lys Leu Glu Ala Glu Ile Glu Asn Thr Lys Leu Ala Lys Glu Gln
           440                 445                 450
   gcc att gga act caa gaa tac gaa aaa gct gca ggt tta cgt gat gaa    1507
   Ala Ile Gly Thr Gln Glu Tyr Glu Lys Ala Ala Gly Leu Arg Asp Glu
       455                 460                 465
   gag aaa aaa ctt cgc gaa cgt ctg caa agt atg aaa cag gaa tgg gaa    1555
   Glu Lys Lys Leu Arg Glu Arg Leu Gln Ser Met Lys Gln Glu Trp Glu
   470                 475                 480                 485
   aat cat aaa gaa gag cac caa gtt cct gta gat gaa gaa gca gtc gct    1603
   Asn His Lys Glu Glu His Gln Val Pro Val Asp Glu Glu Ala Val Ala
                   490                 495                 500
   cag gta gtt tct cta caa aca gga att ccc tca gca agg ctc aca gaa    1651
   Gln Val Val Ser Leu Gln Thr Gly Ile Pro Ser Ala Arg Leu Thr Glu
               505                 510                 515
   gct gaa agt gag aag ctt ctg aag tta gaa gac acg tta aga aga aaa     1699
   Ala Glu Ser Glu Lys Leu Leu Lys Leu Glu Asp Thr Leu Arg Arg Lys
           520                 525                 530
   gtc att ggt caa aat gat gcc gtt acc agc att tgc cgt gcc atc cga     1747
   Val Ile Gly Gln Asn Asp Ala Val Thr Ser Ile Cys Arg Ala Ile Arg
       535                 540                 545
   cgt tct cga aca ggg atc aaa gat cct aac cga cct acg ggc tcc ttc     1795
   Arg Ser Arg Thr Gly Ile Lys Asp Pro Asn Arg Pro Thr Gly Ser Phe
   550                 555                 560                 565
   cta ttc ctt ggg cct acc ggt gta ggg aaa agc ctg ctc gcc caa caa     1843
   Leu Phe Leu Gly Pro Thr Gly Val Gly Lys Ser Leu Leu Ala Gln Gln
                   570                 575                 580
   att gct ata gag atg ttc ggt ggt gaa gac gct ctg att cag gta gac     1891
   Ile Ala Ile Glu Met Phe Gly Gly Glu Asp Ala Leu Ile Gln Val Asp
               585                 590                 595
   atg tca gag tac atg gag aaa ttt gct gct acc aag atg atg gga tca     1939
   Met Ser Glu Tyr Met Glu Lys Phe Ala Ala Thr Lys Met Met Gly Ser
           600                 605                 610
   cct cca gga tat gta ggt cat gaa gaa ggg ggc cac ctt acg gaa cag     1987
   Pro Pro Gly Tyr Val Gly His Glu Glu Gly Gly His Leu Thr Glu Gln
       615                 620                 625
   gta cgt cgc cgt cct tac tgc gtt gtt ctc ttt gat gag ata gaa aag     2035
   Val Arg Arg Arg Pro Tyr Cys Val Val Leu Phe Asp Glu Ile Glu Lys
   630                 635                 640                 645
   gca cac cca gac att atg gac ctg atg ttg caa att tta gag caa gga     2083
   Ala His Pro Asp Ile Met Asp Leu Met Leu Gln Ile Leu Glu Gln Gly
                   650                 655                 660
   cgt ctt act gat tct ttt ggt cgc aaa gtg gat ttc cgt cat gcc att     2131
   Arg Leu Thr Asp Ser Phe Gly Arg Lys Val Asp Phe Arg His Ala Ile
               665                 670                 675
   att atc atg acc tcc aat ttg gga gct gat ctc att cgt aaa agc gga     2179
   Ile Ile Met Thr Ser Asn Leu Gly Ala Asp Leu Ile Arg Lys Ser Gly
           680                 685                 690
   gaa att ggt ttt ggc ttg aag tcc cat atg gac tat aag gtc atc caa     2227
   Glu Ile Gly Phe Gly Leu Lys Ser His Met Asp Tyr Lys Val Ile Gln
       695                 700                 705
   gag aaa atc gaa cat gct atg aag aaa cac tta aag cct gag ttc att     2275
   Glu Lys Ile Glu His Ala Met Lys Lys His Leu Lys Pro Glu Phe Ile
   710                 715                 720                 725
   aac cgt ttg gat gaa agt gtg att ttc cgt ccc ctc gag aaa gaa tct     2323
   Asn Arg Leu Asp Glu Ser Val Ile Phe Arg Pro Leu Glu Lys Glu Ser
                   730                 735                 740
   cta tcg gag atc atc cat tta gag atc aac aaa ctg gac tcg aga ctg     2371
   Leu Ser Glu Ile Ile His Leu Glu Ile Asn Lys Leu Asp Ser Arg Leu
               745                 750                 755
   aaa aac tac caa atg gct ttg aac atc cca gac tct gtg att tcc ttc    2419
   Lys Asn Tyr Gln Met Ala Leu Asn Ile Pro Asp Ser Val Ile Ser Phe
           760                 765                 770
   cta gta acg aag ggg cat tct cca gaa atg gga gca cgt cct cta cgc    2467
   Leu Val Thr Lys Gly His Ser Pro Glu Met Gly Ala Arg Pro Leu Arg
       775                 780                 785
   cgt gtc att gag cag tac ctt gaa gat cct cta gcg gag ctc ttg ctt    2515
   Arg Val Ile Glu Gln Tyr Leu Glu Asp Pro Leu Ala Glu Leu Leu Leu
   790                 795                 800                 805
   aaa gag tcc tgc cgt caa gaa gct cgc aag cta cga gca acc ttg gtt    2563
   Lys Glu Ser Cys Arg Gln Glu Ala Arg Lys Leu Arg Ala Thr Leu Val
                   810                 815                 820
   gaa aat cgc gtt gcc ttt gaa agg gaa gaa gag gag cag gaa gct gct    2611
   Glu Asn Arg Val Ala Phe Glu Arg Glu Glu Glu Glu Gln Glu Ala Ala
               825                 830                 835
   ctc cct agc cct cac ttg gaa tca taggaacgtc gataactcca ctaccaaggc   2665
   Leu Pro Ser Pro His Leu Glu Ser
           840                 845
   aggtatctcc ttgataaaac gctattgttt gtcctggagt taccgccttg acgggttgtg 2725
   aaaatcgcac ctt                                                    2738
<210> 18
   <211> 845
   <212> PRT
   <213> Chlamydia pneumoniae
<220>
   <221> SITE
   <222> (467) ... (492)
   <223> B-cell epitope
<220>
   <221> SITE
   <222> (548)...(563)
   <223> B-cell epitope
<220>
   <221> SITE
   <222> (565)...(574)
   <223> T-cell epitope
<220>
   <221> SITE
   <222> (410) ... (418)
   <223> T-cell epitope
<400> 18
   Met Phe Glu Lys Phe Thr Asn Arg Ala Lys Gln Val Ile Lys Leu Ala
   1 5 10 15
   Lys Lys Glu Ala Gln Arg Leu Asn His Asn Tyr Leu Gly Thr Glu His
   20 25 30
   Ile Leu Leu Gly Leu Leu Lys Leu Gly Gln Gly Val Ala Val Asn Val
           35                  40                  45
   Leu Arg Asn Leu Gly Ile Asp Phe Asp Thr Ala Arg Gln Glu Val Glu
       50                  55                  60
   Arg Leu Ile Gly Tyr Gly Pro Glu Ile Gln Val Tyr Gly Asp Pro Ala
   65                  70                  75                  80
   Leu Thr Gly Arg Val Lys Lys Ser Phe Glu Ser Ala Asn Glu Glu Ala
                   85                  90                  95
   Ser Leu Leu Glu His Asn Tyr Val Gly Thr Glu His Leu Leu Leu Gly
               100                 105                 110
   Ile Leu His Gln Ser Asp Ser Val Ala Leu Gln Val Leu Glu Asn Leu
           115                 120                 125
   His Ile Asp Pro Arg Glu Val Arg Lys Glu Ile Leu Arg Glu Leu Glu
       130                 135                 140
   Thr Phe Asn Leu Gln Leu Pro Pro Ser Ser Ser Ser Ser Ser Ser Ser
   145                 150                 155                 160
   Ser Arg Ser Asn Pro Ser Ser Ser Lys Ser Pro Leu Gly His Ser Leu
                   165                 170                 175
   Gly Ser Asp Lys Asn Glu Lys Leu Ser Ala Leu Lys Ala Tyr Gly Tyr
               180                 185                 190
   Asp Leu Thr Glu Met Val Arg Glu Ser Lys Leu Asp Pro Val Ile Gly
           195                 200                 205
   Arg Ser Ser Glu Val Glu Arg Leu Ile Leu Ile Leu Cys Arg Arg Arg
       210                 215                 220
   Lys Asn Asn Pro Val Leu Ile Gly Glu Ala Gly Val Gly Lys Thr Ala
   225                 230                 235                 240
   Ile Val Glu Gly Leu Ala Gln Lys Ile Ile Leu Asn Glu Val Pro Asp
                   245                 250                 255
   Ala Leu Arg Lys Lys Arg Leu Ile Thr Leu Asp Leu Ala Leu Met Ile
               260                 265                 270
   Ala Gly Thr Lys Tyr Arg Gly Gln Phe Glu Glu Arg Ile Lys Ala Val
           275                 280                 285
   Met Asp Glu Val Arg Lys His Gly Asn Ile Leu Leu Phe Ile Asp Glu
       290                 295                 300
   Leu His Thr Ile Val Gly Ala Gly Ala Ala Glu Gly Ala Ile Asp Ala
   305                 310                 315                 320
   Ser Asn Ile Leu Lys Pro Ala Leu Ala Arg Gly Glu Ile Gln Cys Ile
                   325                 330                 335
   Gly Ala Thr Thr Ile Asp Glu Tyr Arg Lys His Ile Glu Lys Asp Ala
               340                 345                 350
   Ala Leu Glu Arg Arg Phe Gln Lys Ile Val Val His Pro Pro Ser Val
           355                 360                 365
   Asp Glu Thr Ile Glu Ile Leu Arg Gly Leu Lys Lys Lys Tyr Glu Glu
       370                 375                 380
   His His Asn Val Phe Ile Thr Glu Glu Ala Leu Lys Ala Ala Ala Thr
   385                 390                 395                 400
   Leu Ser Asp Gln Tyr Val His Gly Arg Phe Leu Pro Asp Lys Ala Ile
                   405                 410                 415
   Asp Leu Leu Asp Glu Ala Gly Ala Arg Val Arg Val Asn Thr Met Gly
               420                 425                 430
   Gln Pro Thr Asp Leu Met Lys Leu Glu Ala Glu Ile Glu Asn Thr Lys
           435                 440                 445
   Leu Ala Lys Glu Gln Ala Ile Gly Thr Gln Glu Tyr Glu Lys Ala Ala
       450                 455                 460
   Gly Leu Arg Asp Glu Glu Lys Lys Leu Arg Glu Arg Leu Gln Ser Met
   465                 470                 475                 480
   Lys Gln Glu Trp Glu Asn His Lys Glu Glu His Gln Val Pro Val Asp
                   485                 490                 495
   Glu Glu Ala Val Ala Gln Val Val Ser Leu Gln Thr Gly Ile Pro Ser
               500                 505                 510
   Ala Arg Leu Thr Glu Ala Glu Ser Glu Lys Leu Leu Lys Leu Glu Asp
           515                 520                 525
   Thr Leu Arg Arg Lys Val Ile Gly Gln Asn Asp Ala Val Thr Ser Ile
       530                 535                 540
   Cys Arg Ala Ile Arg Arg Ser Arg Thr Gly Ile Lys Asp Pro Asn Arg
   545                 550                 555                 560
   Pro Thr Gly Ser Phe Leu Phe Leu Gly Pro Thr Gly Val Gly Lys Ser
                   565                 570                 575
   Leu Leu Ala Gln Gln Ile Ala Ile Glu Met Phe Gly Gly Glu Asp Ala
               580                 585                 590
   Leu Ile Gln Val Asp Met Ser Glu Tyr Met Glu Lys Phe Ala Ala Thr
           595                 600                 605
   Lys Met Met Gly Ser Pro Pro Gly Tyr Val Gly His Glu Glu Gly Gly
       610                 615                 620
   His Leu Thr Glu Gln Val Arg Arg Arg Pro Tyr Cys Val Val Leu Phe
   625                 630                 635                 640
   Asp Glu Ile Glu Lys Ala His Pro Asp Ile Met Asp Leu Met Leu Gln
                   645                 650                 655
   Ile Leu Glu Gln Gly Arg Leu Thr Asp Ser Phe Gly Arg Lys Val Asp
               660                 665                 670
   Phe Arg His Ala Ile Ile Ile Met Thr Ser Asn Leu Gly Ala Asp Leu
   675 680 685
   Ile Arg Lys Ser Gly Glu Ile Gly Phe Gly Leu Lys Ser His Met Asp
   690 695 700
   Tyr Lys Val Ile Gln Glu Lys Ile Glu His Ala Met Lys Lys His Leu
   705 710 715 720
   Lys Pro Glu Phe Ile Asn Arg Leu Asp Glu Ser Val Ile Phe Arg Pro
   725 730 735
   Leu Glu Lys Glu Ser Leu Ser Glu Ile Ile His Leu Glu Ile Asn Lys
   740 745 750
   Leu Asp Ser Arg Leu Lys Asn Tyr Gln Met Ala Leu Asn Ile Pro Asp
   755 760 765
   Ser Val Ile Ser Phe Leu Val Thr Lys Gly His Ser Pro Glu Met Gly
   770 775 780
   Ala Arg Pro Leu Arg Arg Val Ile Glu Gln Tyr Leu Glu Asp Pro Leu
   785 790 795 800
   Ala Glu Leu Leu Leu Lys Glu Ser Cys Arg Gln Glu Ala Arg Lys Leu
   805 810 815
   Arg Ala Thr Leu Val Glu Asn Arg Val Ala Phe Glu Arg Glu Glu Glu
   820 825 830
   Glu Gln Glu Ala Ala Leu Pro Ser Pro His Leu Glu Ser
   835 840 845
<210> 19
   <211> 509
   <212> DNA
   <213> Chlamydia pneumoniae
<220>
   <221> CDS
   <222> (101) .. (406)
<400> 19
   gattcaggtt ctagtgagct tatgctcatg gaagttcaag tcttcttagc tgcaagaaaa 60
   taacagggac agtaattcga tttttcgaga agggaaactt atg gta aag atc ata   115
                                               Met Val Lys Ile Ile
                                                 1               5
   tca agt gaa aat ttt gac tct ttt att gca tcg ggg ctc gtt ctc gtt   163
   Ser Ser Glu Asn Phe Asp Ser Phe Ile Ala Ser Gly Leu Val Leu Val
                    10                  15                  20
   gat ttc ttt gca gaa tgg tgt ggc ccc tgt cgg atg ctc act cct atc   211
   Asp Phe Phe Ala Glu Trp Cys Gly Pro Cys Arg Met Leu Thr Pro Ile
                25                  30                  35
   tta gaa aat ctt gct gcg gaa ctt cct cat gtc act att gga aaa atc   259
   Leu Glu Asn Leu Ala Ala Glu Leu Pro His Val Thr Ile Gly Lys Ile
            40                  45                  50
   aat ata gat gag aac agc aag cct gca gaa acg tac gaa gtc agc tct   307
   Asn Ile Asp Glu Asn Ser Lys Pro Ala Glu Thr Tyr Glu Val Ser Ser
        55                  60                  65
   att cct acg ctt att ctt ttt aag gat ggg aac gag gtg gct cgg gtc   355
   Ile Pro Thr Leu Ile Leu Phe Lys Asp Gly Asn Glu Val Ala Arg Val
    70                  75                  80                  85
   gta ggt ctt aag gat aaa gaa ttc cta acc aat ctt atc aat aag cac   403
   Val Gly Leu Lys Asp Lys Glu Phe Leu Thr Asn Leu Ile Asn Lys His
                    90                  95                 100
   gct taaaaagacg ctgcaatatt aaaccgtagg attcttttgc aatgctacgg        456
   Ala
   ttttctgcct taccacttca tataaaacga tccctacact ggtagctaaa ttt        509
<210> 20
   <211> 102
   <212> PRT
   <213> Chlamydia pneumoniae
<220>
   <221> SITE
   <222> (543)...(66)
   <223> B-cell epitope
<220>
   <221> SITE
   <222> (40) ... (48)
   <223> T-cell epitope
<220>
   <221> SITE
   <222> (74) ... (83)
   <223> T-cell epitope
<400> 20
   Met Val Lys Ile Ile Ser Ser Glu Asn Phe Asp Ser Phe Ile Ala Ser
   1               5                   10                  15
   Gly Leu Val Leu Val Asp Phe Phe Ala Glu Trp Cys Gly Pro Cys Arg
               20                  25                  30
   Met Leu Thr Pro Ile Leu Glu Asn Leu Ala Ala Glu Leu Pro His Val
           35                  40                  45
   Thr Ile Gly Lys Ile Asn Ile Asp Glu Asn Ser Lys Pro Ala Glu Thr
       50                  55                  60
   Tyr Glu Val Ser Ser Ile Pro Thr Leu Ile Leu Phe Lys Asp Gly Asn
   65                  70                  75                  80
   Glu Val Ala Arg Val Val Gly Leu Lys Asp Lys Glu Phe Leu Thr Asn
                   85                  90                  95
   Leu Ile Asn Lys His Ala
               100
<210> 21
   <211> 43
   <212> DNA
   <213> Artificial Sequence
<220>
   <223> 5' PCR primer
<400> 21
   ataagaatgc ggccgccacc atgaagatgc ataggcttaa acc 43
<210> 22
   <211> 36
   <212> DNA
   <213> Artificial Sequence
<220>
   <223> 3' PCR primer
<400> 22
   gcgccggatc ccacttaaga tatcgatatt tttgag 36
<210> 23
   <211> 45
   <212> DNA
   <213> Artificial Sequence
<220>
   <223> 5' PCR primer
<400> 23
   ataagaatgc ggccgccacc atgcggttgg gaaataagcc tatgc 45
<210> 24
   <211> 35
   <212> DNA
   <213> Artificial Sequence
<220>
   <223> 3' PCR primer
<400> 24
   gcgccggtac cgtaatttaa tactctttga agggc 35
<210> 25
   <211> 49
   <212> DNA
   <213> Artificial Sequence
<220>
   <223> 5' PCR primer
<400> 25
   ataagaatgc ggccgccacc atgctcaccc taggcttgga aagttcttg 49
<210> 26
   <211> 36
   <212> DNA
   <213> Artificial Sequence
<220>
   <223> 3' PCR primer
<400> 26
   gctttggagg atccccggag aggctaagga gaatgg 36
<210> 27
   <211> 44
   <212> DNA
   <213> Artificial Sequence
<220>
   <223> 5' PCR primer
<400> 27
   ataagaatgc ggccgccacc atgaaaaaag ggaaattagg agcc 44
<210> 28
   <211> 33
   <212> DNA
   <213> Artificial Sequence
<220>
   <223> 3' PCR primer
<400> 28
   gcgccggatc cccgaagcag aagtcgttgt ggg 33
<210> 29
   <211> 48
   <212> DNA
   <213> Artificial Sequence
<220>
   <223> 5' PCR primer
<400> 29
   ataagaatgc ggccgccacc atgagaaaac ttattttatg caatccta 48
<210> 30
   <211> 33
   <212> DNA
   <213> Artificial Sequence
<220>
   <223> 3' PCR primer
<400> 30
   gcgccggatc ccagaacaac ggagttcttt tgg 33
<210> 31
   <211> 46
   <212> DNA
   <213> Artificial Sequence
<220>
   <223> 5' PCR primer
<400> 31
   ataagaatgc ggccgccacc atgaataaaa aaaatctaac tatttg 46
<210> 32
   <211> 32
   <212> DNA
   <213> Artificial Sequence
<220>
   <223> 3' PCR primer
<400> 32
   gcgccggatc ccagcgatag cttctggggt cc 32
<210> 33
   <211> 42
   <212> DNA
   <213> Artificial Sequence
<220>
   <223> 5' PCR primer
<400> 33
   ataagaatgc ggccgccacc atgacactgg taccctatgt tg 42
<210> 34
   <211> 35
   <212> DNA
   <213> Artificial Sequence
<220>
   <223> 3' PCR primer
<400> 34
   gcgccggatc ccagtgctac ttgtatcctt attag 35
<210> 35
   <211> 45
   <212> DNA
   <213> Artificial Sequence
<220>
   <223> 5' PCR primer
<400> 35
   ataagaatgc ggccgccacc atgagctacc gtaaacgttc gactc 45
<210> 36
   <211> 35
   <212> DNA
   <213> Artificial Sequence
<220>
   <223> 3' PCR primer
<400> 36
   gcgccggatc cccctcgttc ccccttgttt cggag 35
<210> 37
   <211> 46
   <212> DNA
   <213> Artificial Sequence
<220>
   <223> 5' PCR primer
<400> 37
   ataagaatgc ggccgccacc atgtttgaga agttcactaa tagagc 46
<210> 38
   <211> 34
   <212> DNA
   <213> Artificial Sequence
<220>
   <223> 3' PCR primer
<400> 38
   gcgccggtac cgtgattcca agtgagggct aggg 34
<210> 39
   <211> 42
   <212> DNA
   <213> Artificial Sequence
<220>
   <223> 5' PCR primer
<400> 39
   ataagaatgc ggccgccacc atggtaaaga tcatatcaag tg 42
<210> 40
   <211> 30
   <212> DNA
   <213> Artificial Sequence
<220>
   <223> 3' PCR primer
<400> 40
   gcgccggatc ccagcgtgct tattgataag 30
<210> 41
   <211> 17
   <212> PRT
   <213> Artificial Sequence
<220>
   <223> B-cell epitope of ATP-binding cassette protein
<400> 41
   Val His His Thr Leu Arg Glu Ser Tyr Lys Lys Gly Thr Pro Pro
   1 5 10 15
   Ser Thr
<210> 42
   <211> 16
<212> PRT
   <213> Artificial Sequence
<220>
   <223> B-cell epitope of ATP-binding cassette protein
<400> 42
   Asn Leu Gln Lys Glu Ile Ser Thr Glu Glu Arg Gln Thr Lys Ala
   1 5 10 15
   Arg
<210> 43
   <211> 9
   <212> PRT
   <213> Artificial Sequence
<220>
   <223> T-cell epitope of ATP-binding cassette protein
<400> 43
   Trp Ile Ala Glu Tyr Val Ser Pro Val
   1 5
<210> 44
   <211> 12
   <212> PRT
   <213> Artificial Sequence
<220>
   <223> B-cell epitope of endopeptidase protein
<400> 44
   Lys Gly Asn Asn Ser Ser Pro Arg Ser Pro Ala Pro
   1 5 10
<210> 45
   <211> 9
   <212> PRT
   <213> Artificial Sequence
<220>
   <223> B-cell epitope of endopeptidase protein
<400> 45
   Gly Glu Asn Phe Gln Lys Asn Ser Ser
   1 5
<210> 46
   <211> 9
   <212> PRT
   <213> Artificial Sequence
<220>
   <223> T-cell epitope of endopeptidase protein
<400> 46
   Leu Leu Ile Glu Asp Met Asp Leu Ile
   1 5
<210> 47
   <211> 9
   <212> PRT
   <213> Artificial Sequence
<220>
   <223> T-cell epitope of endopeptidase protein
<400> 47
   Asn Leu Leu Ile Glu Asp Met Asp Leu
   1 5
<210> 48
   <211> 16
   <212> PRT
   <213> Artificial Sequence
<220>
   <223> B-cell epitope of protease protein
<400> 48
   Thr Asp Leu Glu Gly Leu Glu Glu Asp His Lys Asp Ser Pro Trp
   1 5 10 15
   Glu
<210> 49
   <211> 18
   <212> PRT
   <213> Artificial Sequence
<220>
   <223> B-cell epitope of protease protein
<400> 49
   Ser Glu Asn Ala Lys Lys Ser Glu Glu Gln Thr Ser Pro Gln Glu
   1 5 10 15
   Thr Pro Glu
<210> 50
   <211> 9
   <212> PRT
   <213> Artificial Sequence
<220>
   <223> T-cell epitope of protease protein
<400> 50
   Tyr Leu Gly Asp Glu Ile Leu Glu Val
   1 5
<210> 51
   <211> 9
   <212> PRT
   <213> Artificial Sequence
<220>
   <223> T-cell epitope of protease protein
<400> 51
   Tyr Leu Tyr Ser Leu Leu Ser Met Leu
   1 5
<210> 52
   <211> 8
   <212> PRT
   <213> Artificial Sequence
<220>
   <223> B-cell epitope of metalloprotease protein
<400> 52
   Thr Thr Asn Arg Gln Lys Ala Leu
   1 5
<210> 53
   <211> 11
   <212> PRT
   <213> Artificial Sequence
<220>
   <223> B-cell epitope of metalloprotease protein
<400> 53
   Val Asn Ser Ser Asn Ser Asn Arg Leu Arg Glu
   1 5 10
<210> 54
   <211> 9
   <212> PRT
   <213> Artificial Sequence
<220>
   <223> T-cell epitope of metalloprotease protein
<400> 54
   Ser Val Leu Ser Arg Val Asn Tyr Val
   1 5
<210> 55
   <211> 9
   <212> PRT
   <213> Artificial Sequence
<220>
   <223> T-cell epitope of metalloprotease protein
<400> 55
   Lys Leu Ser Ser Leu Ile Pro Gly Leu
   1 5
<210> 56
   <211> 9
   <212> PRT
   <213> Artificial Sequence
<220>
   <223> T-cell epitope of metalloprotease protein
<400> 56
   Ile Leu Ile Gly His Lys Lys His Val
   1 5
<210> 57
   <211> 14
   <212> PRT
   <213> Artificial Sequence
<220>
   <223> B-cell epitope of CLP protease ATPase protein
<400> 57
   Pro Pro Lys Gly Gly Arg Lys His Pro Asn Gln Glu Tyr Ile
   5 10
<210> 58
   <211> 14
   <212> PRT
   <213> Artificial Sequence
<220>
   <223> B-cell epitope of CLP protease ATPase protein
<400> 58
   Ser Asp Asp Gln Ala Asp Leu Ser Gln Lys Thr Arg Asp His
   1 5 10
<210> 59
   <211> 9
   <212> PRT
   <213> Artificial Sequence
<220>
   <223> T-cell epitope of CLP protease ATPase protein
<400> 59
   Lys Ile Leu Asp Val Pro Phe Thr Ile
   1 5
<210> 60
   <211> 9
   <212> PRT
   <213> Artificial Sequence
<220>
   <223> T-cell epitope of CLP protease ATPase protein
<400> 60
   Leu Leu Gln Ala Ala Asp Tyr Asp Val
   1 5
<210> 61
   <211> 9
   <212> PRT
   <213> Artificial Sequence
<220>
   <223> B-cell epitope of CLP protease subunit protein
<400> 61
   Gly Thr Lys Gly Lys Arg His Ala Leu
   1 5
<210> 62
   <211> 11
   <212> PRT
   <213> Artificial Sequence
<220>
   <223> B-cell epitope of CLP protease subunit protein
<400> 62
   Ala Lys Glu Thr Asn Lys Asp Thr Ser Ser Thr
   1 5 10
<210> 63
   <211> 9
   <212> PRT
   <213> Artificial Sequence
<220>
   <223> T-cell epitope of CLP protease subunit protein
<400> 63
   Ala Ile Tyr Asp Thr Ile Arg Phe Leu
   1 5
<210> 64
   <211> 19
   <212> PRT
   <213> Artificial Sequence
<220>
   <223> B-cell epitope of translycolase/transpeptidase protein
<400> 64
   Asp Pro Thr Asn Tyr Lys Glu Tyr Phe Asn Asn Lys Glu Arg Ile
   1 5 10 15
   Glu His Thr Lys
<210> 65
   <211> 17
   <212> PRT
   <213> Artificial Sequence
<220>
   <223> B-cell epitope of translycolase/transpeptidase protein
<400> 65
   Lys Arg Leu Tyr Glu Glu Trp Asn Arg Ser Pro Lys Gln Gly Gly
   1 5 10 15
   Thr Arg
<210> 66
   <211> 9
   <212> PRT
   <213> Artificial Sequence
<220>
   <223> T-cell epitope of translycolase/transpeptidase protein
<400> 66
   Ala Leu Gly Gln His Glu Phe Cys Val
   1 5
<210> 67
   <211> 9
   <212> PRT
   <213> Artificial Sequence
<220>
   <223> T-cell epitope of translycolase/transpeptidase protein
<400> 67
   Ile Leu Ala Thr Gly Ile Gln Met Val
   1 5
<210> 68
   <211> 26
   <212> PRT
   <213> Artificial Sequence
<220>
   <223> B-cell epitope of CLPc protease protein
<400> 68
   Arg Asp Glu Glu Lys Lys Leu Arg Glu Arg Leu Gln Ser Met Lys
   1               5                   10                  15
   Gln Glu Trp Glu Asn His Lys Glu Glu His Gln
                   20                  25
<210> 69
   <211> 16
   <212> PRT
   <213> Artificial Sequence
<220>
   <223> B-cell epitope of CLPc protease protein
<400> 69
   Ile Arg Arg Ser Arg Thr Gly Ile Lys Asp Pro Asn Arg Pro Thr
   1               5                   10                  15
   Gly
<210> 70
   <211> 9
   <212> PRT
   <213> Artificial Sequence
<220>
   <223> T-cell epitope of CLPc protease protein
<400> 70
   Phe Leu Phe Leu Gly Pro Thr Gly Val
   1               5
<210> 71
   <211> 9
   <212> PRT
   <213> Artificial Sequence
<220>
   <223> T-cell epitope of CLPc protease protein
<400> 71
   Phe Leu Pro Asp Lys Ala Ile Asp Leu
   1               5
<210> 72
   <211> 13
   <212> PRT
   <213> Artificial Sequence
<220>
   <223> B-cell epitope of thioredoxin
<400> 72
   Asn Ile Asp Glu Asn Ser Lys Pro Ala Glu Thr Tyr Glu
   1 5 10
<210> 73
   <211> 9
   <212> PRT
   <213> Artificial Sequence
<220>
   <223> B-cell epitope of thioredoxin
<400> 73
   Asn Leu Ala Ala Glu Leu Pro His Val
   1 5
<210> 74
   <211> 9
   <212> PRT
   <213> Artificial Sequence
<220>
   <223> T-cell epitope of thioredoxin
<400> 74
   Ile Leu Phe Lys Asp Gly Asn Glu Val
   1 5